# EUROPEAN PATENT SPECIFICATION

(11) **EP 3 003 361 B1**
(45) Date of publication and mention of the grant of the patent: **03.07.2019**
(21) Application number: 14733889.1
(22) Date of filing: 30.05.2014
(51) Int. Cl.: A61K 39/00, A61K 39/395, A61P 9/10, C07K 16/40

(54) **METHODS FOR REDUCING REMNANT CHOLESTEROL AND OTHER LIPOPROTEIN FRACTIONS BY ADMINISTERING AN INHIBITOR OF PROPROTEIN CONVERTASE SUBTILISIN KEXIN-9 (PCSK9)**
VERFAHREN ZUR REDUZIERUNG VON RESTLICHEM CHOLESTERIN UND ANDEREN LIPOPROTEINFRAKTIONEN DURCH VERABREICHUNG EINES INHIBITORS VON PROPROTEIN-KONVERTASE-SUBTILISIN-KEXIN-9 (PCSK9)
PROCÉDÉS VISANT À RÉDUIRE LE CHOLESTÉROL RESTANT ET D'AUTRES FRACTIONS DE LIPOPROTÉINES PAR L'ADMINISTRATION D'UN INHIBITEUR DE LA PROPROTÉINE CONVERTASE SUBTILISINE KEXINE 9 (PCSK9)

(30) Priority: 30.05.2013 US 201361828753 P; 08.11.2013 US 201361901705 P; 23.12.2013 US 201361919836 P; 04.02.2014 US 201461935358 P; 17.03.2014 US 201461953959 P; 12.05.2014 US 201461991738 P; 29.05.2014 US 201414290544
(43) Date of publication of application: 13.04.2016
(73) Proprietor: Regeneron Pharmaceuticals, Inc., Tarrytown, NY 10591 (US)
(72) Inventor: SWERGOLD, Gary, New Rochelle, NY 10804 (US); SASIELA, William, J., Tarrytown, NY 10591-6706 (US); PORDY, Robert, C., Tarrytown, NY 10591-6706 (US)
(74) Representative: J A Kemp
(86) International application number: PCT/US2014/040163
(87) International publication number: WO 2014/194168

(56) References cited:
- WO-A1-2012/154999
- WO-A1-2013/039969
- TOTH PP ET AL: "Alirocumab, a Proprotein Convertase Subtilisin/Kexin Type 9 Monoclonal Antibody, Reduces Cholesterol Concentrations of Serum Remnant Lipoprotein Fractions, Very Low-Density Lipoproteins and Triglycerides):", CIRCULATION, vol. 128, no. 22, Suppl., 17 November 2013 (2013-11-17), page 17492, XP055136391, ISSN: 0009-7322
- KOREN MICHAEL J ET AL: "EFFECTS OF ALIROCUMAB, A FULLY HUMAN MONOCLONAL ANTIBODY TO PROPROTEIN CONVERTASE SUBTILISIN/KEXIN TYPE 9, ON LIPOPROTEIN PARTICLE CONCENTRATIONS DETERMINED BY NUCLEAR MAGNETIC RESONANCE: SUBSTUDY OF A RANDOMIZED DOUBLE-BLIND PHASE II CLINICAL TRIAL", JOURNAL OF THE AMERICAN COLLEGE OF CARDIOLOGY, vol. 63, no. 12, 30 March 2014 (2014-03-30), XP028600234, ISSN: 0735-1097, DOI: 10.1016/S0735-1097(14)61373-5
- GISSETTE REYES-SOFFER ET AL: "Effects of PCSK9 Inhibition With Alirocumab on Lipoprotein Metabolism in Healthy HumansClinical Perspective", CIRCULATION, vol. 135, no. 4, 16 December 2016 (2016-12-16), pages 352-362, XP055401731, US ISSN: 0009-7322, DOI: 10.1161/CIRCULATIONAHA.116.025253
- DAVID SULLIVAN ET AL: "Effect of a Monoclonal Antibody to PCSK9 on Low-Density Lipoprotein Cholesterol Levels in Statin-Intolerant Patients", JAMA, vol. 308, no. 23, 19 December 2012 (2012-12-19), page 2497, XP055136453, ISSN: 0098-7484, DOI: 10.1001/jama.2012.25790
- ANETTE VARBO ET AL: "Remnant Cholesterol as a Causal Risk Factor for Ischemic Heart Disease", JOURNAL OF THE AMERICAN COLLEGE OF CARDIOLOGY, vol. 61, no. 4, 1 January 2013 (2013-01-01), pages 427-436, XP055136401, ISSN: 0735-1097, DOI: 10.1016/j.jacc.2012.08.1026
- A. B. JORGENSEN ET AL: "Genetically elevated non-fasting triglycerides and calculated remnant cholesterol as causal risk factors for myocardial infarction", EUROPEAN HEART JOURNAL, vol. 34, no. 24, 17 December 2012 (2012-12-17), pages 1826-1833, XP055136634, ISSN: 0195-668X, DOI: 10.1093/eurheartj/ehs431
- KAWASHIRI MASA-AKI ET AL: "Statin Therapy Improves Fractional Catabolic Rate of LDL without Affecting Impaired VLDL and VLDL Remnant Catabolism in Homozygous FH Patient Due to PCSK9 Gene Mutation: Evidence from Kinetic Study with Stable Isotope", CIRCULATION, vol. 126, no. 21, Suppl. S, November 2012 (2012-11), page 13869, XP009179823, & AMERICAN-HEART-ASSOCIATION RESUSCITATION SCIENCE SYMPOSIUM; LOS ANGELES, CA, USA; NOVEMBER 03 -04, 2012
- TOTH PP ET AL: "Alirocumab, a Proprotein Convertase Subtilisin/Kexin Type 9 Monoclonal Antibody, Reduces Cholesterol Concentrations of Serum Remnant Lipoprotein Fractions, Very Low-Density Lipoproteins and Triglycerides):", CIRCULATION, vol. 128, no. 22, Suppl., 17 November 2013 (2013-11-17), page 17492, XP055136391, ISSN: 0009-7322
- TOTH PP ET AL: "Alirocumab, a Proprotein Convertase Subtilisin/Kexin Type 9 Monoclonal Antibody, Reduces Cholesterol Concentrations of Serum Remnant Lipoprotein Fractions, Very Low-Density Lipoproteins and Triglycerides):", CIRCULATION, vol. 128, no. 22, Suppl., 17 November 2013 (2013-11-17), page 17492, XP055136391, ISSN: 0009-7322
- KOREN MICHAEL J ET AL: "EFFECTS OF ALIROCUMAB, A FULLY HUMAN MONOCLONAL ANTIBODY TO PROPROTEIN CONVERTASE SUBTILISIN/KEXIN TYPE 9, ON LIPOPROTEIN PARTICLE CONCENTRATIONS DETERMINED BY NUCLEAR MAGNETIC RESONANCE: SUBSTUDY OF A RANDOMIZED DOUBLE-BLIND PHASE II CLINICAL TRIAL", JOURNAL OF THE AMERICAN COLLEGE OF CARDIOLOGY, vol. 63, no. 12, 30 March 2014 (2014-03-30), XP028600234, ISSN: 0735-1097, DOI: 10.1016/S0735-1097(14)61373-5
- DAVID SULLIVAN ET AL: "Effect of a Monoclonal Antibody to PCSK9 on Low-Density Lipoprotein Cholesterol Levels in Statin-Intolerant Patients", JAMA, vol. 308, no. 23, 19 December 2012 (2012-12-19), page 2497, XP055136453, ISSN: 0098-7484, DOI: 10.1001/jama.2012.25790
- GISSETTE REYES-SOFFER ET AL: "Effects of PCSK9 Inhibition With Alirocumab on Lipoprotein Metabolism in Healthy HumansClinical Perspective", CIRCULATION, vol. 135, no. 4, 16 December 2016 (2016-12-16), pages 352-362, XP055401731, US ISSN: 0009-7322, DOI: 10.1161/CIRCULATIONAHA.116.025253

### SEQUENCE LISTING

<110> REGENERON PHARMACEUTICALS, INC.
<120> Methods for Reducing Remnant Cholesterol and Other Lipoprotein
   Fractions by Administering an Inhibitor of Proprotein Convertase Subtilisin Kexin-9 (PCSK9)
<130> 7008A-WO
<140> To be assigned
   <141> Filed Herewith
<150> 61/828,753
   <151> 2013-05-30
<150> 61/901,705
   <151> 2013-11-08
<150> 61/919,836
   <151> 2013-12-23
<150> 61/935,358
   <151> 2014-02-04
<150> 61/953,959
   <151> 2014-03-17
<150> 61/991,738
   <151> 2014-05-12
<160> 765
<170> FastSEQ for Windows Version 4.0
<210> 1
   <211> 351
   <212> DNA
   <213> Artificial Sequence
<220>
   <223> Synthetic
<400> 1
   caggtccagc tggtgcagtc tgggggaggc ttggtacagc ctggggggtc cctgagactc 60
   tcctgtgcag cctctggatt tactctaagt agttacgaca tgcactgggt ccgccaatct 120
   acaggaaaag gtctggagtg ggtctcagct attggttcta ccggtgacac atactatcca 180
   ggctccgtga agggccgatt caccatcacc agagaaaaag ccaagaactc cgtgtatctt 240
   caaatgaaca gcctgagagc cggggacacg gctgtgtatt actgtgtaag agaggggtgg 300
   gaggtaccct ttgactactg gggccaggga accctggtca ctgtctcctc a          351
<210> 2
   <211> 117
   <212> PRT
   <213> Artificial Sequence
<220>
   <223> Synthetic
<400> 2
   Gln Val Gln Leu Val Gln Ser Gly Gly Gly Leu Val Gln Pro Gly Gly
    1               5                  10                  15
   Ser Leu Arg Leu Ser Cys Ala Ala Ser Gly Phe Thr Leu Ser Ser Tyr
               20                  25                  30
   Asp Met His Trp Val Arg Gln Ser Thr Gly Lys Gly Leu Glu Trp Val
           35                  40                  45
   Ser Ala Ile Gly Ser Thr Gly Asp Thr Tyr Tyr Pro Gly Ser Val Lys
       50                  55                  60
   Gly Arg Phe Thr Ile Thr Arg Glu Lys Ala Lys Asn Ser Val Tyr Leu
   65                  70                  75                  80
   Gln Met Asn Ser Leu Arg Ala Gly Asp Thr Ala Val Tyr Tyr Cys Val
                   85                  90                  95
   Arg Glu Gly Trp Glu Val Pro Phe Asp Tyr Trp Gly Gln Gly Thr Leu
               100                 105                 110
   Val Thr Val Ser Ser
           115
<210> 3
   <211> 24
   <212> DNA
   <213> Artificial Sequence
<220>
   <223> Synthetic
<400> 3
   ggatttactc taagtagtta cgac 24
<210> 4
   <211> 8
   <212> PRT
   <213> Artificial Sequence
<220>
   <223> Synthetic
<400> 4
   Gly Phe Thr Leu Ser Ser Tyr Asp
    1               5
<210> 5
   <211> 21
   <212> DNA
   <213> Artificial Sequence
<220>
   <223> Synthetic
<400> 5
   attggttcta ccggtgacac a 21
<210> 6
   <211> 7
   <212> PRT
   <213> Artificial Sequence
<220>
   <223> Synthetic
<400> 6
   Ile Gly Ser Thr Gly Asp Thr
    1               5
<210> 7
   <211> 33
   <212> DNA
   <213> Artificial Sequence
<220>
   <223> Synthetic
<400> 7
   gtaagagagg ggtgggaggt accctttgac tac 33
<210> 8
   <211> 11
   <212> PRT
   <213> Artificial Sequence
<220>
   <223> Synthetic
<400> 8
   Val Arg Glu Gly Trp Glu Val Pro Phe Asp Tyr
    1               5                   10
<210> 9
   <211> 327
   <212> DNA
   <213> Artificial Sequence
<220>
   <223> Synthetic
<400> 9
   gacatccaga tgacccagtc tccagccacc ctgtctgtgt ctccagggga aagagccgcc 60
   ctctcctgca gggccagtca gagtgttagc agcaacttag cctggtacca ccagaaacct 120
   ggccaggctc ccaggctcct catctatggt gcatccacca gggccactgg tatcccagcc 180
   aggttcagtg gcattgggtc tgggacagag ttcactctca ttatcagcag cctgcagtct 240
   gaagattttg cattttattt ctgtcagcag tataataact ggcctccatt cactttcggc 300
   cctgggacca aggtggagat caaacga                                     327
<210> 10
   <211> 109
   <212> PRT
   <213> Artificial Sequence
<220>
   <223> Synthetic
<400> 10
   Asp Ile Gln Met Thr Gln Ser Pro Ala Thr Leu Ser Val Ser Pro Gly
    1               5                  10                  15
   Glu Arg Ala Ala Leu Ser Cys Arg Ala Ser Gln Ser Val Ser Ser Asn
               20                  25                  30
   Leu Ala Trp Tyr His Gln Lys Pro Gly Gln Ala Pro Arg Leu Leu Ile
           35                  40                  45
   Tyr Gly Ala Ser Thr Arg Ala Thr Gly Ile Pro Ala Arg Phe Ser Gly
       50                  55                  60
   Ile Gly Ser Gly Thr Glu Phe Thr Leu Ile Ile Ser Ser Leu Gln Ser
   65                  70                  75                  80
   Glu Asp Phe Ala Phe Tyr Phe Cys Gln Gln Tyr Asn Asn Trp Pro Pro
                   85                  90                  95
   Phe Thr Phe Gly Pro Gly Thr Lys Val Glu Ile Lys Arg
               100                 105
<210> 11
   <211> 18
   <212> DNA
   <213> Artificial Sequence
<220>
   <223> Synthetic
<400> 11
   cagagtgtta gcagcaac 18
<210> 12
   <211> 6
   <212> PRT
   <213> Artificial Sequence
<220>
   <223> Synthetic
<400> 12
   Gln Ser Val Ser Ser Asn
    1               5
<210> 13
   <211> 9
   <212> DNA
   <213> Artificial Sequence
<220>
   <223> Synthetic
<400> 13
   ggtgcatcc 9
<210> 14
   <211> 3
   <212> PRT
   <213> Artificial Sequence
<220>
   <223> Synthetic
<400> 14
   Gly Ala Ser
    1
<210> 15
   <211> 30
   <212> DNA
   <213> Artificial Sequence
<220>
   <223> Synthetic
<400> 15
   cagcagtata ataactggcc tccattcact 30
<210> 16
   <211> 10
   <212> PRT
   <213> Artificial Sequence
<220>
   <223> Synthetic
<400> 16
   Gln Gln Tyr Asn Asn Trp Pro Pro Phe Thr
    1               5                  10
<210> 17
   <211> 351
   <212> DNA
   <213> Artificial Sequence
<220>
   <223> Synthetic
<400> 17
   gaggtgcagc tggtggagtc tgggggaggc ttggtacagc ctggggggtc cctgagactc 60
   tcctgtgcag cctctggatt tactctaagt agttacgaca tgcactgggt ccgccaatct 120
   acaggaaaag gtctggagtg ggtctcagct attggttcta ccggtgacac atactatcca 180
   ggctccgtga agggccgatt caccatcacc agagaaaaag ccaagaactc cgtgtatctt 240
   caaatgaaca gcctgagagc cggggacacg gctgtgtatt actgtgtaag agaggggtgg 300
   gaggtaccct ttgactactg gggccaggga accctggtca ccgtctcctc a          351
<210> 18
   <211> 117
   <212> PRT
   <213> Artificial Sequence
<220>
   <223> Synthetic
<400> 18
   Glu Val Gln Leu Val Glu Ser Gly Gly Gly Leu Val Gln Pro Gly Gly
    1               5                  10                  15
   Ser Leu Arg Leu Ser Cys Ala Ala Ser Gly Phe Thr Leu Ser Ser Tyr
               20                  25                  30
   Asp Met His Trp Val Arg Gln Ser Thr Gly Lys Gly Leu Glu Trp Val
           35                  40                  45
   Ser Ala Ile Gly Ser Thr Gly Asp Thr Tyr Tyr Pro Gly Ser Val Lys
       50                  55                  60
   Gly Arg Phe Thr Ile Thr Arg Glu Lys Ala Lys Asn Ser Val Tyr Leu
   65                  70                  75                  80
   Gln Met Asn Ser Leu Arg Ala Gly Asp Thr Ala Val Tyr Tyr Cys Val
                   85                  90                  95
   Arg Glu Gly Trp Glu Val Pro Phe Asp Tyr Trp Gly Gln Gly Thr Leu
               100                 105                 110
   Val Thr Val Ser Ser
           115
<210> 19
   <211> 324
   <212> DNA
   <213> Artificial Sequence
<220>
   <223> Synthetic
<400> 19
   gaaatagtga tgacgcagtc tccagccacc ctgtctgtgt ctccagggga aagagccgcc 60
   ctctcctgca gggccagtca gagtgttagc agcaacttag cctggtacca ccagaaacct 120
   ggccaggctc ccaggctcct catctatggt gcatccacca gggccactgg tatcccagcc 180
   aggttcagtg gcattgggtc tgggacagag ttcactctca ttatcagcag cctgcagtct 240
   gaagattttg cattttattt ctgtcagcag tataataact ggcctccatt cactttcggc 300
   cctgggacca aagtggatat caaa                                        324
<210> 20
   <211> 108
   <212> PRT
   <213> Artificial Sequence
<220>
   <223> Synthetic
<400> 20
   Glu Ile Val Met Thr Gln Ser Pro Ala Thr Leu Ser Val Ser Pro Gly
    1               5                  10                  15
   Glu Arg Ala Ala Leu Ser Cys Arg Ala Ser Gln Ser Val Ser Ser Asn
               20                  25                  30
   Leu Ala Trp Tyr His Gln Lys Pro Gly Gln Ala Pro Arg Leu Leu Ile
           35                  40                  45
   Tyr Gly Ala Ser Thr Arg Ala Thr Gly Ile Pro Ala Arg Phe Ser Gly
       50                  55                  60
   Ile Gly Ser Gly Thr Glu Phe Thr Leu Ile Ile Ser Ser Leu Gln Ser
   65                  70                  75                  80
   Glu Asp Phe Ala Phe Tyr Phe Cys Gln Gln Tyr Asn Asn Trp Pro Pro
                   85                  90                  95
   Phe Thr Phe Gly Pro Gly Thr Lys Val Asp Ile Lys
               100                 105
<210> 21
   <211> 351
   <212> DNA
   <213> Artificial Sequence
<220>
   <223> Synthetic
<400> 21
   gaggtgcagc tggtggagtc tgggggaggc ttggtacagc ctggggggtc cctgagactc 60
   tcctgtgcag cctctggatt tactctaagt agttacgaca tgcactgggt ccgccaagct 120
   acaggaaaag gtctggagtg ggtctcagct attggttcta ccggtgacac atactatcca 180
   ggctccgtga agggccgatt caccatctcc agagaaaatg ccaagaactc cttgtatctt 240
   caaatgaaca gcctgagagc cggggacacg gctgtgtatt actgtgtaag agaggggtgg 300
   gaggtaccct ttgactactg gggccaggga accctggtca ccgtctcctc a          351
<210> 22
   <211> 117
   <212> PRT
   <213> Artificial Sequence
<220>
   <223> Synthetic
<400> 22
   Glu Val Gln Leu Val Glu Ser Gly Gly Gly Leu Val Gln Pro Gly Gly
    1               5                   10                  15
   Ser Leu Arg Leu Ser Cys Ala Ala Ser Gly Phe Thr Leu Ser Ser Tyr
               20                  25                  30
   Asp Met His Trp Val Arg Gln Ala Thr Gly Lys Gly Leu Glu Trp Val
           35                  40                  45
   Ser Ala Ile Gly Ser Thr Gly Asp Thr Tyr Tyr Pro Gly Ser Val Lys
       50                  55                  60
   Gly Arg Phe Thr Ile Ser Arg Glu Asn Ala Lys Asn Ser Leu Tyr Leu
   65                  70                  75                  80
   Gln Met Asn Ser Leu Arg Ala Gly Asp Thr Ala Val Tyr Tyr Cys Val
                   85                  90                  95
   Arg Glu Gly Trp Glu Val Pro Phe Asp Tyr Trp Gly Gln Gly Thr Leu
               100                 105                 110
   Val Thr Val Ser Ser
           115
<210> 23
   <211> 324
   <212> DNA
   <213> Artificial Sequence
<220>
   <223> Synthetic
<400> 23
   gaaatagtga tgacgcagtc tccagccacc ctgtctgtgt ctccagggga aagagccacc 60
   ctctcctgca gggccagtca gagtgttagc agcaacttag cctggtacca gcagaaacct 120
   ggccaggctc ccaggctcct catctatggt gcatccacca gggccactgg tatcccagcc 180
   aggttcagtg gcagtgggtc tgggacagag ttcactctca ccatcagcag cctgcagtct 240
   gaagattttg cagtttatta ctgtcagcag tataataact ggcctccatt cactttcggc 300
   cctgggacca aagtggatat caaa                                        324
<210> 24
   <211> 108
   <212> PRT
   <213> Artificial Sequence
<220>
   <223> Synthetic
<400> 24
   Glu Ile Val Met Thr Gln Ser Pro Ala Thr Leu Ser Val Ser Pro Gly
    1               5                   10                  15
   Glu Arg Ala Thr Leu Ser Cys Arg Ala Ser Gln Ser Val Ser Ser Asn
               20                  25                  30
   Leu Ala Trp Tyr Gln Gln Lys Pro Gly Gln Ala Pro Arg Leu Leu Ile
           35                  40                  45
   Tyr Gly Ala Ser Thr Arg Ala Thr Gly Ile Pro Ala Arg Phe Ser Gly
       50                  55                  60
   Ser Gly Ser Gly Thr Glu Phe Thr Leu Thr Ile Ser Ser Leu Gln Ser
   65                  70                  75                  80
   Glu Asp Phe Ala Val Tyr Tyr Cys Gln Gln Tyr Asn Asn Trp Pro Pro
                   85                  90                  95
   Phe Thr Phe Gly Pro Gly Thr Lys Val Asp Ile Lys
               100                 105
<210> 25
   <211> 342
   <212> DNA
   <213> Artificial Sequence
<220>
   <223> Synthetic
<400> 25
   caggtgcagc tggtgcagtc tgggggaggc gtggtccagc ctgggaggtc cctgagactc 60
   tcctgtgcag cgtctggatt caccttcagt agctatggca tgcactgggt ccgccaggct 120
   ccaggcaagg ggctggagtg ggtggcgttt ataggatttg atggaagtaa tatacattat 180
   ggagactccg tgaggggccg aatcatcata tccagagaca attccgagaa cacgttgtat 240
   ctggaaatga acagcctgag agccgaggac acggcaatgt actattgtgc gagagagaag 300
   ggtttagact ggggccaggg aaccacggtc accgtctcct ca                    342
<210> 26
   <211> 114
   <212> PRT
   <213> Artificial Sequence
<220>
   <223> Synthetic
<400> 26
   Gln Val Gln Leu Val Gln Ser Gly Gly Gly Val Val Gln Pro Gly Arg
    1               5                  10                  15
   Ser Leu Arg Leu Ser Cys Ala Ala Ser Gly Phe Thr Phe Ser Ser Tyr
               20                  25                  30
   Gly Met His Trp Val Arg Gln Ala Pro Gly Lys Gly Leu Glu Trp Val
           35                  40                  45
   Ala Phe Ile Gly Phe Asp Gly Ser Asn Ile His Tyr Gly Asp Ser Val
       50                  55                  60
   Arg Gly Arg Ile Ile Ile Ser Arg Asp Asn Ser Glu Asn Thr Leu Tyr
   65                  70                  75                  80
   Leu Glu Met Asn Ser Leu Arg Ala Glu Asp Thr Ala Met Tyr Tyr Cys
                   85                  90                  95
   Ala Arg Glu Lys Gly Leu Asp Trp Gly Gln Gly Thr Thr Val Thr Val
               100                 105                 110
   Ser Ser
<210> 27
   <211> 24
   <212> DNA
   <213> Artificial Sequence
<220>
   <223> Synthetic
<400> 27
   ggattcacct tcagtagcta tggc 24
<210> 28
   <211> 8
   <212> PRT
   <213> Artificial Sequence
<220>
   <223> Synthetic
<400> 28
   Gly Phe Thr Phe Ser Ser Tyr Gly
    1               5
<210> 29
   <211> 24
   <212> DNA
   <213> Artificial Sequence
<220>
   <223> Synthetic
<400> 29
   ataggatttg atggaagtaa tata 24
<210> 30
   <211> 8
   <212> PRT
   <213> Artificial Sequence
<220>
   <223> Synthetic
<400> 30
   Ile Gly Phe Asp Gly Ser Asn Ile
    1               5
<210> 31
   <211> 21
   <212> DNA
   <213> Artificial Sequence
<220>
   <223> Synthetic
<400> 31
   gcgagagaga agggtttaga c 21
<210> 32
   <211> 7
   <212> PRT
   <213> Artificial Sequence
<220>
   <223> Synthetic
<400> 32
   Ala Arg Glu Lys Gly Leu Asp
    1               5
<210> 33
   <211> 321
   <212> DNA
   <213> Artificial Sequence
<220>
   <223> Synthetic
<400> 33
   gccatccaga tgacccagtc tccttccacc ctgtctgcat ctgtaggaga cagagtcacc 60
   atcacttgcc gggccagtca gagtattagt agctggttgg cctggtatca gcagaaacca 120
   gggaaagccc ctaagctcct gatctataag gcgtctagtt tagaaagtgg ggtcccatca 180
   aggttcagcg gcagtggatc tgggacagaa ttcactctca ccatcagcag cctgcagcct 240
   gatgattttg caacttatta ctgccaacag tataatagtt attacacttt tggccagggg 300
   accaaggtgg aaatcaaacg a                                           321
<210> 34
   <211> 107
   <212> PRT
   <213> Artificial Sequence
<220>
   <223> Synthetic
<400> 34
   Ala Ile Gln Met Thr Gln Ser Pro Ser Thr Leu Ser Ala Ser Val Gly
    1               5                  10                  15
   Asp Arg Val Thr Ile Thr Cys Arg Ala Ser Gln Ser Ile Ser Ser Trp
               20                  25                  30
   Leu Ala Trp Tyr Gln Gln Lys Pro Gly Lys Ala Pro Lys Leu Leu Ile
           35                  40                  45
   Tyr Lys Ala Ser Ser Leu Glu Ser Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Asp Asp Phe Ala Thr Tyr Tyr Cys Gln Gln Tyr Asn Ser Tyr Tyr Thr
                   85                  90                  95
   Phe Gly Gln Gly Thr Lys Val Glu Ile Lys Arg
               100                 105
<210> 35
   <211> 18
   <212> DNA
   <213> Artificial Sequence
<220>
   <223> Synthetic
<400> 35
   cagagtatta gtagctgg 18
<210> 36
   <211> 6
   <212> PRT
   <213> Artificial Sequence
<220>
   <223> Synthetic
<400> 36
   Gln Ser Ile Ser Ser Trp
    1               5
<210> 37
   <211> 9
   <212> DNA
   <213> Artificial Sequence
<220>
   <223> Synthetic
<400> 37
   aaggcgtct 9
<210> 38
   <211> 3
   <212> PRT
   <213> Artificial Sequence
<220>
   <223> Synthetic
<400> 38
   Lys Ala Ser
   1
<210> 39
   <211> 24
   <212> DNA
   <213> Artificial Sequence
<220>
   <223> Synthetic
<400> 39
   caacagtata atagttatta cact 24
<210> 40
   <211> 8
   <212> PRT
   <213> Artificial Sequence
<220>
   <223> Synthetic
<400> 40
   Gln Gln Tyr Asn Ser Tyr Tyr Thr
   1 5
<210> 41
   <211> 342
   <212> DNA
   <213> Artificial Sequence
<220>
   <223> Synthetic
<400> 41
   caggtgcagc tggtggagtc tgggggaggc gtggtccagc ctgggaggtc cctgagactc 60
   tcctgtgcag cgtctggatt caccttcagt agctatggca tgcactgggt ccgccaggct 120
   ccaggcaagg ggctggagtg ggtggcgttt ataggatttg atggaagtaa tatacattat 180
   ggagactccg tgaggggccg aatcatcata tccagagaca attccgagaa cacgttgtat 240
   ctggaaatga acagcctgag agccgaggac acggcaatgt actattgtgc gagagagaag 300
   ggtttagact ggggccaggg aaccctggtc accgtctcct ca 342
<210> 42
   <211> 114
   <212> PRT
   <213> Artificial Sequence
<220>
   <223> Synthetic
<400> 42
   Gln Val Gln Leu Val Glu Ser Gly Gly Gly Val Val Gln Pro Gly Arg
    1               5                  10                  15
   Ser Leu Arg Leu Ser Cys Ala Ala Ser Gly Phe Thr Phe Ser Ser Tyr
               20                  25                  30
   Gly Met His Trp Val Arg Gln Ala Pro Gly Lys Gly Leu Glu Trp Val
           35                  40                  45
   Ala Phe Ile Gly Phe Asp Gly Ser Asn Ile His Tyr Gly Asp Ser Val
       50                  55                  60
   Arg Gly Arg Ile Ile Ile Ser Arg Asp Asn Ser Glu Asn Thr Leu Tyr
   65                  70                  75                  80
   Leu Glu Met Asn Ser Leu Arg Ala Glu Asp Thr Ala Met Tyr Tyr Cys
                   85                  90                  95
   Ala Arg Glu Lys Gly Leu Asp Trp Gly Gln Gly Thr Leu Val Thr Val
               100                 105                 110
   Ser Ser
<210> 43
   <211> 318
   <212> DNA
   <213> Artificial Sequence
<220>
   <223> Synthetic
<400> 43
   gacatccaga tgacccagtc tccttccacc ctgtctgcat ctgtaggaga cagagtcacc 60
   atcacttgcc gggccagtca gagtattagt agctggttgg cctggtatca gcagaaacca 120
   gggaaagccc ctaagctcct gatctataag gcgtctagtt tagaaagtgg ggtcccatca 180
   aggttcagcg gcagtggatc tgggacagaa ttcactctca ccatcagcag cctgcagcct 240
   gatgattttg caacttatta ctgccaacag tataatagtt attacacttt tggccagggg 300
   accaagctgg agatcaaa                                               318
<210> 44
   <211> 106
   <212> PRT
   <213> Artificial Sequence
<220>
   <223> Synthetic
<400> 44
   Asp Ile Gln Met Thr Gln Ser Pro Ser Thr Leu Ser Ala Ser Val Gly
    1               5                  10                  15
   Asp Arg Val Thr Ile Thr Cys Arg Ala Ser Gln Ser Ile Ser Ser Trp
               20                  25                  30
   Leu Ala Trp Tyr Gln Gln Lys Pro Gly Lys Ala Pro Lys Leu Leu Ile
           35                  40                  45
   Tyr Lys Ala Ser Ser Leu Glu Ser Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Asp Asp Phe Ala Thr Tyr Tyr Cys Gln Gln Tyr Asn Ser Tyr Tyr Thr
                   85                  90                  95
   Phe Gly Gln Gly Thr Lys Leu Glu Ile Lys
               100                 105
<210> 45
   <211> 342
   <212> DNA
   <213> Artificial Sequence
<220>
   <223> Synthetic
<400> 45
   caggtgcagc tggtggagtc tgggggaggc gtggtccagc ctgggaggtc cctgagactc 60
   tcctgtgcag cctctggatt caccttcagt agctatggca tgcactgggt ccgccaggct 120
   ccaggcaagg ggctggagtg ggtggcagtt ataggatttg atggaagtaa tatatactat 180
   gcagactccg tgaagggccg attcaccatc tccagagaca attccaagaa cacgctgtat 240
   ctgcaaatga acagcctgag agctgaggac acggctgtgt attactgtgc gagagagaag 300
   ggtttagact ggggccaggg aaccctggtc accgtctcct ca                    342
<210> 46
   <211> 114
   <212> PRT
   <213> Artificial Sequence
<220>
   <223> Synthetic
<400> 46
   Gln Val Gln Leu Val Glu Ser Gly Gly Gly Val Val Gln Pro Gly Arg
    1               5                  10                  15
   Ser Leu Arg Leu Ser Cys Ala Ala Ser Gly Phe Thr Phe Ser Ser Tyr
               20                  25                  30
   Gly Met His Trp Val Arg Gln Ala Pro Gly Lys Gly Leu Glu Trp Val
           35                  40                  45
   Ala Val Ile Gly Phe Asp Gly Ser Asn Ile Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                       85                  90                  95
   Ala Arg Glu Lys Gly Leu Asp Trp Gly Gln Gly Thr Leu Val Thr Val
               100                 105                 110
   Ser Ser
<210> 47
   <211> 319
   <212> DNA
   <213> Artificial Sequence
<220>
   <223> Synthetic
<400> 47
   gacatccaga tgacccagtc tccttccacc ctgtctgcat ctgtaggaga cagagtcacc 60
   atcacttgcc gggccagtca gagtattagt agctggttgg cctggtatca gcagaaacca 120
   gggaaagccc ctaagctcct gatctataag gcgtctagtt tagaaagtgg ggtcccatca 180
   aggttcagcg gcagtggatc tgggacagaa ttcactctca ccatcagcag cctgcagcct 240
   gatgattttg caacttatta ctgccaacag tataatagtt attacacttt tggccagggg 300
   accaagctgg agatcaaac                                              319
<210> 48
   <211> 106
   <212> PRT
   <213> Artificial Sequence
<220>
   <223> Synthetic
<400> 48
   Asp Ile Gln Met Thr Gln Ser Pro Ser Thr Leu Ser Ala Ser Val Gly
    1               5                  10                  15
   Asp Arg Val Thr Ile Thr Cys Arg Ala Ser Gln Ser Ile Ser Ser Trp
               20                  25                  30
   Leu Ala Trp Tyr Gln Gln Lys Pro Gly Lys Ala Pro Lys Leu Leu Ile
           35                  40                  45
   Tyr Lys Ala Ser Ser Leu Glu Ser Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Asp Asp Phe Ala Thr Tyr Tyr Cys Gln Gln Tyr Asn Ser Tyr Tyr Thr
                   85                  90                  95
   Phe Gly Gln Gly Thr Lys Leu Glu Ile Lys
               100                 105
<210> 49
   <211> 342
   <212> DNA
   <213> Artificial Sequence
<220>
   <223> Synthetic
<400> 49
   caggtgcagc tgcaggagtc tgggggaggc gtggtccagc ctgggaggtc cctgagactc 60
   tcctgtgcag cgtctggatt caccttcagt agctatggca tgcactgggt ccgccaggct 120
   ccaggcaagg ggctggagtg ggtggcgttt ataggatttg atggaagtaa tatatattat 180
   ggagactccg tgaggggccg aatcatcata tccagagaca attccgagaa cacgttgtat 240
   ctggaaatga acagcctgag agccgaggac acggcagtgt attattgtgc gagagagaag 300
   ggtttagact ggggccaggg aaccctggtc actgtctcct ca                   342
<210> 50
   <211> 114
   <212> PRT
   <213> Artificial Sequence
<220>
   <223> Synthetic
<400> 50
   Gln Val Gln Leu Gln Glu Ser Gly Gly Gly Val Val Gln Pro Gly Arg
    1               5                  10                  15
   Ser Leu Arg Leu Ser Cys Ala Ala Ser Gly Phe Thr Phe Ser Ser Tyr
               20                  25                  30
   Gly Met His Trp Val Arg Gln Ala Pro Gly Lys Gly Leu Glu Trp Val
           35                  40                  45
   Ala Phe Ile Gly Phe Asp Gly Ser Asn Ile Tyr Tyr Gly Asp Ser Val
       50                  55                  60
   Arg Gly Arg Ile Ile Ile Ser Arg Asp Asn Ser Glu Asn Thr Leu Tyr
   65                  70                  75                  80
   Leu Glu Met Asn Ser Leu Arg Ala Glu Asp Thr Ala Val Tyr Tyr Cys
                   85                  90                  95
   Ala Arg Glu Lys Gly Leu Asp Trp Gly Gln Gly Thr Leu Val Thr Val
               100                 105                 110
   Ser Ser
<210> 51
   <211> 24
   <212> DNA
   <213> Artificial Sequence
<220>
   <223> Synthetic
<400> 51
   ggattcacct tcagtagcta tggc 24
<210> 52
   <211> 8
   <212> PRT
   <213> Artificial Sequence
<220>
   <223> Synthetic
<400> 52
   Gly Phe Thr Phe Ser Ser Tyr Gly
   1 5
<210> 53
   <211> 24
   <212> DNA
   <213> Artificial Sequence
<220>
   <223> Synthetic
<400> 53
   ataggatttg atggaagtaa tata 24
<210> 54
   <211> 8
   <212> PRT
   <213> Artificial Sequence
<220>
   <223> Synthetic
<400> 54
   Ile Gly Phe Asp Gly Ser Asn Ile
   1 5
<210> 55
   <211> 21
   <212> DNA
   <213> Artificial Sequence
<220>
   <223> Synthetic
<400> 55
   gcgagagaga agggtttaga c 21
<210> 56
   <211> 7
   <212> PRT
   <213> Artificial Sequence
<220>
   <223> Synthetic
<400> 56
   Ala Arg Glu Lys Gly Leu Asp
   1 5
<210> 57
   <211> 342
   <212> DNA
   <213> Artificial Sequence
<220>
   <223> Synthetic
<400> 57
   gccatccaga tgacccagtc tccagactcc ctggctgtgt ctctgggcga gagggccacc 60
   atcaactgca agtccagcca gagtgttttt cacacctcca acaataagaa ctacttagtt 120
   tggtatcagc agaaaccagg acagcctcct aagttgctcc tttactgggc ctctacccgg 180
   gaatccgggg tccctgaccg attcagtggc agcgggtctg ggacagattt cactctcacc 240
   atcagcagcc tgcaggctga agatgtggca aattattact gtcaccaata ttacagtatt 300
   ccgtggacgt tcggccaagg gaccaaggtg gagatcaaac ga                     342
<210> 58
   <211> 114
   <212> PRT
   <213> Artificial Sequence
<220>
   <223> Synthetic
<400> 58
   Ala Ile Gln Met Thr Gln Ser Pro Asp Ser Leu Ala Val Ser Leu Gly
    1               5                  10                  15
   Glu Arg Ala Thr Ile Asn Cys Lys Ser Ser Gln Ser Val Phe His Thr
               20                  25                  30
   Ser Asn Asn Lys Asn Tyr Leu Val Trp Tyr Gln Gln Lys Pro Gly Gln
           35                  40                  45
   Pro Pro Lys Leu Leu Leu Tyr Trp Ala Ser Thr Arg Glu Ser Gly Val
       50                  55                  60
   Pro Asp Arg Phe Ser Gly Ser Gly Ser Gly Thr Asp Phe Thr Leu Thr
   65                  70                  75                  80
   Ile Ser Ser Leu Gln Ala Glu Asp Val Ala Asn Tyr Tyr Cys His Gln
                   85                  90                  95
   Tyr Tyr Ser Ile Pro Trp Thr Phe Gly Gln Gly Thr Lys Val Glu Ile
               100                 105                 110
   Lys Arg
<210> 59
   <211> 36
   <212> DNA
   <213> Artificial Sequence
<220>
   <223> Synthetic
<400> 59
   cagagtgttt ttcacacctc caacaataag aactac 36
<210> 60
   <211> 12
   <212> PRT
   <213> Artificial Sequence
<220>
   <223> Synthetic
<400> 60
   Gln Ser Val Phe His Thr Ser Asn Asn Lys Asn Tyr
   1 5 10
<210> 61
   <211> 9
   <212> DNA
   <213> Artificial Sequence
<220>
   <223> Synthetic
<400> 61
   tgggcctct 9
<210> 62
   <211> 3
   <212> PRT
   <213> Artificial Sequence
<220>
   <223> Synthetic
<400> 62
   Trp Ala Ser
   1
<210> 63
   <211> 27
   <212> DNA
   <213> Artificial Sequence
<220>
   <223> Synthetic
<400> 63
   caccaatatt acagtattcc gtggacg 27
<210> 64
   <211> 9
   <212> PRT
   <213> Artificial Sequence
<220>
   <223> Synthetic
<400> 64
   His Gln Tyr Tyr Ser Ile Pro Trp Thr
   1 5
<210> 65
   <211> 342
   <212> DNA
   <213> Artificial Sequence
<220>
   <223> Synthetic
<400> 65
   caggtgcagc tggtggagtc tgggggaggc gtggtccagc ctgggaggtc cctgagactc 60
   tcctgtgcag cgtctggatt caccttcagt agctatggca tgcactgggt ccgccaggct 120
   ccaggcaagg ggctggagtg ggtggcgttt ataggatttg atggaagtaa tatatattat 180
   ggagactccg tgaggggccg aatcatcata tccagagaca attccgagaa cacgttgtat 240
   ctggaaatga acagcctgag agccgaggac acggcagtgt attattgtgc gagagagaag 300
   ggtttagact ggggccaggg aaccctggtc accgtctcct ca                    342
<210> 66
   <211> 114
   <212> PRT
   <213> Artificial Sequence
<220>
   <223> Synthetic
<400> 66
   Gln Val Gln Leu Val Glu Ser Gly Gly Gly Val Val Gln Pro Gly Arg
   1               5                   10                  15
   Ser Leu Arg Leu Ser Cys Ala Ala Ser Gly Phe Thr Phe Ser Ser Tyr
               20                  25                  30
   Gly Met His Trp Val Arg Gln Ala Pro Gly Lys Gly Leu Glu Trp Val
           35                  40                  45
   Ala Phe Ile Gly Phe Asp Gly Ser Asn Ile Tyr Tyr Gly Asp Ser Val
       50                  55                  60
   Arg Gly Arg Ile Ile Ile Ser Arg Asp Asn Ser Glu Asn Thr Leu Tyr
   65                  70                  75                  80
   Leu Glu Met Asn Ser Leu Arg Ala Glu Asp Thr Ala Val Tyr Tyr Cys
                   85                  90                  95
   Ala Arg Glu Lys Gly Leu Asp Trp Gly Gln Gly Thr Leu Val Thr Val
               100                 105                 110
   Ser Ser
<210> 67
   <211> 339
   <212> DNA
   <213> Artificial Sequence
<220>
   <223> Synthetic
<400> 67
   gacatcgtga tgacccagtc tccagactcc ctggctgtgt ctctgggcga gagggccacc 60
   atcaactgca agtccagcca gagtgttttt cacacctcca acaataagaa ctacttagtt 120
   tggtatcagc agaaaccagg acagcctcct aagttgctcc tttactgggc ctctacccgg 180
   gaatccgggg tccctgaccg attcagtggc agcgggtctg ggacagattt cactctcacc 240
   atcagcagcc tgcaggctga agatgtggca aattattact gtcaccaata ttacagtatt 300
   ccgtggacgt tcggccaagg gaccaaggtg gaaatcaaa                        339
<210> 68
   <211> 113
   <212> PRT
   <213> Artificial Sequence
<220>
   <223> Synthetic
<400> 68
   Asp Ile Val Met Thr Gln Ser Pro Asp Ser Leu Ala Val Ser Leu Gly
    1               5                   10                  15
   Glu Arg Ala Thr Ile Asn Cys Lys Ser Ser Gln Ser Val Phe His Thr
               20                  25                  30
   Ser Asn Asn Lys Asn Tyr Leu Val Trp Tyr Gln Gln Lys Pro Gly Gln
           35                  40                  45
   Pro Pro Lys Leu Leu Leu Tyr Trp Ala Ser Thr Arg Glu Ser Gly Val
       50                  55                  60
   Pro Asp Arg Phe Ser Gly Ser Gly Ser Gly Thr Asp Phe Thr Leu Thr
   65                  70                  75                  80
   Ile Ser Ser Leu Gln Ala Glu Asp Val Ala Asn Tyr Tyr Cys His Gln
                   85                  90                  95
   Tyr Tyr Ser Ile Pro Trp Thr Phe Gly Gln Gly Thr Lys Val Glu Ile
               100                 105                 110
   Lys
<210> 69
   <211> 342
   <212> DNA
   <213> Artificial Sequence
<220>
   <223> Synthetic
<400> 69
   caggtgcagc tggtggagtc tgggggaggc gtggtccagc ctgggaggtc cctgagactc 60
   tcctgtgcag cctctggatt caccttcagt agctatggca tgcactgggt ccgccaggct 120
   ccaggcaagg ggctggagtg ggtggcagtt ataggatttg atggaagtaa tatatactat 180
   gcagactccg tgaagggccg attcaccatc tccagagaca attccaagaa cacgctgtat 240
   ctgcaaatga acagcctgag agctgaggac acggctgtgt attactgtgc gagagagaag 300
   ggtttagact ggggccaggg aaccctggtc accgtctcct ca                    342
<210> 70
   <211> 114
   <212> PRT
   <213> Artificial Sequence
<220>
   <223> Synthetic
<400> 70
   Gln Val Gln Leu Val Glu Ser Gly Gly Gly Val Val Gln Pro Gly Arg
    1               5                  10                  15
   Ser Leu Arg Leu Ser Cys Ala Ala Ser Gly Phe Thr Phe Ser Ser Tyr
               20                  25                  30
   Gly Met His Trp Val Arg Gln Ala Pro Gly Lys Gly Leu Glu Trp Val
           35                  40                  45
   Ala Val Ile Gly Phe Asp Gly Ser Asn Ile Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Glu Lys Gly Leu Asp Trp Gly Gln Gly Thr Leu Val Thr Val
               100                 105                 110
   Ser Ser
<210> 71
   <211> 339
   <212> DNA
   <213> Artificial Sequence
<220>
   <223> Synthetic
<400> 71
   gacatcgtga tgacccagtc tccagactcc ctggctgtgt ctctgggcga gagggccacc 60
   atcaactgca agtccagcca gagtgttttt cacacctcca acaataagaa ctacttagct 120
   tggtaccagc agaaaccagg acagcctcct aagctgctca tttactgggc ctctacccgg 180
   gaatccgggg tccctgaccg attcagtggc agcgggtctg ggacagattt cactctcacc 240
   atcagcagcc tgcaggctga agatgtggca gtttattact gtcaccaata ttacagtatt 300
   ccgtggacgt tcggccaagg gaccaaggtg gaaatcaaa                        339
<210> 72
   <211> 113
   <212> PRT
   <213> Artificial Sequence
<220>
   <223> Synthetic
<400> 72
   Asp Ile Val Met Thr Gln Ser Pro Asp Ser Leu Ala Val Ser Leu Gly
    1               5                  10                  15
   Glu Arg Ala Thr Ile Asn Cys Lys Ser Ser Gln Ser Val Phe His Thr
               20                  25                  30
   Ser Asn Asn Lys Asn Tyr Leu Ala Trp Tyr Gln Gln Lys Pro Gly Gln
           35                  40                  45
   Pro Pro Lys Leu Leu Ile Tyr Trp Ala Ser Thr Arg Glu Ser Gly Val
       50                  55                  60
   Pro Asp Arg Phe Ser Gly Ser Gly Ser Gly Thr Asp Phe Thr Leu Thr
   65                  70                  75                  80
   Ile Ser Ser Leu Gln Ala Glu Asp Val Ala Val Tyr Tyr Cys His Gln
                   85                  90                  95
   Tyr Tyr Ser Ile Pro Trp Thr Phe Gly Gln Gly Thr Lys Val Glu Ile
               100                 105                 110
   Lys
<210> 73
   <211> 354
   <212> DNA
   <213> Artificial Sequence
<220>
   <223> Synthetic
<400> 73
   gaagtgcagc tggtgcagtc tgggggaggc ttggtacagc ctggggggtc cctgagactc 60
   tcctgtgcag cctctggatt cacctttaac aactatgcca tgaactgggt ccgccaggct 120
   ccagggaagg gactggactg ggtctcaact attagtggta gcggtggtac tacaaactac 180
   gcagactccg tgaagggccg tttcattatt tcccgagaca gttccaaaca cacgctgtat 240
   ctgcaaatga acagcctgag agccgaggac acggccgtat attactgtgc gaaagattct 300
   aactggggaa atttcgatct ctggggccgt ggcaccacgg tcactgtctc ctca       354
<210> 74
   <211> 118
   <212> PRT
   <213> Artificial Sequence
<220>
   <223> Synthetic
<400> 74
   Glu Val Gln Leu Val Gln Ser Gly Gly Gly Leu Val Gln Pro Gly Gly
    1               5                  10                  15
   Ser Leu Arg Leu Ser Cys Ala Ala Ser Gly Phe Thr Phe Asn Asn Tyr
               20                  25                  30
   Ala Met Asn Trp Val Arg Gln Ala Pro Gly Lys Gly Leu Asp Trp Val
           35                  40                  45
   Ser Thr Ile Ser Gly Ser Gly Gly Thr Thr Asn Tyr Ala Asp Ser Val
       50                  55                  60
   Lys Gly Arg Phe Ile Ile Ser Arg Asp Ser Ser Lys His Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Asp Ser Asn Trp Gly Asn Phe Asp Leu Trp Gly Arg Gly Thr
               100                 105                 110
   Thr Val Thr Val Ser Ser
           115
<210> 75
   <211> 24
   <212> DNA
   <213> Artificial Sequence
<220>
   <223> Synthetic
<400> 75
   ggattcacct ttaacaacta tgcc 24
<210> 76
   <211> 8
   <212> PRT
   <213> Artificial Sequence
<220>
   <223> Synthetic
<400> 76
   Gly Phe Thr Phe Asn Asn Tyr Ala
    1               5
<210> 77
   <211> 24
   <212> DNA
   <213> Artificial Sequence
<220>
   <223> Synthetic
<400> 77
   attagtggta gcggtggtac taca 24
<210> 78
   <211> 8
   <212> PRT
   <213> Artificial Sequence
<220>
   <223> Synthetic
<400> 78
   Ile Ser Gly Ser Gly Gly Thr Thr
    1               5
<210> 79
   <211> 33
   <212> DNA
   <213> Artificial Sequence
<220>
   <223> Synthetic
<400> 79
   gcgaaagatt ctaactgggg aaatttcgat ctc 33
<210> 80
   <211> 11
   <212> PRT
   <213> Artificial Sequence
<220>
   <223> Synthetic
<400> 80
   Ala Lys Asp Ser Asn Trp Gly Asn Phe Asp Leu
    1               5                   10
<210> 81
   <211> 342
   <212> DNA
   <213> Artificial Sequence
<220>
   <223> Synthetic
<400> 81
   gacatccaga tgacccagtc tccagactcc ctggctgtgt ctctgggcga gagggccacc 60
   atcaactgca agtccagcca gagtgtttta tacaggtcca acaataggaa cttcttaggt 120
   tggtaccagc agaaaccagg gcagcctcct aatctactca tttactgggc atctacccgg 180
   gaatccgggg tccctgaccg attcagtggc agcgggtctg ggacagattt cactctcacc 240
   atcagcagcc tgcaggctga agatgtggca gtttattact gtcaacaata ttatactact 300
   ccgtacactt ttggccaggg gaccaaggtg gaaatcaaac ga                    342
<210> 82
   <211> 114
   <212> PRT
   <213> Artificial Sequence
<220>
   <223> Synthetic
<400> 82
   Asp Ile Gln Met Thr Gln Ser Pro Asp Ser Leu Ala Val Ser Leu Gly
    1               5                  10                  15
   Glu Arg Ala Thr Ile Asn Cys Lys Ser Ser Gln Ser Val Leu Tyr Arg
               20                  25                  30
   Ser Asn Asn Arg Asn Phe Leu Gly Trp Tyr Gln Gln Lys Pro Gly Gln
           35                  40                  45
   Pro Pro Asn Leu Leu Ile Tyr Trp Ala Ser Thr Arg Glu Ser Gly Val
       50                  55                  60
   Pro Asp Arg Phe Ser Gly Ser Gly Ser Gly Thr Asp Phe Thr Leu Thr
   65                  70                  75                  80
   Ile Ser Ser Leu Gln Ala Glu Asp Val Ala Val Tyr Tyr Cys Gln Gln
                   85                  90                  95
   Tyr Tyr Thr Thr Pro Tyr Thr Phe Gly Gln Gly Thr Lys Val Glu Ile
               100                 105                 110
   Lys Arg
<210> 83
   <211> 36
   <212> DNA
   <213> Artificial Sequence
<220>
   <223> Synthetic
<400> 83
   cagagtgttt tatacaggtc caacaatagg aacttc 36
<210> 84
   <211> 12
   <212> PRT
   <213> Artificial Sequence
<220>
   <223> Synthetic
<400> 84
   Gln Ser Val Leu Tyr Arg Ser Asn Asn Arg Asn Phe
    1               5                  10
<210> 85
   <211> 9
   <212> DNA
   <213> Artificial Sequence
<220>
   <223> Synthetic
<400> 85
   tgggcatct 9
<210> 86
   <211> 3
   <212> PRT
   <213> Artificial Sequence
<220>
   <223> Synthetic
<400> 86
   Trp Ala Ser
    1
<210> 87
   <211> 27
   <212> DNA
   <213> Artificial Sequence
<220>
   <223> Synthetic
<400> 87
   caacaatatt atactactcc gtacact 27
<210> 88
   <211> 9
   <212> PRT
   <213> Artificial Sequence
<220>
   <223> Synthetic
<400> 88
   Gln Gln Tyr Tyr Thr Thr Pro Tyr Thr
    1               5
<210> 89
   <211> 354
   <212> DNA
   <213> Artificial Sequence
<220>
   <223> Synthetic
<400> 89
   gaggtgcagc tggtggagtc tgggggaggc ttggtacagc ctggggggtc cctgagactc 60
   tcctgtgcag cctctggatt cacctttaac aactatgcca tgaactgggt ccgccaggct 120
   ccaggaaagg gactggactg ggtctcaact attagtggta gcggtggtac tacaaactac 180
   gcagactccg tgaagggccg tttcattatt tcccgagaca gttccaaaca cacgctgtat 240
   ctgcaaatga acagcctgag agccgaggac acggccgtat attactgtgc gaaagattct 300
   aactggggaa atttcgatct ctggggccgt ggcaccctgg tcactgtctc ctca       354
<210> 90
   <211> 118
   <212> PRT
   <213> Artificial Sequence
<220>
   <223> Synthetic
<400> 90
<210> 91
   <211> 339
   <212> DNA
   <213> Artificial Sequence
<220>
   <223> Synthetic
<400> 91
   gacatcgtga tgacccagtc tccagactcc ctggctgtgt ctctgggcga gagggccacc 60
   atcaactgca agtccagcca gagtgtttta tacaggtcca acaataggaa cttcttaggt 120
   tggtaccagc agaaaccagg gcagcctcct aatctactca tttactgggc atctacccgg 180
   gaatccgggg tccctgaccg attcagtggc agcgggtctg ggacagattt cactctcacc 240
   atcagcagcc tgcaggctga agatgtggca gtttattact gtcaacaata ttatactact 300
   ccgtacactt ttggccaggg gaccaagctg gagatcaaa                        339
<210> 92
   <211> 113
   <212> PRT
   <213> Artificial Sequence
<220>
   <223> Synthetic
<400> 92
<210> 93
   <211> 354
   <212> DNA
   <213> Artificial Sequence
<220>
   <223> Synthetic
<400> 93
   gaggtgcagc tggtggagtc tgggggaggc ttggtacagc ctggggggtc cctgagactc 60
   tcctgtgcag cctctggatt cacctttaac aactatgcca tgagctgggt ccgccaggct 120
   ccagggaagg ggctggagtg ggtctcagct attagtggta gcggtggtac tacatactac 180
   gcagactccg tgaagggccg gttcaccatc tccagagaca attccaagaa cacgctgtat 240
   ctgcaaatga acagcctgag agccgaggac acggccgtat attactgtgc gaaagattct 300
   aactggggaa atttcgatct ctggggccgt ggcaccctgg tcactgtctc ctca       354
<210> 94
   <211> 118
   <212> PRT
   <213> Artificial Sequence
<220>
   <223> Synthetic
<400> 94
   Glu Val Gln Leu Val Glu Ser Gly Gly Gly Leu Val Gln Pro Gly Gly
    1               5                   10                  15
   Ser Leu Arg Leu Ser Cys Ala Ala Ser Gly Phe Thr Phe Asn Asn Tyr
               20                  25                  30
   Ala Met Ser Trp Val Arg Gln Ala Pro Gly Lys Gly Leu Glu Trp Val
           35                  40                  45
   Ser Ala Ile Ser Gly Ser Gly Gly Thr Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Asp Ser Asn Trp Gly Asn Phe Asp Leu Trp Gly Arg Gly Thr
               100                 105                 110
   Leu Val Thr Val Ser Ser
           115
<210> 95
   <211> 339
   <212> DNA
   <213> Artificial Sequence
<220>
   <223> Synthetic
<400> 95
   gacatcgtga tgacccagtc tccagactcc ctggctgtgt ctctgggcga gagggccacc 60
   atcaactgca agtccagcca gagtgtttta tacaggtcca acaataggaa cttcttagct 120
   tggtaccagc agaaaccagg acagcctcct aagctgctca tttactgggc atctacccgg 180
   gaatccgggg tccctgaccg attcagtggc agcgggtctg ggacagattt cactctcacc 240
   atcagcagcc tgcaggctga agatgtggca gtttattact gtcaacaata ttatactact 300
   ccgtacactt ttggccaggg gaccaagctg gagatcaaa                        339
<210> 96
   <211> 113
   <212> PRT
   <213> Artificial Sequence
<220>
   <223> Synthetic
<400> 96
   Asp Ile Val Met Thr Gln Ser Pro Asp Ser Leu Ala Val Ser Leu Gly
    1               5                   10                  15
   Glu Arg Ala Thr Ile Asn Cys Lys Ser Ser Gln Ser Val Leu Tyr Arg
               20                  25                  30
   Ser Asn Asn Arg Asn Phe Leu Ala Trp Tyr Gln Gln Lys Pro Gly Gln
           35                  40                  45
   Pro Pro Lys Leu Leu Ile Tyr Trp Ala Ser Thr Arg Glu Ser Gly Val
       50                  55                  60
   Pro Asp Arg Phe Ser Gly Ser Gly Ser Gly Thr Asp Phe Thr Leu Thr
   65                  70                  75                  80
   Ile Ser Ser Leu Gln Ala Glu Asp Val Ala Val Tyr Tyr Cys Gln Gln
                   85                  90                  95
   Tyr Tyr Thr Thr Pro Tyr Thr Phe Gly Gln Gly Thr Lys Leu Glu Ile
               100                 105                 110
   Lys
<210> 97
   <211> 351
   <212> DNA
   <213> Artificial Sequence
<220>
   <223> Synthetic
<400> 97
   caggtgcagc tggtgcagtc tgggggaggc ttggtacagc ctggggggtc cctgagactc 60
   tcctgtgcag tctctggatt caccctcagt agctacgata tgcactgggt ccgccaacct 120
   acaggaaaag gtctggagtg ggtctcagct attggttcta ctggtgacac atactatcca 180
   ggctccgtga agggccgatt caccatctcc agagaaaatg ccaagaactc cttgtatctt 240
   caaatgaaca gcctgagagc cggggacacg gctgtgtatt actgtgcaag agagggatgg 300
   gacgtaccct ttgacttctg gggccaggga accctggtca ccgtctcctc a          351
<210> 98
   <211> 117
   <212> PRT
   <213> Artificial Sequence
<220>
   <223> Synthetic
<400> 98
   Gln Val Gln Leu Val Gln Ser Gly Gly Gly Leu Val Gln Pro Gly Gly
    1               5                  10                  15
   Ser Leu Arg Leu Ser Cys Ala Val Ser Gly Phe Thr Leu Ser Ser Tyr
               20                  25                  30
   Asp Met His Trp Val Arg Gln Pro Thr Gly Lys Gly Leu Glu Trp Val
           35                  40                  45
   Ser Ala Ile Gly Ser Thr Gly Asp Thr Tyr Tyr Pro Gly Ser Val Lys
       50                  55                  60
   Gly Arg Phe Thr Ile Ser Arg Glu Asn Ala Lys Asn Ser Leu Tyr Leu
   65                  70                  75                  80
   Gln Met Asn Ser Leu Arg Ala Gly Asp Thr Ala Val Tyr Tyr Cys Ala
                   85                  90                  95
   Arg Glu Gly Trp Asp Val Pro Phe Asp Phe Trp Gly Gln Gly Thr Leu
               100                 105                 110
   Val Thr Val Ser Ser
           115
<210> 99
   <211> 24
   <212> DNA
   <213> Artificial Sequence
<220>
   <223> Synthetic
<400> 99
   ggattcaccc tcagtagcta cgat 24
<210> 100
   <211> 8
   <212> PRT
   <213> Artificial Sequence
<220>
   <223> Synthetic
<400> 100
   Gly Phe Thr Leu Ser Ser Tyr Asp
   1 5
<210> 101
   <211> 21
   <212> DNA
   <213> Artificial Sequence
<220>
   <223> Synthetic
<400> 101
   attggttcta ctggtgacac a 21
<210> 102
   <211> 7
   <212> PRT
   <213> Artificial Sequence
<220>
   <223> Synthetic
<400> 102
   Ile Gly Ser Thr Gly Asp Thr
   1 5
<210> 103
   <211> 33
   <212> DNA
   <213> Artificial Sequence
<220>
   <223> Synthetic
<400> 103
   gcaagagagg gatgggacgt accctttgac ttc 33
<210> 104
   <211> 11
   <212> PRT
   <213> Artificial Sequence
<220>
   <223> Synthetic
<400> 104
   Ala Arg Glu Gly Trp Asp Val Pro Phe Asp Phe
    1               5                   10
<210> 105
   <211> 324
   <212> DNA
   <213> Artificial Sequence
<220>
   <223> Synthetic
<400> 105
   gccatccagt tgacccagtc tccatcctcc ctgtctgcat ctgtaggaga cagagtcacc 60
   atcacttgcc gggcaagtca ggacattaga aatgatttag gctggtatca gcagaaacca 120
   gggaaagccc ctaagctcct gatctatgct gcatccagtt tacaaagtgg ggtcccatca 180
   cggttcagcg gcagtggatc tggcacagat ttcactctca ccatcagcag cctgcagcct 240
   gaagattttg caacttatta ctgtctacaa gattacaatt acccgtggac gttcggccaa 300
   gggaccaagg tggagatcaa acga                                        324
<210> 106
   <211> 108
   <212> PRT
   <213> Artificial Sequence
<220>
   <223> Synthetic
<400> 106
   Ala Ile Gln Leu Thr Gln Ser Pro Ser Ser Leu Ser Ala Ser Val Gly
    1              5                   10                  15
   Asp Arg Val Thr Ile Thr Cys Arg Ala Ser Gln Asp Ile Arg Asn Asp
               20                  25                  30
   Leu Gly Trp Tyr Gln Gln Lys Pro Gly Lys Ala Pro Lys Leu Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Leu Gln Asp Tyr Asn Tyr Pro Trp
                   85                  90                  95
   Thr Phe Gly Gln Gly Thr Lys Val Glu Ile Lys Arg
               100                 105
<210> 107
   <211> 18
   <212> DNA
   <213> Artificial Sequence
<220>
   <223> Synthetic
<400> 107
   caggacatta gaaatgat 18
<210> 108
   <211> 6
   <212> PRT
   <213> Artificial Sequence
<220>
   <223> Synthetic
<400> 108
   Gln Asp Ile Arg Asn Asp
    1               5
<210> 109
   <211> 9
   <212> DNA
   <213> Artificial Sequence
<220>
   <223> Synthetic
<400> 109
   gctgcatcc 9
<210> 110
   <211> 3
   <212> PRT
   <213> Artificial Sequence
<220>
   <223> Synthetic
<400> 110
   Ala Ala Ser
   1
<210> 111
   <211> 27
   <212> DNA
   <213> Artificial Sequence
<220>
   <223> Synthetic
<400> 111
   ctacaagatt acaattaccc gtggacg 27
<210> 112
   <211> 9
   <212> PRT
   <213> Artificial Sequence
<220>
   <223> Synthetic
<400> 112
   Leu Gln Asp Tyr Asn Tyr Pro Trp Thr
   1 5
<210> 113
   <211> 351
   <212> DNA
   <213> Artificial Sequence
<220>
   <223> Synthetic
<400> 113
   gaggtgcagc tggtggagtc tgggggaggc ttggtacagc ctggggggtc cctgagactc 60
   tcctgtgcag tctctggatt caccctcagt agctacgata tgcactgggt ccgccaacct 120
   acaggaaaag gtctggagtg ggtctcagct attggttcta ctggtgacac atactatcca 180
   ggctccgtga agggccgatt caccatctcc agagaaaatg ccaagaactc cttgtatctt 240
   caaatgaaca gcctgagagc cggggacacg gctgtgtatt actgtgcaag agagggatgg 300
   gacgtaccct ttgacttctg gggccaggga accctggtca ccgtctcctc a 351
<210> 114
   <211> 117
   <212> PRT
   <213> Artificial Sequence
<220>
   <223> Synthetic
<400> 114
   Glu Val Gln Leu Val Glu Ser Gly Gly Gly Leu Val Gln Pro Gly Gly
    1              5                   10                  15
   Ser Leu Arg Leu Ser Cys Ala Val Ser Gly Phe Thr Leu Ser Ser Tyr
               20                  25                  30
   Asp Met His Trp Val Arg Gln Pro Thr Gly Lys Gly Leu Glu Trp Val
           35                  40                  45
   Ser Ala Ile Gly Ser Thr Gly Asp Thr Tyr Tyr Pro Gly Ser Val Lys
       50                  55                  60
   Gly Arg Phe Thr Ile Ser Arg Glu Asn Ala Lys Asn Ser Leu Tyr Leu
   65                  70                  75                  80
   Gln Met Asn Ser Leu Arg Ala Gly Asp Thr Ala Val Tyr Tyr Cys Ala
                   85                  90                  95
   Arg Glu Gly Trp Asp Val Pro Phe Asp Phe Trp Gly Gln Gly Thr Leu
               100                 105                 110
   Val Thr Val Ser Ser
           115
<210> 115
   <211> 321
   <212> DNA
   <213> Artificial Sequence
<220>
   <223> Synthetic
<400> 115
   gccatccaga tgacccagtc tccatcctcc ctgtctgcat ctgtaggaga cagagtcacc 60
   atcacttgcc gggcaagtca ggacattaga aatgatttag gctggtatca gcagaaacca 120
   gggaaagccc ctaagctcct gatctatgct gcatccagtt tacaaagtgg ggtcccatca 180
   cggttcagcg gcagtggatc tggcacagat ttcactctca ccatcagcag cctgcagcct 240
   gaagattttg caacttatta ctgtctacaa gattacaatt acccgtggac gttcggccaa 300
   gggaccaagg tggaaatcaa a                                           321
<210> 116
   <211> 107
   <212> PRT
   <213> Artificial Sequence
<220>
   <223> Synthetic
<400> 116
   Ala Ile Gln Met Thr Gln Ser Pro Ser Ser Leu Ser Ala Ser Val Gly
    1              5                   10                  15
   Asp Arg Val Thr Ile Thr Cys Arg Ala Ser Gln Asp Ile Arg Asn Asp
               20                  25                  30
   Leu Gly Trp Tyr Gln Gln Lys Pro Gly Lys Ala Pro Lys Leu Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Leu Gln Asp Tyr Asn Tyr Pro Trp
                   85                  90                  95
   Thr Phe Gly Gln Gly Thr Lys Val Glu Ile Lys
               100                 105
<210> 117
   <211> 351
   <212> DNA
   <213> Artificial Sequence
<220>
   <223> Synthetic
<400> 117
   gaggtgcagc tggtggagtc tgggggaggc ttggtacagc ctggggggtc cctgagactc 60
   tcctgtgcag cctctggatt caccctcagt agctacgata tgcactgggt ccgccaagct 120
   acaggaaaag gtctggagtg ggtctcagct attggttcta ctggtgacac atactatcca 180
   ggctccgtga agggccgatt caccatctcc agagaaaatg ccaagaactc cttgtatctt 240
   caaatgaaca gcctgagagc cggggacacg gctgtgtatt actgtgcaag agagggatgg 300
   gacgtaccct ttgacttctg gggccaggga accctggtca ccgtctcctc a          351
<210> 118
   <211> 117
   <212> PRT
   <213> Artificial Sequence
<220>
   <223> Synthetic
<400> 118
   Glu Val Gln Leu Val Glu Ser Gly Gly Gly Leu Val Gln Pro Gly Gly
    1              5                   10                  15
   Ser Leu Arg Leu Ser Cys Ala Ala Ser Gly Phe Thr Leu Ser Ser Tyr
               20                  25                  30
   Asp Met His Trp Val Arg Gln Ala Thr Gly Lys Gly Leu Glu Trp Val
           35                  40                  45
   Ser Ala Ile Gly Ser Thr Gly Asp Thr Tyr Tyr Pro Gly Ser Val Lys
       50                  55                  60
   Gly Arg Phe Thr Ile Ser Arg Glu Asn Ala Lys Asn Ser Leu Tyr Leu
   65                  70                  75                  80
   Gln Met Asn Ser Leu Arg Ala Gly Asp Thr Ala Val Tyr Tyr Cys Ala
                       85                  90                  95
   Arg Glu Gly Trp Asp Val Pro Phe Asp Phe Trp Gly Gln Gly Thr Leu
                   100                 105                 110
   Val Thr Val Ser Ser
               115
<210> 119
   <211> 321
   <212> DNA
   <213> Artificial Sequence
<220>
   <223> Synthetic
<400> 119
   gccatccaga tgacccagtc tccatcctcc ctgtctgcat ctgtaggaga cagagtcacc 60
   atcacttgcc gggcaagtca ggacattaga aatgatttag gctggtatca gcagaaacca 120
   gggaaagccc ctaagctcct gatctatgct gcatccagtt tacaaagtgg ggtcccatca 180
   aggttcagcg gcagtggatc tggcacagat ttcactctca ccatcagcag cctgcagcct 240
   gaagattttg caacttatta ctgtctacaa gattacaatt acccgtggac gttcggccaa 300
   gggaccaagg tggaaatcaa a                                           321
<210> 120
   <211> 107
   <212> PRT
   <213> Artificial Sequence
<220>
   <223> Synthetic
<400> 120
   Ala Ile Gln Met Thr Gln Ser Pro Ser Ser Leu Ser Ala Ser Val Gly
    1               5                  10                  15
   Asp Arg Val Thr Ile Thr Cys Arg Ala Ser Gln Asp Ile Arg Asn Asp
                   20                  25                  30
   Leu Gly Trp Tyr Gln Gln Lys Pro Gly Lys Ala Pro Lys Leu Leu Ile
               35                  40                  45
   Tyr Ala Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Leu Gln Asp Tyr Asn Tyr Pro Trp
                   85                  90                  95
   Thr Phe Gly Gln Gly Thr Lys Val Glu Ile Lys
                   100                 105
<210> 121
   <211> 384
   <212> DNA
   <213> Artificial Sequence
<220>
   <223> Synthetic
<400> 121
   caggtgcagc tgcaggagtc ggggccagga ctggtgaagc cttcggagac cctgtccctc 60
   acctgcactg tctctgggga ctccatcaat acttactact ggagctggtt ccggcagccc 120
   ccagggaagg gactggagtg gattgggtat atctattata gtggaaccac caactacaac 180
   ccctccctca agagtcgagt caccatatca atagacacgc ccaggaacca gttctccctg 240
   aagctgatct ctgtgaccgc agcggacacg gccgtgtatt actgtgcgag agagaggatt 300
   actatgattc ggggagttac cctctactat tactcctacg gtatggacgt ctggggccaa 360
   gggaccacgg tcaccgtctc ctca                                        384
<210> 122
   <211> 128
   <212> PRT
   <213> Artificial Sequence
<220>
   <223> Synthetic
<400> 122
   Gln Val Gln Leu Gln Glu Ser Gly Pro Gly Leu Val Lys Pro Ser Glu
    1               5                  10                  15
   Thr Leu Ser Leu Thr Cys Thr Val Ser Gly Asp Ser Ile Asn Thr Tyr
               20                  25                  30
   Tyr Trp Ser Trp Phe Arg Gln Pro Pro Gly Lys Gly Leu Glu Trp Ile
           35                  40                  45
   Gly Tyr Ile Tyr Tyr Ser Gly Thr Thr Asn Tyr Asn Pro Ser Leu Lys
       50                  55                  60
   Ser Arg Val Thr Ile Ser Ile Asp Thr Pro Arg Asn Gln Phe Ser Leu
   65                  70                  75                  80
   Lys Leu Ile Ser Val Thr Ala Ala Asp Thr Ala Val Tyr Tyr Cys Ala
                   85                  90                  95
   Arg Glu Arg Ile Thr Met Ile Arg Gly Val Thr Leu Tyr Tyr Tyr Ser
               100                 105                 110
   Tyr Gly Met Asp Val Trp Gly Gln Gly Thr Thr Val Thr Val Ser Ser
           115                 120                 125
<210> 123
   <211> 24
   <212> DNA
   <213> Artificial Sequence
<220>
   <223> Synthetic
<400> 123
   ggggactcca tcaatactta ctac 24
<210> 124
   <211> 8
   <212> PRT
   <213> Artificial Sequence
<220>
   <223> Synthetic
<400> 124
   Gly Asp Ser Ile Asn Thr Tyr Tyr
   1               5
<210> 125
   <211> 21
   <212> DNA
   <213> Artificial Sequence
<220>
   <223> Synthetic
<400> 125
   atctattata gtggaaccac c 21
<210> 126
   <211> 7
   <212> PRT
   <213> Artificial Sequence
<220>
   <223> Synthetic
<400> 126
   Ile Tyr Tyr Ser Gly Thr Thr
   1               5
<210> 127
   <211> 66
   <212> DNA
   <213> Artificial Sequence
<220>
   <223> Synthetic
<400> 127
   gcgagagaga ggattactat gattcgggga gttaccctct actattactc ctacggtatg 60
   gacgtc                                                            66
<210> 128
   <211> 22
   <212> PRT
   <213> Artificial Sequence
<220>
   <223> Synthetic
<400> 128
   Ala Arg Glu Arg Ile Thr Met Ile Arg Gly Val Thr Leu Tyr Tyr Tyr
    1              5                   10                  15
   Ser Tyr Gly Met Asp Val
               20
<210> 129
   <211> 324
   <212> DNA
   <213> Artificial Sequence
<220>
   <223> Synthetic
<400> 129
   gacatccaga tgacccagtc tccatccttc ctgtctgcat ctgtaggaga cagagtcacc 60
   atcacttgct gggccagtca ggacattagc agttatttag cctggtatca gcaaaaacca 120
   gggatagccc ctaagctcct gatctatgct gcatccactt tgcaaagtgg ggtcccatca 180
   aggttcggcg gcagtggatc tgggacagaa ttcactctca caatcagcag cctgcagcct 240
   gaagattttg caacttatta ctgtcaacag cttaatagtt accctcggac gttcggccaa 300
   gggaccaagg tggaaatcaa acga                                        324
<210> 130
   <211> 108
   <212> PRT
   <213> Artificial Sequence
<220>
   <223> Synthetic
<400> 130
   Asp Ile Gln Met Thr Gln Ser Pro Ser Phe Leu Ser Ala Ser Val Gly
    1              5                   10                  15
   Asp Arg Val Thr Ile Thr Cys Trp Ala Ser Gln Asp Ile Ser Ser Tyr
               20                  25                  30
   Leu Ala Trp Tyr Gln Gln Lys Pro Gly Ile Ala Pro Lys Leu Leu Ile
           35                  40                  45
   Tyr Ala Ala Ser Thr Leu Gln Ser Gly Val Pro Ser Arg Phe Gly Gly
       50                  55                  60
   Ser Gly Ser Gly Thr Glu Phe Thr Leu Thr Ile Ser Ser Leu Gln Pro
   65                  70                  75                  80
   Glu Asp Phe Ala Thr Tyr Tyr Cys Gln Gln Leu Asn Ser Tyr Pro Arg
                   85                  90                  95
   Thr Phe Gly Gln Gly Thr Lys Val Glu Ile Lys Arg
               100                 105
<210> 131
   <211> 18
   <212> DNA
   <213> Artificial Sequence
<220>
   <223> Synthetic
<400> 131
   caggacatta gcagttat 18
<210> 132
   <211> 6
   <212> PRT
   <213> Artificial Sequence
<220>
   <223> Synthetic
<400> 132
   Gln Asp Ile Ser Ser Tyr
   1 5
<210> 133
   <211> 9
   <212> DNA
   <213> Artificial Sequence
<220>
   <223> Synthetic
<400> 133
   gctgcatcc 9
<210> 134
   <211> 3
   <212> PRT
   <213> Artificial Sequence
<220>
   <223> Synthetic
<400> 134
   Ala Ala Ser
   1
<210> 135
   <211> 27
   <212> DNA
   <213> Artificial Sequence
<220>
   <223> Synthetic
<400> 135
   caacagctta atagttaccc tcggacg 27
<210> 136
   <211> 9
   <212> PRT
   <213> Artificial Sequence
<220>
   <223> Synthetic
<400> 136
   Gln Gln Leu Asn Ser Tyr Pro Arg Thr
    1               5
<210> 137
   <211> 384
   <212> DNA
   <213> Artificial Sequence
<220>
   <223> Synthetic
<400> 137
   caggtgcagc tgcaggagtc ggggccagga ctggtgaagc cttcggagac cctgtccctc 60
   acctgcactg tctctgggga ctccatcaat acttactact ggagctggtt ccggcagccc 120
   ccagggaagg gactggagtg gattgggtat atctattata gtggaaccac caactacaac 180
   ccctccctca agagtcgagt caccatatca atagacacgc ccaggaacca gttctccctg 240
   aagctgatct ctgtgaccgc agcggacacg gccgtgtatt actgtgcgag agagaggatt 300
   actatgattc ggggagttac cctctactat tactcctacg gtatggacgt ctggggccaa 360
   gggaccacgg tcaccgtctc ctca                                        384
<210> 138
   <211> 128
   <212> PRT
   <213> Artificial Sequence
<220>
   <223> Synthetic
<400> 138
   Gln Val Gln Leu Gln Glu Ser Gly Pro Gly Leu Val Lys Pro Ser Glu
    1               5                  10                  15
   Thr Leu Ser Leu Thr Cys Thr Val Ser Gly Asp Ser Ile Asn Thr Tyr
               20                  25                  30
   Tyr Trp Ser Trp Phe Arg Gln Pro Pro Gly Lys Gly Leu Glu Trp Ile
           35                  40                  45
   Gly Tyr Ile Tyr Tyr Ser Gly Thr Thr Asn Tyr Asn Pro Ser Leu Lys
       50                  55                  60
   Ser Arg Val Thr Ile Ser Ile Asp Thr Pro Arg Asn Gln Phe Ser Leu
   65                  70                  75                  80
   Lys Leu Ile Ser Val Thr Ala Ala Asp Thr Ala Val Tyr Tyr Cys Ala
                   85                  90                  95
   Arg Glu Arg Ile Thr Met Ile Arg Gly Val Thr Leu Tyr Tyr Tyr Ser
               100                 105                 110
   Tyr Gly Met Asp Val Trp Gly Gln Gly Thr Thr Val Thr Val Ser Ser
           115                 120                 125
<210> 139
   <211> 321
   <212> DNA
   <213> Artificial Sequence
<220>
   <223> Synthetic
<400> 139
   gacatccaga tgacccagtc tccatccttc ctgtctgcat ctgtaggaga cagagtcacc 60
   atcacttgct gggccagtca ggacattagc agttatttag cctggtatca gcaaaaacca 120
   gggatagccc ctaagctcct gatctatgct gcatccactt tgcaaagtgg ggtcccatca 180
   aggttcggcg gcagtggatc tgggacagaa ttcactctca caatcagcag cctgcagcct 240
   gaagattttg caacttatta ctgtcaacag cttaatagtt accctcggac gttcggccaa 300
   gggaccaagg tggaaatcaa a                                           321
<210> 140
   <211> 107
   <212> PRT
   <213> Artificial Sequence
<220>
   <223> Synthetic
<400> 140
   Asp Ile Gln Met Thr Gln Ser Pro Ser Phe Leu Ser Ala Ser Val Gly
    1               5                  10                  15
   Asp Arg Val Thr Ile Thr Cys Trp Ala Ser Gln Asp Ile Ser Ser Tyr
               20                  25                  30
   Leu Ala Trp Tyr Gln Gln Lys Pro Gly Ile Ala Pro Lys Leu Leu Ile
           35                  40                  45
   Tyr Ala Ala Ser Thr Leu Gln Ser Gly Val Pro Ser Arg Phe Gly Gly
       50                  55                  60
   Ser Gly Ser Gly Thr Glu Phe Thr Leu Thr Ile Ser Ser Leu Gln Pro
   65                  70                  75                  80
   Glu Asp Phe Ala Thr Tyr Tyr Cys Gln Gln Leu Asn Ser Tyr Pro Arg
                     85                  90                  95
   Thr Phe Gly Gln Gly Thr Lys Val Glu Ile Lys
               100                 105
<210> 141
   <211> 384
   <212> DNA
   <213> Artificial Sequence
<220>
   <223> Synthetic
<400> 141
   caggtgcagc tgcaggagtc gggcccagga ctggtgaagc cttcggagac cctgtccctc 60
   acctgcactg tctctgggga ctccatcaat acttactact ggagctggat ccggcagccc 120
   ccagggaagg gactggagtg gattgggtat atctattata gtggaaccac caactacaac 180
   ccctccctca agagtcgagt caccatatca gtagacacgt ccaagaacca gttctccctg 240
   aagctgagct ctgtgaccgc tgcggacacg gccgtgtatt actgtgcgag agagaggatt 300
   actatgattc ggggagttac cctctactat tactcctacg gtatggacgt ctggggccaa 360
   gggaccacgg tcaccgtctc ctca 384
<210> 142
   <211> 128
   <212> PRT
   <213> Artificial Sequence
<220>
   <223> Synthetic
<400> 142
   Gln Val Gln Leu Gln Glu Ser Gly Pro Gly Leu Val Lys Pro Ser Glu
   1 5 10 15
   Thr Leu Ser Leu Thr Cys Thr Val Ser Gly Asp Ser Ile Asn Thr Tyr
   20 25 30
   Tyr Trp Ser Trp Ile Arg Gln Pro Pro Gly Lys Gly Leu Glu Trp Ile
   35 40 45
   Gly Tyr Ile Tyr Tyr Ser Gly Thr Thr Asn Tyr Asn Pro Ser Leu Lys
   50 55 60
   Ser Arg Val Thr Ile Ser Val Asp Thr Ser Lys Asn Gln Phe Ser Leu
   65 70 75 80
   Lys Leu Ser Ser Val Thr Ala Ala Asp Thr Ala Val Tyr Tyr Cys Ala
   85 90 95
   Arg Glu Arg Ile Thr Met Ile Arg Gly Val Thr Leu Tyr Tyr Tyr Ser
   100 105 110
   Tyr Gly Met Asp Val Trp Gly Gln Gly Thr Thr Val Thr Val Ser Ser
   115 120 125
<210> 143
   <211> 321
   <212> DNA
   <213> Artificial Sequence
<220>
   <223> Synthetic
<400> 143
   gacatccaga tgacccagtc tccatcctcc ctgtctgcat ctgtaggaga cagagtcacc 60
   atcacttgcc gggcaagtca ggacattagc agttatttag gctggtatca gcagaaacca 120
   gggaaagccc ctaagcgcct gatctatgct gcatccagtt tgcaaagtgg ggtcccatca 180
   aggttcagcg gcagtggatc tgggacagaa ttcactctca caatcagcag cctgcagcct 240
   gaagattttg caacttatta ctgtcaacag cttaatagtt accctcggac gttcggccaa 300
   gggaccaagg tggaaatcaa a 321
<210> 144
   <211> 107
   <212> PRT
   <213> Artificial Sequence
<220>
   <223> Synthetic
<400> 144
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Asp Ile Ser Ser Tyr
               20                  25                  30
   Leu Gly Trp Tyr Gln Gln Lys Pro Gly Lys Ala Pro Lys Arg Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Glu Asp Phe Ala Thr Tyr Tyr Cys Gln Gln Leu Asn Ser Tyr Pro Arg
                   85                  90                  95
   Thr Phe Gly Gln Gly Thr Lys Val Glu Ile Lys
               100                 105
<210> 145
   <211> 378
   <212> DNA
   <213> Artificial Sequence
<220>
   <223> Synthetic
<400> 145
   caggtgcagc tggtgcagtc tggagctgag gtgaagaagc ctggggcctc agtgaaggtc 60
   tcctgcaagg cttctggtta cacctttacc aactatggta tcagctgggt gcgacaggcc 120
   cctggacaag gacttgagtt aatgggatgg attagtggtt acaatggtaa cacaaactat 180
   gcacaagaac tccaggccag agtcaccatg accacagaca catccacgag cacagcctac 240
   atggagctga ggaacctgag atctgacgac acggccgtat attactgtgc gagagataga 300
   gtcgttgtag cagctgctaa ttactacttt tattctatgg acgtctgggg ccaagggacc 360
   acggtcaccg tctcctca                                               378
<210> 146
   <211> 126
   <212> PRT
   <213> Artificial Sequence
<220>
   <223> Synthetic
<400> 146
   Gln Val Gln Leu Val Gln Ser Gly Ala Glu Val Lys Lys Pro Gly Ala
    1               5                  10                  15
   Ser Val Lys Val Ser Cys Lys Ala Ser Gly Tyr Thr Phe Thr Asn Tyr
               20                  25                  30
   Gly Ile Ser Trp Val Arg Gln Ala Pro Gly Gln Gly Leu Glu Leu Met
           35                  40                  45
   Gly Trp Ile Ser Gly Tyr Asn Gly Asn Thr Asn Tyr Ala Gln Glu Leu
       50                  55                  60
   Gln Ala Arg Val Thr Met Thr Thr Asp Thr Ser Thr Ser Thr Ala Tyr
   65                  70                  75                  80
   Met Glu Leu Arg Asn Leu Arg Ser Asp Asp Thr Ala Val Tyr Tyr Cys
                   85                  90                  95
   Ala Arg Asp Arg Val Val Val Ala Ala Ala Asn Tyr Tyr Phe Tyr Ser
               100                 105                 110
   Met Asp Val Trp Gly Gln Gly Thr Thr Val Thr Val Ser Ser
           115                 120                 125
<210> 147
   <211> 24
   <212> DNA
   <213> Artificial Sequence
<220>
   <223> Synthetic
<400> 147
   ggttacacct ttaccaacta tggt 24
<210> 148
   <211> 8
   <212> PRT
   <213> Artificial Sequence
<220>
   <223> Synthetic
<400> 148
   Gly Tyr Thr Phe Thr Asn Tyr Gly
    1               5
<210> 149
   <211> 24
   <212> DNA
   <213> Artificial Sequence
<220>
   <223> Synthetic
<400> 149
   attagtggtt acaatggtaa caca 24
<210> 150
   <211> 8
   <212> PRT
   <213> Artificial Sequence
<220>
   <223> Synthetic
<400> 150
   Ile Ser Gly Tyr Asn Gly Asn Thr
   1               5
<210> 151
   <211> 57
   <212> DNA
   <213> Artificial Sequence
<220>
   <223> Synthetic
<400> 151
   gcgagagata gagtcgttgt agcagctgct aattactact tttattctat ggacgtc 57
<210> 152
   <211> 19
   <212> PRT
   <213> Artificial Sequence
<220>
   <223> Synthetic
<400> 152
   Ala Arg Asp Arg Val Val Val Ala Ala Ala Asn Tyr Tyr Phe Tyr Ser
   1               5                   10                  15
   Met Asp Val
<210> 153
   <211> 339
   <212> DNA
   <213> Artificial Sequence
<220>
   <223> Synthetic
<400> 153
   gccatccaga tgacccagtc tccactctcc ctgtccgtca cccttggaca gccggcctcc 60
   atctcctgca ggtctagtca aagcctcgta tacagtgatg gagacaccta cttgaattgg 120
   tttcagcaga ggccaggcca atctccaagg cgcctaattt ataaggtttc taaccgggac 180
   tctggggtcc cagacagatt cagcggcagt gggtcaggca ctgctttcac actgaaaatc 240
   agcggggtgg aggccgagga tgttggggtt tactactgca tgcaagctac acactggcct 300
   cggacgttcg gccaagggac caaggtggaa atcaaacga 339
<210> 154
   <211> 113
   <212> PRT
   <213> Artificial Sequence
<220>
   <223> Synthetic
<400> 154
   Ala Ile Gln Met Thr Gln Ser Pro Leu Ser Leu Ser Val Thr Leu Gly
    1               5                  10                  15
   Gln Pro Ala Ser Ile Ser Cys Arg Ser Ser Gln Ser Leu Val Tyr Ser
               20                  25                  30
   Asp Gly Asp Thr Tyr Leu Asn Trp Phe Gln Gln Arg Pro Gly Gln Ser
           35                  40                  45
   Pro Arg Arg Leu Ile Tyr Lys Val Ser Asn Arg Asp Ser Gly Val Pro
       50                  55                  60
   Asp Arg Phe Ser Gly Ser Gly Ser Gly Thr Ala Phe Thr Leu Lys Ile
   65                  70                  75                  80
   Ser Gly Val Glu Ala Glu Asp Val Gly Val Tyr Tyr Cys Met Gln Ala
                   85                  90                  95
   Thr His Trp Pro Arg Thr Phe Gly Gln Gly Thr Lys Val Glu Ile Lys
               100                 105                 110
   Arg
<210> 155
   <211> 33
   <212> DNA
   <213> Artificial Sequence
<220>
   <223> Synthetic
<400> 155
   caaagcctcg tatacagtga tggagacacc tac 33
<210> 156
   <211> 11
   <212> PRT
   <213> Artificial Sequence
<220>
   <223> Synthetic
<400> 156
   Gln Ser Leu Val Tyr Ser Asp Gly Asp Thr Tyr
    1               5                  10
<210> 157
   <211> 9
   <212> DNA
   <213> Artificial Sequence
<220>
   <223> Synthetic
<400> 157
   aaggtttct 9
<210> 158
   <211> 3
   <212> PRT
   <213> Artificial Sequence
<220>
   <223> Synthetic
<400> 158
   Lys Val Ser
   1
<210> 159
   <211> 27
   <212> DNA
   <213> Artificial Sequence
<220>
   <223> Synthetic
<400> 159
   atgcaagcta cacactggcc tcggacg 27
<210> 160
   <211> 9
   <212> PRT
   <213> Artificial Sequence
<220>
   <223> Synthetic
<400> 160
   Met Gln Ala Thr His Trp Pro Arg Thr
   1 5
<210> 161
   <211> 378
   <212> DNA
   <213> Artificial Sequence
<220>
   <223> Synthetic
<400> 161
   caggttcagc tggtgcagtc tggagctgag gtgaagaagc ctggggcctc agtgaaggtc 60
   tcctgcaagg cttctggtta cacctttacc aactatggta tcagctgggt gcgacaggcc 120
   cctggacaag gacttgagtt aatgggatgg attagtggtt acaatggtaa cacaaactat 180
   gcacaagaac tccaggccag agtcaccatg accacagaca catccacgag cacagcctac 240
   atggagctga ggaacctgag atctgacgac acggccgtat attactgtgc gagagataga 300
   gtcgttgtag cagctgctaa ttactacttt tattctatgg acgtctgggg ccaagggacc 360
   acggtcaccg tctcctca                                               378
<210> 162
   <211> 126
   <212> PRT
   <213> Artificial Sequence
<220>
   <223> Synthetic
<400> 162
   Gln Val Gln Leu Val Gln Ser Gly Ala Glu Val Lys Lys Pro Gly Ala
    1               5                  10                  15
   Ser Val Lys Val Ser Cys Lys Ala Ser Gly Tyr Thr Phe Thr Asn Tyr
               20                  25                  30
   Gly Ile Ser Trp Val Arg Gln Ala Pro Gly Gln Gly Leu Glu Leu Met
           35                  40                  45
   Gly Trp Ile Ser Gly Tyr Asn Gly Asn Thr Asn Tyr Ala Gln Glu Leu
       50                  55                  60
   Gln Ala Arg Val Thr Met Thr Thr Asp Thr Ser Thr Ser Thr Ala Tyr
   65                  70                  75                  80
   Met Glu Leu Arg Asn Leu Arg Ser Asp Asp Thr Ala Val Tyr Tyr Cys
                   85                  90                  95
   Ala Arg Asp Arg Val Val Val Ala Ala Ala Asn Tyr Tyr Phe Tyr Ser
               100                 105                 110
   Met Asp Val Trp Gly Gln Gly Thr Thr Val Thr Val Ser Ser
           115                 120                 125
<210> 163
   <211> 336
   <212> DNA
   <213> Artificial Sequence
<220>
   <223> Synthetic
<400> 163
   gatgttgtga tgactcagtc tccactctcc ctgtccgtca cccttggaca gccggcctcc 60
   atctcctgca ggtctagtca aagcctcgta tacagtgatg gagacaccta cttgaattgg 120
   tttcagcaga ggccaggcca atctccaagg cgcctaattt ataaggtttc taaccgggac 180
   tctggggtcc cagacagatt cagcggcagt gggtcaggca ctgctttcac actgaaaatc 240
   agcggggtgg aggccgagga tgttggggtt tactactgca tgcaagctac acactggcct 300
   cggacgttcg gccaagggac caaggtggaa atcaaa                           336
<210> 164
   <211> 112
   <212> PRT
   <213> Artificial Sequence
<220>
   <223> Synthetic
<400> 164
   Asp Val Val Met Thr Gln Ser Pro Leu Ser Leu Ser Val Thr Leu Gly
    1               5                  10                  15
   Gln Pro Ala Ser Ile Ser Cys Arg Ser Ser Gln Ser Leu Val Tyr Ser
               20                  25                  30
   Asp Gly Asp Thr Tyr Leu Asn Trp Phe Gln Gln Arg Pro Gly Gln Ser
           35                  40                  45
   Pro Arg Arg Leu Ile Tyr Lys Val Ser Asn Arg Asp Ser Gly Val Pro
       50                  55                  60
   Asp Arg Phe Ser Gly Ser Gly Ser Gly Thr Ala Phe Thr Leu Lys Ile
   65                  70                  75                  80
   Ser Gly Val Glu Ala Glu Asp Val Gly Val Tyr Tyr Cys Met Gln Ala
                   85                  90                  95
   Thr His Trp Pro Arg Thr Phe Gly Gln Gly Thr Lys Val Glu Ile Lys
               100                 105                 110
<210> 165
   <211> 378
   <212> DNA
   <213> Artificial Sequence
<220>
   <223> Synthetic
<400> 165
   caggttcagc tggtgcagtc tggagctgag gtgaagaagc ctggggcctc agtgaaggtc 60
   tcctgcaagg cttctggtta cacctttacc aactatggta tcagctgggt gcgacaggcc 120
   cctggacaag ggcttgagtg gatgggatgg attagtggtt acaatggtaa cacaaactat 180
   gcacagaagc tccagggcag agtcaccatg accacagaca catccacgag cacagcctac 240
   atggagctga ggagcctgag atctgacgac acggccgtgt attactgtgc gagagataga 300
   gtcgttgtag cagctgctaa ttactacttt tattctatgg acgtctgggg ccaagggacc 360
   acggtcaccg tctcctca                                                378
<210> 166
   <211> 126
   <212> PRT
   <213> Artificial Sequence
<220>
   <223> Synthetic
<400> 166
   Gln Val Gln Leu Val Gln Ser Gly Ala Glu Val Lys Lys Pro Gly Ala
    1               5                  10                  15
   Ser Val Lys Val Ser Cys Lys Ala Ser Gly Tyr Thr Phe Thr Asn Tyr
               20                  25                  30
   Gly Ile Ser Trp Val Arg Gln Ala Pro Gly Gln Gly Leu Glu Trp Met
           35                  40                  45
   Gly Trp Ile Ser Gly Tyr Asn Gly Asn Thr Asn Tyr Ala Gln Lys Leu
       50                  55                  60
   Gln Gly Arg Val Thr Met Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Asp Arg Val Val Val Ala Ala Ala Asn Tyr Tyr Phe Tyr Ser
               100                 105                 110
   Met Asp Val Trp Gly Gln Gly Thr Thr Val Thr Val Ser Ser
           115                 120                 125
<210> 167
   <211> 336
   <212> DNA
   <213> Artificial Sequence
<220>
   <223> Synthetic
<400> 167
   gatgttgtga tgactcagtc tccactctcc ctgcccgtca cccttggaca gccggcctcc 60
   atctcctgca ggtctagtca aagcctcgta tacagtgatg gagacaccta cttgaattgg 120
   tttcagcaga ggccaggcca atctccaagg cgcctaattt ataaggtttc taaccgggac 180
   tctggggtcc cagacagatt cagcggcagt gggtcaggca ctgatttcac actgaaaatc 240
   agcagggtgg aggctgagga tgttggggtt tattactgca tgcaagctac acactggcct 300
   cggacgttcg gccaagggac caaggtggaa atcaaa                           336
<210> 168
   <211> 112
   <212> PRT
   <213> Artificial Sequence
<220>
   <223> Synthetic
<400> 168
   Asp Val Val Met Thr Gln Ser Pro Leu Ser Leu Pro Val Thr Leu Gly
    1               5                  10                  15
   Gln Pro Ala Ser Ile Ser Cys Arg Ser Ser Gln Ser Leu Val Tyr Ser
                   20                  25                  30
   Asp Gly Asp Thr Tyr Leu Asn Trp Phe Gln Gln Arg Pro Gly Gln Ser
               35                  40                  45
   Pro Arg Arg Leu Ile Tyr Lys Val Ser Asn Arg Asp Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ala
                   85                  90                  95
   Thr His Trp Pro Arg Thr Phe Gly Gln Gly Thr Lys Val Glu Ile Lys
               100                 105                 110
<210> 169
   <211> 375
   <212> DNA
   <213> Artificial Sequence
<220>
   <223> Synthetic
<400> 169
   caggtccact tgaaggagtc tggtcctacg ctggtgaaac ccacacagac cctcacgctg 60
   acctgcacct tctctggatt ctcactcatc actagtggag tgggtgtggg ctggattcgt 120
   cagccccccg gaaaggccct ggagtggctt gcactcattt attggaatgg tgataagcgc 180
   tacagcccat ctctgaagag caggctcacc atcaccaagg acacctccaa aaaccaggtg 240
   gtccttacaa tgaccaacat ggaccctgtg gacacagcca catattactg tgcacacagg 300
   ataactgaaa ctagttacta cttctactac ggtatggacg tctggggcca agggaccacg 360
   gtcaccgtct cctca                                                  375
<210> 170
   <211> 125
   <212> PRT
   <213> Artificial Sequence
<220>
   <223> Synthetic
<400> 170
   Gln Val His Leu Lys Glu Ser Gly Pro Thr Leu Val Lys Pro Thr Gln
    1               5                  10                  15
   Thr Leu Thr Leu Thr Cys Thr Phe Ser Gly Phe Ser Leu Ile Thr Ser
               20                  25                  30
   Gly Val Gly Val Gly Trp Ile Arg Gln Pro Pro Gly Lys Ala Leu Glu
           35                  40                  45
   Trp Leu Ala Leu Ile Tyr Trp Asn Gly Asp Lys Arg Tyr Ser Pro Ser
       50                  55                  60
   Leu Lys Ser Arg Leu Thr Ile Thr Lys Asp Thr Ser Lys Asn Gln Val
   65                  70                  75                  80
   Val Leu Thr Met Thr Asn Met Asp Pro Val Asp Thr Ala Thr Tyr Tyr
                   85                  90                  95
   Cys Ala His Arg Ile Thr Glu Thr Ser Tyr Tyr Phe Tyr Tyr Gly Met
               100                 105                 110
   Asp Val Trp Gly Gln Gly Thr Thr Val Thr Val Ser Ser
           115                 120                 125
<210> 171
   <211> 30
   <212> DNA
   <213> Artificial Sequence
<220>
   <223> Synthetic
<400> 171
   ggattctcac tcatcactag tggagtgggt 30
<210> 172
   <211> 10
   <212> PRT
   <213> Artificial Sequence
<220>
   <223> Synthetic
<400> 172
   Gly Phe Ser Leu Ile Thr Ser Gly Val Gly
    1               5                  10
<210> 173
   <211> 21
   <212> DNA
   <213> Artificial Sequence
<220>
   <223> Synthetic
<400> 173
   atttattgga atggtgataa g 21
<210> 174
   <211> 7
   <212> PRT
   <213> Artificial Sequence
<220>
   <223> Synthetic
<400> 174
   Ile Tyr Trp Asn Gly Asp Lys
    1               5
<210> 175
   <211> 51
   <212> DNA
   <213> Artificial Sequence
<220>
   <223> Synthetic
<400> 175
   gcacacagga taactgaaac tagttactac ttctactacg gtatggacgt c 51
<210> 176
   <211> 17
   <212> PRT
   <213> Artificial Sequence
<220>
   <223> Synthetic
<400> 176
   Ala His Arg Ile Thr Glu Thr Ser Tyr Tyr Phe Tyr Tyr Gly Met Asp
    1               5                   10                  15
   Val
<210> 177
   <211> 339
   <212> DNA
   <213> Artificial Sequence
<220>
   <223> Synthetic
<400> 177
   gacatccaga tgacccagtc tccactctcc ctgcccgtca cccctggaga gccggcctcc 60
   atctcctgca ggtctagtca gagcctcctg catagtcatg gatacgacta tttggattgg 120
   tacctgcaga agccagggca gtctccacag ctcctgatct atttgggttc taatcgggcc 180
   tccggggtcc ctgacaggtt cagtggcagt ggatcaggca cagattttac actgaaaatc 240
   agcagagtgg aggctgagga tgttggggtt tattactgca tgcaagctct acaaactccg 300
   ctcactttcg gcggagggac caaggtggaa atcaaacga                        339
<210> 178
   <211> 113
   <212> PRT
   <213> Artificial Sequence
<220>
   <223> Synthetic
<400> 178
   Asp Ile Gln Met Thr Gln Ser Pro Leu Ser Leu Pro Val Thr Pro Gly
    1               5                  10                  15
   Glu Pro Ala Ser Ile Ser Cys Arg Ser Ser Gln Ser Leu Leu His Ser
               20                  25                  30
   His Gly Tyr Asp Tyr Leu Asp Trp Tyr Leu Gln Lys Pro Gly Gln Ser
           35                  40                  45
   Pro Gln Leu Leu Ile Tyr Leu Gly Ser Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ala
                   85                  90                  95
   Leu Gln Thr Pro Leu Thr Phe Gly Gly Gly Thr Lys Val Glu Ile Lys
               100                 105                 110
   Arg
<210> 179
   <211> 33
   <212> DNA
   <213> Artificial Sequence
<220>
   <223> Synthetic
<400> 179
   cagagcctcc tgcatagtca tggatacgac tat 33
<210> 180
   <211> 11
   <212> PRT
   <213> Artificial Sequence
<220>
   <223> Synthetic
<400> 180
   Gln Ser Leu Leu His Ser His Gly Tyr Asp Tyr
    1               5                  10
<210> 181
   <211> 9
   <212> DNA
   <213> Artificial Sequence
<220>
   <223> Synthetic
<400> 181
   ttgggttct 9
<210> 182
   <211> 3
   <212> PRT
   <213> Artificial Sequence
<220>
   <223> Synthetic
<400> 182
   Leu Gly Ser
   1
<210> 183
   <211> 27
   <212> DNA
   <213> Artificial Sequence
<220>
   <223> Synthetic
<400> 183
   atgcaagctc tacaaactcc gctcact 27
<210> 184
   <211> 9
   <212> PRT
   <213> Artificial Sequence
<220>
   <223> Synthetic
<400> 184
   Met Gln Ala Leu Gln Thr Pro Leu Thr
   1 5
<210> 185
   <211> 375
   <212> DNA
   <213> Artificial Sequence
<220>
   <223> Synthetic
<400> 185
   cagatcacct tgaaggagtc tggtcctacg ctggtgaaac ccacacagac cctcacgctg 60
   acctgcacct tctctggatt ctcactcatc actagtggag tgggtgtggg ctggattcgt 120
   cagccccccg gaaaggccct ggagtggctt gcactcattt attggaatgg tgataagcgc 180
   tacagcccat ctctgaagag caggctcacc atcaccaagg acacctccaa aaaccaggtg 240
   gtccttacaa tgaccaacat ggaccctgtg gacacagcca catattactg tgcacacagg 300
   ataactgaaa ctagttacta cttctactac ggtatggacg tctggggcca agggaccacg 360
   gtcaccgtct cctca 375
<210> 186
   <211> 125
   <212> PRT
   <213> Artificial Sequence
<220>
   <223> Synthetic
<400> 186
   Gln Ile Thr Leu Lys Glu Ser Gly Pro Thr Leu Val Lys Pro Thr Gln
    1               5                  10                  15
   Thr Leu Thr Leu Thr Cys Thr Phe Ser Gly Phe Ser Leu Ile Thr Ser
               20                  25                  30
   Gly Val Gly Val Gly Trp Ile Arg Gln Pro Pro Gly Lys Ala Leu Glu
           35                  40                  45
   Trp Leu Ala Leu Ile Tyr Trp Asn Gly Asp Lys Arg Tyr Ser Pro Ser
       50                  55                  60
   Leu Lys Ser Arg Leu Thr Ile Thr Lys Asp Thr Ser Lys Asn Gln Val
   65                  70                  75                  80
   Val Leu Thr Met Thr Asn Met Asp Pro Val Asp Thr Ala Thr Tyr Tyr
                   85                  90                  95
   Cys Ala His Arg Ile Thr Glu Thr Ser Tyr Tyr Phe Tyr Tyr Gly Met
               100                 105                 110
   Asp Val Trp Gly Gln Gly Thr Thr Val Thr Val Ser Ser
           115                 120                 125
<210> 187
   <211> 336
   <212> DNA
   <213> Artificial Sequence
<220>
   <223> Synthetic
<400> 187
   gatattgtga tgactcagtc tccactctcc ctgcccgtca cccctggaga gccggcctcc 60
   atctcctgca ggtctagtca gagcctcctg catagtcatg gatacgacta tttggattgg 120
   tacctgcaga agccagggca gtctccacag ctcctgatct atttgggttc taatcgggcc 180
   tccggggtcc ctgacaggtt cagtggcagt ggatcaggca cagattttac actgaaaatc 240
   agcagagtgg aggctgagga tgttggggtt tattactgca tgcaagctct acaaactccg 300
   ctcactttcg gcggagggac caaggtggag atcaaa                           336
<210> 188
   <211> 112
   <212> PRT
   <213> Artificial Sequence
<220>
   <223> Synthetic
<400> 188
   Asp Ile Val Met Thr Gln Ser Pro Leu Ser Leu Pro Val Thr Pro Gly
    1               5                  10                  15
   Glu Pro Ala Ser Ile Ser Cys Arg Ser Ser Gln Ser Leu Leu His Ser
               20                  25                  30
   His Gly Tyr Asp Tyr Leu Asp Trp Tyr Leu Gln Lys Pro Gly Gln Ser
           35                  40                  45
   Pro Gln Leu Leu Ile Tyr Leu Gly Ser Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ala
                   85                  90                  95
   Leu Gln Thr Pro Leu Thr Phe Gly Gly Gly Thr Lys Val Glu Ile Lys
               100                 105                 110
<210> 189
   <211> 375
   <212> DNA
   <213> Artificial Sequence
<220>
   <223> Synthetic
<400> 189
   cagatcacct tgaaggagtc tggtcctacg ctggtgaaac ccacacagac cctcacgctg 60
   acctgcacct tctctggatt ctcactcatc actagtggag tgggtgtggg ctggatccgt 120
   cagcccccag gaaaggccct ggagtggctt gcactcattt attggaatgg tgataagcgc 180
   tacagcccat ctctgaagag caggctcacc atcaccaagg acacctccaa aaaccaggtg 240
   gtccttacaa tgaccaacat ggaccctgtg gacacagcca catattactg tgcacacagg 300
   ataactgaaa ctagttacta cttctactac ggtatggacg tctggggcca agggaccacg 360
   gtcaccgtct cctca                                                  375
<210> 190
   <211> 125
   <212> PRT
   <213> Artificial Sequence
<220>
   <223> Synthetic
<400> 190
   Gln Ile Thr Leu Lys Glu Ser Gly Pro Thr Leu Val Lys Pro Thr Gln
    1               5                  10                  15
   Thr Leu Thr Leu Thr Cys Thr Phe Ser Gly Phe Ser Leu Ile Thr Ser
                   20                  25                  30
   Gly Val Gly Val Gly Trp Ile Arg Gln Pro Pro Gly Lys Ala Leu Glu
               35                  40                  45
   Trp Leu Ala Leu Ile Tyr Trp Asn Gly Asp Lys Arg Tyr Ser Pro Ser
       50                  55                  60
   Leu Lys Ser Arg Leu Thr Ile Thr Lys Asp Thr Ser Lys Asn Gln Val
   65                  70                  75                  80
   Val Leu Thr Met Thr Asn Met Asp Pro Val Asp Thr Ala Thr Tyr Tyr
                   85                  90                  95
   Cys Ala His Arg Ile Thr Glu Thr Ser Tyr Tyr Phe Tyr Tyr Gly Met
               100                 105                 110
   Asp Val Trp Gly Gln Gly Thr Thr Val Thr Val Ser Ser
           115                 120                 125
<210> 191
   <211> 336
   <212> DNA
   <213> Artificial Sequence
<220>
   <223> Synthetic
<400> 191
   gatattgtga tgactcagtc tccactctcc ctgcccgtca cccctggaga gccggcctcc 60
   atctcctgca ggtctagtca gagcctcctg catagtcatg gatacgacta tttggattgg 120
   tacctgcaga agccagggca gtctccacag ctcctgatct atttgggttc taatcgggcc 180
   tccggggtcc ctgacaggtt cagtggcagt ggatcaggca cagattttac actgaaaatc 240
   agcagagtgg aggctgagga tgttggggtt tattactgca tgcaagctct acaaactccg 300
   ctcactttcg gcggagggac caaggtggag atcaaa                           336
<210> 192
   <211> 112
   <212> PRT
   <213> Artificial Sequence
<220>
   <223> Synthetic
<400> 192
   Asp Ile Val Met Thr Gln Ser Pro Leu Ser Leu Pro Val Thr Pro Gly
    1               5                  10                  15
   Glu Pro Ala Ser Ile Ser Cys Arg Ser Ser Gln Ser Leu Leu His Ser
               20                  25                  30
   His Gly Tyr Asp Tyr Leu Asp Trp Tyr Leu Gln Lys Pro Gly Gln Ser
           35                  40                  45
   Pro Gln Leu Leu Ile Tyr Leu Gly Ser Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ala
                   85                  90                  95
   Leu Gln Thr Pro Leu Thr Phe Gly Gly Gly Thr Lys Val Glu Ile Lys
               100                 105                 110
<210> 193
   <211> 375
   <212> DNA
   <213> Artificial Sequence
<220>
   <223> Synthetic
<400> 193
   cagatcacct tgaaggagtc tggtcctact ctggtgaaac cctcacagac cctcacgctg 60
   acctgcacct tctctgggtt ctcactcagc actagtggag tgggtgtggg ctggatccgt 120
   cagcccccag gaaaggccct ggagtggctt gcactcattt attggaattc tgataagcgc 180
   tacagcccat ctctgaagag caggctcacc atcaccaagg acacctccaa aaaccaggta 240
   gtccttacaa tgaccaacat ggaccctgtg gacacagcca catattactg tgcacacaga 300
   catgacagct cgtcctacta cttctactac ggtatggacg tctggggcca agggatcacg 360
   gtcaccgtct cctca                                                  375
<210> 194
   <211> 125
   <212> PRT
   <213> Artificial Sequence
<220>
   <223> Synthetic
<400> 194
   Gln Ile Thr Leu Lys Glu Ser Gly Pro Thr Leu Val Lys Pro Ser Gln
    1               5                  10                  15
   Thr Leu Thr Leu Thr Cys Thr Phe Ser Gly Phe Ser Leu Ser Thr Ser
               20                  25                  30
   Gly Val Gly Val Gly Trp Ile Arg Gln Pro Pro Gly Lys Ala Leu Glu
           35                  40                  45
   Trp Leu Ala Leu Ile Tyr Trp Asn Ser Asp Lys Arg Tyr Ser Pro Ser
       50                  55                  60
   Leu Lys Ser Arg Leu Thr Ile Thr Lys Asp Thr Ser Lys Asn Gln Val
   65                  70                  75                  80
   Val Leu Thr Met Thr Asn Met Asp Pro Val Asp Thr Ala Thr Tyr Tyr
                   85                  90                  95
   Cys Ala His Arg His Asp Ser Ser Ser Tyr Tyr Phe Tyr Tyr Gly Met
               100                 105                 110
   Asp Val Trp Gly Gln Gly Ile Thr Val Thr Val Ser Ser
           115                 120                 125
<210> 195
   <211> 30
   <212> DNA
   <213> Artificial Sequence
<220>
   <223> Synthetic
<400> 195
   gggttctcac tcagcactag tggagtgggt 30
<210> 196
   <211> 10
   <212> PRT
   <213> Artificial Sequence
<220>
   <223> Synthetic
<400> 196
   Gly Phe Ser Leu Ser Thr Ser Gly Val Gly
   1               5                   10
<210> 197
   <211> 21
   <212> DNA
   <213> Artificial Sequence
<220>
   <223> Synthetic
<400> 197
   atttattgga attctgataa g 21
<210> 198
   <211> 7
   <212> PRT
   <213> Artificial Sequence
<220>
   <223> Synthetic
<400> 198
   Ile Tyr Trp Asn Ser Asp Lys
   1               5
<210> 199
   <211> 51
   <212> DNA
   <213> Artificial Sequence
<220>
   <223> Synthetic
<400> 199
   gcacacagac atgacagctc gtcctactac ttctactacg gtatggacgt c 51
<210> 200
   <211> 17
   <212> PRT
   <213> Artificial Sequence
<220>
   <223> Synthetic
<400> 200
   Ala His Arg His Asp Ser Ser Ser Tyr Tyr Phe Tyr Tyr Gly Met Asp
    1               5                  10                  15
   Val
<210> 201
   <211> 339
   <212> DNA
   <213> Artificial Sequence
<220>
   <223> Synthetic
<400> 201
   gacatccaga tgacccagtc tccgctctcc ctgcccgtca cccctggaga gccggcctcc 60
   atctcctgca ggtctagtca gagcctcctc catagtcatg gatacaacta tttggattgg 120
   tacctgcaga agccagggca gtctccacaa ctcctgatct atttgggttc taatcgggcc 180
   tccggggtcc ctgacaggtt cagtggcggt ggatcaggca cagattttac actgaaaatc 240
   agcagagtgg aggctgagga tgttgggatt tattactgca tgcaagctct acagactcct 300
   ctcactttcg gcggagggac caaggtggag atcaaacga                        339
<210> 202
   <211> 113
   <212> PRT
   <213> Artificial Sequence
<220>
   <223> Synthetic
<400> 202
   Asp Ile Gln Met Thr Gln Ser Pro Leu Ser Leu Pro Val Thr Pro Gly
    1               5                  10                  15
   Glu Pro Ala Ser Ile Ser Cys Arg Ser Ser Gln Ser Leu Leu His Ser
               20                  25                  30
   His Gly Tyr Asn Tyr Leu Asp Trp Tyr Leu Gln Lys Pro Gly Gln Ser
           35                  40                  45
   Pro Gln Leu Leu Ile Tyr Leu Gly Ser Asn Arg Ala Ser Gly Val Pro
       50                  55                  60
   Asp Arg Phe Ser Gly Gly Gly Ser Gly Thr Asp Phe Thr Leu Lys Ile
   65                  70                  75                  80
   Ser Arg Val Glu Ala Glu Asp Val Gly Ile Tyr Tyr Cys Met Gln Ala
                   85                  90                  95
   Leu Gln Thr Pro Leu Thr Phe Gly Gly Gly Thr Lys Val Glu Ile Lys
               100                 105                 110
   Arg
<210> 203
   <211> 33
   <212> DNA
   <213> Artificial Sequence
<220>
   <223> Synthetic
<400> 203
   cagagcctcc tccatagtca tggatacaac tat 33
<210> 204
   <211> 11
   <212> PRT
   <213> Artificial Sequence
<220>
   <223> Synthetic
<400> 204
   Gln Ser Leu Leu His Ser His Gly Tyr Asn Tyr
   1 5 10
<210> 205
   <211> 9
   <212> DNA
   <213> Artificial Sequence
<220>
   <223> Synthetic
<400> 205
   ttgggttct 9
<210> 206
   <211> 3
   <212> PRT
   <213> Artificial Sequence
<220>
   <223> Synthetic
<400> 206
   Leu Gly Ser
   1
<210> 207
   <211> 27
   <212> DNA
   <213> Artificial Sequence
<220>
   <223> Synthetic
<400> 207
   atgcaagctc tacagactcc tctcact 27
<210> 208
   <211> 9
   <212> PRT
   <213> Artificial Sequence
<220>
   <223> Synthetic
<400> 208
   Met Gln Ala Leu Gln Thr Pro Leu Thr
    1               5
<210> 209
   <211> 375
   <212> DNA
   <213> Artificial Sequence
<220>
   <223> Synthetic
<400> 209
   cagatcacct tgaaggagtc tggtcctact ctggtgaaac cctcacagac cctcacgctg 60
   acctgcacct tctctgggtt ctcactcagc actagtggag tgggtgtggg ctggatccgt 120
   cagcccccag gaaaggccct ggagtggctt gcactcattt attggaattc tgataagcgc 180
   tacagcccat ctctgaagag caggctcacc atcaccaagg acacctccaa aaaccaggta 240
   gtccttacaa tgaccaacat ggaccctgtg gacacagcca catattactg tgcacacaga 300
   catgacagct cgtcctacta cttctactac ggtatggacg tctggggcca agggaccacg 360
   gtcaccgtct cctca                                                  375
<210> 210
   <211> 125
   <212> PRT
   <213> Artificial Sequence
<220>
   <223> Synthetic
<400> 210
   Gln Ile Thr Leu Lys Glu Ser Gly Pro Thr Leu Val Lys Pro Ser Gln
    1               5                  10                  15
   Thr Leu Thr Leu Thr Cys Thr Phe Ser Gly Phe Ser Leu Ser Thr Ser
               20                  25                  30
   Gly Val Gly Val Gly Trp Ile Arg Gln Pro Pro Gly Lys Ala Leu Glu
           35                  40                  45
   Trp Leu Ala Leu Ile Tyr Trp Asn Ser Asp Lys Arg Tyr Ser Pro Ser
       50                  55                  60
   Leu Lys Ser Arg Leu Thr Ile Thr Lys Asp Thr Ser Lys Asn Gln Val
   65                  70                  75                  80
   Val Leu Thr Met Thr Asn Met Asp Pro Val Asp Thr Ala Thr Tyr Tyr
                   85                  90                  95
   Cys Ala His Arg His Asp Ser Ser Ser Tyr Tyr Phe Tyr Tyr Gly Met
               100                 105                 110
   Asp Val Trp Gly Gln Gly Thr Thr Val Thr Val Ser Ser
           115                 120                 125
<210> 211
   <211> 336
   <212> DNA
   <213> Artificial Sequence
<220>
   <223> Synthetic
<400> 211
   gatattgtga tgactcagtc tccgctctcc ctgcccgtca cccctggaga gccggcctcc 60
   atctcctgca ggtctagtca gagcctcctc catagtcatg gatacaacta tttggattgg 120
   tacctgcaga agccagggca gtctccacaa ctcctgatct atttgggttc taatcgggcc 180
   tccggggtcc ctgacaggtt cagtggcggt ggatcaggca cagattttac actgaaaatc 240
   agcagagtgg aggctgagga tgttgggatt tattactgca tgcaagctct acagactcct 300
   ctcactttcg gcggagggac caaggtggag atcaaa                           336
<210> 212
   <211> 112
   <212> PRT
   <213> Artificial Sequence
<220>
   <223> Synthetic
<400> 212
   Asp Ile Val Met Thr Gln Ser Pro Leu Ser Leu Pro Val Thr Pro Gly
    1               5                  10                  15
   Glu Pro Ala Ser Ile Ser Cys Arg Ser Ser Gln Ser Leu Leu His Ser
               20                  25                  30
   His Gly Tyr Asn Tyr Leu Asp Trp Tyr Leu Gln Lys Pro Gly Gln Ser
           35                  40                  45
   Pro Gln Leu Leu Ile Tyr Leu Gly Ser Asn Arg Ala Ser Gly Val Pro
       50                  55                  60
   Asp Arg Phe Ser Gly Gly Gly Ser Gly Thr Asp Phe Thr Leu Lys Ile
   65                  70                  75                  80
   Ser Arg Val Glu Ala Glu Asp Val Gly Ile Tyr Tyr Cys Met Gln Ala
                   85                  90                  95
   Leu Gln Thr Pro Leu Thr Phe Gly Gly Gly Thr Lys Val Glu Ile Lys
               100                 105                 110
<210> 213
   <211> 375
   <212> DNA
   <213> Artificial Sequence
<220>
   <223> Synthetic
<400> 213
   cagatcacct tgaaggagtc tggtcctacg ctggtgaaac ccacacagac cctcacgctg 60
   acctgcacct tctctgggtt ctcactcagc actagtggag tgggtgtggg ctggatccgt 120
   cagcccccag gaaaggccct ggagtggctt gcactcattt attggaattc tgataagcgc 180
   tacagcccat ctctgaagag caggctcacc atcaccaagg acacctccaa aaaccaggtg 240
   gtccttacaa tgaccaacat ggaccctgtg gacacagcca catattactg tgcacacaga 300
   catgacagct cgtcctacta cttctactac ggtatggacg tctggggcca agggaccacg 360
   gtcaccgtct cctca                                                  375
<210> 214
   <211> 125
   <212> PRT
   <213> Artificial Sequence
<220>
   <223> Synthetic
<400> 214
   Gln Ile Thr Leu Lys Glu Ser Gly Pro Thr Leu Val Lys Pro Thr Gln
    1               5                  10                  15
   Thr Leu Thr Leu Thr Cys Thr Phe Ser Gly Phe Ser Leu Ser Thr Ser
               20                  25                  30
   Gly Val Gly Val Gly Trp Ile Arg Gln Pro Pro Gly Lys Ala Leu Glu
           35                  40                  45
   Trp Leu Ala Leu Ile Tyr Trp Asn Ser Asp Lys Arg Tyr Ser Pro Ser
       50                  55                  60
   Leu Lys Ser Arg Leu Thr Ile Thr Lys Asp Thr Ser Lys Asn Gln Val
   65                  70                  75                  80
   Val Leu Thr Met Thr Asn Met Asp Pro Val Asp Thr Ala Thr Tyr Tyr
                   85                  90                  95
   Cys Ala His Arg His Asp Ser Ser Ser Tyr Tyr Phe Tyr Tyr Gly Met
               100                 105                 110
   Asp Val Trp Gly Gln Gly Thr Thr Val Thr Val Ser Ser
           115                 120                 125
<210> 215
   <211> 336
   <212> DNA
   <213> Artificial Sequence
<220>
   <223> Synthetic
<400> 215
   gatattgtga tgactcagtc tccactctcc ctgcccgtca cccctggaga gccggcctcc 60
   atctcctgca ggtctagtca gagcctcctc catagtcatg gatacaacta tttggattgg 120
   tacctgcaga agccagggca gtctccacag ctcctgatct atttgggttc taatcgggcc 180
   tccggggtcc ctgacaggtt cagtggcagt ggatcaggca cagattttac actgaaaatc 240
   agcagagtgg aggctgagga tgttggggtt tattactgca tgcaagctct acagactcct 300
   ctcactttcg gcggagggac caaggtggag atcaaa                           336
<210> 216
   <211> 112
   <212> PRT
   <213> Artificial Sequence
<220>
   <223> Synthetic
<400> 216
   Asp Ile Val Met Thr Gln Ser Pro Leu Ser Leu Pro Val Thr Pro Gly
    1               5                  10                  15
   Glu Pro Ala Ser Ile Ser Cys Arg Ser Ser Gln Ser Leu Leu His Ser
               20                  25                  30
   His Gly Tyr Asn Tyr Leu Asp Trp Tyr Leu Gln Lys Pro Gly Gln Ser
           35                  40                  45
   Pro Gln Leu Leu Ile Tyr Leu Gly Ser Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ala
                   85                  90                  95
   Leu Gln Thr Pro Leu Thr Phe Gly Gly Gly Thr Lys Val Glu Ile Lys
               100                 105                 110
<210> 217
   <211> 381
   <212> DNA
   <213> Artificial Sequence
<220>
   <223> Synthetic
<400> 217
   gagatgcaac tggtggagtc tgggggaggc ttggtccagc ctggggggtc cctgagactc 60
   tcctgtgcag cctctggatt cacctttagt agtcactgga tgaagtgggt ccgccaggct 120
   ccagggaagg ggctggagtg ggtggccaac ataaaccaag atggaagtga gaaatactat 180
   gtggactctg tgaagggccg attcaccatc tccagagaca acgccaagaa ctcactgttt 240
   ctgcaaatga acagcctgag agccgaggac acggctgtgt attactgtgc gagagatatt 300
   gtactaatgg tctatgatat ggactactac tactacggta tggacgtctg gggccaaggg 360
   accacggtca ccgtctcctc a                                          381
<210> 218
   <211> 127
   <212> PRT
   <213> Artificial Sequence
<220>
   <223> Synthetic
<400> 218
   Glu Met Gln Leu Val Glu Ser Gly Gly Gly Leu Val Gln Pro Gly Gly
    1               5                  10                  15
   Ser Leu Arg Leu Ser Cys Ala Ala Ser Gly Phe Thr Phe Ser Ser His
               20                  25                  30
   Trp Met Lys Trp Val Arg Gln Ala Pro Gly Lys Gly Leu Glu Trp Val
           35                  40                  45
   Ala Asn Ile Asn Gln Asp Gly Ser Glu Lys Tyr Tyr Val Asp Ser Val
       50                  55                  60
   Lys Gly Arg Phe Thr Ile Ser Arg Asp Asn Ala Lys Asn Ser Leu Phe
   65                  70                  75                  80
   Leu Gln Met Asn Ser Leu Arg Ala Glu Asp Thr Ala Val Tyr Tyr Cys
                   85                  90                  95
   Ala Arg Asp Ile Val Leu Met Val Tyr Asp Met Asp Tyr Tyr Tyr Tyr
               100                 105                 110
   Gly Met Asp Val Trp Gly Gln Gly Thr Thr Val Thr Val Ser Ser
           115                 120                 125
<210> 219
   <211> 24
   <212> DNA
   <213> Artificial Sequence
<220>
   <223> Synthetic
<400> 219
   ggattcacct ttagtagtca ctgg 24
<210> 220
   <211> 8
   <212> PRT
   <213> Artificial Sequence
<220>
   <223> Synthetic
<400> 220
   Gly Phe Thr Phe Ser Ser His Trp
    1               5
<210> 221
   <211> 24
   <212> DNA
   <213> Artificial Sequence
<220>
   <223> Synthetic
<400> 221
   ataaaccaag atggaagtga gaaa 24
<210> 222
   <211> 8
   <212> PRT
   <213> Artificial Sequence
<220>
   <223> Synthetic
<400> 222
   Ile Asn Gln Asp Gly Ser Glu Lys
    1               5
<210> 223
   <211> 60
   <212> DNA
   <213> Artificial Sequence
<220>
   <223> Synthetic
<400> 223
   gcgagagata ttgtactaat ggtctatgat atggactact actactacgg tatggacgtc 60
<210> 224
   <211> 20
   <212> PRT
   <213> Artificial Sequence
<220>
   <223> Synthetic
<400> 224
   Ala Arg Asp Ile Val Leu Met Val Tyr Asp Met Asp Tyr Tyr Tyr Tyr
    1               5                  10                  15
   Gly Met Asp Val
               20
<210> 225
   <211> 336
   <212> DNA
   <213> Artificial Sequence
<220>
   <223> Synthetic
<400> 225
   gatattgtga tgactcagtc tccactctcc ctgcccgtca cccctggaga gccggcctcc 60
   atctcctgca ggtctagtca gagcctcctg catagtaatg gaaacaacta tttggattgg 120
   tacctgcaga agccagggca gtctccacag ctcctgatct atttgggttc taatcgggcc 180
   tccggggtcc ctgacaggtt cagtggcagt ggatcaggca cagattttac actgaaaatc 240
   agcagagtgg aggctgagga tgttggggtt tattactgca tgcaaactct acaaactccg 300
   ctcactttcg gcggagggac caaggtggag atcaaa                           336
<210> 226
   <211> 112
   <212> PRT
   <213> Artificial Sequence
<220>
   <223> Synthetic
<400> 226
   Asp Ile Val Met Thr Gln Ser Pro Leu Ser Leu Pro Val Thr Pro Gly
    1               5                  10                  15
   Glu Pro Ala Ser Ile Ser Cys Arg Ser Ser Gln Ser Leu Leu His Ser
               20                  25                  30
   Asn Gly Asn Asn Tyr Leu Asp Trp Tyr Leu Gln Lys Pro Gly Gln Ser
           35                  40                  45
   Pro Gln Leu Leu Ile Tyr Leu Gly Ser Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Thr
                   85                  90                  95
   Leu Gln Thr Pro Leu Thr Phe Gly Gly Gly Thr Lys Val Glu Ile Lys
               100                 105                 110
<210> 227
   <211> 33
   <212> DNA
   <213> Artificial Sequence
<220>
   <223> Synthetic
<400> 227
   cagagcctcc tgcatagtaa tggaaacaac tat 33
<210> 228
   <211> 11
   <212> PRT
   <213> Artificial Sequence
<220>
   <223> Synthetic
<400> 228
   Gln Ser Leu Leu His Ser Asn Gly Asn Asn Tyr
    1               5                  10
<210> 229
   <211> 9
   <212> DNA
   <213> Artificial Sequence
<220>
   <223> Synthetic
<400> 229
   ttgggttct 9
<210> 230
   <211> 3
   <212> PRT
   <213> Artificial Sequence
<220>
   <223> Synthetic
<400> 230
   Leu Gly Ser
   1
<210> 231
   <211> 27
   <212> DNA
   <213> Artificial Sequence
<220>
   <223> Synthetic
<400> 231
   atgcaaactc tacaaactcc gctcact 27
<210> 232
   <211> 9
   <212> PRT
   <213> Artificial Sequence
<220>
   <223> Synthetic
<400> 232
   Met Gln Thr Leu Gln Thr Pro Leu Thr
   1 5
<210> 233
   <211> 381
   <212> DNA
   <213> Artificial Sequence
<220>
   <223> Synthetic
<400> 233
   gaggtgcagc tggtggagtc tgggggaggc ttggtccagc ctggggggtc cctgagactc 60
   tcctgtgcag cctctggatt cacctttagt agtcactgga tgaagtgggt ccgccaggct 120
   ccagggaagg ggctggagtg ggtggccaac ataaaccaag atggaagtga gaaatactat 180
   gtggactctg tgaagggccg attcaccatc tccagagaca acgccaagaa ctcactgttt 240
   ctgcaaatga acagcctgag agccgaggac acggctgtgt attactgtgc gagagatatt 300
   gtactaatgg tctatgatat ggactactac tactacggta tggacgtctg ggccaagggg 360
   accacggtca ccgtctcctc a 381
<210> 234
   <211> 127
   <212> PRT
   <213> Artificial Sequence
<220>
   <223> Synthetic
<400> 234
   Glu Val Gln Leu Val Glu Ser Gly Gly Gly Leu Val Gln Pro Gly Gly
    1               5                  10                  15
   Ser Leu Arg Leu Ser Cys Ala Ala Ser Gly Phe Thr Phe Ser Ser His
               20                  25                  30
   Trp Met Lys Trp Val Arg Gln Ala Pro Gly Lys Gly Leu Glu Trp Val
           35                  40                  45
   Ala Asn Ile Asn Gln Asp Gly Ser Glu Lys Tyr Tyr Val Asp Ser Val
       50                  55                  60
   Lys Gly Arg Phe Thr Ile Ser Arg Asp Asn Ala Lys Asn Ser Leu Phe
   65                  70                  75                  80
   Leu Gln Met Asn Ser Leu Arg Ala Glu Asp Thr Ala Val Tyr Tyr Cys
                   85                  90                  95
   Ala Arg Asp Ile Val Leu Met Val Tyr Asp Met Asp Tyr Tyr Tyr Tyr
               100                 105                 110
   Gly Met Asp Val Trp Gly Gln Gly Thr Thr Val Thr Val Ser Ser
           115                 120                 125
<210> 235
   <211> 336
   <212> DNA
   <213> Artificial Sequence
<220>
   <223> Synthetic
<400> 235
   gatattgtga tgactcagtc tccactctcc ctgcccgtca cccctggaga gccggcctcc 60
   atctcctgca ggtctagtca gagcctcctg catagtaatg gaaacaacta tttggattgg 120
   tacctgcaga agccagggca gtctccacag ctcctgatct atttgggttc taatcgggcc 180
   tccggggtcc ctgacaggtt cagtggcagt ggatcaggca cagattttac actgaaaatc 240
   agcagagtgg aggctgagga tgttggggtt tattactgca tgcaaactct acaaactccg 300
   ctcactttcg gcggagggac caaggtggag atcaaa                           336
<210> 236
   <211> 112
   <212> PRT
   <213> Artificial Sequence
<220>
   <223> Synthetic
<400> 236
   Asp Ile Val Met Thr Gln Ser Pro Leu Ser Leu Pro Val Thr Pro Gly
    1               5                  10                  15
   Glu Pro Ala Ser Ile Ser Cys Arg Ser Ser Gln Ser Leu Leu His Ser
               20                  25                  30
   Asn Gly Asn Asn Tyr Leu Asp Trp Tyr Leu Gln Lys Pro Gly Gln Ser
           35                  40                  45
   Pro Gln Leu Leu Ile Tyr Leu Gly Ser Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Thr
                   85                  90                  95
   Leu Gln Thr Pro Leu Thr Phe Gly Gly Gly Thr Lys Val Glu Ile Lys
               100                 105                 110
<210> 237
   <211> 381
   <212> DNA
   <213> Artificial Sequence
<220>
   <223> Synthetic
<400> 237
   gaggtgcagc tggtggagtc tgggggaggc ttggtccagc ctggggggtc cctgagactc 60
   tcctgtgcag cctctggatt cacctttagt agtcactgga tgagctgggt ccgccaggct 120
   ccagggaagg ggctggagtg ggtggccaac ataaaccaag atggaagtga gaaatactat 180
   gtggactctg tgaagggccg attcaccatc tccagagaca acgccaagaa ctcactgtat 240
   ctgcaaatga acagcctgag agccgaggac acggctgtgt attactgtgc gagagatatt 300
   gtactaatgg tctatgatat ggactactac tactacggta tggacgtctg ggggcaaggg 360
   accacggtca ccgtctcctc a                                            381
<210> 238
   <211> 127
   <212> PRT
   <213> Artificial Sequence
<220>
   <223> Synthetic
<400> 238
   Glu Val Gln Leu Val Glu Ser Gly Gly Gly Leu Val Gln Pro Gly Gly
    1               5                  10                  15
   Ser Leu Arg Leu Ser Cys Ala Ala Ser Gly Phe Thr Phe Ser Ser His
               20                  25                  30
   Trp Met Ser Trp Val Arg Gln Ala Pro Gly Lys Gly Leu Glu Trp Val
           35                  40                  45
   Ala Asn Ile Asn Gln Asp Gly Ser Glu Lys Tyr Tyr Val Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Arg Asp Ile Val Leu Met Val Tyr Asp Met Asp Tyr Tyr Tyr Tyr
               100                 105                 110
   Gly Met Asp Val Trp Gly Gln Gly Thr Thr Val Thr Val Ser Ser
           115                 120                 125
<210> 239
   <211> 336
   <212> DNA
   <213> Artificial Sequence
<220>
   <223> Synthetic
<400> 239
   gatattgtga tgactcagtc tccactctcc ctgcccgtca cccctggaga gccggcctcc 60
   atctcctgca ggtctagtca gagcctcctg catagtaatg gaaacaacta tttggattgg 120
   tacctgcaga agccagggca gtctccacag ctcctgatct atttgggttc taatcgggcc 180
   tccggggtcc ctgacaggtt cagtggcagt ggatcaggca cagattttac actgaaaatc 240
   agcagagtgg aggctgagga tgttggggtt tattactgca tgcaaactct acaaactccg 300
   ctcactttcg gcggagggac caaggtggag atcaaa                           336
<210> 240
   <211> 112
   <212> PRT
   <213> Artificial Sequence
<220>
   <223> Synthetic
<400> 240
   Asp Ile Val Met Thr Gln Ser Pro Leu Ser Leu Pro Val Thr Pro Gly
    1               5                  10                  15
   Glu Pro Ala Ser Ile Ser Cys Arg Ser Ser Gln Ser Leu Leu His Ser
               20                  25                  30
   Asn Gly Asn Asn Tyr Leu Asp Trp Tyr Leu Gln Lys Pro Gly Gln Ser
           35                  40                  45
   Pro Gln Leu Leu Ile Tyr Leu Gly Ser Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Thr
                   85                  90                  95
   Leu Gln Thr Pro Leu Thr Phe Gly Gly Gly Thr Lys Val Glu Ile Lys
               100                 105                 110
<210> 241
   <211> 381
   <212> DNA
   <213> Artificial Sequence
<220>
   <223> Synthetic
<400> 241
   caggtgcagc tggtggagtc tgggggaggc gtggtccagc ctgggaggtc cctgagactc 60
   tcctgtgcag tctctggatt caccttcagt agctatggca tgcactgggt ccgccaggct 120
   ccaggcaagg ggctggagtg ggtggcagct atatcatatg atggaagtaa taaatactat 180
   gtagactccg tgaagggccg attcaccatc tccagagaca attccaagaa aacgctgtat 240
   ctgcaaatga acagcctgag agctgaggac acggctgtgt ataattgtgc gaaaaatatt 300
   gtactagtga tgtatgatat agactatcac tactatggga tggacgtctg gggccaaggg 360
   accacggtca ccgtctcctc a                                           381
<210> 242
   <211> 127
   <212> PRT
   <213> Artificial Sequence
<220>
   <223> Synthetic
<400> 242
   Gln Val Gln Leu Val Glu Ser Gly Gly Gly Val Val Gln Pro Gly Arg
    1               5                  10                  15
   Ser Leu Arg Leu Ser Cys Ala Val Ser Gly Phe Thr Phe Ser Ser Tyr
               20                  25                  30
   Gly Met His Trp Val Arg Gln Ala Pro Gly Lys Gly Leu Glu Trp Val
           35                  40                  45
   Ala Ala Ile Ser Tyr Asp Gly Ser Asn Lys Tyr Tyr Val Asp Ser Val
       50                  55                  60
   Lys Gly Arg Phe Thr Ile Ser Arg Asp Asn Ser Lys Lys Thr Leu Tyr
   65                  70                  75                  80
   Leu Gln Met Asn Ser Leu Arg Ala Glu Asp Thr Ala Val Tyr Asn Cys
                   85                  90                  95
   Ala Lys Asn Ile Val Leu Val Met Tyr Asp Ile Asp Tyr His Tyr Tyr
               100                 105                 110
   Gly Met Asp Val Trp Gly Gln Gly Thr Thr Val Thr Val Ser Ser
           115                 120                 125
<210> 243
   <211> 24
   <212> DNA
   <213> Artificial Sequence
<220>
   <223> Synthetic
<400> 243
   ggattcacct tcagtagcta tggc 24
<210> 244
   <211> 8
   <212> PRT
   <213> Artificial Sequence
<220>
   <223> Synthetic
<400> 244
   Gly Phe Thr Phe Ser Ser Tyr Gly
    1               5
<210> 245
   <211> 24
   <212> DNA
   <213> Artificial Sequence
<220>
   <223> Synthetic
<400> 245
   atatcatatg atggaagtaa taaa 24
<210> 246
   <211> 8
   <212> PRT
   <213> Artificial Sequence
<220>
   <223> Synthetic
<400> 246
   Ile Ser Tyr Asp Gly Ser Asn Lys
    1               5
<210> 247
   <211> 60
   <212> DNA
   <213> Artificial Sequence
<220>
   <223> Synthetic
<400> 247
   gcgaaaaata ttgtactagt gatgtatgat atagactatc actactatgg gatggacgtc 60
<210> 248
   <211> 20
   <212> PRT
   <213> Artificial Sequence
<220>
   <223> Synthetic
<400> 248
   Ala Lys Asn Ile Val Leu Val Met Tyr Asp Ile Asp Tyr His Tyr Tyr
    1               5                  10                  15
   Gly Met Asp Val
               20
<210> 249
   <211> 336
   <212> DNA
   <213> Artificial Sequence
<220>
   <223> Synthetic
<400> 249
   gatattgtga tgactcagtc tccactctcc ctgcccgtca cccctggaga gccggcctcc 60
   atctcctgca ggtctagtca gagcctcctg catagtaatg gatacaacta tttggattgg 120
   tacctgcaga agccagggca gtctccacaa ctcctgatct atttgggttt taatcgggcc 180
   tccggggtcc ctgacaggtt cagtggcagt ggatcaggca cagattttac actgaaaatc 240
   agcagagtgg aggctgagga tgttggggtt tattactgca tgcaagctct acaaactcct 300
   ctcactttcg gcggagggac caaggtggag atcaga                          336
<210> 250
   <211> 112
   <212> PRT
   <213> Artificial Sequence
<220>
   <223> Synthetic
<400> 250
   Asp Ile Val Met Thr Gln Ser Pro Leu Ser Leu Pro Val Thr Pro Gly
    1               5                  10                  15
   Glu Pro Ala Ser Ile Ser Cys Arg Ser Ser Gln Ser Leu Leu His Ser
               20                  25                  30
   Asn Gly Tyr Asn Tyr Leu Asp Trp Tyr Leu Gln Lys Pro Gly Gln Ser
           35                  40                  45
   Pro Gln Leu Leu Ile Tyr Leu Gly Phe Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ala
                   85                  90                  95
   Leu Gln Thr Pro Leu Thr Phe Gly Gly Gly Thr Lys Val Glu Ile Arg
               100                 105                 110
<210> 251
   <211> 33
   <212> DNA
   <213> Artificial Sequence
<220>
   <223> Synthetic
<400> 251
   cagagcctcc tgcatagtaa tggatacaac tat 33
<210> 252
   <211> 11
   <212> PRT
   <213> Artificial Sequence
<220>
   <223> Synthetic
<400> 252
   Gln Ser Leu Leu His Ser Asn Gly Tyr Asn Tyr
    1               5                  10
<210> 253
   <211> 9
   <212> DNA
   <213> Artificial Sequence
<220>
   <223> Synthetic
<400> 253
   ttgggtttt 9
<210> 254
   <211> 3
   <212> PRT
   <213> Artificial Sequence
<220>
   <223> Synthetic
<400> 254
   Leu Gly Phe
   1
<210> 255
   <211> 27
   <212> DNA
   <213> Artificial Sequence
<220>
   <223> Synthetic
<400> 255
   atgcaagctc tacaaactcc tctcact 27
<210> 256
   <211> 9
   <212> PRT
   <213> Artificial Sequence
<220>
   <223> Synthetic
<400> 256
   Met Gln Ala Leu Gln Thr Pro Leu Thr
   1 5
<210> 257
   <211> 381
   <212> DNA
   <213> Artificial Sequence
<220>
   <223> Synthetic
<400> 257
   caggtgcagc tggtggagtc tgggggaggc gtggtccagc ctgggaggtc cctgagactc 60
   tcctgtgcag tctctggatt caccttcagt agctatggca tgcactgggt ccgccaggct 120
   ccaggcaagg ggctggagtg ggtggcagct atatcatatg atggaagtaa taaatactat 180
   gtagactccg tgaagggccg attcaccatc tccagagaca attccaagaa aacgctgtat 240
   ctgcaaatga acagcctgag agctgaggac acggctgtgt ataattgtgc gaaaaatatt 300
   gtactagtga tgtatgatat agactatcac tactatggga tggacgtctg gggccaaggg 360
   accacggtca ccgtctcctc a                                           381
<210> 258
   <211> 127
   <212> PRT
   <213> Artificial Sequence
<220>
   <223> Synthetic
<400> 258
   Gln Val Gln Leu Val Glu Ser Gly Gly Gly Val Val Gln Pro Gly Arg
    1               5                  10                  15
   Ser Leu Arg Leu Ser Cys Ala Val Ser Gly Phe Thr Phe Ser Ser Tyr
               20                  25                  30
   Gly Met His Trp Val Arg Gln Ala Pro Gly Lys Gly Leu Glu Trp Val
           35                  40                  45
   Ala Ala Ile Ser Tyr Asp Gly Ser Asn Lys Tyr Tyr Val Asp Ser Val
       50                  55                  60
   Lys Gly Arg Phe Thr Ile Ser Arg Asp Asn Ser Lys Lys Thr Leu Tyr
   65                  70                  75                  80
   Leu Gln Met Asn Ser Leu Arg Ala Glu Asp Thr Ala Val Tyr Asn Cys
                   85                  90                  95
   Ala Lys Asn Ile Val Leu Val Met Tyr Asp Ile Asp Tyr His Tyr Tyr
               100                 105                 110
   Gly Met Asp Val Trp Gly Gln Gly Thr Thr Val Thr Val Ser Ser
           115                 120                 125
<210> 259
   <211> 336
   <212> DNA
   <213> Artificial Sequence
<220>
   <223> Synthetic
<400> 259
   gatattgtga tgactcagtc tccactctcc ctgcccgtca cccctggaga gccggcctcc 60
   atctcctgca ggtctagtca gagcctcctg catagtaatg gatacaacta tttggattgg 120
   tacctgcaga agccagggca gtctccacaa ctcctgatct atttgggttt taatcgggcc 180
   tccggggtcc ctgacaggtt cagtggcagt ggatcaggca cagattttac actgaaaatc 240
   agcagagtgg aggctgagga tgttggggtt tattactgca tgcaagctct acaaactcct 300
   ctcactttcg gcggagggac caaggtggag atcaaa                          336
<210> 260
   <211> 112
   <212> PRT
   <213> Artificial Sequence
<220>
   <223> Synthetic
<400> 260
   Asp Ile Val Met Thr Gln Ser Pro Leu Ser Leu Pro Val Thr Pro Gly
    1               5                  10                  15
   Glu Pro Ala Ser Ile Ser Cys Arg Ser Ser Gln Ser Leu Leu His Ser
               20                  25                  30
   Asn Gly Tyr Asn Tyr Leu Asp Trp Tyr Leu Gln Lys Pro Gly Gln Ser
           35                  40                  45
   Pro Gln Leu Leu Ile Tyr Leu Gly Phe Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ala
                   85                  90                  95
   Leu Gln Thr Pro Leu Thr Phe Gly Gly Gly Thr Lys Val Glu Ile Lys
               100                 105                 110
<210> 261
   <211> 381
   <212> DNA
   <213> Artificial Sequence
<220>
   <223> Synthetic
<400> 261
   caggtgcagc tggtggagtc tgggggaggc gtggtccagc ctgggaggtc cctgagactc 60
   tcctgtgcag cctctggatt caccttcagt agctatggca tgcactgggt ccgccaggct 120
   ccaggcaagg ggctggagtg ggtggcagtt atatcatatg atggaagtaa taaatactat 180
   gcagactccg tgaagggccg attcaccatc tccagagaca attccaagaa cacgctgtat 240
   ctgcaaatga acagcctgag agctgaggac acggctgtgt attactgtgc gaaaaatatt 300
   gtactagtga tgtatgatat agactatcac tactatggga tggacgtctg ggggcaaggg 360
   accacggtca ccgtctcctc a                                          381
<210> 262
   <211> 127
   <212> PRT
   <213> Artificial Sequence
<220>
   <223> Synthetic
<400> 262
   Gln Val Gln Leu Val Glu Ser Gly Gly Gly Val Val Gln Pro Gly Arg
    1               5                   10                  15
   Ser Leu Arg Leu Ser Cys Ala Ala Ser Gly Phe Thr Phe Ser Ser Tyr
               20                  25                  30
   Gly Met His Trp Val Arg Gln Ala Pro Gly Lys Gly Leu Glu Trp Val
           35                  40                  45
   Ala Val Ile Ser Tyr Asp Gly Ser Asn Lys Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Asn Ile Val Leu Val Met Tyr Asp Ile Asp Tyr His Tyr Tyr
               100                 105                 110
   Gly Met Asp Val Trp Gly Gln Gly Thr Thr Val Thr Val Ser Ser
           115                 120                 125
<210> 263
   <211> 336
   <212> DNA
   <213> Artificial Sequence
<220>
   <223> Synthetic
<400> 263
   gatattgtga tgactcagtc tccactctcc ctgcccgtca cccctggaga gccggcctcc 60
   atctcctgca ggtctagtca gagcctcctg catagtaatg gatacaacta tttggattgg 120
   tacctgcaga agccagggca gtctccacag ctcctgatct atttgggttt taatcgggcc 180
   tccggggtcc ctgacaggtt cagtggcagt ggatcaggca cagattttac actgaaaatc 240
   agcagagtgg aggctgagga tgttggggtt tattactgca tgcaagctct acaaactcct 300
   ctcactttcg gcggagggac caaggtggag atcaaa                           336
<210> 264
   <211> 112
   <212> PRT
   <213> Artificial Sequence
<220>
   <223> Synthetic
<400> 264
   Asp Ile Val Met Thr Gln Ser Pro Leu Ser Leu Pro Val Thr Pro Gly
    1               5                  10                  15
   Glu Pro Ala Ser Ile Ser Cys Arg Ser Ser Gln Ser Leu Leu His Ser
               20                  25                  30
   Asn Gly Tyr Asn Tyr Leu Asp Trp Tyr Leu Gln Lys Pro Gly Gln Ser
           35                  40                  45
   Pro Gln Leu Leu Ile Tyr Leu Gly Phe Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ala
                   85                  90                  95
   Leu Gln Thr Pro Leu Thr Phe Gly Gly Gly Thr Lys Val Glu Ile Lys
               100                 105                 110
<210> 265
   <211> 381
   <212> DNA
   <213> Artificial Sequence
<220>
   <223> Synthetic
<400> 265
   caggtgcagc tggtggagtc tgggggaggc gtggtccagc ctgggaggtc cctgagactc 60
   tcctgtgcag tctctggatt caccttcagt agctatggca tgcactgggt ccgccaggct 120
   ccaggcaagg ggctggagtg ggtggcagct atatcatatg atggaagtaa taaatactat 180
   gtagactccg tgaagggccg attcaccatc tccagagaca attccaagaa aacgctgtat 240
   ctgcaaatga acagcctgag agctgaggac acggctgtgt ataattgtgc gaaaaatatt 300
   gtactagtga tgtatgatat agactatcac tactatggga tggacgtctg gggccaaggg 360
   accacggtca ccgtctcctc a                                           381
<210> 266
   <211> 127
   <212> PRT
   <213> Artificial Sequence
<220>
   <223> Synthetic
<400> 266
   Gln Val Gln Leu Val Glu Ser Gly Gly Gly Val Val Gln Pro Gly Arg
    1               5                  10                  15
   Ser Leu Arg Leu Ser Cys Ala Val Ser Gly Phe Thr Phe Ser Ser Tyr
               20                  25                  30
   Gly Met His Trp Val Arg Gln Ala Pro Gly Lys Gly Leu Glu Trp Val
           35                  40                  45
   Ala Ala Ile Ser Tyr Asp Gly Ser Asn Lys Tyr Tyr Val Asp Ser Val
       50                  55                  60
   Lys Gly Arg Phe Thr Ile Ser Arg Asp Asn Ser Lys Lys Thr Leu Tyr
   65                  70                  75                  80
   Leu Gln Met Asn Ser Leu Arg Ala Glu Asp Thr Ala Val Tyr Asn Cys
                   85                  90                  95
   Ala Lys Asn Ile Val Leu Val Met Tyr Asp Ile Asp Tyr His Tyr Tyr
               100                 105                 110
   Gly Met Asp Val Trp Gly Gln Gly Thr Thr Val Thr Val Ser Ser
           115                 120                 125
<210> 267
   <211> 24
   <212> DNA
   <213> Artificial Sequence
<220>
   <223> Synthetic
<400> 267
   ggattcacct tcagtagcta tggc 24
<210> 268
   <211> 8
   <212> PRT
   <213> Artificial Sequence
<220>
   <223> Synthetic
<400> 268
   Gly Phe Thr Phe Ser Ser Tyr Gly
    1               5
<210> 269
   <211> 24
   <212> DNA
   <213> Artificial Sequence
<220>
   <223> Synthetic
<400> 269
   atatcatatg atggaagtaa taaa 24
<210> 270
   <211> 8
   <212> PRT
   <213> Artificial Sequence
<220>
   <223> Synthetic
<400> 270
   Ile Ser Tyr Asp Gly Ser Asn Lys
    1               5
<210> 271
   <211> 60
   <212> DNA
   <213> Artificial Sequence
<220>
   <223> Synthetic
<400> 271
   gcgaaaaata ttgtactagt gatgtatgat atagactatc actactatgg gatggacgtc 60
<210> 272
   <211> 20
   <212> PRT
   <213> Artificial Sequence
<220>
   <223> Synthetic
<400> 272
   Ala Lys Asn Ile Val Leu Val Met Tyr Asp Ile Asp Tyr His Tyr Tyr
    1               5                  10                  15
   Gly Met Asp Val
               20
<210> 273
   <211> 336
   <212> DNA
   <213> Artificial Sequence
<220>
   <223> Synthetic
<400> 273
   gatattgtga tgactcagtc tccactctcc ctgcccgtca cccctggaga gccggcctcc 60
   atctcctgca ggtctagtca gagcctcctg catagtaatg gatacaacta tttggattgg 120
   tacctgcaga agccagggca gtctccacaa ctcctgatct atttgggttt taatcgggcc 180
   tccggggtcc ctgacaggtt cagtggcagt ggatcaggca cagattttac actgaaaatc 240
   agcagagtgg aggctgagga tgttggggtt tattactgca tgcaagctct acaaactcct 300
   ctcactttcg gcggagggac caaggtggag atcaga                           336
<210> 274
   <211> 112
   <212> PRT
   <213> Artificial Sequence
<220>
   <223> Synthetic
<400> 274
   Asp Ile Val Met Thr Gln Ser Pro Leu Ser Leu Pro Val Thr Pro Gly
    1               5                  10                  15
   Glu Pro Ala Ser Ile Ser Cys Arg Ser Ser Gln Ser Leu Leu His Ser
               20                  25                  30
   Asn Gly Tyr Asn Tyr Leu Asp Trp Tyr Leu Gln Lys Pro Gly Gln Ser
           35                  40                  45
   Pro Gln Leu Leu Ile Tyr Leu Gly Phe Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ala
                   85                  90                  95
   Leu Gln Thr Pro Leu Thr Phe Gly Gly Gly Thr Lys Val Glu Ile Arg
               100                 105                 110
<210> 275
   <211> 33
   <212> DNA
   <213> Artificial Sequence
<220>
   <223> Synthetic
<400> 275
   cagagcctcc tgcatagtaa tggatacaac tat 33
<210> 276
   <211> 11
   <212> PRT
   <213> Artificial Sequence
<220>
   <223> Synthetic
<400> 276
   Gln Ser Leu Leu His Ser Asn Gly Tyr Asn Tyr
    1               5                  10
<210> 277
   <211> 9
   <212> DNA
   <213> Artificial Sequence
<220>
   <223> Synthetic
<400> 277
   ttgggtttt 9
<210> 278
   <211> 3
   <212> PRT
   <213> Artificial Sequence
<220>
   <223> Synthetic
<400> 278
   Leu Gly Phe
    1
<210> 279
   <211> 27
   <212> DNA
   <213> Artificial Sequence
<220>
   <223> Synthetic
<400> 279
   atgcaagctc tacaaactcc tctcact 27
<210> 280
   <211> 9
   <212> PRT
   <213> Artificial Sequence
<220>
   <223> Synthetic
<400> 280
   Met Gln Ala Leu Gln Thr Pro Leu Thr
    1               5
<210> 281
   <211> 381
   <212> DNA
   <213> Artificial Sequence
<220>
   <223> Synthetic
<400> 281
   caggtgcagc tggtggagtc tgggggaggc gtggtccagc ctgggaggtc cctgagactc 60
   tcctgtgcag tctctggatt caccttcagt agctatggca tgcactgggt ccgccaggct 120
   ccaggcaagg ggctggagtg ggtggcagct atatcatatg atggaagtaa taaatactat 180
   gtagactccg tgaagggccg attcaccatc tccagagaca attccaagaa aacgctgtat 240
   ctgcaaatga acagcctgag agctgaggac acggctgtgt ataattgtgc gaaaaatatt 300
   gtactagtga tgtatgatat agactatcac tactatggga tggacgtctg gggccaaggg 360
   accacggtca ccgtctcctc a                                           381
<210> 282
   <211> 127
   <212> PRT
   <213> Artificial Sequence
<220>
   <223> Synthetic
<400> 282
   Gln Val Gln Leu Val Glu Ser Gly Gly Gly Val Val Gln Pro Gly Arg
    1               5                  10                  15
   Ser Leu Arg Leu Ser Cys Ala Val Ser Gly Phe Thr Phe Ser Ser Tyr
               20                  25                  30
   Gly Met His Trp Val Arg Gln Ala Pro Gly Lys Gly Leu Glu Trp Val
           35                  40                  45
   Ala Ala Ile Ser Tyr Asp Gly Ser Asn Lys Tyr Tyr Val Asp Ser Val
       50                  55                  60
   Lys Gly Arg Phe Thr Ile Ser Arg Asp Asn Ser Lys Lys Thr Leu Tyr
   65                  70                  75                      80
   Leu Gln Met Asn Ser Leu Arg Ala Glu Asp Thr Ala Val Tyr Asn Cys
                   85                  90                  95
   Ala Lys Asn Ile Val Leu Val Met Tyr Asp Ile Asp Tyr His Tyr Tyr
               100                 105                 110
   Gly Met Asp Val Trp Gly Gln Gly Thr Thr Val Thr Val Ser Ser
           115                 120                 125
<210> 283
   <211> 336
   <212> DNA
   <213> Artificial Sequence
<220>
   <223> Synthetic
<400> 283
   gatattgtga tgactcagtc tccactctcc ctgcccgtca cccctggaga gccggcctcc 60
   atctcctgca ggtctagtca gagcctcctg catagtaatg gatacaacta tttggattgg 120
   tacctgcaga agccagggca gtctccacaa ctcctgatct atttgggttt taatcgggcc 180
   tccggggtcc ctgacaggtt cagtggcagt ggatcaggca cagattttac actgaaaatc 240
   agcagagtgg aggctgagga tgttggggtt tattactgca tgcaagctct acaaactcct 300
   ctcactttcg gcggagggac caaggtggag atcaaa                           336
<210> 284
   <211> 112
   <212> PRT
   <213> Artificial Sequence
<220>
   <223> Synthetic
<400> 284
   Asp Ile Val Met Thr Gln Ser Pro Leu Ser Leu Pro Val Thr Pro Gly
    1               5                  10                  15
   Glu Pro Ala Ser Ile Ser Cys Arg Ser Ser Gln Ser Leu Leu His Ser
               20                  25                  30
   Asn Gly Tyr Asn Tyr Leu Asp Trp Tyr Leu Gln Lys Pro Gly Gln Ser
           35                  40                  45
   Pro Gln Leu Leu Ile Tyr Leu Gly Phe Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ala
                   85                  90                  95
   Leu Gln Thr Pro Leu Thr Phe Gly Gly Gly Thr Lys Val Glu Ile Lys
               100                 105                 110
<210> 285
   <211> 381
   <212> DNA
   <213> Artificial Sequence
<220>
   <223> Synthetic
<400> 285
   caggtgcagc tggtggagtc tgggggaggc gtggtccagc ctgggaggtc cctgagactc 60
   tcctgtgcag cctctggatt caccttcagt agctatggca tgcactgggt ccgccaggct 120
   ccaggcaagg ggctggagtg ggtggcagtt atatcatatg atggaagtaa taaatactat 180
   gcagactccg tgaagggccg attcaccatc tccagagaca attccaagaa cacgctgtat 240
   ctgcaaatga acagcctgag agctgaggac acggctgtgt attactgtgc gaaaaatatt 300
   gtactagtga tgtatgatat agactatcac tactatggga tggacgtctg ggggcaaggg 360
   accacggtca ccgtctcctc a                                           381
<210> 286
   <211> 127
   <212> PRT
   <213> Artificial Sequence
<220>
   <223> Synthetic
<400> 286
   Gln Val Gln Leu Val Glu Ser Gly Gly Gly Val Val Gln Pro Gly Arg
    1               5                  10                  15
   Ser Leu Arg Leu Ser Cys Ala Ala Ser Gly Phe Thr Phe Ser Ser Tyr
               20                  25                  30
   Gly Met His Trp Val Arg Gln Ala Pro Gly Lys Gly Leu Glu Trp Val
           35                  40                  45
   Ala Val Ile Ser Tyr Asp Gly Ser Asn Lys Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Asn Ile Val Leu Val Met Tyr Asp Ile Asp Tyr His Tyr Tyr
               100                 105                 110
   Gly Met Asp Val Trp Gly Gln Gly Thr Thr Val Thr Val Ser Ser
           115                 120                 125
<210> 287
   <211> 336
   <212> DNA
   <213> Artificial Sequence
<220>
   <223> Synthetic
<400> 287
   gatattgtga tgactcagtc tccactctcc ctgcccgtca cccctggaga gccggcctcc 60
   atctcctgca ggtctagtca gagcctcctg catagtaatg gatacaacta tttggattgg 120
   tacctgcaga agccagggca gtctccacag ctcctgatct atttgggttt taatcgggcc 180
   tccggggtcc ctgacaggtt cagtggcagt ggatcaggca cagattttac actgaaaatc 240
   agcagagtgg aggctgagga tgttggggtt tattactgca tgcaagctct acaaactcct 300
   ctcactttcg gcggagggac caaggtggag atcaaa                           336
<210> 288
   <211> 112
   <212> PRT
   <213> Artificial Sequence
<220>
   <223> Synthetic
<400> 288
   Asp Ile Val Met Thr Gln Ser Pro Leu Ser Leu Pro Val Thr Pro Gly
    1               5                  10                  15
   Glu Pro Ala Ser Ile Ser Cys Arg Ser Ser Gln Ser Leu Leu His Ser
               20                  25                  30
   Asn Gly Tyr Asn Tyr Leu Asp Trp Tyr Leu Gln Lys Pro Gly Gln Ser
           35                  40                  45
   Pro Gln Leu Leu Ile Tyr Leu Gly Phe Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ala
                   85                  90                  95
   Leu Gln Thr Pro Leu Thr Phe Gly Gly Gly Thr Lys Val Glu Ile Lys
               100                 105                 110
<210> 289
   <211> 372
   <212> DNA
   <213> Artificial Sequence
<220>
   <223> Synthetic
<400> 289
   cagatcacct tgaaggagtc tggtcctacg ctggtaaaac ccacacagac cctcacgctg 60
   acctgcacct tctctgggtt ctcactcagc gctagtggag tgggtgtggg ctggttccgt 120
   cagcccccag gaaaggccct ggagtggctt gcactcattt attggaatga tgataagcgt 180
   tacagcccat ctctaaagaa cagcctcacc atcaccaagg acacctccaa aaaccaggtg 240
   gtccttacaa tgaccaacat ggaccctgtg gacacagcca catattactg tgcacacaga 300
   atacatctat ggtcctactt ctactacggt atggacgtct ggggccaagg gaccacggtc 360
   accgtctcct ca                                                     372
<210> 290
   <211> 124
   <212> PRT
   <213> Artificial Sequence
<220>
   <223> Synthetic
<400> 290
   Gln Ile Thr Leu Lys Glu Ser Gly Pro Thr Leu Val Lys Pro Thr Gln
    1               5                  10                  15
   Thr Leu Thr Leu Thr Cys Thr Phe Ser Gly Phe Ser Leu Ser Ala Ser
               20                  25                  30
   Gly Val Gly Val Gly Trp Phe Arg Gln Pro Pro Gly Lys Ala Leu Glu
           35                  40                  45
   Trp Leu Ala Leu Ile Tyr Trp Asn Asp Asp Lys Arg Tyr Ser Pro Ser
       50                  55                  60
   Leu Lys Asn Ser Leu Thr Ile Thr Lys Asp Thr Ser Lys Asn Gln Val
   65                  70                  75                  80
   Val Leu Thr Met Thr Asn Met Asp Pro Val Asp Thr Ala Thr Tyr Tyr
                   85                  90                  95
   Cys Ala His Arg Ile His Leu Trp Ser Tyr Phe Tyr Tyr Gly Met Asp
               100                 105                 110
   Val Trp Gly Gln Gly Thr Thr Val Thr Val Ser Ser
           115                 120
<210> 291
   <211> 30
   <212> DNA
   <213> Artificial Sequence
<220>
   <223> Synthetic
<400> 291
   gggttctcac tcagcgctag tggagtgggt 30
<210> 292
   <211> 10
   <212> PRT
   <213> Artificial Sequence
<220>
   <223> Synthetic
<400> 292
   Gly Phe Ser Leu Ser Ala Ser Gly Val Gly
    1               5                  10
<210> 293
   <211> 21
   <212> DNA
   <213> Artificial Sequence
<220>
   <223> Synthetic
<400> 293
   atttattgga atgatgataa g 21
<210> 294
   <211> 7
   <212> PRT
   <213> Artificial Sequence
<220>
   <223> Synthetic
<400> 294
   Ile Tyr Trp Asn Asp Asp Lys
   1 5
<210> 295
   <211> 48
   <212> DNA
   <213> Artificial Sequence
<220>
   <223> Synthetic
<400> 295
   gcacacagaa tacatctatg gtcctacttc tactacggta tggacgtc 48
<210> 296
   <211> 16
   <212> PRT
   <213> Artificial Sequence
<220>
   <223> Synthetic
<400> 296
   Ala His Arg Ile His Leu Trp Ser Tyr Phe Tyr Tyr Gly Met Asp Val
   1 5 10 15
<210> 297
   <211> 336
   <212> DNA
   <213> Artificial Sequence
<220>
   <223> Synthetic
<400> 297
   gatattgtga tgactcagtc tccactctcc ctgcccgtca cccctggaga gccggcctcc 60
   atctcctgca ggtctagtca gactctcctg catagtaatg gatacaacta tttcgattgg 120
   tacctgcaga agccagggca gtctccacag ctcctgatct atttgggttc taatcgggcc 180
   tccggggtcc ctgacagatt cagtggcagt ggatcaggca cagattttac actgaaaatc 240
   agcagagtgg aggctgagga tgttggaatt tattactgca tgcaagctct acaaactcct 300
   ctcactttcg gcggagggac caaggtggag atcaga 336
<210> 298
   <211> 112
   <212> PRT
   <213> Artificial Sequence
<220>
   <223> Synthetic
<400> 298
   Asp Ile Val Met Thr Gln Ser Pro Leu Ser Leu Pro Val Thr Pro Gly
    1               5                  10                  15
   Glu Pro Ala Ser Ile Ser Cys Arg Ser Ser Gln Thr Leu Leu His Ser
               20                  25                  30
   Asn Gly Tyr Asn Tyr Phe Asp Trp Tyr Leu Gln Lys Pro Gly Gln Ser
           35                  40                  45
   Pro Gln Leu Leu Ile Tyr Leu Gly Ser Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Ile Tyr Tyr Cys Met Gln Ala
                   85                  90                  95
   Leu Gln Thr Pro Leu Thr Phe Gly Gly Gly Thr Lys Val Glu Ile Arg
               100                 105                 110
<210> 299
   <211> 33
   <212> DNA
   <213> Artificial Sequence
<220>
   <223> Synthetic
<400> 299
   cagactctcc tgcatagtaa tggatacaac tat 33
<210> 300
   <211> 11
   <212> PRT
   <213> Artificial Sequence
<220>
   <223> Synthetic
<400> 300
   Gln Thr Leu Leu His Ser Asn Gly Tyr Asn Tyr
    1               5                  10
<210> 301
   <211> 9
   <212> DNA
   <213> Artificial Sequence
<220>
   <223> Synthetic
<400> 301
   ttgggttct 9
<210> 302
   <211> 3
   <212> PRT
   <213> Artificial Sequence
<220>
   <223> Synthetic
<400> 302
   Leu Gly Ser
    1
<210> 303
   <211> 27
   <212> DNA
   <213> Artificial Sequence
<220>
   <223> Synthetic
<400> 303
   atgcaagctc tacaaactcc tctcact 27
<210> 304
   <211> 9
   <212> PRT
   <213> Artificial Sequence
<220>
   <223> Synthetic
<400> 304
   Met Gln Ala Leu Gln Thr Pro Leu Thr
    1               5
<210> 305
   <211> 372
   <212> DNA
   <213> Artificial Sequence
<220>
   <223> Synthetic
<400> 305
   cagatcacct tgaaggagtc tggtcctacg ctggtaaaac ccacacagac cctcacgctg 60
   acctgcacct tctctgggtt ctcactcagc gctagtggag tgggtgtggg ctggttccgt 120
   cagcccccag gaaaggccct ggagtggctt gcactcattt attggaatga tgataagcgt 180
   tacagcccat ctctaaagaa cagcctcacc atcaccaagg acacctccaa aaaccaggtg 240
   gtccttacaa tgaccaacat ggaccctgtg gacacagcca catattactg tgcacacaga 300
   atacatctat ggtcctactt ctactacggt atggacgtct ggggccaagg gaccacggtc 360
   accgtctcct ca                                                     372
<210> 306
   <211> 124
   <212> PRT
   <213> Artificial Sequence
<220>
   <223> Synthetic
<400> 306
   Gln Ile Thr Leu Lys Glu Ser Gly Pro Thr Leu Val Lys Pro Thr Gln
    1               5                  10                  15
   Thr Leu Thr Leu Thr Cys Thr Phe Ser Gly Phe Ser Leu Ser Ala Ser
               20                  25                  30
   Gly Val Gly Val Gly Trp Phe Arg Gln Pro Pro Gly Lys Ala Leu Glu
           35                  40                  45
   Trp Leu Ala Leu Ile Tyr Trp Asn Asp Asp Lys Arg Tyr Ser Pro Ser
       50                  55                  60
   Leu Lys Asn Ser Leu Thr Ile Thr Lys Asp Thr Ser Lys Asn Gln Val
   65                  70                  75                  80
   Val Leu Thr Met Thr Asn Met Asp Pro Val Asp Thr Ala Thr Tyr Tyr
                   85                  90                  95
   Cys Ala His Arg Ile His Leu Trp Ser Tyr Phe Tyr Tyr Gly Met Asp
               100                 105                 110
   Val Trp Gly Gln Gly Thr Thr Val Thr Val Ser Ser
           115                 120
<210> 307
   <211> 336
   <212> DNA
   <213> Artificial Sequence
<220>
   <223> Synthetic
<400> 307
   gatattgtga tgactcagtc tccactctcc ctgcccgtca cccctggaga gccggcctcc 60
   atctcctgca ggtctagtca gactctcctg catagtaatg gatacaacta tttcgattgg 120
   tacctgcaga agccagggca gtctccacag ctcctgatct atttgggttc taatcgggcc 180
   tccggggtcc ctgacagatt cagtggcagt ggatcaggca cagattttac actgaaaatc 240
   agcagagtgg aggctgagga tgttggaatt tattactgca tgcaagctct acaaactcct 300
   ctcactttcg gcggagggac caaggtggag atcaaa                           336
<210> 308
   <211> 112
   <212> PRT
   <213> Artificial Sequence
<220>
   <223> Synthetic
<400> 308
   Asp Ile Val Met Thr Gln Ser Pro Leu Ser Leu Pro Val Thr Pro Gly
    1               5                  10                  15
   Glu Pro Ala Ser Ile Ser Cys Arg Ser Ser Gln Thr Leu Leu His Ser
               20                  25                  30
   Asn Gly Tyr Asn Tyr Phe Asp Trp Tyr Leu Gln Lys Pro Gly Gln Ser
           35                  40                  45
   Pro Gln Leu Leu Ile Tyr Leu Gly Ser Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Ile Tyr Tyr Cys Met Gln Ala
                   85                  90                  95
   Leu Gln Thr Pro Leu Thr Phe Gly Gly Gly Thr Lys Val Glu Ile Lys
               100                 105                 110
<210> 309
   <211> 372
   <212> DNA
   <213> Artificial Sequence
<220>
   <223> Synthetic
<400> 309
   cagatcacct tgaaggagtc tggtcctacg ctggtgaaac ccacacagac cctcacgctg 60
   acctgcacct tctctgggtt ctcactcagc gctagtggag tgggtgtggg ctggatccgt 120
   cagcccccag gaaaggccct ggagtggctt gcactcattt attggaatga tgataagcgc 180
   tacagcccat ctctgaagag caggctcacc atcaccaagg acacctccaa aaaccaggtg 240
   gtccttacaa tgaccaacat ggaccctgtg gacacagcca catattactg tgcacacaga 300
   atacatctat ggtcctactt ctactacggt atggacgtct gggggcaagg gaccacggtc 360
   accgtctcct ca                                                     372
<210> 310
   <211> 124
   <212> PRT
   <213> Artificial Sequence
<220>
   <223> Synthetic
<400> 310
   Gln Ile Thr Leu Lys Glu Ser Gly Pro Thr Leu Val Lys Pro Thr Gln
    1               5                   10                  15
   Thr Leu Thr Leu Thr Cys Thr Phe Ser Gly Phe Ser Leu Ser Ala Ser
               20                  25                  30
   Gly Val Gly Val Gly Trp Ile Arg Gln Pro Pro Gly Lys Ala Leu Glu
           35                  40                  45
   Trp Leu Ala Leu Ile Tyr Trp Asn Asp Asp Lys Arg Tyr Ser Pro Ser
       50                  55                  60
   Leu Lys Ser Arg Leu Thr Ile Thr Lys Asp Thr Ser Lys Asn Gln Val
   65                  70                  75                  80
   Val Leu Thr Met Thr Asn Met Asp Pro Val Asp Thr Ala Thr Tyr Tyr
                   85                  90                  95
   Cys Ala His Arg Ile His Leu Trp Ser Tyr Phe Tyr Tyr Gly Met Asp
               100                 105                 110
   Val Trp Gly Gln Gly Thr Thr Val Thr Val Ser Ser
           115                 120
<210> 311
   <211> 336
   <212> DNA
   <213> Artificial Sequence
<220>
   <223> Synthetic
<400> 311
   gatattgtga tgactcagtc tccactctcc ctgcccgtca cccctggaga gccggcctcc 60
   atctcctgca ggtctagtca gactctcctg catagtaatg gatacaacta tttggattgg 120
   tacctgcaga agccagggca gtctccacag ctcctgatct atttgggttc taatcgggcc 180
   tccggggtcc ctgacaggtt cagtggcagt ggatcaggca cagattttac actgaaaatc 240
   agcagagtgg aggctgagga tgttggggtt tattactgca tgcaagctct acaaactcct 300
   ctcactttcg gcggagggac caaggtggag atcaaa                           336
<210> 312
   <211> 112
   <212> PRT
   <213> Artificial Sequence
<220>
   <223> Synthetic
<400> 312
   Asp Ile Val Met Thr Gln Ser Pro Leu Ser Leu Pro Val Thr Pro Gly
    1               5                  10                  15
   Glu Pro Ala Ser Ile Ser Cys Arg Ser Ser Gln Thr Leu Leu His Ser
               20                  25                  30
   Asn Gly Tyr Asn Tyr Leu Asp Trp Tyr Leu Gln Lys Pro Gly Gln Ser
           35                  40                  45
   Pro Gln Leu Leu Ile Tyr Leu Gly Ser Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ala
                   85                  90                  95
   Leu Gln Thr Pro Leu Thr Phe Gly Gly Gly Thr Lys Val Glu Ile Lys
               100                 105                 110
<210> 313
   <211> 381
   <212> DNA
   <213> Artificial Sequence
<220>
   <223> Synthetic
<400> 313
   caggttcagc tggtgcagtc tggacctgag gtgaagaacc ctggggcctc agtgaaggtc 60
   tcctgcaagg cttctggtta cacctttacc acctatggta tcagttgggt acgacaggcc 120
   cctggacaag ggcttgagtg gatgggatgg atcagcggtt acaatggtaa aacaaacgat 180
   gcacagaagt tccaggacag agtcgccatg accacagaca catccacgag cacagcctac 240
   atggagctga ggagcctgag atctgacgac acggccattt attactgttc gagagatcgt 300
   ttagtagtac cacctgccct taattattcc tactacgtta tggacgtctg gggccaaggg 360
   accacggtca ccgtctcctc a                                           381
<210> 314
   <211> 127
   <212> PRT
   <213> Artificial Sequence
<220>
   <223> Synthetic
<400> 314
   Gln Val Gln Leu Val Gln Ser Gly Pro Glu Val Lys Asn Pro Gly Ala
    1               5                  10                  15
   Ser Val Lys Val Ser Cys Lys Ala Ser Gly Tyr Thr Phe Thr Thr Tyr
               20                  25                  30
   Gly Ile Ser Trp Val Arg Gln Ala Pro Gly Gln Gly Leu Glu Trp Met
           35                  40                  45
   Gly Trp Ile Ser Gly Tyr Asn Gly Lys Thr Asn Asp Ala Gln Lys Phe
       50                  55                  60
   Gln Asp Arg Val Ala Met Thr Thr Asp Thr Ser Thr Ser Thr Ala Tyr
   65                  70                  75                  80
   Met Glu Leu Arg Ser Leu Arg Ser Asp Asp Thr Ala Ile Tyr Tyr Cys
                   85                  90                  95
   Ser Arg Asp Arg Leu Val Val Pro Pro Ala Leu Asn Tyr Ser Tyr Tyr
               100                 105                 110
   Val Met Asp Val Trp Gly Gln Gly Thr Thr Val Thr Val Ser Ser
           115                 120                 125
<210> 315
   <211> 24
   <212> DNA
   <213> Artificial Sequence
<220>
   <223> Synthetic
<400> 315
   ggttacacct ttaccaccta tggt 24
<210> 316
   <211> 8
   <212> PRT
   <213> Artificial Sequence
<220>
   <223> Synthetic
<400> 316
   Gly Tyr Thr Phe Thr Thr Tyr Gly
    1               5
<210> 317
   <211> 24
   <212> DNA
   <213> Artificial Sequence
<220>
   <223> Synthetic
<400> 317
   atcagcggtt acaatggtaa aaca 24
<210> 318
   <211> 8
   <212> PRT
   <213> Artificial Sequence
<220>
   <223> Synthetic
<400> 318
   Ile Ser Gly Tyr Asn Gly Lys Thr
    1               5
<210> 319
   <211> 60
   <212> DNA
   <213> Artificial Sequence
<220>
   <223> Synthetic
<400> 319
   tcgagagatc gtttagtagt accacctgcc cttaattatt cctactacgt tatggacgtc 60
<210> 320
   <211> 20
   <212> PRT
   <213> Artificial Sequence
<220>
   <223> Synthetic
<400> 320
   Ser Arg Asp Arg Leu Val Val Pro Pro Ala Leu Asn Tyr Ser Tyr Tyr
   1               5                   10                  15
   Val Met Asp Val
               20
<210> 321
   <211> 336
   <212> DNA
   <213> Artificial Sequence
<220>
   <223> Synthetic
<400> 321
   gatgttgtga tgactcagtc tccactctcc ctgcccgtca cccttggaca gccggcctcc 60
   atctcctgca ggtctagtca aagcctcgta tacagtgatg gaaacaccta cttgaattgg 120
   tctcagcaga ggccaggtca atctccaagg cgcctaattt ataaggtttc taaccgggac 180
   tctggggtcc cagacagatt cagcggcagt gggtcaggca ctgatttcac actgaaaatc 240
   agcagggtgg aggctgagga tgttggggtt tattactgca tgcaaggtac acactggccg 300
   tacacttttg gccaggggac caagctggag atcaaa                           336
<210> 322
   <211> 112
   <212> PRT
   <213> Artificial Sequence
<220>
   <223> Synthetic
<400> 322
   Asp Val Val Met Thr Gln Ser Pro Leu Ser Leu Pro Val Thr Leu Gly
    1               5                  10                  15
   Gln Pro Ala Ser Ile Ser Cys Arg Ser Ser Gln Ser Leu Val Tyr Ser
               20                  25                  30
   Asp Gly Asn Thr Tyr Leu Asn Trp Ser Gln Gln Arg Pro Gly Gln Ser
           35                  40                  45
   Pro Arg Arg Leu Ile Tyr Lys Val Ser Asn Arg Asp Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Gly
                   85                  90                  95
   Thr His Trp Pro Tyr Thr Phe Gly Gln Gly Thr Lys Leu Glu Ile Lys
               100                 105                 110
<210> 323
   <211> 33
   <212> DNA
   <213> Artificial Sequence
<220>
   <223> Synthetic
<400> 323
   caaagcctcg tatacagtga tggaaacacc tac 33
<210> 324
   <211> 11
   <212> PRT
   <213> Artificial Sequence
<220>
   <223> Synthetic
<400> 324
   Gln Ser Leu Val Tyr Ser Asp Gly Asn Thr Tyr
    1               5                  10
<210> 325
   <211> 9
   <212> DNA
   <213> Artificial Sequence
<220>
   <223> Synthetic
<400> 325
   aaggtttct 9
<210> 326
   <211> 3
   <212> PRT
   <213> Artificial Sequence
<220>
   <223> Synthetic
<400> 326
   Lys Val Ser
   1
<210> 327
   <211> 27
   <212> DNA
   <213> Artificial Sequence
<220>
   <223> Synthetic
<400> 327
   atgcaaggta cacactggcc gtacact 27
<210> 328
   <211> 9
   <212> PRT
   <213> Artificial Sequence
<220>
   <223> Synthetic
<400> 328
   Met Gln Gly Thr His Trp Pro Tyr Thr
   1 5
<210> 329
   <211> 381
   <212> DNA
   <213> Artificial Sequence
<220>
   <223> Synthetic
<400> 329
   caggttcagc tggtgcagtc tggacctgag gtgaagaacc ctggggcctc agtgaaggtc 60
   tcctgcaagg cttctggtta cacctttacc acctatggta tcagttgggt acgacaggcc 120
   cctggacaag ggcttgagtg gatgggatgg atcagcggtt acaatggtaa aacaaacgat 180
   gcacagaagt tccaggacag agtcgccatg accacagaca catccacgag cacagcctac 240
   atggagctga ggagcctgag atctgacgac acggccattt attactgttc gagagatcgt 300
   ttagtagtac cacctgccct taattattcc tactacgtta tggacgtctg gggccaaggg 360
   accacggtca ccgtctcctc a 381
<210> 330
   <211> 127
   <212> PRT
   <213> Artificial Sequence
<220>
   <223> Synthetic
<400> 330
   Gln Val Gln Leu Val Gln Ser Gly Pro Glu Val Lys Asn Pro Gly Ala
    1               5                  10                  15
   Ser Val Lys Val Ser Cys Lys Ala Ser Gly Tyr Thr Phe Thr Thr Tyr
               20                  25                  30
   Gly Ile Ser Trp Val Arg Gln Ala Pro Gly Gln Gly Leu Glu Trp Met
           35                  40                  45
   Gly Trp Ile Ser Gly Tyr Asn Gly Lys Thr Asn Asp Ala Gln Lys Phe
       50                  55                  60
   Gln Asp Arg Val Ala Met Thr Thr Asp Thr Ser Thr Ser Thr Ala Tyr
   65                  70                  75                  80
   Met Glu Leu Arg Ser Leu Arg Ser Asp Asp Thr Ala Ile Tyr Tyr Cys
                   85                  90                  95
   Ser Arg Asp Arg Leu Val Val Pro Pro Ala Leu Asn Tyr Ser Tyr Tyr
               100                 105                 110
   Val Met Asp Val Trp Gly Gln Gly Thr Thr Val Thr Val Ser Ser
           115                 120                 125
<210> 331
   <211> 336
   <212> DNA
   <213> Artificial Sequence
<220>
   <223> Synthetic
<400> 331
   gatgttgtga tgactcagtc tccactctcc ctgcccgtca cccttggaca gccggcctcc 60
   atctcctgca ggtctagtca aagcctcgta tacagtgatg gaaacaccta cttgaattgg 120
   tctcagcaga ggccaggtca atctccaagg cgcctaattt ataaggtttc taaccgggac 180
   tctggggtcc cagacagatt cagcggcagt gggtcaggca ctgatttcac actgaaaatc 240
   agcagggtgg aggctgagga tgttggggtt tattactgca tgcaaggtac acactggccg 300
   tacacttttg gccaggggac caagctggag atcaaa                           336
<210> 332
   <211> 112
   <212> PRT
   <213> Artificial Sequence
<220>
   <223> Synthetic
<400> 332
   Asp Val Val Met Thr Gln Ser Pro Leu Ser Leu Pro Val Thr Leu Gly
    1               5                  10                  15
   Gln Pro Ala Ser Ile Ser Cys Arg Ser Ser Gln Ser Leu Val Tyr Ser
               20                  25                  30
   Asp Gly Asn Thr Tyr Leu Asn Trp Ser Gln Gln Arg Pro Gly Gln Ser
           35                  40                  45
   Pro Arg Arg Leu Ile Tyr Lys Val Ser Asn Arg Asp Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Gly
                   85                  90                  95
   Thr His Trp Pro Tyr Thr Phe Gly Gln Gly Thr Lys Leu Glu Ile Lys
               100                 105                 110
<210> 333
   <211> 381
   <212> DNA
   <213> Artificial Sequence
<220>
   <223> Synthetic
<400> 333
   caggttcagc tggtgcagtc tggagctgag gtgaagaagc ctggggcctc agtgaaggtc 60
   tcctgcaagg cttctggtta cacctttacc acctatggta tcagctgggt gcgacaggcc 120
   cctggacaag ggcttgagtg gatgggatgg atcagcggtt acaatggtaa aacaaactat 180
   gcacagaagc tccagggcag agtcaccatg accacagaca catccacgag cacagcctac 240
   atggagctga ggagcctgag atctgacgac acggccgtgt attactgttc gagagatcgt 300
   ttagtagtac cacctgccct taattattcc tactacgtta tggacgtctg ggggcaaggg 360
   accacggtca ccgtctcctc a                                            381
<210> 334
   <211> 127
   <212> PRT
   <213> Artificial Sequence
<220>
   <223> Synthetic
<400> 334
   Gln Val Gln Leu Val Gln Ser Gly Ala Glu Val Lys Lys Pro Gly Ala
    1               5                  10                  15
   Ser Val Lys Val Ser Cys Lys Ala Ser Gly Tyr Thr Phe Thr Thr Tyr
               20                  25                  30
   Gly Ile Ser Trp Val Arg Gln Ala Pro Gly Gln Gly Leu Glu Trp Met
           35                  40                  45
   Gly Trp Ile Ser Gly Tyr Asn Gly Lys Thr Asn Tyr Ala Gln Lys Leu
       50                  55                  60
   Gln Gly Arg Val Thr Met Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ser Arg Asp Arg Leu Val Val Pro Pro Ala Leu Asn Tyr Ser Tyr Tyr
               100                 105                 110
   Val Met Asp Val Trp Gly Gln Gly Thr Thr Val Thr Val Ser Ser
           115                 120                 125
<210> 335
   <211> 336
   <212> DNA
   <213> Artificial Sequence
<220>
   <223> Synthetic
<400> 335
   gatgttgtga tgactcagtc tccactctcc ctgcccgtca cccttggaca gccggcctcc 60
   atctcctgca ggtctagtca aagcctcgta tacagtgatg gaaacaccta cttgaattgg 120
   tttcagcaga ggccaggcca atctccaagg cgcctaattt ataaggtttc taaccgggac 180
   tctggggtcc cagacagatt cagcggcagt gggtcaggca ctgatttcac actgaaaatc 240
   agcagggtgg aggctgagga tgttggggtt tattactgca tgcaaggtac acactggccg 300
   tacacttttg gccaggggac caagctggag atcaaa                           336
<210> 336
   <211> 112
   <212> PRT
   <213> Artificial Sequence
<220>
   <223> Synthetic
<400> 336
   Asp Val Val Met Thr Gln Ser Pro Leu Ser Leu Pro Val Thr Leu Gly
    1               5                  10                  15
   Gln Pro Ala Ser Ile Ser Cys Arg Ser Ser Gln Ser Leu Val Tyr Ser
               20                  25                  30
   Asp Gly Asn Thr Tyr Leu Asn Trp Phe Gln Gln Arg Pro Gly Gln Ser
           35                  40                  45
   Pro Arg Arg Leu Ile Tyr Lys Val Ser Asn Arg Asp Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Gly
                   85                  90                  95
   Thr His Trp Pro Tyr Thr Phe Gly Gln Gly Thr Lys Leu Glu Ile Lys
               100                 105                 110
<210> 337
   <211> 354
   <212> DNA
   <213> Artificial Sequence
<220>
   <223> Synthetic
<400> 337
   gaggtgcagc tggtggagtc tgggggaggc ctggtcaagc ctggggggtc cctgagactc 60
   tcctgtgcag cctctggatt caccttcagt agctatagca tggactgggt ccgccaggct 120
   ccagggaagg ggctggagtg ggtctcatcc attagtagta gtagtagtta catatactac 180
   gcagactctg tgaagggccg attcaccatc tccagagaca ccgccaagaa ctcactgtat 240
   ctgcaaatga acagcctgag agacgaggac acggctgttt attactgtgc gagagagggc 300
   agtagcagac tttttgacta ctggggccag ggaaccctgg tcaccgtctc ctca       354
<210> 338
   <211> 118
   <212> PRT
   <213> Artificial Sequence
<220>
   <223> Synthetic
<400> 338
   Glu Val Gln Leu Val Glu Ser Gly Gly Gly Leu Val Lys Pro Gly Gly
    1               5                  10                  15
   Ser Leu Arg Leu Ser Cys Ala Ala Ser Gly Phe Thr Phe Ser Ser Tyr
               20                  25                  30
   Ser Met Asp Trp Val Arg Gln Ala Pro Gly Lys Gly Leu Glu Trp Val
           35                  40                  45
   Ser Ser Ile Ser Ser Ser Ser Ser Tyr Ile Tyr Tyr Ala Asp Ser Val
       50                  55                  60
   Lys Gly Arg Phe Thr Ile Ser Arg Asp Thr Ala Lys Asn Ser Leu Tyr
   65                  70                  75                  80
   Leu Gln Met Asn Ser Leu Arg Asp Glu Asp Thr Ala Val Tyr Tyr Cys
                   85                  90                  95
   Ala Arg Glu Gly Ser Ser Arg Leu Phe Asp Tyr Trp Gly Gln Gly Thr
               100                 105                 110
   Leu Val Thr Val Ser Ser
           115
<210> 339
   <211> 24
   <212> DNA
   <213> Artificial Sequence
<220>
   <223> Synthetic
<400> 339
   ggattcacct tcagtagcta tagc 24
<210> 340
   <211> 8
   <212> PRT
   <213> Artificial Sequence
<220>
   <223> Synthetic
<400> 340
   Gly Phe Thr Phe Ser Ser Tyr Ser
   1 5
<210> 341
   <211> 24
   <212> DNA
   <213> Artificial Sequence
<220>
   <223> Synthetic
<400> 341
   attagtagta gtagtagtta cata 24
<210> 342
   <211> 8
   <212> PRT
   <213> Artificial Sequence
<220>
   <223> Synthetic
<400> 342
   Ile Ser Ser Ser Ser Ser Tyr Ile
   1 5
<210> 343
   <211> 33
   <212> DNA
   <213> Artificial Sequence
<220>
   <223> Synthetic
<400> 343
   gcgagagagg gcagtagcag actttttgac tac 33
<210> 344
   <211> 11
   <212> PRT
   <213> Artificial Sequence
<220>
   <223> Synthetic
<400> 344
   Ala Arg Glu Gly Ser Ser Arg Leu Phe Asp Tyr
    1               5                   10
<210> 345
   <211> 321
   <212> DNA
   <213> Artificial Sequence
<220>
   <223> Synthetic
<400> 345
   gacatccaga tgacccagtc tccttccacc ctgtctgcat ctgtaggaga cagagtcacc 60
   atcacttgcc gggccagtca gagtattagt agctggttgg cctggtatca gcagagacca 120
   gggaaagccc ctaagctcct gatctataag gcgtctagtt tagaaggtgg agtcccatca 180
   aggttcagcg gcagtggatc tgggacagaa ttcactctca ccatcagcag cctgcagcct 240
   gaggattttg caacttatta ctgccaacag tataatagtt attggtacac ttttggccag 300
   gggaccaagc tggagatcaa a                                           321
<210> 346
   <211> 107
   <212> PRT
   <213> Artificial Sequence
<220>
   <223> Synthetic
<400> 346
   Asp Ile Gln Met Thr Gln Ser Pro Ser Thr Leu Ser Ala Ser Val Gly
    1               5                  10                  15
   Asp Arg Val Thr Ile Thr Cys Arg Ala Ser Gln Ser Ile Ser Ser Trp
               20                  25                  30
   Leu Ala Trp Tyr Gln Gln Arg Pro Gly Lys Ala Pro Lys Leu Leu Ile
           35                  40                  45
   Tyr Lys Ala Ser Ser Leu Glu Gly Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Glu Asp Phe Ala Thr Tyr Tyr Cys Gln Gln Tyr Asn Ser Tyr Trp Tyr
                   85                  90                  95
   Thr Phe Gly Gln Gly Thr Lys Leu Glu Ile Lys
               100                 105
<210> 347
   <211> 18
   <212> DNA
   <213> Artificial Sequence
<220>
   <223> Synthetic
<400> 347
   cagagtatta gtagctgg 18
<210> 348
   <211> 6
   <212> PRT
   <213> Artificial Sequence
<220>
   <223> Synthetic
<400> 348
   Gln Ser Ile Ser Ser Trp
   1 5
<210> 349
   <211> 9
   <212> DNA
   <213> Artificial Sequence
<220>
   <223> Synthetic
<400> 349
   aaggcgtct 9
<210> 350
   <211> 3
   <212> PRT
   <213> Artificial Sequence
<220>
   <223> Synthetic
<400> 350
   Lys Ala Ser
   1
<210> 351
   <211> 27
   <212> DNA
   <213> Artificial Sequence
<220>
   <223> Synthetic
<400> 351
   caacagtata atagttattg gtacact 27
<210> 352
   <211> 9
   <212> PRT
   <213> Artificial Sequence
<220>
   <223> Synthetic
<400> 352
   Gln Gln Tyr Asn Ser Tyr Trp Tyr Thr
   1               5
<210> 353
   <211> 354
   <212> DNA
   <213> Artificial Sequence
<220>
   <223> Synthetic
<400> 353
   gaggtgcagc tggtggagtc tgggggaggc ctggtcaagc ctggggggtc cctgagactc 60
   tcctgtgcag cctctggatt caccttcagt agctatagca tggactgggt ccgccaggct 120
   ccagggaagg ggctggagtg ggtctcatcc attagtagta gtagtagtta catatactac 180
   gcagactctg tgaagggccg attcaccatc tccagagaca ccgccaagaa ctcactgtat 240
   ctgcaaatga acagcctgag agacgaggac acggctgttt attactgtgc gagagagggc 300
   agtagcagac tttttgacta ctggggccag ggaaccctgg tcaccgtctc ctca       354
<210> 354
   <211> 118
   <212> PRT
   <213> Artificial Sequence
<220>
   <223> Synthetic
<400> 354
   Glu Val Gln Leu Val Glu Ser Gly Gly Gly Leu Val Lys Pro Gly Gly
    1               5                  10                  15
   Ser Leu Arg Leu Ser Cys Ala Ala Ser Gly Phe Thr Phe Ser Ser Tyr
               20                  25                  30
   Ser Met Asp Trp Val Arg Gln Ala Pro Gly Lys Gly Leu Glu Trp Val
           35                  40                  45
   Ser Ser Ile Ser Ser Ser Ser Ser Tyr Ile Tyr Tyr Ala Asp Ser Val
       50                  55                  60
   Lys Gly Arg Phe Thr Ile Ser Arg Asp Thr Ala Lys Asn Ser Leu Tyr
   65                  70                  75                  80
   Leu Gln Met Asn Ser Leu Arg Asp Glu Asp Thr Ala Val Tyr Tyr Cys
                   85                  90                  95
   Ala Arg Glu Gly Ser Ser Arg Leu Phe Asp Tyr Trp Gly Gln Gly Thr
               100                 105                 110
   Leu Val Thr Val Ser Ser
           115
<210> 355
   <211> 321
   <212> DNA
   <213> Artificial Sequence
<220>
   <223> Synthetic
<400> 355
   gacatccaga tgacccagtc tccttccacc ctgtctgcat ctgtaggaga cagagtcacc 60
   atcacttgcc gggccagtca gagtattagt agctggttgg cctggtatca gcagagacca 120
   gggaaagccc ctaagctcct gatctataag gcgtctagtt tagaaggtgg agtcccatca 180
   aggttcagcg gcagtggatc tgggacagaa ttcactctca ccatcagcag cctgcagcct 240
   gaggattttg caacttatta ctgccaacag tataatagtt attggtacac ttttggccag 300
   gggaccaagc tggagatcaa a                                           321
<210> 356
   <211> 107
   <212> PRT
   <213> Artificial Sequence
<220>
   <223> Synthetic
<400> 356
   Asp Ile Gln Met Thr Gln Ser Pro Ser Thr Leu Ser Ala Ser Val Gly
    1               5                  10                  15
   Asp Arg Val Thr Ile Thr Cys Arg Ala Ser Gln Ser Ile Ser Ser Trp
               20                  25                  30
   Leu Ala Trp Tyr Gln Gln Arg Pro Gly Lys Ala Pro Lys Leu Leu Ile
           35                  40                  45
   Tyr Lys Ala Ser Ser Leu Glu Gly Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Glu Asp Phe Ala Thr Tyr Tyr Cys Gln Gln Tyr Asn Ser Tyr Trp Tyr
                   85                  90                  95
   Thr Phe Gly Gln Gly Thr Lys Leu Glu Ile Lys
               100                 105
<210> 357
   <211> 354
   <212> DNA
   <213> Artificial Sequence
<220>
   <223> Synthetic
<400> 357
   gaggtgcagc tggtggagtc tgggggaggc ctggtcaagc ctggggggtc cctgagactc 60
   tcctgtgcag cctctggatt caccttcagt agctatagca tgaactgggt ccgccaggct 120
   ccagggaagg ggctggagtg ggtctcatcc attagtagta gtagtagtta catatactac 180
   gcagactcag tgaagggccg attcaccatc tccagagaca acgccaagaa ctcactgtat 240
   ctgcaaatga acagcctgag agccgaggac acggctgtgt attactgtgc gagagagggc 300
   agtagcagac tttttgacta ctggggccaa ggaaccctgg tcaccgtctc ctca       354
<210> 358
   <211> 118
   <212> PRT
   <213> Artificial Sequence
<220>
   <223> Synthetic
<400> 358
   Glu Val Gln Leu Val Glu Ser Gly Gly Gly Leu Val Lys Pro Gly Gly
    1               5                  10                  15
   Ser Leu Arg Leu Ser Cys Ala Ala Ser Gly Phe Thr Phe Ser Ser Tyr
               20                  25                  30
   Ser Met Asn Trp Val Arg Gln Ala Pro Gly Lys Gly Leu Glu Trp Val
           35                  40                  45
   Ser Ser Ile Ser Ser Ser Ser Ser Tyr Ile Tyr Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Arg Glu Gly Ser Ser Arg Leu Phe Asp Tyr Trp Gly Gln Gly Thr
               100                 105                 110
   Leu Val Thr Val Ser Ser
           115
<210> 359
   <211> 321
   <212> DNA
   <213> Artificial Sequence
<220>
   <223> Synthetic
<400> 359
   gacatccaga tgacccagtc tccttccacc ctgtctgcat ctgtaggaga cagagtcacc 60
   atcacttgcc gggccagtca gagtattagt agctggttgg cctggtatca gcagaaacca 120
   gggaaagccc ctaagctcct gatctataag gcgtctagtt tagaaagtgg ggtcccatca 180
   aggttcagcg gcagtggatc tgggacagaa ttcactctca ccatcagcag cctgcagcct 240
   gatgattttg caacttatta ctgccaacag tataatagtt attggtacac ttttggccag 300
   gggaccaagc tggagatcaa a                                           321
<210> 360
   <211> 107
   <212> PRT
   <213> Artificial Sequence
<220>
   <223> Synthetic
<400> 360
   Asp Ile Gln Met Thr Gln Ser Pro Ser Thr Leu Ser Ala Ser Val Gly
    1               5                  10                  15
   Asp Arg Val Thr Ile Thr Cys Arg Ala Ser Gln Ser Ile Ser Ser Trp
               20                  25                  30
   Leu Ala Trp Tyr Gln Gln Lys Pro Gly Lys Ala Pro Lys Leu Leu Ile
           35                  40                  45
   Tyr Lys Ala Ser Ser Leu Glu Ser Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Asp Asp Phe Ala Thr Tyr Tyr Cys Gln Gln Tyr Asn Ser Tyr Trp Tyr
                   85                  90                  95
   Thr Phe Gly Gln Gly Thr Lys Leu Glu Ile Lys
               100                 105
<210> 361
   <211> 384
   <212> DNA
   <213> Artificial Sequence
<220>
   <223> Synthetic
<400> 361
   caggtgcacc tggtggagtc tgggggaggc ttggtcaagc ctggagggtc cctgagactc 60
   tcctgtgcag cctctggatt caccttcagt gaccactaca tgagctggat ccgccaggct 120
   ccagggaagg ggctggagtg gatttcatac attagtaatg atggtggtac caaatactat 180
   gtggactctg tggagggccg attcatcatt tccagggaca acgccaagaa ctcattgtat 240
   ctacatatga acagcctcag agccgacgac acggccgtgt attactgtgc gagagatcag 300
   ggatatattg gctacgactc gtattattac tattcctacg gtatggacgt ctggggccaa 360
   gggaccacgg tcaccgtcgc ctca                                        384
<210> 362
   <211> 128
   <212> PRT
   <213> Artificial Sequence
<220>
   <223> Synthetic
<400> 362
   Gln Val His Leu Val Glu Ser Gly Gly Gly Leu Val Lys Pro Gly Gly
    1               5                  10                  15
   Ser Leu Arg Leu Ser Cys Ala Ala Ser Gly Phe Thr Phe Ser Asp His
               20                  25                  30
   Tyr Met Ser Trp Ile Arg Gln Ala Pro Gly Lys Gly Leu Glu Trp Ile
           35                  40                  45
   Ser Tyr Ile Ser Asn Asp Gly Gly Thr Lys Tyr Tyr Val Asp Ser Val
       50                  55                  60
   Glu Gly Arg Phe Ile Ile Ser Arg Asp Asn Ala Lys Asn Ser Leu Tyr
   65                  70                  75                  80
   Leu His Met Asn Ser Leu Arg Ala Asp Asp Thr Ala Val Tyr Tyr Cys
                   85                  90                  95
   Ala Arg Asp Gln Gly Tyr Ile Gly Tyr Asp Ser Tyr Tyr Tyr Tyr Ser
               100                 105                 110
   Tyr Gly Met Asp Val Trp Gly Gln Gly Thr Thr Val Thr Val Ala Ser
           115                 120                 125
<210> 363
   <211> 24
   <212> DNA
   <213> Artificial Sequence
<220>
   <223> Synthetic
<400> 363
   ggattcacct tcagtgacca ctac 24
<210> 364
   <211> 8
   <212> PRT
   <213> Artificial Sequence
<220>
   <223> Synthetic
<400> 364
   Gly Phe Thr Phe Ser Asp His Tyr
    1               5
<210> 365
   <211> 24
   <212> DNA
   <213> Artificial Sequence
<220>
   <223> Synthetic
<400> 365
   attagtaatg atggtggtac caaa 24
<210> 366
   <211> 8
   <212> PRT
   <213> Artificial Sequence
<220>
   <223> Synthetic
<400> 366
   Ile Ser Asn Asp Gly Gly Thr Lys
    1               5
<210> 367
   <211> 63
   <212> DNA
   <213> Artificial Sequence
<220>
   <223> Synthetic
<400> 367
   gcgagagatc agggatatat tggctacgac tcgtattatt actattccta cggtatggac 60
   gtc                                                               63
<210> 368
   <211> 21
   <212> PRT
   <213> Artificial Sequence
<220>
   <223> Synthetic
<400> 368
   Ala Arg Asp Gln Gly Tyr Ile Gly Tyr Asp Ser Tyr Tyr Tyr Tyr Ser
    1               5                  10                  15
   Tyr Gly Met Asp Val
               20
<210> 369
   <211> 321
   <212> DNA
   <213> Artificial Sequence
<220>
   <223> Synthetic
<400> 369
   aaaattgtgt tgacgcagtc tccaggcacc ctgcctttgt ttccagggga aagagccacc 60
   ctctcctgta gggccagtca gagtgttaac aacaaattct tagcctggta ccagcagaaa 120
   tctggccagg ctcccaggct cctcatctat ggtgcatcca gcagggccac tggcatccca 180
   gacaggttca gtggcagtgg gtctgggacc gacttcactc tcaccatcag cggactggag 240
   cctgaagatt ttgaagtgta ttattgtcaa gtatatggta actcactcac tctcggcgga 300
   gggaccaagg tggagatcaa g                                           321
<210> 370
   <211> 107
   <212> PRT
   <213> Artificial Sequence
<220>
   <223> Synthetic
<400> 370
   Lys Ile Val Leu Thr Gln Ser Pro Gly Thr Leu Pro Leu Phe Pro Gly
    1               5                  10                  15
   Glu Arg Ala Thr Leu Ser Cys Arg Ala Ser Gln Ser Val Asn Asn Lys
               20                  25                  30
   Phe Leu Ala Trp Tyr Gln Gln Lys Ser Gly Gln Ala Pro Arg Leu Leu
           35                  40                  45
   Ile Tyr Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Gly Leu Glu
   65                  70                  75                  80
   Pro Glu Asp Phe Glu Val Tyr Tyr Cys Gln Val Tyr Gly Asn Ser Leu
                   85                  90                  95
   Thr Leu Gly Gly Gly Thr Lys Val Glu Ile Lys
               100                 105
<210> 371
   <211> 21
   <212> DNA
   <213> Artificial Sequence
<220>
   <223> Synthetic
<400> 371
   cagagtgtta acaacaaatt c 21
<210> 372
   <211> 7
   <212> PRT
   <213> Artificial Sequence
<220>
   <223> Synthetic
<400> 372
   Gln Ser Val Asn Asn Lys Phe
    1               5
<210> 373
   <211> 9
   <212> DNA
   <213> Artificial Sequence
<220>
   <223> Synthetic
<400> 373
   ggtgcatcc 9
<210> 374
   <211> 3
   <212> PRT
   <213> Artificial Sequence
<220>
   <223> Synthetic
<400> 374
   Gly Ala Ser
   1
<210> 375
   <211> 24
   <212> DNA
   <213> Artificial Sequence
<220>
   <223> Synthetic
<400> 375
   caagtatatg gtaactcact cact 24
<210> 376
   <211> 8
   <212> PRT
   <213> Artificial Sequence
<220>
   <223> Synthetic
<400> 376
   Gln Val Tyr Gly Asn Ser Leu Thr
   1 5
<210> 377
   <211> 384
   <212> DNA
   <213> Artificial Sequence
<220>
   <223> Synthetic
<400> 377
   caggtgcagc tggtggagtc tgggggaggc ttggtcaagc ctggagggtc cctgagactc 60
   tcctgtgcag cctctggatt caccttcagt gaccactaca tgagctggat ccgccaggct 120
   ccagggaagg ggctggagtg gatttcatac attagtaatg atggtggtac caaatactat 180
   gtggactctg tggagggccg attcatcatt tccagggaca acgccaagaa ctcattgtat 240
   ctacatatga acagcctcag agccgacgac acggccgtgt attactgtgc gagagatcag 300
   ggatatattg gctacgactc gtattattac tattcctacg gtatggacgt ctggggccaa 360
   gggaccacgg tcaccgtctc ctca                                        384
<210> 378
   <211> 128
   <212> PRT
   <213> Artificial Sequence
<220>
   <223> Synthetic
<400> 378
   Gln Val Gln Leu Val Glu Ser Gly Gly Gly Leu Val Lys Pro Gly Gly
    1               5                  10                  15
   Ser Leu Arg Leu Ser Cys Ala Ala Ser Gly Phe Thr Phe Ser Asp His
               20                  25                  30
   Tyr Met Ser Trp Ile Arg Gln Ala Pro Gly Lys Gly Leu Glu Trp Ile
           35                  40                  45
   Ser Tyr Ile Ser Asn Asp Gly Gly Thr Lys Tyr Tyr Val Asp Ser Val
       50                  55                  60
   Glu Gly Arg Phe Ile Ile Ser Arg Asp Asn Ala Lys Asn Ser Leu Tyr
   65                  70                  75                  80
   Leu His Met Asn Ser Leu Arg Ala Asp Asp Thr Ala Val Tyr Tyr Cys
                   85                  90                  95
   Ala Arg Asp Gln Gly Tyr Ile Gly Tyr Asp Ser Tyr Tyr Tyr Tyr Ser
               100                 105                 110
   Tyr Gly Met Asp Val Trp Gly Gln Gly Thr Thr Val Thr Val Ser Ser
           115                 120                 125
<210> 379
   <211> 321
   <212> DNA
   <213> Artificial Sequence
<220>
   <223> Synthetic
<400> 379
   gaaattgtgt tgacgcagtc tccaggcacc ctgcctttgt ttccagggga aagagccacc 60
   ctctcctgta gggccagtca gagtgttaac aacaaattct tagcctggta ccagcagaaa 120
   tctggccagg ctcccaggct cctcatctat ggtgcatcca gcagggccac tggcatccca 180
   gacaggttca gtggcagtgg gtctgggacc gacttcactc tcaccatcag cggactggag 240
   cctgaagatt ttgaagtgta ttattgtcaa gtatatggta actcactcac tctcggcgga 300
   gggaccaagg tggagatcaa a                                           321
<210> 380
   <211> 107
   <212> PRT
   <213> Artificial Sequence
<220>
   <223> Synthetic
<400> 380
   Glu Ile Val Leu Thr Gln Ser Pro Gly Thr Leu Pro Leu Phe Pro Gly
    1               5                  10                  15
   Glu Arg Ala Thr Leu Ser Cys Arg Ala Ser Gln Ser Val Asn Asn Lys
               20                  25                  30
   Phe Leu Ala Trp Tyr Gln Gln Lys Ser Gly Gln Ala Pro Arg Leu Leu
           35                  40                  45
   Ile Tyr Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Gly Leu Glu
   65                  70                  75                  80
   Pro Glu Asp Phe Glu Val Tyr Tyr Cys Gln Val Tyr Gly Asn Ser Leu
                   85                  90                  95
   Thr Leu Gly Gly Gly Thr Lys Val Glu Ile Lys
               100                 105
<210> 381
   <211> 384
   <212> DNA
   <213> Artificial Sequence
<220>
   <223> Synthetic
<400> 381
   caggtgcagc tggtggagtc tgggggaggc ttggtcaagc ctggagggtc cctgagactc 60
   tcctgtgcag cctctggatt caccttcagt gaccactaca tgagctggat ccgccaggct 120
   ccagggaagg ggctggagtg ggtttcatac attagtaatg atggtggtac caaatactac 180
   gcagactctg tgaagggccg attcaccatc tccagggaca acgccaagaa ctcactgtat 240
   ctgcaaatga acagcctgag agccgaggac acggccgtgt attactgtgc gagagatcag 300
   ggatatattg gctacgactc gtattattac tattcctacg gtatggacgt ctgggggcaa 360
   gggaccacgg tcaccgtctc ctca                                        384
<210> 382
   <211> 128
   <212> PRT
   <213> Artificial Sequence
<220>
   <223> Synthetic
<400> 382
   Gln Val Gln Leu Val Glu Ser Gly Gly Gly Leu Val Lys Pro Gly Gly
    1               5                  10                  15
   Ser Leu Arg Leu Ser Cys Ala Ala Ser Gly Phe Thr Phe Ser Asp His
               20                  25                  30
   Tyr Met Ser Trp Ile Arg Gln Ala Pro Gly Lys Gly Leu Glu Trp Val
           35                  40                  45
   Ser Tyr Ile Ser Asn Asp Gly Gly Thr Lys Tyr Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Arg Asp Gln Gly Tyr Ile Gly Tyr Asp Ser Tyr Tyr Tyr Tyr Ser
               100                 105                 110
   Tyr Gly Met Asp Val Trp Gly Gln Gly Thr Thr Val Thr Val Ser Ser
           115                 120                 125
<210> 383
   <211> 321
   <212> DNA
   <213> Artificial Sequence
<220>
   <223> Synthetic
<400> 383
   gaaattgtgt tgacgcagtc tccaggcacc ctgtctttgt ctccagggga aagagccacc 60
   ctctcctgca gggccagtca gagtgttaac aacaaattct tagcctggta ccagcagaaa 120
   cctggccagg ctcccaggct cctcatctat ggtgcatcca gcagggccac tggcatccca 180
   gacaggttca gtggcagtgg gtctgggaca gacttcactc tcaccatcag cagactggag 240
   cctgaagatt ttgcagtgta ttactgtcaa gtatatggta actcactcac tttcggcgga 300
   gggaccaagg tggagatcaa a                                           321
<210> 384
   <211> 107
   <212> PRT
   <213> Artificial Sequence
<220>
   <223> Synthetic
<400> 384
   Glu Ile Val Leu Thr Gln Ser Pro Gly Thr Leu Ser Leu Ser Pro Gly
    1               5                  10                  15
   Glu Arg Ala Thr Leu Ser Cys Arg Ala Ser Gln Ser Val Asn Asn Lys
               20                  25                  30
   Phe Leu Ala Trp Tyr Gln Gln Lys Pro Gly Gln Ala Pro Arg Leu Leu
           35                  40                  45
   Ile Tyr Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Val Tyr Gly Asn Ser Leu
                   85                  90                  95
   Thr Phe Gly Gly Gly Thr Lys Val Glu Ile Lys
               100                 105
<210> 385
   <211> 360
   <212> DNA
   <213> Artificial Sequence
<220>
   <223> Synthetic
<400> 385
   gaggtgcaga aggtggagtc tgggggaggc ctggtcaagc cgggggggtc cctgagactc 60
   tcctgtacag cctctggatt caccttcagt acttataaca tgaattgggt ccgccaggct 120
   ccagggaagg gactggagtg ggtctcatcc attaggagta gtagtaatta catatactac 180
   gcagactcag tgaagggccg attcaccatc tccagagaca acgccaagaa ttcactgtat 240
   ctgcaaatga acagcctgag agccgatgac acggctgtgt attactgtgc gagagatggc 300
   agcagttggt acgactactc tgactactgg ggccagggaa ccctggtcac cgtctcctca 360
<210> 386
   <211> 120
   <212> PRT
   <213> Artificial Sequence
<220>
   <223> Synthetic
<400> 386
   Glu Val Gln Lys Val Glu Ser Gly Gly Gly Leu Val Lys Pro Gly Gly
    1               5                  10                  15
   Ser Leu Arg Leu Ser Cys Thr Ala Ser Gly Phe Thr Phe Ser Thr Tyr
               20                  25                  30
   Asn Met Asn Trp Val Arg Gln Ala Pro Gly Lys Gly Leu Glu Trp Val
           35                  40                  45
   Ser Ser Ile Arg Ser Ser Ser Asn Tyr Ile Tyr Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Asp Asp Thr Ala Val Tyr Tyr Cys
                   85                  90                  95
   Ala Arg Asp Gly Ser Ser Trp Tyr Asp Tyr Ser Asp Tyr Trp Gly Gln
               100                 105                 110
   Gly Thr Leu Val Thr Val Ser Ser
           115                 120
<210> 387
   <211> 24
   <212> DNA
   <213> Artificial Sequence
<220>
   <223> Synthetic
<400> 387
   ggattcacct tcagtactta taac 24
<210> 388
   <211> 8
   <212> PRT
   <213> Artificial Sequence
<220>
   <223> Synthetic
<400> 388
   Gly Phe Thr Phe Ser Thr Tyr Asn
    1               5
<210> 389
   <211> 24
   <212> DNA
   <213> Artificial Sequence
<220>
   <223> Synthetic
<400> 389
   attaggagta gtagtaatta cata 24
<210> 390
   <211> 8
   <212> PRT
   <213> Artificial Sequence
<220>
   <223> Synthetic
<400> 390
   Ile Arg Ser Ser Ser Asn Tyr Ile
    1               5
<210> 391
   <211> 39
   <212> DNA
   <213> Artificial Sequence
<220>
   <223> Synthetic
<400> 391
   gcgagagatg gcagcagttg gtacgactac tctgactac 39
<210> 392
   <211> 13
   <212> PRT
   <213> Artificial Sequence
<220>
   <223> Synthetic
<400> 392
   Ala Arg Asp Gly Ser Ser Trp Tyr Asp Tyr Ser Asp Tyr
   1 5 10
<210> 393
   <211> 321
   <212> DNA
   <213> Artificial Sequence
<220>
   <223> Synthetic
<400> 393
   gacatccaga tgacccagtc tccttccacc ctgtctgcat ctgtaggaga cagagtcacc 60
   atcacttgcc gggccagtca gagtattagt agctggttgg cctggtatca acagatacca 120
   gggaaagccc ctaaactcct gatctataag gcgtctagtt tagaaaatgg ggtcccatca 180
   aggttcagcg gcagtggatc tgggacagaa ttcactctca tcatcagcag cctgcagcct 240
   gatgattttg caacttatta ctgccaacag tatattagtt attctcggac gttcggccaa 300
   gggaccaagg tggaaatcaa a 321
<210> 394
   <211> 107
   <212> PRT
   <213> Artificial Sequence
<220>
   <223> Synthetic
<400> 394
   Asp Ile Gln Met Thr Gln Ser Pro Ser Thr Leu Ser Ala Ser Val Gly
    1               5                  10                  15
   Asp Arg Val Thr Ile Thr Cys Arg Ala Ser Gln Ser Ile Ser Ser Trp
               20                  25                  30
   Leu Ala Trp Tyr Gln Gln Ile Pro Gly Lys Ala Pro Lys Leu Leu Ile
           35                  40                  45
   Tyr Lys Ala Ser Ser Leu Glu Asn Gly Val Pro Ser Arg Phe Ser Gly
       50                  55                  60
   Ser Gly Ser Gly Thr Glu Phe Thr Leu Ile Ile Ser Ser Leu Gln Pro
   65                  70                  75                  80
   Asp Asp Phe Ala Thr Tyr Tyr Cys Gln Gln Tyr Ile Ser Tyr Ser Arg
                   85                  90                  95
   Thr Phe Gly Gln Gly Thr Lys Val Glu Ile Lys
               100                 105
<210> 395
   <211> 18
   <212> DNA
   <213> Artificial Sequence
<220>
   <223> Synthetic
<400> 395
   cagagtatta gtagctgg 18
<210> 396
   <211> 6
   <212> PRT
   <213> Artificial Sequence
<220>
   <223> Synthetic
<400> 396
   Gln Ser Ile Ser Ser Trp
    1               5
<210> 397
   <211> 9
   <212> DNA
   <213> Artificial Sequence
<220>
   <223> Synthetic
<400> 397
   aaggcgtct 9
<210> 398
   <211> 3
   <212> PRT
   <213> Artificial Sequence
<220>
   <223> Synthetic
<400> 398
   Lys Ala Ser
   1
<210> 399
   <211> 27
   <212> DNA
   <213> Artificial Sequence
<220>
   <223> Synthetic
<400> 399
   caacagtata ttagttattc tcggacg 27
<210> 400
   <211> 9
   <212> PRT
   <213> Artificial Sequence
<220>
   <223> Synthetic
<400> 400
   Gln Gln Tyr Ile Ser Tyr Ser Arg Thr
   1 5
<210> 401
   <211> 360
   <212> DNA
   <213> Artificial Sequence
<220>
   <223> Synthetic
<400> 401
   gaggtgcagc tggtggagtc tgggggaggc ctggtcaagc cgggggggtc cctgagactc 60
   tcctgtacag cctctggatt caccttcagt acttataaca tgaattgggt ccgccaggct 120
   ccagggaagg gactggagtg ggtctcatcc attaggagta gtagtaatta catatactac 180
   gcagactcag tgaagggccg attcaccatc tccagagaca acgccaagaa ttcactgtat 240
   ctgcaaatga acagcctgag agccgatgac acggctgtgt attactgtgc gagagatggc 300
   agcagttggt acgactactc tgactactgg ggccagggaa ccctggtcac cgtctcctca 360
<210> 402
   <211> 120
   <212> PRT
   <213> Artificial Sequence
<220>
   <223> Synthetic
<400> 402
   Glu Val Gln Leu Val Glu Ser Gly Gly Gly Leu Val Lys Pro Gly Gly
    1               5                  10                  15
   Ser Leu Arg Leu Ser Cys Thr Ala Ser Gly Phe Thr Phe Ser Thr Tyr
               20                  25                  30
   Asn Met Asn Trp Val Arg Gln Ala Pro Gly Lys Gly Leu Glu Trp Val
           35                  40                  45
   Ser Ser Ile Arg Ser Ser Ser Asn Tyr Ile Tyr Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Asp Asp Thr Ala Val Tyr Tyr Cys
                   85                  90                  95
   Ala Arg Asp Gly Ser Ser Trp Tyr Asp Tyr Ser Asp Tyr Trp Gly Gln
               100                 105                 110
   Gly Thr Leu Val Thr Val Ser Ser
           115                 120
<210> 403
   <211> 321
   <212> DNA
   <213> Artificial Sequence
<220>
   <223> Synthetic
<400> 403
   gacatccaga tgacccagtc tccttccacc ctgtctgcat ctgtaggaga cagagtcacc 60
   atcacttgcc gggccagtca gagtattagt agctggttgg cctggtatca acagatacca 120
   gggaaagccc ctaaactcct gatctataag gcgtctagtt tagaaaatgg ggtcccatca 180
   aggttcagcg gcagtggatc tgggacagaa ttcactctca tcatcagcag cctgcagcct 240
   gatgattttg caacttatta ctgccaacag tatattagtt attctcggac gttcggccaa 300
   gggaccaagg tggaaatcaa a                                           321
<210> 404
   <211> 107
   <212> PRT
   <213> Artificial Sequence
<220>
   <223> Synthetic
<400> 404
   Asp Ile Gln Met Thr Gln Ser Pro Ser Thr Leu Ser Ala Ser Val Gly
    1              5                   10                  15
   Asp Arg Val Thr Ile Thr Cys Arg Ala Ser Gln Ser Ile Ser Ser Trp
               20                  25                  30
   Leu Ala Trp Tyr Gln Gln Ile Pro Gly Lys Ala Pro Lys Leu Leu Ile
           35                  40                  45
   Tyr Lys Ala Ser Ser Leu Glu Asn Gly Val Pro Ser Arg Phe Ser Gly
       50                  55                  60
   Ser Gly Ser Gly Thr Glu Phe Thr Leu Ile Ile Ser Ser Leu Gln Pro
   65                  70                  75                  80
   Asp Asp Phe Ala Thr Tyr Tyr Cys Gln Gln Tyr Ile Ser Tyr Ser Arg
                   85                  90                  95
   Thr Phe Gly Gln Gly Thr Lys Val Glu Ile Lys
               100                 105
<210> 405
   <211> 360
   <212> DNA
   <213> Artificial Sequence
<220>
   <223> Synthetic
<400> 405
   gaggtgcagc tggtggagtc tgggggaggc ctggtcaagc ctggggggtc cctgagactc 60
   tcctgtgcag cctctggatt caccttcagt acttataaca tgaactgggt ccgccaggct 120
   ccagggaagg ggctggagtg ggtctcatcc attaggagta gtagtaatta catatactac 180
   gcagactcag tgaagggccg attcaccatc tccagagaca acgccaagaa ctcactgtat 240
   ctgcaaatga acagcctgag agccgaggac acggctgtgt attactgtgc gagagatggc 300
   agcagttggt acgactactc tgactactgg ggccaaggaa ccctggtcac cgtctcctca 360
<210> 406
   <211> 120
   <212> PRT
   <213> Artificial Sequence
<220>
   <223> Synthetic
<400> 406
   Glu Val Gln Leu Val Glu Ser Gly Gly Gly Leu Val Lys Pro Gly Gly
    1               5                   10                  15
   Ser Leu Arg Leu Ser Cys Ala Ala Ser Gly Phe Thr Phe Ser Thr Tyr
               20                  25                  30
   Asn Met Asn Trp Val Arg Gln Ala Pro Gly Lys Gly Leu Glu Trp Val
           35                  40                  45
   Ser Ser Ile Arg Ser Ser Ser Asn Tyr Ile Tyr Tyr Ala Asp Ser Val
       50                  55                  60
   Lys Gly Arg Phe Thr Ile Ser Arg Asp Asn Ala Lys Asn Ser Leu Tyr
   65                  70                  75                      80
   Leu Gln Met Asn Ser Leu Arg Ala Glu Asp Thr Ala Val Tyr Tyr Cys
                   85                  90                  95
   Ala Arg Asp Gly Ser Ser Trp Tyr Asp Tyr Ser Asp Tyr Trp Gly Gln
               100                 105                 110
   Gly Thr Leu Val Thr Val Ser Ser
           115                 120
<210> 407
   <211> 321
   <212> DNA
   <213> Artificial Sequence
<220>
   <223> Synthetic
<400> 407
   gacatccaga tgacccagtc tccttccacc ctgtctgcat ctgtaggaga cagagtcacc 60
   atcacttgcc gggccagtca gagtattagt agctggttgg cctggtatca gcagaaacca 120
   gggaaagccc ctaagctcct gatctataag gcgtctagtt tagaaagtgg ggtcccatca 180
   aggttcagcg gcagtggatc tgggacagaa ttcactctca ccatcagcag cctgcagcct 240
   gatgattttg caacttatta ctgccaacag tatattagtt attctcggac gttcggccaa 300
   gggaccaagg tggaaatcaa a                                           321
<210> 408
   <211> 107
   <212> PRT
   <213> Artificial Sequence
<220>
   <223> Synthetic
<400> 408
   Asp Ile Gln Met Thr Gln Ser Pro Ser Thr Leu Ser Ala Ser Val Gly
    1               5                  10                  15
   Asp Arg Val Thr Ile Thr Cys Arg Ala Ser Gln Ser Ile Ser Ser Trp
               20                  25                  30
   Leu Ala Trp Tyr Gln Gln Lys Pro Gly Lys Ala Pro Lys Leu Leu Ile
           35                  40                  45
   Tyr Lys Ala Ser Ser Leu Glu Ser Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Asp Asp Phe Ala Thr Tyr Tyr Cys Gln Gln Tyr Ile Ser Tyr Ser Arg
                   85                  90                  95
   Thr Phe Gly Gln Gly Thr Lys Val Glu Ile Lys
                       100                 105
<210> 409
   <211> 360
   <212> DNA
   <213> Artificial Sequence
<220>
   <223> Synthetic
<400> 409
   gaggtgcagc tggtggagtc tgggggaggc ctggtcaagc cgggggggtc cctgagactc 60
   tcctgtacag cctctggatt caccttcagt acttataaca tgaattgggt ccgccaggct 120
   ccagggaagg gactggagtg ggtctcatcc attaggagta gtagtaatta catatactac 180
   gcagactcag tgaagggccg attcaccatc tccagagaca acgccaagaa ttcactgtat 240
   ctgcaaatga acagcctgag agccgatgac acggctgtgt attactgtgc gagagatggc 300
   agcagttggt acgactactc tgactactgg ggccagggaa ccctggtcac cgtctcctca 360
<210> 410
   <211> 120
   <212> PRT
   <213> Artificial Sequence
<220>
   <223> Synthetic
<400> 410
   Glu Val Gln Leu Val Glu Ser Gly Gly Gly Leu Val Lys Pro Gly Gly
    1               5                  10                  15
   Ser Leu Arg Leu Ser Cys Thr Ala Ser Gly Phe Thr Phe Ser Thr Tyr
               20                  25                  30
   Asn Met Asn Trp Val Arg Gln Ala Pro Gly Lys Gly Leu Glu Trp Val
           35                  40                  45
   Ser Ser Ile Arg Ser Ser Ser Asn Tyr Ile Tyr Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Asp Asp Thr Ala Val Tyr Tyr Cys
                   85                  90                  95
   Ala Arg Asp Gly Ser Ser Trp Tyr Asp Tyr Ser Asp Tyr Trp Gly Gln
               100                 105                 110
   Gly Thr Leu Val Thr Val Ser Ser
           115                 120
<210> 411
   <211> 24
   <212> DNA
   <213> Artificial Sequence
<220>
   <223> Synthetic
<400> 411
   ggattcacct tcagtactta taac 24
<210> 412
   <211> 8
   <212> PRT
   <213> Artificial Sequence
<220>
   <223> Synthetic
<400> 412
   Gly Phe Thr Phe Ser Thr Tyr Asn
    1               5
<210> 413
   <211> 24
   <212> DNA
   <213> Artificial Sequence
<220>
   <223> Synthetic
<400> 413
   attaggagta gtagtaatta cata 24
<210> 414
   <211> 8
   <212> PRT
   <213> Artificial Sequence
<220>
   <223> Synthetic
<400> 414
   Ile Arg Ser Ser Ser Asn Tyr Ile
   1               5
<210> 415
   <211> 39
   <212> DNA
   <213> Artificial Sequence
<220>
   <223> Synthetic
<400> 415
   gcgagagatg gcagcagttg gtacgactac tctgactac 39
<210> 416
   <211> 13
   <212> PRT
   <213> Artificial Sequence
<220>
   <223> Synthetic
<400> 416
   Ala Arg Asp Gly Ser Ser Trp Tyr Asp Tyr Ser Asp Tyr
   1               5                   10
<210> 417
   <211> 321
   <212> DNA
   <213> Artificial Sequence
<220>
   <223> Synthetic
<400> 417
   gacatccaga tgacccagtc tccttccacc ctgtctgcat ctgtaggaga cagagtcacc 60
   atcacttgcc gggccagtca gagtattagt agctggttgg cctggtatca acagatacca 120
   gggaaagccc ctaaactcct gatctataag gcgtctagtt tagaaaatgg ggtcccatca 180
   aggttcagcg gcagtggatc tgggacagaa ttcactctca tcatcagcag cctgcagcct 240
   gatgattttg caacttatta ctgccaacag tatattagtt attctcggac gttcggccaa 300
   gggaccaagg tggaaatcaa a                                           321
<210> 418
   <211> 107
   <212> PRT
   <213> Artificial Sequence
<220>
   <223> Synthetic
<400> 418
   Asp Ile Gln Met Thr Gln Ser Pro Ser Thr Leu Ser Ala Ser Val Gly
    1               5                  10                  15
   Asp Arg Val Thr Ile Thr Cys Arg Ala Ser Gln Ser Ile Ser Ser Trp
               20                  25                  30
   Leu Ala Trp Tyr Gln Gln Ile Pro Gly Lys Ala Pro Lys Leu Leu Ile
           35                  40                  45
   Tyr Lys Ala Ser Ser Leu Glu Asn Gly Val Pro Ser Arg Phe Ser Gly
       50                  55                  60
   Ser Gly Ser Gly Thr Glu Phe Thr Leu Ile Ile Ser Ser Leu Gln Pro
   65                  70                  75                  80
   Asp Asp Phe Ala Thr Tyr Tyr Cys Gln Gln Tyr Ile Ser Tyr Ser Arg
                   85                  90                  95
   Thr Phe Gly Gln Gly Thr Lys Val Glu Ile Lys
               100                 105
<210> 419
   <211> 18
   <212> DNA
   <213> Artificial Sequence
<220>
   <223> Synthetic
<400> 419
   cagagtatta gtagctgg 18
<210> 420
   <211> 6
   <212> PRT
   <213> Artificial Sequence
<220>
   <223> Synthetic
<400> 420
   Gln Ser Ile Ser Ser Trp
    1               5
<210> 421
   <211> 9
   <212> DNA
   <213> Artificial Sequence
<220>
   <223> Synthetic
<400> 421
   aaggcgtct 9
<210> 422
   <211> 3
   <212> PRT
   <213> Artificial Sequence
<220>
   <223> Synthetic
<400> 422
   Lys Ala Ser
   1
<210> 423
   <211> 27
   <212> DNA
   <213> Artificial Sequence
<220>
   <223> Synthetic
<400> 423
   caacagtata ttagttattc tcggacg 27
<210> 424
   <211> 9
   <212> PRT
   <213> Artificial Sequence
<220>
   <223> Synthetic
<400> 424
   Gln Gln Tyr Ile Ser Tyr Ser Arg Thr
   1 5
<210> 425
   <211> 360
   <212> DNA
   <213> Artificial Sequence
<220>
   <223> Synthetic
<400> 425
   gaggtgcagc tggtggagtc tgggggaggc ctggtcaagc cgggggggtc cctgagactc 60
   tcctgtacag cctctggatt caccttcagt acttataaca tgaattgggt ccgccaggct 120
   ccagggaagg gactggagtg ggtctcatcc attaggagta gtagtaatta catatactac 180
   gcagactcag tgaagggccg attcaccatc tccagagaca acgccaagaa ttcactgtat 240
   ctgcaaatga acagcctgag agccgatgac acggctgtgt attactgtgc gagagatggc 300
   agcagttggt acgactactc tgactactgg ggccagggaa ccctggtcac cgtctcctca 360
<210> 426
   <211> 120
   <212> PRT
   <213> Artificial Sequence
<220>
   <223> Synthetic
<400> 426
   Glu Val Gln Leu Val Glu Ser Gly Gly Gly Leu Val Lys Pro Gly Gly
    1               5                  10                  15
   Ser Leu Arg Leu Ser Cys Thr Ala Ser Gly Phe Thr Phe Ser Thr Tyr
               20                  25                  30
   Asn Met Asn Trp Val Arg Gln Ala Pro Gly Lys Gly Leu Glu Trp Val
           35                  40                  45
   Ser Ser Ile Arg Ser Ser Ser Asn Tyr Ile Tyr Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Asp Asp Thr Ala Val Tyr Tyr Cys
                   85                  90                  95
   Ala Arg Asp Gly Ser Ser Trp Tyr Asp Tyr Ser Asp Tyr Trp Gly Gln
               100                 105                 110
   Gly Thr Leu Val Thr Val Ser Ser
           115                 120
<210> 427
   <211> 321
   <212> DNA
   <213> Artificial Sequence
<220>
   <223> Synthetic
<400> 427
   gacatccaga tgacccagtc tccttccacc ctgtctgcat ctgtaggaga cagagtcacc 60
   atcacttgcc gggccagtca gagtattagt agctggttgg cctggtatca acagatacca 120
   gggaaagccc ctaaactcct gatctataag gcgtctagtt tagaaaatgg ggtcccatca 180
   aggttcagcg gcagtggatc tgggacagaa ttcactctca tcatcagcag cctgcagcct 240
   gatgattttg caacttatta ctgccaacag tatattagtt attctcggac gttcggccaa 300
   gggaccaagg tggaaatcaa a                                           321
<210> 428
   <211> 107
   <212> PRT
   <213> Artificial Sequence
<220>
   <223> Synthetic
<400> 428
   Asp Ile Gln Met Thr Gln Ser Pro Ser Thr Leu Ser Ala Ser Val Gly
    1               5                  10                  15
   Asp Arg Val Thr Ile Thr Cys Arg Ala Ser Gln Ser Ile Ser Ser Trp
               20                  25                  30
   Leu Ala Trp Tyr Gln Gln Ile Pro Gly Lys Ala Pro Lys Leu Leu Ile
           35                  40                  45
   Tyr Lys Ala Ser Ser Leu Glu Asn Gly Val Pro Ser Arg Phe Ser Gly
       50                  55                  60
   Ser Gly Ser Gly Thr Glu Phe Thr Leu Ile Ile Ser Ser Leu Gln Pro
   65                  70                  75                  80
   Asp Asp Phe Ala Thr Tyr Tyr Cys Gln Gln Tyr Ile Ser Tyr Ser Arg
                   85                  90                  95
   Thr Phe Gly Gln Gly Thr Lys Val Glu Ile Lys
               100                 105
<210> 429
   <211> 360
   <212> DNA
   <213> Artificial Sequence
<220>
   <223> Synthetic
<400> 429
   gaggtgcagc tggtggagtc tgggggaggc ctggtcaagc ctggggggtc cctgagactc 60
   tcctgtgcag cctctggatt caccttcagt acttataaca tgaactgggt ccgccaggct 120
   ccagggaagg ggctggagtg ggtctcatcc attaggagta gtagtaatta catatactac 180
   gcagactcag tgaagggccg attcaccatc tccagagaca acgccaagaa ctcactgtat 240
   ctgcaaatga acagcctgag agccgaggac acggctgtgt attactgtgc gagagatggc 300
   agcagttggt acgactactc tgactactgg ggccaaggaa ccctggtcac cgtctcctca 360
<210> 430
   <211> 120
   <212> PRT
   <213> Artificial Sequence
<220>
   <223> Synthetic
<400> 430
   Glu Val Gln Leu Val Glu Ser Gly Gly Gly Leu Val Lys Pro Gly Gly
    1               5                  10                  15
   Ser Leu Arg Leu Ser Cys Ala Ala Ser Gly Phe Thr Phe Ser Thr Tyr
               20                  25                  30
   Asn Met Asn Trp Val Arg Gln Ala Pro Gly Lys Gly Leu Glu Trp Val
           35                  40                  45
   Ser Ser Ile Arg Ser Ser Ser Asn Tyr Ile Tyr Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Arg Asp Gly Ser Ser Trp Tyr Asp Tyr Ser Asp Tyr Trp Gly Gln
               100                 105                 110
   Gly Thr Leu Val Thr Val Ser Ser
           115                 120
<210> 431
   <211> 321
   <212> DNA
   <213> Artificial Sequence
<220>
   <223> Synthetic
<400> 431
   gacatccaga tgacccagtc tccttccacc ctgtctgcat ctgtaggaga cagagtcacc 60
   atcacttgcc gggccagtca gagtattagt agctggttgg cctggtatca gcagaaacca 120
   gggaaagccc ctaagctcct gatctataag gcgtctagtt tagaaagtgg ggtcccatca 180
   aggttcagcg gcagtggatc tgggacagaa ttcactctca ccatcagcag cctgcagcct 240
   gatgattttg caacttatta ctgccaacag tatattagtt attctcggac gttcggccaa 300
   gggaccaagg tggaaatcaa a                                           321
<210> 432
   <211> 107
   <212> PRT
   <213> Artificial Sequence
<220>
   <223> Synthetic
<400> 432
   Asp Ile Gln Met Thr Gln Ser Pro Ser Thr Leu Ser Ala Ser Val Gly
    1              5                   10                  15
   Asp Arg Val Thr Ile Thr Cys Arg Ala Ser Gln Ser Ile Ser Ser Trp
               20                  25                  30
   Leu Ala Trp Tyr Gln Gln Lys Pro Gly Lys Ala Pro Lys Leu Leu Ile
           35                  40                  45
   Tyr Lys Ala Ser Ser Leu Glu Ser Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                      80
   Asp Asp Phe Ala Thr Tyr Tyr Cys Gln Gln Tyr Ile Ser Tyr Ser Arg
                   85                  90                      95
   Thr Phe Gly Gln Gly Thr Lys Val Glu Ile Lys
               100                 105
<210> 433
   <211> 360
   <212> DNA
   <213> Artificial Sequence
<220>
   <223> Synthetic
<400> 433
   gaggtgcagc tggtggagtc tgggggaggc ctggtcaagc cgggggggtc cctgagactc 60
   tcctgtacag cctctggatt caccttcagt acttataaca tgaattgggt ccgccaggct 120
   ccagggaagg gactggagtg ggtctcatcc attaggagta gtagtaatta catatactac 180
   gcagactcag tgaagggccg attcaccatc tccagagaca acgccaagag ttcactgtat 240
   ctgcaaatga acagcctgag agccgaggac acggctgtgt attactgtgc gagagatggc 300
   agcagttggt acgactactc tgactactgg ggccagggaa ccctggtcac cgtctcctca 360
<210> 434
   <211> 120
   <212> PRT
   <213> Artificial Sequence
<220>
   <223> Synthetic
<400> 434
   Glu Val Gln Leu Val Glu Ser Gly Gly Gly Leu Val Lys Pro Gly Gly
    1               5                  10                  15
   Ser Leu Arg Leu Ser Cys Thr Ala Ser Gly Phe Thr Phe Ser Thr Tyr
               20                  25                  30
   Asn Met Asn Trp Val Arg Gln Ala Pro Gly Lys Gly Leu Glu Trp Val
           35                  40                  45
   Ser Ser Ile Arg Ser Ser Ser Asn Tyr Ile Tyr Tyr Ala Asp Ser Val
       50                  55                  60
   Lys Gly Arg Phe Thr Ile Ser Arg Asp Asn Ala Lys Ser Ser Leu Tyr
   65                  70                  75                  80
   Leu Gln Met Asn Ser Leu Arg Ala Glu Asp Thr Ala Val Tyr Tyr Cys
                   85                  90                  95
   Ala Arg Asp Gly Ser Ser Trp Tyr Asp Tyr Ser Asp Tyr Trp Gly Gln
               100                 105                 110
   Gly Thr Leu Val Thr Val Ser Ser
           115                 120
<210> 435
   <211> 24
   <212> DNA
   <213> Artificial Sequence
<220>
   <223> Synthetic
<400> 435
   ggattcacct tcagtactta taac 24
<210> 436
   <211> 8
   <212> PRT
   <213> Artificial Sequence
<220>
   <223> Synthetic
<400> 436
   Gly Phe Thr Phe Ser Thr Tyr Asn
    1               5
<210> 437
   <211> 24
   <212> DNA
   <213> Artificial Sequence
<220>
   <223> Synthetic
<400> 437
   attaggagta gtagtaatta cata 24
<210> 438
   <211> 8
   <212> PRT
   <213> Artificial Sequence
<220>
   <223> Synthetic
<400> 438
   Ile Arg Ser Ser Ser Asn Tyr Ile
   1               5
<210> 439
   <211> 39
   <212> DNA
   <213> Artificial Sequence
<220>
   <223> Synthetic
<400> 439
   gcgagagatg gcagcagttg gtacgactac tctgactac 39
<210> 440
   <211> 13
   <212> PRT
   <213> Artificial Sequence
<220>
   <223> Synthetic
<400> 440
   Ala Arg Asp Gly Ser Ser Trp Tyr Asp Tyr Ser Asp Tyr
   1               5                   10
<210> 441
   <211> 321
   <212> DNA
   <213> Artificial Sequence
<220>
   <223> Synthetic
<400> 441
   gacatccaga tgacccagtc tccttccacc ctgtctgcat ctgtaggaga cagagtcacc 60
   atcacttgcc gggccagtca gagtattagt agctggttgg cctggtatca acaggtacca 120
   gggaaagccc ctaaactcct gatctataag gcgtctagtt tagaaaatgg ggtcccatca 180
   aggttcagcg gcagtggatc tgggacagaa ttcactctca tcatcagcag cctgcagcct 240
   gatgattttg caacttatta ctgccaacag tatattagtt attctcggac gttcggccaa 300
   gggaccaagg tggaaatcaa a                                           321
<210> 442
   <211> 107
   <212> PRT
   <213> Artificial Sequence
<220>
   <223> Synthetic
<400> 442
   Asp Ile Gln Met Thr Gln Ser Pro Ser Thr Leu Ser Ala Ser Val Gly
    1               5                  10                  15
   Asp Arg Val Thr Ile Thr Cys Arg Ala Ser Gln Ser Ile Ser Ser Trp
               20                  25                  30
   Leu Ala Trp Tyr Gln Gln Val Pro Gly Lys Ala Pro Lys Leu Leu Ile
           35                  40                  45
   Tyr Lys Ala Ser Ser Leu Glu Asn Gly Val Pro Ser Arg Phe Ser Gly
       50                  55                  60
   Ser Gly Ser Gly Thr Glu Phe Thr Leu Ile Ile Ser Ser Leu Gln Pro
   65                  70                  75                  80
   Asp Asp Phe Ala Thr Tyr Tyr Cys Gln Gln Tyr Ile Ser Tyr Ser Arg
                   85                  90                  95
   Thr Phe Gly Gln Gly Thr Lys Val Glu Ile Lys
               100                 105
<210> 443
   <211> 18
   <212> DNA
   <213> Artificial Sequence
<220>
   <223> Synthetic
<400> 443
   cagagtatta gtagctgg 18
<210> 444
   <211> 6
   <212> PRT
   <213> Artificial Sequence
<220>
   <223> Synthetic
<400> 444
   Gln Ser Ile Ser Ser Trp
    1               5
<210> 445
   <211> 9
   <212> DNA
   <213> Artificial Sequence
<220>
   <223> Synthetic
<400> 445
   aaggcgtct 9
<210> 446
   <211> 3
   <212> PRT
   <213> Artificial Sequence
<220>
   <223> Synthetic
<400> 446
   Lys Ala Ser
   1
<210> 447
   <211> 27
   <212> DNA
   <213> Artificial Sequence
<220>
   <223> Synthetic
<400> 447
   caacagtata ttagttattc tcggacg 27
<210> 448
   <211> 9
   <212> PRT
   <213> Artificial Sequence
<220>
   <223> Synthetic
<400> 448
   Gln Gln Tyr Ile Ser Tyr Ser Arg Thr
   1 5
<210> 449
   <211> 360
   <212> DNA
   <213> Artificial Sequence
<220>
   <223> Synthetic
<400> 449
   gaggtgcagc tggtggagtc tgggggaggc ctggtcaagc cgggggggtc cctgagactc 60
   tcctgtacag cctctggatt caccttcagt acttataaca tgaattgggt ccgccaggct 120
   ccagggaagg gactggagtg ggtctcatcc attaggagta gtagtaatta catatactac 180
   gcagactcag tgaagggccg attcaccatc tccagagaca acgccaagag ttcactgtat 240
   ctgcaaatga acagcctgag agccgaggac acggctgtgt attactgtgc gagagatggc 300
   agcagttggt acgactactc tgactactgg ggccagggaa ccctggtcac cgtctcctca 360
<210> 450
   <211> 120
   <212> PRT
   <213> Artificial Sequence
<220>
   <223> Synthetic
<400> 450
   Glu Val Gln Leu Val Glu Ser Gly Gly Gly Leu Val Lys Pro Gly Gly
    1               5                  10                  15
   Ser Leu Arg Leu Ser Cys Thr Ala Ser Gly Phe Thr Phe Ser Thr Tyr
               20                  25                  30
   Asn Met Asn Trp Val Arg Gln Ala Pro Gly Lys Gly Leu Glu Trp Val
           35                  40                  45
   Ser Ser Ile Arg Ser Ser Ser Asn Tyr Ile Tyr Tyr Ala Asp Ser Val
       50                  55                  60
   Lys Gly Arg Phe Thr Ile Ser Arg Asp Asn Ala Lys Ser Ser Leu Tyr
   65                  70                  75                  80
   Leu Gln Met Asn Ser Leu Arg Ala Glu Asp Thr Ala Val Tyr Tyr Cys
                   85                  90                  95
   Ala Arg Asp Gly Ser Ser Trp Tyr Asp Tyr Ser Asp Tyr Trp Gly Gln
               100                 105                 110
   Gly Thr Leu Val Thr Val Ser Ser
           115                 120
<210> 451
   <211> 321
   <212> DNA
   <213> Artificial Sequence
<220>
   <223> Synthetic
<400> 451
   gacatccaga tgacccagtc tccttccacc ctgtctgcat ctgtaggaga cagagtcacc 60
   atcacttgcc gggccagtca gagtattagt agctggttgg cctggtatca acaggtacca 120
   gggaaagccc ctaaactcct gatctataag gcgtctagtt tagaaaatgg ggtcccatca 180
   aggttcagcg gcagtggatc tgggacagaa ttcactctca tcatcagcag cctgcagcct 240
   gatgattttg caacttatta ctgccaacag tatattagtt attctcggac gttcggccaa 300
   gggaccaagg tggaaatcaa a                                           321
<210> 452
   <211> 107
   <212> PRT
   <213> Artificial Sequence
<220>
   <223> Synthetic
<400> 452
   Asp Ile Gln Met Thr Gln Ser Pro Ser Thr Leu Ser Ala Ser Val Gly
    1               5                  10                  15
   Asp Arg Val Thr Ile Thr Cys Arg Ala Ser Gln Ser Ile Ser Ser Trp
               20                  25                  30
   Leu Ala Trp Tyr Gln Gln Val Pro Gly Lys Ala Pro Lys Leu Leu Ile
           35                  40                  45
   Tyr Lys Ala Ser Ser Leu Glu Asn Gly Val Pro Ser Arg Phe Ser Gly
       50                  55                  60
   Ser Gly Ser Gly Thr Glu Phe Thr Leu Ile Ile Ser Ser Leu Gln Pro
   65                  70                  75                  80
   Asp Asp Phe Ala Thr Tyr Tyr Cys Gln Gln Tyr Ile Ser Tyr Ser Arg
                   85                  90                  95
   Thr Phe Gly Gln Gly Thr Lys Val Glu Ile Lys
               100                 105
<210> 453
   <211> 360
   <212> DNA
   <213> Artificial Sequence
<220>
   <223> Synthetic
<400> 453
   gaggtgcagc tggtggagtc tgggggaggc ctggtcaagc ctggggggtc cctgagactc 60
   tcctgtgcag cctctggatt caccttcagt acttataaca tgaactgggt ccgccaggct 120
   ccagggaagg ggctggagtg ggtctcatcc attaggagta gtagtaatta catatactac 180
   gcagactcag tgaagggccg attcaccatc tccagagaca acgccaagaa ctcactgtat 240
   ctgcaaatga acagcctgag agccgaggac acggctgtgt attactgtgc gagagatggc 300
   agcagttggt acgactactc tgactactgg ggccagggaa ccctggtcac cgtctcctca 360
<210> 454
   <211> 120
   <212> PRT
   <213> Artificial Sequence
<220>
   <223> Synthetic
<400> 454
   Glu Val Gln Leu Val Glu Ser Gly Gly Gly Leu Val Lys Pro Gly Gly
    1               5                  10                  15
   Ser Leu Arg Leu Ser Cys Ala Ala Ser Gly Phe Thr Phe Ser Thr Tyr
               20                  25                  30
   Asn Met Asn Trp Val Arg Gln Ala Pro Gly Lys Gly Leu Glu Trp Val
           35                  40                  45
   Ser Ser Ile Arg Ser Ser Ser Asn Tyr Ile Tyr Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Arg Asp Gly Ser Ser Trp Tyr Asp Tyr Ser Asp Tyr Trp Gly Gln
               100                 105                 110
   Gly Thr Leu Val Thr Val Ser Ser
           115                 120
<210> 455
   <211> 321
   <212> DNA
   <213> Artificial Sequence
<220>
   <223> Synthetic
<400> 455
   gacatccaga tgacccagtc tccttccacc ctgtctgcat ctgtaggaga cagagtcacc 60
   atcacttgcc gggccagtca gagtattagt agctggttgg cctggtatca gcagaaacca 120
   gggaaagccc ctaagctcct gatctataag gcgtctagtt tagaaagtgg ggtcccatca 180
   aggttcagcg gcagtggatc tgggacagaa ttcactctca ccatcagcag cctgcagcct 240
   gatgattttg caacttatta ctgccaacag tatattagtt attctcggac gttcggccaa 300
   gggaccaagg tggaaatcaa a                                           321
<210> 456
   <211> 107
   <212> PRT
   <213> Artificial Sequence
<220>
   <223> Synthetic
<400> 456
   Asp Ile Gln Met Thr Gln Ser Pro Ser Thr Leu Ser Ala Ser Val Gly
    1               5                  10                  15
   Asp Arg Val Thr Ile Thr Cys Arg Ala Ser Gln Ser Ile Ser Ser Trp
               20                  25                  30
   Leu Ala Trp Tyr Gln Gln Lys Pro Gly Lys Ala Pro Lys Leu Leu Ile
           35                  40                  45
   Tyr Lys Ala Ser Ser Leu Glu Ser Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Asp Asp Phe Ala Thr Tyr Tyr Cys Gln Gln Tyr Ile Ser Tyr Ser Arg
                   85                  90                  95
   Thr Phe Gly Gln Gly Thr Lys Val Glu Ile Lys
               100                 105
<210> 457
   <211> 360
   <212> DNA
   <213> Artificial Sequence
<220>
   <223> Synthetic
<400> 457
   gaggtgcagc tggtggagtc tgggggaggc ctggtcaagc cgggggggtc cctgagactc 60
   tcctgtacag cctctggatt caccttcagt acttataaca tgaattgggt ccgccaggct 120
   ccagggaagg gactggagtg ggtctcatcc attaggagta gtagtaatta catatactac 180
   gcagactcag tgaagggccg attcaccatc tccagagaca acgccaagaa ttcactgtat 240
   ctgcaaatga acagcctgag agccgatgac acggctgtgt attactgtgc gagagatggc 300
   agcagttggt acgactactc tgactactgg ggccagggaa ccctggtcac cgtctcctca 360
<210> 458
   <211> 120
   <212> PRT
   <213> Artificial Sequence
<220>
   <223> Synthetic
<400> 458
   Glu Val Gln Leu Val Glu Ser Gly Gly Gly Leu Val Lys Pro Gly Gly
    1               5                  10                  15
   Ser Leu Arg Leu Ser Cys Thr Ala Ser Gly Phe Thr Phe Ser Thr Tyr
               20                  25                  30
   Asn Met Asn Trp Val Arg Gln Ala Pro Gly Lys Gly Leu Glu Trp Val
           35                  40                  45
   Ser Ser Ile Arg Ser Ser Ser Asn Tyr Ile Tyr Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Asp Asp Thr Ala Val Tyr Tyr Cys
                   85                  90                  95
   Ala Arg Asp Gly Ser Ser Trp Tyr Asp Tyr Ser Asp Tyr Trp Gly Gln
                      100                 105                 110
   Gly Thr Leu Val Thr Val Ser Ser
               115                 120
<210> 459
   <211> 24
   <212> DNA
   <213> Artificial Sequence
<220>
   <223> Synthetic
<400> 459
   ggattcacct tcagtactta taac 24
<210> 460
   <211> 8
   <212> PRT
   <213> Artificial Sequence
<220>
   <223> Synthetic
<400> 460
   Gly Phe Thr Phe Ser Thr Tyr Asn
    1               5
<210> 461
   <211> 24
   <212> DNA
   <213> Artificial Sequence
<220>
   <223> Synthetic
<400> 461
   attaggagta gtagtaatta cata 24
<210> 462
   <211> 8
   <212> PRT
   <213> Artificial Sequence
<220>
   <223> Synthetic
<400> 462
   Ile Arg Ser Ser Ser Asn Tyr Ile
    1               5
<210> 463
   <211> 39
   <212> DNA
   <213> Artificial Sequence
<220>
   <223> Synthetic
<400> 463
   gcgagagatg gcagcagttg gtacgactac tctgactac 39
<210> 464
   <211> 13
   <212> PRT
   <213> Artificial Sequence
<220>
   <223> Synthetic
<400> 464
   Ala Arg Asp Gly Ser Ser Trp Tyr Asp Tyr Ser Asp Tyr
   1 5 10
<210> 465
   <211> 321
   <212> DNA
   <213> Artificial Sequence
<220>
   <223> Synthetic
<400> 465
   gacatccaga tgacccagtc tccttccacc ctgtctgcat ctgtaggaga cagagtcacc 60
   atcacttgcc gggccagtca gagtattagt agctggttgg cctggtatca acagatacca 120
   gggaaagccc ctaaactcct gatctataag gcgtctagtt tagaaaatgg ggtcccatca 180
   aggttcagcg gcagtggatc tgggacagaa ttcactctca tcatcagcag cctgcagcct 240
   gatgattttg caacttatta ctgccaacag tatattagtt attctcggac gttcggccaa 300
   gggaccaagg tggaaatcaa a 321
<210> 466
   <211> 107
   <212> PRT
   <213> Artificial Sequence
<220>
   <223> Synthetic
<400> 466
   Asp Ile Gln Met Thr Gln Ser Pro Ser Thr Leu Ser Ala Ser Val Gly
    1               5                  10                  15
   Asp Arg Val Thr Ile Thr Cys Arg Ala Ser Gln Ser Ile Ser Ser Trp
               20                  25                  30
   Leu Ala Trp Tyr Gln Gln Ile Pro Gly Lys Ala Pro Lys Leu Leu Ile
           35                  40                  45
   Tyr Lys Ala Ser Ser Leu Glu Asn Gly Val Pro Ser Arg Phe Ser Gly
       50                  55                  60
   Ser Gly Ser Gly Thr Glu Phe Thr Leu Ile Ile Ser Ser Leu Gln Pro
   65                  70                  75                  80
   Asp Asp Phe Ala Thr Tyr Tyr Cys Gln Gln Tyr Ile Ser Tyr Ser Arg
                   85                  90                  95
   Thr Phe Gly Gln Gly Thr Lys Val Glu Ile Lys
               100                 105
<210> 467
   <211> 18
   <212> DNA
   <213> Artificial Sequence
<220>
   <223> Synthetic
<400> 467
   cagagtatta gtagctgg 18
<210> 468
   <211> 6
   <212> PRT
   <213> Artificial Sequence
<220>
   <223> Synthetic
<400> 468
   Gln Ser Ile Ser Ser Trp
    1               5
<210> 469
   <211> 9
   <212> DNA
   <213> Artificial Sequence
<220>
   <223> Synthetic
<400> 469
   aaggcgtct 9
<210> 470
   <211> 3
   <212> PRT
   <213> Artificial Sequence
<220>
   <223> Synthetic
<400> 470
   Lys Ala Ser
   1
<210> 471
   <211> 27
   <212> DNA
   <213> Artificial Sequence
<220>
   <223> Synthetic
<400> 471
   caacagtata ttagttattc tcggacg 27
<210> 472
   <211> 9
   <212> PRT
   <213> Artificial Sequence
<220>
   <223> Synthetic
<400> 472
   Gln Gln Tyr Ile Ser Tyr Ser Arg Thr
   1 5
<210> 473
   <211> 360
   <212> DNA
   <213> Artificial Sequence
<220>
   <223> Synthetic
<400> 473
   gaggtgcagc tggtggagtc tgggggaggc ctggtcaagc cgggggggtc cctgagactc 60
   tcctgtacag cctctggatt caccttcagt acttataaca tgaattgggt ccgccaggct 120
   ccagggaagg gactggagtg ggtctcatcc attaggagta gtagtaatta catatactac 180
   gcagactcag tgaagggccg attcaccatc tccagagaca acgccaagaa ttcactgtat 240
   ctgcaaatga acagcctgag agccgatgac acggctgtgt attactgtgc gagagatggc 300
   agcagttggt acgactactc tgactactgg ggccagggaa ccctggtcac cgtctcctca 360
<210> 474
   <211> 120
   <212> PRT
   <213> Artificial Sequence
<220>
   <223> Synthetic
<400> 474
   Glu Val Gln Leu Val Glu Ser Gly Gly Gly Leu Val Lys Pro Gly Gly
    1              5                   10                  15
   Ser Leu Arg Leu Ser Cys Thr Ala Ser Gly Phe Thr Phe Ser Thr Tyr
               20                  25                  30
   Asn Met Asn Trp Val Arg Gln Ala Pro Gly Lys Gly Leu Glu Trp Val
           35                  40                  45
   Ser Ser Ile Arg Ser Ser Ser Asn Tyr Ile Tyr Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Asp Asp Thr Ala Val Tyr Tyr Cys
                   85                  90                  95
   Ala Arg Asp Gly Ser Ser Trp Tyr Asp Tyr Ser Asp Tyr Trp Gly Gln
               100                 105                 110
   Gly Thr Leu Val Thr Val Ser Ser
           115                 120
<210> 475
   <211> 321
   <212> DNA
   <213> Artificial Sequence
<220>
   <223> Synthetic
<400> 475
   gacatccaga tgacccagtc tccttccacc ctgtctgcat ctgtaggaga cagagtcacc 60
   atcacttgcc gggccagtca gagtattagt agctggttgg cctggtatca acagatacca 120
   gggaaagccc ctaaactcct gatctataag gcgtctagtt tagaaaatgg ggtcccatca 180
   aggttcagcg gcagtggatc tgggacagaa ttcactctca tcatcagcag cctgcagcct 240
   gatgattttg caacttatta ctgccaacag tatattagtt attctcggac gttcggccaa 300
   gggaccaagg tggaaatcaa a                                           321
<210> 476
   <211> 107
   <212> PRT
   <213> Artificial Sequence
<220>
   <223> Synthetic
<400> 476
   Asp Ile Gln Met Thr Gln Ser Pro Ser Thr Leu Ser Ala Ser Val Gly
    1               5                  10                  15
   Asp Arg Val Thr Ile Thr Cys Arg Ala Ser Gln Ser Ile Ser Ser Trp
               20                  25                  30
   Leu Ala Trp Tyr Gln Gln Ile Pro Gly Lys Ala Pro Lys Leu Leu Ile
           35                  40                  45
   Tyr Lys Ala Ser Ser Leu Glu Asn Gly Val Pro Ser Arg Phe Ser Gly
       50                  55                  60
   Ser Gly Ser Gly Thr Glu Phe Thr Leu Ile Ile Ser Ser Leu Gln Pro
   65                  70                  75                  80
   Asp Asp Phe Ala Thr Tyr Tyr Cys Gln Gln Tyr Ile Ser Tyr Ser Arg
                   85                  90                  95
   Thr Phe Gly Gln Gly Thr Lys Val Glu Ile Lys
               100                 105
<210> 477
   <211> 360
   <212> DNA
   <213> Artificial Sequence
<220>
   <223> Synthetic
<400> 477
   gaggtgcagc tggtggagtc tgggggaggc ctggtcaagc ctggggggtc cctgagactc 60
   tcctgtgcag cctctggatt caccttcagt acttataaca tgaactgggt ccgccaggct 120
   ccagggaagg ggctggagtg ggtctcatcc attaggagta gtagtaatta catatactac 180
   gcagactcag tgaagggccg attcaccatc tccagagaca acgccaagaa ctcactgtat 240
   ctgcaaatga acagcctgag agccgaggac acggctgtgt attactgtgc gagagatggc 300
   agcagttggt acgactactc tgactactgg ggccaaggaa ccctggtcac cgtctcctca 360
<210> 478
   <211> 120
   <212> PRT
   <213> Artificial Sequence
<220>
   <223> Synthetic
<400> 478
   Glu Val Gln Leu Val Glu Ser Gly Gly Gly Leu Val Lys Pro Gly Gly
    1               5                  10                  15
   Ser Leu Arg Leu Ser Cys Ala Ala Ser Gly Phe Thr Phe Ser Thr Tyr
               20                  25                  30
   Asn Met Asn Trp Val Arg Gln Ala Pro Gly Lys Gly Leu Glu Trp Val
           35                  40                  45
   Ser Ser Ile Arg Ser Ser Ser Asn Tyr Ile Tyr Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Arg Asp Gly Ser Ser Trp Tyr Asp Tyr Ser Asp Tyr Trp Gly Gln
               100                 105                 110
   Gly Thr Leu Val Thr Val Ser Ser
           115                 120
<210> 479
   <211> 321
   <212> DNA
   <213> Artificial Sequence
<220>
   <223> Synthetic
<400> 479
   gacatccaga tgacccagtc tccttccacc ctgtctgcat ctgtaggaga cagagtcacc 60
   atcacttgcc gggccagtca gagtattagt agctggttgg cctggtatca gcagaaacca 120
   gggaaagccc ctaagctcct gatctataag gcgtctagtt tagaaagtgg ggtcccatca 180
   aggttcagcg gcagtggatc tgggacagaa ttcactctca ccatcagcag cctgcagcct 240
   gatgattttg caacttatta ctgccaacag tatattagtt attctcggac gttcggccaa 300
   gggaccaagg tggaaatcaa a                                           321
<210> 480
   <211> 107
   <212> PRT
   <213> Artificial Sequence
<220>
   <223> Synthetic
<400> 480
   Asp Ile Gln Met Thr Gln Ser Pro Ser Thr Leu Ser Ala Ser Val Gly
    1               5                  10                  15
   Asp Arg Val Thr Ile Thr Cys Arg Ala Ser Gln Ser Ile Ser Ser Trp
               20                  25                  30
   Leu Ala Trp Tyr Gln Gln Lys Pro Gly Lys Ala Pro Lys Leu Leu Ile
           35                  40                  45
   Tyr Lys Ala Ser Ser Leu Glu Ser Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Asp Asp Phe Ala Thr Tyr Tyr Cys Gln Gln Tyr Ile Ser Tyr Ser Arg
                   85                  90                  95
   Thr Phe Gly Gln Gly Thr Lys Val Glu Ile Lys
               100                 105
<210> 481
   <211> 354
   <212> DNA
   <213> Artificial Sequence
<220>
   <223> Synthetic
<400> 481
   gaggtgcaac tagtggagtc tgggggaggc ttggtacagc ctggggggtc cctgagactc 60
   tcctgtgtag tctctggatt caccttcggt gactacgaca tgcactgggt ccgtcaagct 120
   acaggaagag gtctggagtg ggtctcaggt attgctcctg ctggtgacac atcctataca 180
   ggctccgtga agggccgatt caccatctcc agagagaatg ccaagaactc cttgcatctt 240
   caaatgaaca gcctgacaac cggggacacg gctatatatt attgtgctag agaggatata 300
   gcagtgcctg gttttgatta ctggggccag ggaaccctgg tcaccgtctc ctca       354
<210> 482
   <211> 118
   <212> PRT
   <213> Artificial Sequence
<220>
   <223> Synthetic
<400> 482
   Glu Val Gln Leu Val Glu Ser Gly Gly Gly Leu Val Gln Pro Gly Gly
    1               5                  10                  15
   Ser Leu Arg Leu Ser Cys Val Val Ser Gly Phe Thr Phe Gly Asp Tyr
               20                  25                  30
   Asp Met His Trp Val Arg Gln Ala Thr Gly Arg Gly Leu Glu Trp Val
           35                  40                  45
   Ser Gly Ile Ala Pro Ala Gly Asp Thr Ser Tyr Thr Gly Ser Val Lys
       50                  55                  60
   Gly Arg Phe Thr Ile Ser Arg Glu Asn Ala Lys Asn Ser Leu His Leu
   65                  70                  75                  80
   Gln Met Asn Ser Leu Thr Thr Gly Asp Thr Ala Ile Tyr Tyr Cys Ala
                   85                  90                  95
   Arg Glu Asp Ile Ala Val Pro Gly Phe Asp Tyr Trp Gly Gln Gly Thr
               100                 105                 110
   Leu Val Thr Val Ser Ser
           115
<210> 483
   <211> 24
   <212> DNA
   <213> Artificial Sequence
<220>
   <223> Synthetic
<400> 483
   ggattcacct tcggtgacta cgac 24
<210> 484
   <211> 8
   <212> PRT
   <213> Artificial Sequence
<220>
   <223> Synthetic
<400> 484
   Gly Phe Thr Phe Gly Asp Tyr Asp
    1               5
<210> 485
   <211> 21
   <212> DNA
   <213> Artificial Sequence
<220>
   <223> Synthetic
<400> 485
   attgctcctg ctggtgacac a 21
<210> 486
   <211> 7
   <212> PRT
   <213> Artificial Sequence
<220>
   <223> Synthetic
<400> 486
   Ile Ala Pro Ala Gly Asp Thr
   1 5
<210> 487
   <211> 36
   <212> DNA
   <213> Artificial Sequence
<220>
   <223> Synthetic
<400> 487
   gctagagagg atatagcagt gcctggtttt gattac 36
<210> 488
   <211> 12
   <212> PRT
   <213> Artificial Sequence
<220>
   <223> Synthetic
<400> 488
   Ala Arg Glu Asp Ile Ala Val Pro Gly Phe Asp Tyr
   1 5 10
<210> 489
   <211> 324
   <212> DNA
   <213> Artificial Sequence
<220>
   <223> Synthetic
<400> 489
   gaaatagtga tgacgcagtc tccagccacc ctgtctgtgt ctccagggga acgaggcacc 60
   ctctcctgca gggccagtca gagtgttagc agcaacttag cctggtacca gcagaaacct 120
   ggccaggctc ccagactcct catctatggt gcatccacga gggccactgg cttcccagcc 180
   aggttcagtg gcagtgggtc tgggacagag ttcactctca ccatcagcag cctgcagtct 240
   gaagattttg cagtttatta ctgtcagcag tataataagt ggcctccgtt cactttcggc 300
   cctgggacca aagtggattt caaa 324
<210> 490
   <211> 108
   <212> PRT
   <213> Artificial Sequence
<220>
   <223> Synthetic
<400> 490
   Glu Ile Val Met Thr Gln Ser Pro Ala Thr Leu Ser Val Ser Pro Gly
    1               5                  10                  15
   Glu Arg Gly Thr Leu Ser Cys Arg Ala Ser Gln Ser Val Ser Ser Asn
               20                  25                  30
   Leu Ala Trp Tyr Gln Gln Lys Pro Gly Gln Ala Pro Arg Leu Leu Ile
           35                  40                  45
   Tyr Gly Ala Ser Thr Arg Ala Thr Gly Phe Pro Ala Arg Phe Ser Gly
       50                  55                  60
   Ser Gly Ser Gly Thr Glu Phe Thr Leu Thr Ile Ser Ser Leu Gln Ser
   65                  70                  75                  80
   Glu Asp Phe Ala Val Tyr Tyr Cys Gln Gln Tyr Asn Lys Trp Pro Pro
                   85                  90                  95
   Phe Thr Phe Gly Pro Gly Thr Lys Val Asp Phe Lys
               100                 105
<210> 491
   <211> 18
   <212> DNA
   <213> Artificial Sequence
<220>
   <223> Synthetic
<400> 491
   cagagtgtta gcagcaac 18
<210> 492
   <211> 6
   <212> PRT
   <213> Artificial Sequence
<220>
   <223> Synthetic
<400> 492
   Gln Ser Val Ser Ser Asn
    1               5
<210> 493
   <211> 9
   <212> DNA
   <213> Artificial Sequence
<220>
   <223> Synthetic
<400> 493
   ggtgcatcc 9
<210> 494
   <211> 3
   <212> PRT
   <213> Artificial Sequence
<220>
   <223> Synthetic
<400> 494
   Gly Ala Ser
   1
<210> 495
   <211> 30
   <212> DNA
   <213> Artificial Sequence
<220>
   <223> Synthetic
<400> 495
   cagcagtata ataagtggcc tccgttcact 30
<210> 496
   <211> 10
   <212> PRT
   <213> Artificial Sequence
<220>
   <223> Synthetic
<400> 496
   Gln Gln Tyr Asn Lys Trp Pro Pro Phe Thr
   1 5 10
<210> 497
   <211> 354
   <212> DNA
   <213> Artificial Sequence
<220>
   <223> Synthetic
<400> 497
   gaggtgcaac tagtggagtc tgggggaggc ttggtacagc ctggggggtc cctgagactc 60
   tcctgtgtag tctctggatt caccttcggt gactacgaca tgcactgggt ccgtcaagct 120
   acaggaagag gtctggagtg ggtctcaggt attgctcctg ctggtgacac atcctataca 180
   ggctccgtga agggccgatt caccatctcc agagagaatg ccaagaactc cttgcatctt 240
   caaatgaaca gcctgacaac cggggacacg gctatatatt attgtgctag agaggatata 300
   gcagtgcctg gttttgatta ctggggccag ggaaccctgg tcaccgtctc ctca 354
<210> 498
   <211> 118
   <212> PRT
   <213> Artificial Sequence
<220>
   <223> Synthetic
<400> 498
   Glu Val Gln Leu Val Glu Ser Gly Gly Gly Leu Val Gln Pro Gly Gly
    1               5                   10                  15
   Ser Leu Arg Leu Ser Cys Val Val Ser Gly Phe Thr Phe Gly Asp Tyr
               20                  25                  30
   Asp Met His Trp Val Arg Gln Ala Thr Gly Arg Gly Leu Glu Trp Val
           35                  40                  45
   Ser Gly Ile Ala Pro Ala Gly Asp Thr Ser Tyr Thr Gly Ser Val Lys
       50                  55                  60
   Gly Arg Phe Thr Ile Ser Arg Glu Asn Ala Lys Asn Ser Leu His Leu
   65                  70                  75                  80
   Gln Met Asn Ser Leu Thr Thr Gly Asp Thr Ala Ile Tyr Tyr Cys Ala
                   85                  90                  95
   Arg Glu Asp Ile Ala Val Pro Gly Phe Asp Tyr Trp Gly Gln Gly Thr
               100                 105                 110
   Leu Val Thr Val Ser Ser
           115
<210> 499
   <211> 324
   <212> DNA
   <213> Artificial Sequence
<220>
   <223> Synthetic
<400> 499
   gaaatagtga tgacgcagtc tccagccacc ctgtctgtgt ctccagggga acgaggcacc 60
   ctctcctgca gggccagtca gagtgttagc agcaacttag cctggtacca gcagaaacct 120
   ggccaggctc ccagactcct catctatggt gcatccacga gggccactgg cttcccagcc 180
   aggttcagtg gcagtgggtc tgggacagag ttcactctca ccatcagcag cctgcagtct 240
   gaagattttg cagtttatta ctgtcagcag tataataagt ggcctccgtt cactttcggc 300
   cctgggacca aagtggatat caaa                                        324
<210> 500
   <211> 108
   <212> PRT
   <213> Artificial Sequence
<220>
   <223> Synthetic
<400> 500
   Glu Ile Val Met Thr Gln Ser Pro Ala Thr Leu Ser Val Ser Pro Gly
    1               5                  10                  15
   Glu Arg Gly Thr Leu Ser Cys Arg Ala Ser Gln Ser Val Ser Ser Asn
               20                  25                  30
   Leu Ala Trp Tyr Gln Gln Lys Pro Gly Gln Ala Pro Arg Leu Leu Ile
           35                  40                  45
   Tyr Gly Ala Ser Thr Arg Ala Thr Gly Phe Pro Ala Arg Phe Ser Gly
       50                  55                  60
   Ser Gly Ser Gly Thr Glu Phe Thr Leu Thr Ile Ser Ser Leu Gln Ser
   65                  70                  75                  80
   Glu Asp Phe Ala Val Tyr Tyr Cys Gln Gln Tyr Asn Lys Trp Pro Pro
                   85                  90                  95
   Phe Thr Phe Gly Pro Gly Thr Lys Val Asp Ile Lys
               100                 105
<210> 501
   <211> 354
   <212> DNA
   <213> Artificial Sequence
<220>
   <223> Synthetic
<400> 501
   gaggtgcagc tggtggagtc tgggggaggc ttggtacagc ctggggggtc cctgagactc 60
   tcctgtgcag cctctggatt caccttcggt gactacgaca tgcactgggt ccgccaagct 120
   acaggaaaag gtctggagtg ggtctcagct attgctcctg ctggtgacac atactatcca 180
   ggctccgtga agggccgatt caccatctcc agagaaaatg ccaagaactc cttgtatctt 240
   caaatgaaca gcctgagagc cggggacacg gctgtgtatt actgtgctag agaggatata 300
   gcagtgcctg gttttgatta ctggggccaa ggaaccctgg tcaccgtctc ctca       354
<210> 502
   <211> 118
   <212> PRT
   <213> Artificial Sequence
<220>
   <223> Synthetic
<400> 502
   Glu Val Gln Leu Val Glu Ser Gly Gly Gly Leu Val Gln Pro Gly Gly
    1               5                   10                  15
   Ser Leu Arg Leu Ser Cys Ala Ala Ser Gly Phe Thr Phe Gly Asp Tyr
               20                  25                  30
   Asp Met His Trp Val Arg Gln Ala Thr Gly Lys Gly Leu Glu Trp Val
           35                  40                  45
   Ser Ala Ile Ala Pro Ala Gly Asp Thr Tyr Tyr Pro Gly Ser Val Lys
       50                  55                  60
   Gly Arg Phe Thr Ile Ser Arg Glu Asn Ala Lys Asn Ser Leu Tyr Leu
   65                  70                  75                  80
   Gln Met Asn Ser Leu Arg Ala Gly Asp Thr Ala Val Tyr Tyr Cys Ala
                   85                  90                  95
   Arg Glu Asp Ile Ala Val Pro Gly Phe Asp Tyr Trp Gly Gln Gly Thr
               100                 105                 110
   Leu Val Thr Val Ser Ser
           115
<210> 503
   <211> 324
   <212> DNA
   <213> Artificial Sequence
<220>
   <223> Synthetic
<400> 503
   gaaatagtga tgacgcagtc tccagccacc ctgtctgtgt ctccagggga aagagccacc 60
   ctctcctgca gggccagtca gagtgttagc agcaacttag cctggtacca gcagaaacct 120
   ggccaggctc ccaggctcct catctatggt gcatccacca gggccactgg tatcccagcc 180
   aggttcagtg gcagtgggtc tgggacagag ttcactctca ccatcagcag cctgcagtct 240
   gaagattttg cagtttatta ctgtcagcag tataataagt ggcctccgtt cactttcggc 300
   cctgggacca aagtggatat caaa                                         324
<210> 504
   <211> 108
   <212> PRT
   <213> Artificial Sequence
<220>
   <223> Synthetic
<400> 504
   Glu Ile Val Met Thr Gln Ser Pro Ala Thr Leu Ser Val Ser Pro Gly
    1               5                  10                  15
   Glu Arg Ala Thr Leu Ser Cys Arg Ala Ser Gln Ser Val Ser Ser Asn
               20                  25                  30
   Leu Ala Trp Tyr Gln Gln Lys Pro Gly Gln Ala Pro Arg Leu Leu Ile
           35                  40                  45
   Tyr Gly Ala Ser Thr Arg Ala Thr Gly Ile Pro Ala Arg Phe Ser Gly
       50                  55                  60
   Ser Gly Ser Gly Thr Glu Phe Thr Leu Thr Ile Ser Ser Leu Gln Ser
   65                  70                  75                  80
   Glu Asp Phe Ala Val Tyr Tyr Cys Gln Gln Tyr Asn Lys Trp Pro Pro
                   85                  90                  95
   Phe Thr Phe Gly Pro Gly Thr Lys Val Asp Ile Lys
               100                 105
<210> 505
   <211> 378
   <212> DNA
   <213> Artificial Sequence
<220>
   <223> Synthetic
<400> 505
   caaattctgc tggtgcaatc tggacctgag gtgaaggagc ctggggcctc agtgaaggtc 60
   tcctgcaagg cttctggtta cacctttacc aactacgcta tcagctgggt gcgacaggtc 120
   cctggacaag ggcttgagtg gatgggatgg gtcagcgctt acaatggtca cacaaactat 180
   gcacatgaag tccagggcag agtcaccatg accacagaca catccacgac cacagcctac 240
   atggagctga ggagcctgag atctgacgac acggccatgt attactgtgc gagagggggt 300
   gtagtcgtgc cagttgctcc ccacttctac aacggtatgg acgtctgggg ccaagggacc 360
   acggtcaccg tctcctca                                               378
<210> 506
   <211> 126
   <212> PRT
   <213> Artificial Sequence
<220>
   <223> Synthetic
<400> 506
   Gln Ile Leu Leu Val Gln Ser Gly Pro Glu Val Lys Glu Pro Gly Ala
    1               5                  10                  15
   Ser Val Lys Val Ser Cys Lys Ala Ser Gly Tyr Thr Phe Thr Asn Tyr
               20                  25                  30
   Ala Ile Ser Trp Val Arg Gln Val Pro Gly Gln Gly Leu Glu Trp Met
           35                  40                  45
   Gly Trp Val Ser Ala Tyr Asn Gly His Thr Asn Tyr Ala His Glu Val
       50                  55                  60
   Gln Gly Arg Val Thr Met Thr Thr Asp Thr Ser Thr Thr Thr Ala Tyr
   65                  70                  75                  80
   Met Glu Leu Arg Ser Leu Arg Ser Asp Asp Thr Ala Met Tyr Tyr Cys
                   85                  90                  95
   Ala Arg Gly Gly Val Val Val Pro Val Ala Pro His Phe Tyr Asn Gly
               100                 105                 110
   Met Asp Val Trp Gly Gln Gly Thr Thr Val Thr Val Ser Ser
           115                 120                 125
<210> 507
   <211> 24
   <212> DNA
   <213> Artificial Sequence
<220>
   <223> Synthetic
<400> 507
   ggttacacct ttaccaacta cgct 24
<210> 508
   <211> 8
   <212> PRT
   <213> Artificial Sequence
<220>
   <223> Synthetic
<400> 508
   Gly Tyr Thr Phe Thr Asn Tyr Ala
    1               5
<210> 509
   <211> 24
   <212> DNA
   <213> Artificial Sequence
<220>
   <223> Synthetic
<400> 509
   gtcagcgctt acaatggtca caca 24
<210> 510
   <211> 8
   <212> PRT
   <213> Artificial Sequence
<220>
   <223> Synthetic
<400> 510
   Val Ser Ala Tyr Asn Gly His Thr
    1               5
<210> 511
   <211> 57
   <212> DNA
   <213> Artificial Sequence
<220>
   <223> Synthetic
<400> 511
   gcgagagggg gtgtagtcgt gccagttgct ccccacttct acaacggtat ggacgtc 57
<210> 512
   <211> 19
   <212> PRT
   <213> Artificial Sequence
<220>
   <223> Synthetic
<400> 512
   Ala Arg Gly Gly Val Val Val Pro Val Ala Pro His Phe Tyr Asn Gly
    1               5                  10                  15
   Met Asp Val
<210> 513
   <211> 336
   <212> DNA
   <213> Artificial Sequence
<220>
   <223> Synthetic
<400> 513
   gatattgtga tgactcagtt tccactctcc ctgcccgtca cccctggaga gccggcctcc 60
   atctcctgca ggtctagtca gagcctcctg catattaatg aatacaacta tttggattgg 120
   tacctaaaga agccagggca gtctccacag ctcctgatct atttgggttt taatcgggcc 180
   tccggggtcc ctgacaggtt cagtggcagt ggatcaggca cagattttac actgaaaatc 240
   agcagagtgg aggctgagga tgttggggtc tattactgca tgcaagctct tcaaactccg 300
   tggacgttag gccaagggac caaggtggaa atcaaa                           336
<210> 514
   <211> 112
   <212> PRT
   <213> Artificial Sequence
<220>
   <223> Synthetic
<400> 514
   Asp Ile Val Met Thr Gln Phe Pro Leu Ser Leu Pro Val Thr Pro Gly
   1               5                   10                  15
   Glu Pro Ala Ser Ile Ser Cys Arg Ser Ser Gln Ser Leu Leu His Ile
               20                  25                  30
   Asn Glu Tyr Asn Tyr Leu Asp Trp Tyr Leu Lys Lys Pro Gly Gln Ser
           35                  40                  45
   Pro Gln Leu Leu Ile Tyr Leu Gly Phe Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ala
                   85                  90                  95
   Leu Gln Thr Pro Trp Thr Leu Gly Gln Gly Thr Lys Val Glu Ile Lys
               100                 105                 110
<210> 515
   <211> 33
   <212> DNA
   <213> Artificial Sequence
<220>
   <223> Synthetic
<400> 515
   cagagcctcc tgcatattaa tgaatacaac tat 33
<210> 516
   <211> 11
   <212> PRT
   <213> Artificial Sequence
<220>
   <223> Synthetic
<400> 516
   Gln Ser Leu Leu His Ile Asn Glu Tyr Asn Tyr
   1               5                   10
<210> 517
   <211> 9
   <212> DNA
   <213> Artificial Sequence
<220>
   <223> Synthetic
<400> 517
   ttgggtttt 9
<210> 518
   <211> 3
   <212> PRT
   <213> Artificial Sequence
<220>
   <223> Synthetic
<400> 518
   Leu Gly Phe
   1
<210> 519
   <211> 27
   <212> DNA
   <213> Artificial Sequence
<220>
   <223> Synthetic
<400> 519
   atgcaagctc ttcaaactcc gtggacg 27
<210> 520
   <211> 9
   <212> PRT
   <213> Artificial Sequence
<220>
   <223> Synthetic
<400> 520
   Met Gln Ala Leu Gln Thr Pro Trp Thr
   1 5
<210> 521
   <211> 378
   <212> DNA
   <213> Artificial Sequence
<220>
   <223> Synthetic
<400> 521
   caggttcagc tggtgcagtc tggacctgag gtgaaggagc ctggggcctc agtgaaggtc 60
   tcctgcaagg cttctggtta cacctttacc aactacgcta tcagctgggt gcgacaggtc 120
   cctggacaag ggcttgagtg gatgggatgg gtcagcgctt acaatggtca cacaaactat 180
   gcacatgaag tccagggcag agtcaccatg accacagaca catccacgac cacagcctac 240
   atggagctga ggagcctgag atctgacgac acggccatgt attactgtgc gagagggggt 300
   gtagtcgtgc cagttgctcc ccacttctac aacggtatgg acgtctgggg ccaagggacc 360
   acggtcaccg tctcctca                                                378
<210> 522
   <211> 126
   <212> PRT
   <213> Artificial Sequence
<220>
   <223> Synthetic
<400> 522
   Gln Val Gln Leu Val Gln Ser Gly Pro Glu Val Lys Glu Pro Gly Ala
    1               5                  10                  15
   Ser Val Lys Val Ser Cys Lys Ala Ser Gly Tyr Thr Phe Thr Asn Tyr
               20                  25                  30
   Ala Ile Ser Trp Val Arg Gln Val Pro Gly Gln Gly Leu Glu Trp Met
           35                  40                  45
   Gly Trp Val Ser Ala Tyr Asn Gly His Thr Asn Tyr Ala His Glu Val
       50                  55                  60
   Gln Gly Arg Val Thr Met Thr Thr Asp Thr Ser Thr Thr Thr Ala Tyr
   65                  70                  75                  80
   Met Glu Leu Arg Ser Leu Arg Ser Asp Asp Thr Ala Met Tyr Tyr Cys
                   85                  90                  95
   Ala Arg Gly Gly Val Val Val Pro Val Ala Pro His Phe Tyr Asn Gly
               100                 105                 110
   Met Asp Val Trp Gly Gln Gly Thr Thr Val Thr Val Ser Ser
           115                 120                 125
<210> 523
   <211> 336
   <212> DNA
   <213> Artificial Sequence
<220>
   <223> Synthetic
<400> 523
   gatattgtga tgactcagtc tccactctcc ctgcccgtca cccctggaga gccggcctcc 60
   atctcctgca ggtctagtca gagcctcctg catattaatg aatacaacta tttggattgg 120
   tacctaaaga agccagggca gtctccacag ctcctgatct atttgggttt taatcgggcc 180
   tccggggtcc ctgacaggtt cagtggcagt ggatcaggca cagattttac actgaaaatc 240
   agcagagtgg aggctgagga tgttggggtc tattactgca tgcaagctct tcaaactccg 300
   tggacgttag gccaagggac caaggtggaa atcaaa                           336
<210> 524
   <211> 112
   <212> PRT
   <213> Artificial Sequence
<220>
   <223> Synthetic
<400> 524
   Asp Ile Val Met Thr Gln Ser Pro Leu Ser Leu Pro Val Thr Pro Gly
    1               5                  10                  15
   Glu Pro Ala Ser Ile Ser Cys Arg Ser Ser Gln Ser Leu Leu His Ile
               20                  25                  30
   Asn Glu Tyr Asn Tyr Leu Asp Trp Tyr Leu Lys Lys Pro Gly Gln Ser
           35                  40                  45
   Pro Gln Leu Leu Ile Tyr Leu Gly Phe Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ala
                   85                  90                  95
   Leu Gln Thr Pro Trp Thr Leu Gly Gln Gly Thr Lys Val Glu Ile Lys
               100                 105                 110
<210> 525
   <211> 378
   <212> DNA
   <213> Artificial Sequence
<220>
   <223> Synthetic
<400> 525
   caggttcagc tggtgcagtc tggagctgag gtgaagaagc ctggggcctc agtgaaggtc 60
   tcctgcaagg cttctggtta cacctttacc aactacgcta tcagctgggt gcgacaggcc 120
   cctggacaag ggcttgagtg gatgggatgg gtcagcgctt acaatggtca cacaaactat 180
   gcacagaagc tccagggcag agtcaccatg accacagaca catccacgag cacagcctac 240
   atggagctga ggagcctgag atctgacgac acggccgtgt attactgtgc gagagggggt 300
   gtagtcgtgc cagttgctcc ccacttctac aacggtatgg acgtctgggg gcaagggacc 360
   acggtcaccg tctcctca                                                378
<210> 526
   <211> 126
   <212> PRT
   <213> Artificial Sequence
<220>
   <223> Synthetic
<400> 526
   Gln Val Gln Leu Val Gln Ser Gly Ala Glu Val Lys Lys Pro Gly Ala
    1               5                  10                  15
   Ser Val Lys Val Ser Cys Lys Ala Ser Gly Tyr Thr Phe Thr Asn Tyr
               20                  25                  30
   Ala Ile Ser Trp Val Arg Gln Ala Pro Gly Gln Gly Leu Glu Trp Met
           35                  40                  45
   Gly Trp Val Ser Ala Tyr Asn Gly His Thr Asn Tyr Ala Gln Lys Leu
       50                  55                  60
   Gln Gly Arg Val Thr Met Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Gly Gly Val Val Val Pro Val Ala Pro His Phe Tyr Asn Gly
               100                 105                 110
   Met Asp Val Trp Gly Gln Gly Thr Thr Val Thr Val Ser Ser
           115                 120                 125
<210> 527
   <211> 336
   <212> DNA
   <213> Artificial Sequence
<220>
   <223> Synthetic
<400> 527
   gatattgtga tgactcagtc tccactctcc ctgcccgtca cccctggaga gccggcctcc 60
   atctcctgca ggtctagtca gagcctcctg catattaatg aatacaacta tttggattgg 120
   tacctgcaga agccagggca gtctccacag ctcctgatct atttgggttc taatcgggcc 180
   tccggggtcc ctgacaggtt cagtggcagt ggatcaggca cagattttac actgaaaatc 240
   agcagagtgg aggctgagga tgttggggtt tattactgca tgcaagctct tcaaactccg 300
   tggacgttcg gccaagggac caaggtggaa atcaaa                           336
<210> 528
   <211> 112
   <212> PRT
   <213> Artificial Sequence
<220>
   <223> Synthetic
<400> 528
   Asp Ile Val Met Thr Gln Ser Pro Leu Ser Leu Pro Val Thr Pro Gly
    1               5                  10                  15
   Glu Pro Ala Ser Ile Ser Cys Arg Ser Ser Gln Ser Leu Leu His Ile
               20                  25                  30
   Asn Glu Tyr Asn Tyr Leu Asp Trp Tyr Leu Gln Lys Pro Gly Gln Ser
           35                  40                  45
   Pro Gln Leu Leu Ile Tyr Leu Gly Ser Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ala
                   85                  90                  95
   Leu Gln Thr Pro Trp Thr Phe Gly Gln Gly Thr Lys Val Glu Ile Lys
               100                 105                 110
<210> 529
   <211> 351
   <212> DNA
   <213> Artificial Sequence
<220>
   <223> Synthetic
<400> 529
   gaggtgcagc tggtggagtc tgggggaggc ttggtacagc ctggggggtc cctgagactc 60
   tcctgtgcag cctctggatt caccctaagt agctacgaca tgcactgggt ccgccaagca 120
   acaggaaaag gtctggagtg ggtctcagct attggcagta ctggtgacac atactataca 180
   ggctccgtga tgggccgatt caccatctcc agagacgctg ccaaaaactc cttctatctt 240
   gaaatgaaca gcctgagagt cggggacacg gctgtatatt actgtgcaag agagggaata 300
   agaacaccct atgattattg gggccaggga gcccgggtca ccgtctcctc a          351
<210> 530
   <211> 117
   <212> PRT
   <213> Artificial Sequence
<220>
   <223> Synthetic
<400> 530
   Glu Val Gln Leu Val Glu Ser Gly Gly Gly Leu Val Gln Pro Gly Gly
    1               5                  10                  15
   Ser Leu Arg Leu Ser Cys Ala Ala Ser Gly Phe Thr Leu Ser Ser Tyr
               20                  25                  30
   Asp Met His Trp Val Arg Gln Ala Thr Gly Lys Gly Leu Glu Trp Val
           35                  40                  45
   Ser Ala Ile Gly Ser Thr Gly Asp Thr Tyr Tyr Thr Gly Ser Val Met
       50                  55                  60
   Gly Arg Phe Thr Ile Ser Arg Asp Ala Ala Lys Asn Ser Phe Tyr Leu
   65                  70                  75                  80
   Glu Met Asn Ser Leu Arg Val Gly Asp Thr Ala Val Tyr Tyr Cys Ala
                   85                  90                  95
   Arg Glu Gly Ile Arg Thr Pro Tyr Asp Tyr Trp Gly Gln Gly Ala Arg
               100                 105                 110
   Val Thr Val Ser Ser
           115
<210> 531
   <211> 24
   <212> DNA
   <213> Artificial Sequence
<220>
   <223> Synthetic
<400> 531
   ggattcaccc taagtagcta cgac 24
<210> 532
   <211> 8
   <212> PRT
   <213> Artificial Sequence
<220>
   <223> Synthetic
<400> 532
   Gly Phe Thr Leu Ser Ser Tyr Asp
    1               5
<210> 533
   <211> 21
   <212> DNA
   <213> Artificial Sequence
<220>
   <223> Synthetic
<400> 533
   attggcagta ctggtgacac a 21
<210> 534
   <211> 7
   <212> PRT
   <213> Artificial Sequence
<220>
   <223> Synthetic
<400> 534
   Ile Gly Ser Thr Gly Asp Thr
   1 5
<210> 535
   <211> 33
   <212> DNA
   <213> Artificial Sequence
<220>
   <223> Synthetic
<400> 535
   gcaagagagg gaataagaac accctatgat tat 33
<210> 536
   <211> 11
   <212> PRT
   <213> Artificial Sequence
<220>
   <223> Synthetic
<400> 536
   Ala Arg Glu Gly Ile Arg Thr Pro Tyr Asp Tyr
   1 5 10
<210> 537
   <211> 324
   <212> DNA
   <213> Artificial Sequence
<220>
   <223> Synthetic
<400> 537
   gaaatagtga tgacgcagtc tccagccacc ctgtctgtgt ctccagggga aagagccacc 60
   ctctcctgca gggccagtca gagtgttagc agcaatgtag cctggtacca gcagaaacct 120
   ggccaggctc ccaggctcct catctatggt gcatccacca gggccactgg tatcccagcc 180
   aggttcagtg gcagtgggtc tgggacagaa ttcactctca ccatcagcag cctgcagtct 240
   gaagattttg cagtttatta ctgtcagcag tataataatt ggcctccatt cactttcggc 300
   cctgggacca aagtggatat caaa 324
<210> 538
   <211> 108
   <212> PRT
   <213> Artificial Sequence
<220>
   <223> Synthetic
<400> 538
   Glu Ile Val Met Thr Gln Ser Pro Ala Thr Leu Ser Val Ser Pro Gly
    1               5                  10                  15
   Glu Arg Ala Thr Leu Ser Cys Arg Ala Ser Gln Ser Val Ser Ser Asn
               20                  25                  30
   Val Ala Trp Tyr Gln Gln Lys Pro Gly Gln Ala Pro Arg Leu Leu Ile
           35                  40                  45
   Tyr Gly Ala Ser Thr Arg Ala Thr Gly Ile Pro Ala Arg Phe Ser Gly
       50                  55                  60
   Ser Gly Ser Gly Thr Glu Phe Thr Leu Thr Ile Ser Ser Leu Gln Ser
   65                  70                  75                  80
   Glu Asp Phe Ala Val Tyr Tyr Cys Gln Gln Tyr Asn Asn Trp Pro Pro
                   85                  90                  95
   Phe Thr Phe Gly Pro Gly Thr Lys Val Asp Ile Lys
               100                 105
<210> 539
   <211> 18
   <212> DNA
   <213> Artificial Sequence
<220>
   <223> Synthetic
<400> 539
   cagagtgtta gcagcaat 18
<210> 540
   <211> 6
   <212> PRT
   <213> Artificial Sequence
<220>
   <223> Synthetic
<400> 540
   Gln Ser Val Ser Ser Asn
    1               5
<210> 541
   <211> 9
   <212> DNA
   <213> Artificial Sequence
<220>
   <223> Synthetic
<400> 541
   ggtgcatcc 9
<210> 542
   <211> 3
   <212> PRT
   <213> Artificial Sequence
<220>
   <223> Synthetic
<400> 542
   Gly Ala Ser
   1
<210> 543
   <211> 30
   <212> DNA
   <213> Artificial Sequence
<220>
   <223> Synthetic
<400> 543
   cagcagtata ataattggcc tccattcact 30
<210> 544
   <211> 10
   <212> PRT
   <213> Artificial Sequence
<220>
   <223> Synthetic
<400> 544
   Gln Gln Tyr Asn Asn Trp Pro Pro Phe Thr
   1               5                   10
<210> 545
   <211> 351
   <212> DNA
   <213> Artificial Sequence
<220>
   <223> Synthetic
<400> 545
   gaggtgcagc tggtggagtc tgggggaggc ttggtacagc ctggggggtc cctgagactc 60
   tcctgtgcag cctctggatt caccctaagt agctacgaca tgcactgggt ccgccaagca 120
   acaggaaaag gtctggagtg ggtctcagct attggcagta ctggtgacac atactataca 180
   ggctccgtga tgggccgatt caccatctcc agagacgctg ccaaaaactc cttctatctt 240
   gaaatgaaca gcctgagagt cggggacacg gctgtatatt actgtgcaag agagggaata 300
   agaacaccct atgattattg gggccaggga accctggtca ccgtctcctc a          351
<210> 546
   <211> 117
   <212> PRT
   <213> Artificial Sequence
<220>
   <223> Synthetic
<400> 546
   Glu Val Gln Leu Val Glu Ser Gly Gly Gly Leu Val Gln Pro Gly Gly
    1               5                  10                  15
   Ser Leu Arg Leu Ser Cys Ala Ala Ser Gly Phe Thr Leu Ser Ser Tyr
               20                  25                  30
   Asp Met His Trp Val Arg Gln Ala Thr Gly Lys Gly Leu Glu Trp Val
           35                  40                  45
   Ser Ala Ile Gly Ser Thr Gly Asp Thr Tyr Tyr Thr Gly Ser Val Met
       50                  55                  60
   Gly Arg Phe Thr Ile Ser Arg Asp Ala Ala Lys Asn Ser Phe Tyr Leu
   65                  70                  75                  80
   Glu Met Asn Ser Leu Arg Val Gly Asp Thr Ala Val Tyr Tyr Cys Ala
                   85                  90                  95
   Arg Glu Gly Ile Arg Thr Pro Tyr Asp Tyr Trp Gly Gln Gly Thr Leu
               100                 105                 110
   Val Thr Val Ser Ser
           115
<210> 547
   <211> 324
   <212> DNA
   <213> Artificial Sequence
<220>
   <223> Synthetic
<400> 547
   gaaatagtga tgacgcagtc tccagccacc ctgtctgtgt ctccagggga aagagccacc 60
   ctctcctgca gggccagtca gagtgttagc agcaatgtag cctggtacca gcagaaacct 120
   ggccaggctc ccaggctcct catctatggt gcatccacca gggccactgg tatcccagcc 180
   aggttcagtg gcagtgggtc tgggacagaa ttcactctca ccatcagcag cctgcagtct 240
   gaagattttg cagtttatta ctgtcagcag tataataatt ggcctccatt cactttcggc 300
   cctgggacca aagtggatat caaa                                        324
<210> 548
   <211> 108
   <212> PRT
   <213> Artificial Sequence
<220>
   <223> Synthetic
<400> 548
   Glu Ile Val Met Thr Gln Ser Pro Ala Thr Leu Ser Val Ser Pro Gly
    1               5                  10                  15
   Glu Arg Ala Thr Leu Ser Cys Arg Ala Ser Gln Ser Val Ser Ser Asn
               20                  25                  30
   Val Ala Trp Tyr Gln Gln Lys Pro Gly Gln Ala Pro Arg Leu Leu Ile
           35                  40                  45
   Tyr Gly Ala Ser Thr Arg Ala Thr Gly Ile Pro Ala Arg Phe Ser Gly
       50                  55                  60
   Ser Gly Ser Gly Thr Glu Phe Thr Leu Thr Ile Ser Ser Leu Gln Ser
   65                  70                  75                  80
   Glu Asp Phe Ala Val Tyr Tyr Cys Gln Gln Tyr Asn Asn Trp Pro Pro
                   85                  90                  95
   Phe Thr Phe Gly Pro Gly Thr Lys Val Asp Ile Lys
               100                 105
<210> 549
   <211> 351
   <212> DNA
   <213> Artificial Sequence
<220>
   <223> Synthetic
<400> 549
   gaggtgcagc tggtggagtc tgggggaggc ttggtacagc ctggggggtc cctgagactc 60
   tcctgtgcag cctctggatt caccctaagt agctacgaca tgcactgggt ccgccaagct 120
   acaggaaaag gtctggagtg ggtctcagct attggcagta ctggtgacac atactatcca 180
   ggctccgtga agggccgatt caccatctcc agagaaaatg ccaagaactc cttgtatctt 240
   caaatgaaca gcctgagagc cggggacacg gctgtgtatt actgtgcaag agagggaata 300
   agaacaccct atgattattg gggccaagga accctggtca ccgtctcctc a          351
<210> 550
   <211> 117
   <212> PRT
   <213> Artificial Sequence
<220>
   <223> Synthetic
<400> 550
   Glu Val Gln Leu Val Glu Ser Gly Gly Gly Leu Val Gln Pro Gly Gly
    1               5                  10                  15
   Ser Leu Arg Leu Ser Cys Ala Ala Ser Gly Phe Thr Leu Ser Ser Tyr
               20                  25                  30
   Asp Met His Trp Val Arg Gln Ala Thr Gly Lys Gly Leu Glu Trp Val
           35                  40                  45
   Ser Ala Ile Gly Ser Thr Gly Asp Thr Tyr Tyr Pro Gly Ser Val Lys
       50                  55                  60
   Gly Arg Phe Thr Ile Ser Arg Glu Asn Ala Lys Asn Ser Leu Tyr Leu
   65                  70                  75                  80
   Gln Met Asn Ser Leu Arg Ala Gly Asp Thr Ala Val Tyr Tyr Cys Ala
                   85                  90                  95
   Arg Glu Gly Ile Arg Thr Pro Tyr Asp Tyr Trp Gly Gln Gly Thr Leu
               100                 105                 110
   Val Thr Val Ser Ser
           115
<210> 551
   <211> 324
   <212> DNA
   <213> Artificial Sequence
<220>
   <223> Synthetic
<400> 551
   gaaatagtga tgacgcagtc tccagccacc ctgtctgtgt ctccagggga aagagccacc 60
   ctctcctgca gggccagtca gagtgttagc agcaatttag cctggtacca gcagaaacct 120
   ggccaggctc ccaggctcct catctatggt gcatccacca gggccactgg tatcccagcc 180
   aggttcagtg gcagtgggtc tgggacagag ttcactctca ccatcagcag cctgcagtct 240
   gaagattttg cagtttatta ctgtcagcag tataataatt ggcctccatt cactttcggc 300
   cctgggacca aagtggatat caaa                                        324
<210> 552
   <211> 108
   <212> PRT
   <213> Artificial Sequence
<220>
   <223> Synthetic
<400> 552
   Glu Ile Val Met Thr Gln Ser Pro Ala Thr Leu Ser Val Ser Pro Gly
    1               5                  10                  15
   Glu Arg Ala Thr Leu Ser Cys Arg Ala Ser Gln Ser Val Ser Ser Asn
               20                  25                  30
   Leu Ala Trp Tyr Gln Gln Lys Pro Gly Gln Ala Pro Arg Leu Leu Ile
           35                  40                  45
   Tyr Gly Ala Ser Thr Arg Ala Thr Gly Ile Pro Ala Arg Phe Ser Gly
       50                  55                  60
   Ser Gly Ser Gly Thr Glu Phe Thr Leu Thr Ile Ser Ser Leu Gln Ser
   65                  70                  75                  80
   Glu Asp Phe Ala Val Tyr Tyr Cys Gln Gln Tyr Asn Asn Trp Pro Pro
                   85                  90                  95
   Phe Thr Phe Gly Pro Gly Thr Lys Val Asp Ile Lys
               100                 105
<210> 553
   <211> 351
   <212> DNA
   <213> Artificial Sequence
<220>
   <223> Synthetic
<400> 553
   gaggtgcagc tggtggagtc tgggggaggc ttggtacagc ctggggggtc cctgagactc 60
   tcctgtgcag cctctggatt caccctaagt agctacgaca tgcactgggt ccgccaagca 120
   acaggaaaag gtctggagtg ggtctcagct attggcagta ctggtgacac atactataca 180
   ggctccgtga tgggccgatt caccatctcc agagacgctg ccaaaaactc cttctatctt 240
   gaaatgaaca gcctgagagt cggggacacg gctgtatatt actgtgcaag agagggaata 300
   agaacaccct atgattattg gggccaggga gcccgggtca ccgtctcctc a          351
<210> 554
   <211> 117
   <212> PRT
   <213> Artificial Sequence
<220>
   <223> Synthetic
<400> 554
   Glu Val Gln Leu Val Glu Ser Gly Gly Gly Leu Val Gln Pro Gly Gly
    1               5                  10                  15
   Ser Leu Arg Leu Ser Cys Ala Ala Ser Gly Phe Thr Leu Ser Ser Tyr
               20                  25                  30
   Asp Met His Trp Val Arg Gln Ala Thr Gly Lys Gly Leu Glu Trp Val
           35                  40                  45
   Ser Ala Ile Gly Ser Thr Gly Asp Thr Tyr Tyr Thr Gly Ser Val Met
       50                  55                  60
   Gly Arg Phe Thr Ile Ser Arg Asp Ala Ala Lys Asn Ser Phe Tyr Leu
   65                  70                  75                  80
   Glu Met Asn Ser Leu Arg Val Gly Asp Thr Ala Val Tyr Tyr Cys Ala
                   85                  90                  95
   Arg Glu Gly Ile Arg Thr Pro Tyr Asp Tyr Trp Gly Gln Gly Ala Arg
               100                 105                 110
   Val Thr Val Ser Ser
           115
<210> 555
   <211> 24
   <212> DNA
   <213> Artificial Sequence
<220>
   <223> Synthetic
<400> 555
   ggattcaccc taagtagcta cgac 24
<210> 556
   <211> 8
   <212> PRT
   <213> Artificial Sequence
<220>
   <223> Synthetic
<400> 556
   Gly Phe Thr Leu Ser Ser Tyr Asp
   1               5
<210> 557
   <211> 21
   <212> DNA
   <213> Artificial Sequence
<220>
   <223> Synthetic
<400> 557
   attggcagta ctggtgacac a 21
<210> 558
   <211> 7
   <212> PRT
   <213> Artificial Sequence
<220>
   <223> Synthetic
<400> 558
   Ile Gly Ser Thr Gly Asp Thr
   1               5
<210> 559
   <211> 33
   <212> DNA
   <213> Artificial Sequence
<220>
   <223> Synthetic
<400> 559
   gcaagagagg gaataagaac accctatgat tat 33
<210> 560
   <211> 11
   <212> PRT
   <213> Artificial Sequence
<220>
   <223> Synthetic
<400> 560
   Ala Arg Glu Gly Ile Arg Thr Pro Tyr Asp Tyr
    1               5                   10
<210> 561
   <211> 324
   <212> DNA
   <213> Artificial Sequence
<220>
   <223> Synthetic
<400> 561
   gaaatagtga tgacgcagtc tccagccacc ctgtctgtgt ctccagggga aagagccacc 60
   ctctcctgca gggccagtca gagtgttagc agcaatgtag cctggtacca gcagaaacct 120
   ggccaggctc ccaggctcct catctatggt gcatccacca gggccactgg tatcccagcc 180
   aggttcagtg gcagtgggtc tgggacagaa ttcactctca ccatcagcag cctgcagtct 240
   gaagattttg cagtttatta ctgtcagcag tataataatt ggcctccatt cactttcggc 300
   cctgggacca aagtggatat caaa                                         324
<210> 562
   <211> 108
   <212> PRT
   <213> Artificial Sequence
<220>
   <223> Synthetic
<400> 562
   Glu Ile Val Met Thr Gln Ser Pro Ala Thr Leu Ser Val Ser Pro Gly
    1               5                  10                  15
   Glu Arg Ala Thr Leu Ser Cys Arg Ala Ser Gln Ser Val Ser Ser Asn
               20                  25                  30
   Val Ala Trp Tyr Gln Gln Lys Pro Gly Gln Ala Pro Arg Leu Leu Ile
           35                  40                  45
   Tyr Gly Ala Ser Thr Arg Ala Thr Gly Ile Pro Ala Arg Phe Ser Gly
       50                  55                  60
   Ser Gly Ser Gly Thr Glu Phe Thr Leu Thr Ile Ser Ser Leu Gln Ser
   65                  70                  75                  80
   Glu Asp Phe Ala Val Tyr Tyr Cys Gln Gln Tyr Asn Asn Trp Pro Pro
                   85                  90                  95
   Phe Thr Phe Gly Pro Gly Thr Lys Val Asp Ile Lys
               100                 105
<210> 563
   <211> 18
   <212> DNA
   <213> Artificial Sequence
<220>
   <223> Synthetic
<400> 563
   cagagtgtta gcagcaat 18
<210> 564
   <211> 6
   <212> PRT
   <213> Artificial Sequence
<220>
   <223> Synthetic
<400> 564
   Gln Ser Val Ser Ser Asn
   1 5
<210> 565
   <211> 9
   <212> DNA
   <213> Artificial Sequence
<220>
   <223> Synthetic
<400> 565
   ggtgcatcc 9
<210> 566
   <211> 3
   <212> PRT
   <213> Artificial Sequence
<220>
   <223> Synthetic
<400> 566
   Gly Ala Ser
   1
<210> 567
   <211> 30
   <212> DNA
   <213> Artificial Sequence
<220>
   <223> Synthetic
<400> 567
   cagcagtata ataattggcc tccattcact 30
<210> 568
   <211> 10
   <212> PRT
   <213> Artificial Sequence
<220>
   <223> Synthetic
<400> 568
   Gln Gln Tyr Asn Asn Trp Pro Pro Phe Thr
    1               5                  10
<210> 569
   <211> 351
   <212> DNA
   <213> Artificial Sequence
<220>
   <223> Synthetic
<400> 569
   gaggtgcagc tggtggagtc tgggggaggc ttggtacagc ctggggggtc cctgagactc 60
   tcctgtgcag cctctggatt caccctaagt agctacgaca tgcactgggt ccgccaagca 120
   acaggaaaag gtctggagtg ggtctcagct attggcagta ctggtgacac atactataca 180
   ggctccgtga tgggccgatt caccatctcc agagacgctg ccaaaaactc cttctatctt 240
   gaaatgaaca gcctgagagt cggggacacg gctgtatatt actgtgcaag agagggaata 300
   agaacaccct atgattattg gggccaggga accctggtca ccgtctcctc a          351
<210> 570
   <211> 117
   <212> PRT
   <213> Artificial Sequence
<220>
   <223> Synthetic
<400> 570
   Glu Val Gln Leu Val Glu Ser Gly Gly Gly Leu Val Gln Pro Gly Gly
    1               5                  10                  15
   Ser Leu Arg Leu Ser Cys Ala Ala Ser Gly Phe Thr Leu Ser Ser Tyr
               20                  25                  30
   Asp Met His Trp Val Arg Gln Ala Thr Gly Lys Gly Leu Glu Trp Val
           35                  40                  45
   Ser Ala Ile Gly Ser Thr Gly Asp Thr Tyr Tyr Thr Gly Ser Val Met
       50                  55                  60
   Gly Arg Phe Thr Ile Ser Arg Asp Ala Ala Lys Asn Ser Phe Tyr Leu
   65                  70                  75                  80
   Glu Met Asn Ser Leu Arg Val Gly Asp Thr Ala Val Tyr Tyr Cys Ala
                   85                  90                  95
   Arg Glu Gly Ile Arg Thr Pro Tyr Asp Tyr Trp Gly Gln Gly Thr Leu
               100                 105                 110
   Val Thr Val Ser Ser
           115
<210> 571
   <211> 324
   <212> DNA
   <213> Artificial Sequence
<220>
   <223> Synthetic
<400> 571
   gaaatagtga tgacgcagtc tccagccacc ctgtctgtgt ctccagggga aagagccacc 60
   ctctcctgca gggccagtca gagtgttagc agcaatgtag cctggtacca gcagaaacct 120
   ggccaggctc ccaggctcct catctatggt gcatccacca gggccactgg tatcccagcc 180
   aggttcagtg gcagtgggtc tgggacagaa ttcactctca ccatcagcag cctgcagtct 240
   gaagattttg cagtttatta ctgtcagcag tataataatt ggcctccatt cactttcggc 300
   cctgggacca aagtggatat caaa                                        324
<210> 572
   <211> 108
   <212> PRT
   <213> Artificial Sequence
<220>
   <223> Synthetic
<400> 572
   Glu Ile Val Met Thr Gln Ser Pro Ala Thr Leu Ser Val Ser Pro Gly
    1              5                   10                  15
   Glu Arg Ala Thr Leu Ser Cys Arg Ala Ser Gln Ser Val Ser Ser Asn
               20                  25                  30
   Val Ala Trp Tyr Gln Gln Lys Pro Gly Gln Ala Pro Arg Leu Leu Ile
           35                  40                  45
   Tyr Gly Ala Ser Thr Arg Ala Thr Gly Ile Pro Ala Arg Phe Ser Gly
       50                  55                  60
   Ser Gly Ser Gly Thr Glu Phe Thr Leu Thr Ile Ser Ser Leu Gln Ser
   65                  70                  75                  80
   Glu Asp Phe Ala Val Tyr Tyr Cys Gln Gln Tyr Asn Asn Trp Pro Pro
                   85                  90                  95
   Phe Thr Phe Gly Pro Gly Thr Lys Val Asp Ile Lys
               100                 105
<210> 573
   <211> 351
   <212> DNA
   <213> Artificial Sequence
<220>
   <223> Synthetic
<400> 573
   gaggtgcagc tggtggagtc tgggggaggc ttggtacagc ctggggggtc cctgagactc 60
   tcctgtgcag cctctggatt caccctaagt agctacgaca tgcactgggt ccgccaagct 120
   acaggaaaag gtctggagtg ggtctcagct attggcagta ctggtgacac atactatcca 180
   ggctccgtga agggccgatt caccatctcc agagaaaatg ccaagaactc cttgtatctt 240
   caaatgaaca gcctgagagc cggggacacg gctgtgtatt actgtgcaag agagggaata 300
   agaacaccct atgattattg gggccaagga accctggtca ccgtctcctc a          351
<210> 574
   <211> 117
   <212> PRT
   <213> Artificial Sequence
<220>
   <223> Synthetic
<400> 574
   Glu Val Gln Leu Val Glu Ser Gly Gly Gly Leu Val Gln Pro Gly Gly
    1               5                  10                  15
   Ser Leu Arg Leu Ser Cys Ala Ala Ser Gly Phe Thr Leu Ser Ser Tyr
               20                  25                  30
   Asp Met His Trp Val Arg Gln Ala Thr Gly Lys Gly Leu Glu Trp Val
           35                  40                  45
   Ser Ala Ile Gly Ser Thr Gly Asp Thr Tyr Tyr Pro Gly Ser Val Lys
       50                  55                  60
   Gly Arg Phe Thr Ile Ser Arg Glu Asn Ala Lys Asn Ser Leu Tyr Leu
   65                  70                  75                  80
   Gln Met Asn Ser Leu Arg Ala Gly Asp Thr Ala Val Tyr Tyr Cys Ala
                   85                  90                  95
   Arg Glu Gly Ile Arg Thr Pro Tyr Asp Tyr Trp Gly Gln Gly Thr Leu
               100                 105                 110
   Val Thr Val Ser Ser
           115
<210> 575
   <211> 324
   <212> DNA
   <213> Artificial Sequence
<220>
   <223> Synthetic
<400> 575
   gaaatagtga tgacgcagtc tccagccacc ctgtctgtgt ctccagggga aagagccacc 60
   ctctcctgca gggccagtca gagtgttagc agcaatttag cctggtacca gcagaaacct 120
   ggccaggctc ccaggctcct catctatggt gcatccacca gggccactgg tatcccagcc 180
   aggttcagtg gcagtgggtc tgggacagag ttcactctca ccatcagcag cctgcagtct 240
   gaagattttg cagtttatta ctgtcagcag tataataatt ggcctccatt cactttcggc 300
   cctgggacca aagtggatat caaa                                        324
<210> 576
   <211> 108
   <212> PRT
   <213> Artificial Sequence
<220>
   <223> Synthetic
<400> 576
   Glu Ile Val Met Thr Gln Ser Pro Ala Thr Leu Ser Val Ser Pro Gly
    1               5                  10                  15
   Glu Arg Ala Thr Leu Ser Cys Arg Ala Ser Gln Ser Val Ser Ser Asn
               20                  25                  30
   Leu Ala Trp Tyr Gln Gln Lys Pro Gly Gln Ala Pro Arg Leu Leu Ile
           35                  40                  45
   Tyr Gly Ala Ser Thr Arg Ala Thr Gly Ile Pro Ala Arg Phe Ser Gly
       50                  55                  60
   Ser Gly Ser Gly Thr Glu Phe Thr Leu Thr Ile Ser Ser Leu Gln Ser
   65                  70                  75                  80
   Glu Asp Phe Ala Val Tyr Tyr Cys Gln Gln Tyr Asn Asn Trp Pro Pro
                   85                  90                  95
   Phe Thr Phe Gly Pro Gly Thr Lys Val Asp Ile Lys
               100                 105
<210> 577
   <211> 363
   <212> DNA
   <213> Artificial Sequence
<220>
   <223> Synthetic
<400> 577
   gaagtgcagc tggtggagtc tgggggaggc ttggtacagc ctggcaggtc cctgagactc 60
   tcctgtgcag cctctggatt cacctttgat gattatgcca tgcactgggt ccggcaagct 120
   ccagggaagg gcctggagtg ggtctcaggt attaattgga acagtggtag cataggctat 180
   gcggactctg tgaagggccg attcaccatc tccagagaca acgccaagca ctccctgtat 240
   ctgcaaatga acagtctgag acctgaggac acggccttgt attactgtgt aaaagaggtg 300
   actacgggat actactacgg tatggacgtc tggggccaag ggaccacggt caccgtctcc 360
   tca                                                               363
<210> 578
   <211> 121
   <212> PRT
   <213> Artificial Sequence
<220>
   <223> Synthetic
<400> 578
   Glu Val Gln Leu Val Glu Ser Gly Gly Gly Leu Val Gln Pro Gly Arg
    1               5                  10                  15
   Ser Leu Arg Leu Ser Cys Ala Ala Ser Gly Phe Thr Phe Asp Asp Tyr
               20                  25                  30
   Ala Met His Trp Val Arg Gln Ala Pro Gly Lys Gly Leu Glu Trp Val
           35                  40                  45
   Ser Gly Ile Asn Trp Asn Ser Gly Ser Ile Gly Tyr Ala Asp Ser Val
       50                  55                  60
   Lys Gly Arg Phe Thr Ile Ser Arg Asp Asn Ala Lys His Ser Leu Tyr
   65                  70                  75                  80
   Leu Gln Met Asn Ser Leu Arg Pro Glu Asp Thr Ala Leu Tyr Tyr Cys
                   85                  90                  95
   Val Lys Glu Val Thr Thr Gly Tyr Tyr Tyr Gly Met Asp Val Trp Gly
               100                 105                 110
   Gln Gly Thr Thr Val Thr Val Ser Ser
           115                 120
<210> 579
   <211> 24
   <212> DNA
   <213> Artificial Sequence
<220>
   <223> Synthetic
<400> 579
   ggattcacct ttgatgatta tgcc 24
<210> 580
   <211> 8
   <212> PRT
   <213> Artificial Sequence
<220>
   <223> Synthetic
<400> 580
   Gly Phe Thr Phe Asp Asp Tyr Ala
    1               5
<210> 581
   <211> 24
   <212> DNA
   <213> Artificial Sequence
<220>
   <223> Synthetic
<400> 581
   attaattgga acagtggtag cata 24
<210> 582
   <211> 8
   <212> PRT
   <213> Artificial Sequence
<220>
   <223> Synthetic
<400> 582
   Ile Asn Trp Asn Ser Gly Ser Ile
   1 5
<210> 583
   <211> 42
   <212> DNA
   <213> Artificial Sequence
<220>
   <223> Synthetic
<400> 583
   gtaaaagagg tgactacggg atactactac ggtatggacg tc 42
<210> 584
   <211> 14
   <212> PRT
   <213> Artificial Sequence
<220>
   <223> Synthetic
<400> 584
   Val Lys Glu Val Thr Thr Gly Tyr Tyr Tyr Gly Met Asp Val
   1 5 10
<210> 585
   <211> 321
   <212> DNA
   <213> Artificial Sequence
<220>
   <223> Synthetic
<400> 585
   gacatccagt tgacccagtc tccatccttc ctgtctgcat ctgtaggaga cagagtcacc 60
   atcacttgct gggccagtca gggcattagc agttatttag cctggtatca gaaaaaacca 120
   gggaaagccc ctaacctcct gatctatgat gcatccactt tgcaaagtgg ggtcccatca 180
   aggttcagcg gcagtggatc tgggacagaa ttcactctca cactcagcag cctgcagcct 240
   gaagattttg caacttatta ctgtcaacag cttaatattt acccattcac tttcggccct 300
   gggaccaaag tggatatcaa a 321
<210> 586
   <211> 107
   <212> PRT
   <213> Artificial Sequence
<220>
   <223> Synthetic
<400> 586
   Asp Ile Gln Leu Thr Gln Ser Pro Ser Phe Leu Ser Ala Ser Val Gly
    1               5                  10                  15
   Asp Arg Val Thr Ile Thr Cys Trp Ala Ser Gln Gly Ile Ser Ser Tyr
               20                  25                  30
   Leu Ala Trp Tyr Gln Lys Lys Pro Gly Lys Ala Pro Asn Leu Leu Ile
           35                  40                  45
   Tyr Asp Ala Ser Thr Leu Gln Ser Gly Val Pro Ser Arg Phe Ser Gly
       50                  55                  60
   Ser Gly Ser Gly Thr Glu Phe Thr Leu Thr Leu Ser Ser Leu Gln Pro
   65                  70                  75                  80
   Glu Asp Phe Ala Thr Tyr Tyr Cys Gln Gln Leu Asn Ile Tyr Pro Phe
                   85                  90                  95
   Thr Phe Gly Pro Gly Thr Lys Val Asp Ile Lys
               100                 105
<210> 587
   <211> 18
   <212> DNA
   <213> Artificial Sequence
<220>
   <223> Synthetic
<400> 587
   cagggcatta gcagttat 18
<210> 588
   <211> 6
   <212> PRT
   <213> Artificial Sequence
<220>
   <223> Synthetic
<400> 588
   Gln Gly Ile Ser Ser Tyr
    1               5
<210> 589
   <211> 9
   <212> DNA
   <213> Artificial Sequence
<220>
   <223> Synthetic
<400> 589
   gatgcatcc 9
<210> 590
   <211> 3
   <212> PRT
   <213> Artificial Sequence
<220>
   <223> Synthetic
<400> 590
   Asp Ala Ser
   1
<210> 591
   <211> 27
   <212> DNA
   <213> Artificial Sequence
<220>
   <223> Synthetic
<400> 591
   caacagctta atatttaccc attcact 27
<210> 592
   <211> 9
   <212> PRT
   <213> Artificial Sequence
<220>
   <223> Synthetic
<400> 592
   Gln Gln Leu Asn Ile Tyr Pro Phe Thr
   1 5
<210> 593
   <211> 363
   <212> DNA
   <213> Artificial Sequence
<220>
   <223> Synthetic
<400> 593
   gaagtgcagc tggtggagtc tgggggaggc ttggtacagc ctggcaggtc cctgagactc 60
   tcctgtgcag cctctggatt cacctttgat gattatgcca tgcactgggt ccggcaagct 120
   ccagggaagg gcctggagtg ggtctcaggt attaattgga acagtggtag cataggctat 180
   gcggactctg tgaagggccg attcaccatc tccagagaca acgccaagca ctccctgtat 240
   ctgcaaatga acagtctgag acctgaggac acggccttgt attactgtgt aaaagaggtg 300
   actacgggat actactacgg tatggacgtc tggggccaag ggaccacggt caccgtctcc 360
   tca                                                               363
<210> 594
   <211> 121
   <212> PRT
   <213> Artificial Sequence
<220>
   <223> Synthetic
<400> 594
   Glu Val Gln Leu Val Glu Ser Gly Gly Gly Leu Val Gln Pro Gly Arg
    1               5                  10                  15
   Ser Leu Arg Leu Ser Cys Ala Ala Ser Gly Phe Thr Phe Asp Asp Tyr
               20                  25                  30
   Ala Met His Trp Val Arg Gln Ala Pro Gly Lys Gly Leu Glu Trp Val
           35                  40                  45
   Ser Gly Ile Asn Trp Asn Ser Gly Ser Ile Gly Tyr Ala Asp Ser Val
       50                  55                  60
   Lys Gly Arg Phe Thr Ile Ser Arg Asp Asn Ala Lys His Ser Leu Tyr
   65                  70                  75                  80
   Leu Gln Met Asn Ser Leu Arg Pro Glu Asp Thr Ala Leu Tyr Tyr Cys
                   85                  90                  95
   Val Lys Glu Val Thr Thr Gly Tyr Tyr Tyr Gly Met Asp Val Trp Gly
               100                 105                 110
   Gln Gly Thr Thr Val Thr Val Ser Ser
           115                 120
<210> 595
   <211> 321
   <212> DNA
   <213> Artificial Sequence
<220>
   <223> Synthetic
<400> 595
   gacatccagt tgacccagtc tccatccttc ctgtctgcat ctgtaggaga cagagtcacc 60
   atcacttgct gggccagtca gggcattagc agttatttag cctggtatca gaaaaaacca 120
   gggaaagccc ctaacctcct gatctatgat gcatccactt tgcaaagtgg ggtcccatca 180
   aggttcagcg gcagtggatc tgggacagaa ttcactctca cactcagcag cctgcagcct 240
   gaagattttg caacttatta ctgtcaacag cttaatattt acccattcac tttcggccct 300
   gggaccaaag tggatatcaa a                                           321
<210> 596
   <211> 107
   <212> PRT
   <213> Artificial Sequence
<220>
   <223> Synthetic
<400> 596
   Asp Ile Gln Leu Thr Gln Ser Pro Ser Phe Leu Ser Ala Ser Val Gly
    1               5                  10                  15
   Asp Arg Val Thr Ile Thr Cys Trp Ala Ser Gln Gly Ile Ser Ser Tyr
               20                  25                  30
   Leu Ala Trp Tyr Gln Lys Lys Pro Gly Lys Ala Pro Asn Leu Leu Ile
           35                  40                  45
   Tyr Asp Ala Ser Thr Leu Gln Ser Gly Val Pro Ser Arg Phe Ser Gly
       50                  55                  60
   Ser Gly Ser Gly Thr Glu Phe Thr Leu Thr Leu Ser Ser Leu Gln Pro
   65                  70                  75                  80
   Glu Asp Phe Ala Thr Tyr Tyr Cys Gln Gln Leu Asn Ile Tyr Pro Phe
                   85                  90                  95
   Thr Phe Gly Pro Gly Thr Lys Val Asp Ile Lys
               100                 105
<210> 597
   <211> 363
   <212> DNA
   <213> Artificial Sequence
<220>
   <223> Synthetic
<400> 597
   gaagtgcagc tggtggagtc tgggggaggc ttggtacagc ctggcaggtc cctgagactc 60
   tcctgtgcag cctctggatt cacctttgat gattatgcca tgcactgggt ccggcaagct 120
   ccagggaagg gcctggagtg ggtctcaggt attaattgga acagtggtag cataggctat 180
   gcggactctg tgaagggccg attcaccatc tccagagaca acgccaagaa ctccctgtat 240
   ctgcaaatga acagtctgag agctgaggac acggccttgt attactgtgt aaaagaggtg 300
   actacgggat actactacgg tatggacgtc tgggggcaag ggaccacggt caccgtctcc 360
   tca                                                               363
<210> 598
   <211> 121
   <212> PRT
   <213> Artificial Sequence
<220>
   <223> Synthetic
<400> 598
   Glu Val Gln Leu Val Glu Ser Gly Gly Gly Leu Val Gln Pro Gly Arg
    1               5                  10                  15
   Ser Leu Arg Leu Ser Cys Ala Ala Ser Gly Phe Thr Phe Asp Asp Tyr
               20                  25                  30
   Ala Met His Trp Val Arg Gln Ala Pro Gly Lys Gly Leu Glu Trp Val
           35                  40                  45
   Ser Gly Ile Asn Trp Asn Ser Gly Ser Ile Gly Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Leu Tyr Tyr Cys
                   85                  90                  95
   Val Lys Glu Val Thr Thr Gly Tyr Tyr Tyr Gly Met Asp Val Trp Gly
               100                 105                 110
   Gln Gly Thr Thr Val Thr Val Ser Ser
           115                 120
<210> 599
   <211> 321
   <212> DNA
   <213> Artificial Sequence
<220>
   <223> Synthetic
<400> 599
   gacatccagt tgacccagtc tccatccttc ctgtctgcat ctgtaggaga cagagtcacc 60
   atcacttgcc gggccagtca gggcattagc agttatttag cctggtatca gcaaaaacca 120
   gggaaagccc ctaagctcct gatctatgat gcatccactt tgcaaagtgg ggtcccatca 180
   aggttcagcg gcagtggatc tgggacagaa ttcactctca caatcagcag cctgcagcct 240
   gaagattttg caacttatta ctgtcaacag cttaatattt acccattcac tttcggccct 300
   gggaccaaag tggatatcaa a                                           321
<210> 600
   <211> 107
   <212> PRT
   <213> Artificial Sequence
<220>
   <223> Synthetic
<400> 600
   Asp Ile Gln Leu Thr Gln Ser Pro Ser Phe Leu Ser Ala Ser Val Gly
    1               5                  10                  15
   Asp Arg Val Thr Ile Thr Cys Arg Ala Ser Gln Gly Ile Ser Ser Tyr
               20                  25                  30
   Leu Ala Trp Tyr Gln Gln Lys Pro Gly Lys Ala Pro Lys Leu Leu Ile
           35                  40                  45
   Tyr Asp Ala Ser Thr Leu Gln Ser Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Glu Asp Phe Ala Thr Tyr Tyr Cys Gln Gln Leu Asn Ile Tyr Pro Phe
                   85                  90                  95
   Thr Phe Gly Pro Gly Thr Lys Val Asp Ile Lys
               100                 105
<210> 601
   <211> 366
   <212> DNA
   <213> Artificial Sequence
<220>
   <223> Synthetic
<400> 601
   gaggtgcagt tgttggagtc tgggggaggc ttggtacagc ctggggggtc cctgagactc 60
   tcctgtgcag cctctggatt cacgtttagt agctatgcca tgaactgggt ccgccaggct 120
   ccagggaagg ggctggattg ggtctcaggt atcagtggta atggtggtag cacctactac 180
   gcagactccg tgaagggccg gttcaccatc tccagagaca tttccaagaa cacgctgtat 240
   gtgcaaatgc acagcctgag agtcgaggac acggccgttt actactgtgc gaaagcccgt 300
   tattacgatt tttggggggg gaatttcgat ctctggggcc gtggcaccca ggtcactgtc 360
   tcctca                                                            366
<210> 602
   <211> 122
   <212> PRT
   <213> Artificial Sequence
<220>
   <223> Synthetic
<400> 602
   Glu Val Gln Leu Leu Glu Ser Gly Gly Gly Leu Val Gln Pro Gly Gly
    1               5                  10                  15
   Ser Leu Arg Leu Ser Cys Ala Ala Ser Gly Phe Thr Phe Ser Ser Tyr
               20                  25                  30
   Ala Met Asn Trp Val Arg Gln Ala Pro Gly Lys Gly Leu Asp Trp Val
           35                  40                  45
   Ser Gly Ile Ser Gly Asn Gly Gly Ser Thr Tyr Tyr Ala Asp Ser Val
       50                  55                  60
   Lys Gly Arg Phe Thr Ile Ser Arg Asp Ile Ser Lys Asn Thr Leu Tyr
   65                  70                  75                  80
   Val Gln Met His Ser Leu Arg Val Glu Asp Thr Ala Val Tyr Tyr Cys
                   85                  90                  95
   Ala Lys Ala Arg Tyr Tyr Asp Phe Trp Gly Gly Asn Phe Asp Leu Trp
               100                 105                 110
   Gly Arg Gly Thr Gln Val Thr Val Ser Ser
           115                 120
<210> 603
   <211> 24
   <212> DNA
   <213> Artificial Sequence
<220>
   <223> Synthetic
<400> 603
   ggattcacgt ttagtagcta tgcc 24
<210> 604
   <211> 8
   <212> PRT
   <213> Artificial Sequence
<220>
   <223> Synthetic
<400> 604
   Gly Phe Thr Phe Ser Ser Tyr Ala
   1 5
<210> 605
   <211> 24
   <212> DNA
   <213> Artificial Sequence
<220>
   <223> Synthetic
<400> 605
   atcagtggta atggtggtag cacc 24
<210> 606
   <211> 8
   <212> PRT
   <213> Artificial Sequence
<220>
   <223> Synthetic
<400> 606
   Ile Ser Gly Asn Gly Gly Ser Thr
   1 5
<210> 607
   <211> 45
   <212> DNA
   <213> Artificial Sequence
<220>
   <223> Synthetic
<400> 607
   gcgaaagccc gttattacga tttttggggg gggaatttcg atctc 45
<210> 608
   <211> 15
   <212> PRT
   <213> Artificial Sequence
<220>
   <223> Synthetic
<400> 608
   Ala Lys Ala Arg Tyr Tyr Asp Phe Trp Gly Gly Asn Phe Asp Leu
    1               5                  10                  15
<210> 609
   <211> 324
   <212> DNA
   <213> Artificial Sequence
<220>
   <223> Synthetic
<400> 609
   gaaattgtgt tgacgcagtc tccaggcacc ctgtctttgt ctccagggga aagagccacc 60
   ctctcctgca gggccagtca gagtgttagc atcaggtact tagcctggta tcagcagaaa 120
   cctggccagg ctcccaggct cctcatctat ggtgcatcca gcagggccac tggcatccca 180
   gacaggttca gtgtcagtgt gtctgggaca gacttcactc tcaccatcac tagactggag 240
   cctgaagatt ttgcagtcta ttactgtcag caatatggta gttcaccgct cactttcggc 300
   ggagggacca aggtggagat caaa                                        324
<210> 610
   <211> 108
   <212> PRT
   <213> Artificial Sequence
<220>
   <223> Synthetic
<400> 610
   Glu Ile Val Leu Thr Gln Ser Pro Gly Thr Leu Ser Leu Ser Pro Gly
    1               5                  10                  15
   Glu Arg Ala Thr Leu Ser Cys Arg Ala Ser Gln Ser Val Ser Ile Arg
               20                  25                  30
   Tyr Leu Ala Trp Tyr Gln Gln Lys Pro Gly Gln Ala Pro Arg Leu Leu
           35                  40                  45
   Ile Tyr Gly Ala Ser Ser Arg Ala Thr Gly Ile Pro Asp Arg Phe Ser
       50                  55                  60
   Val Ser Val Ser Gly Thr Asp Phe Thr Leu Thr Ile Thr Arg Leu Glu
   65                  70                  75                  80
   Pro Glu Asp Phe Ala Val Tyr Tyr Cys Gln Gln Tyr Gly Ser Ser Pro
                   85                  90                  95
   Leu Thr Phe Gly Gly Gly Thr Lys Val Glu Ile Lys
               100                 105
<210> 611
   <211> 21
   <212> DNA
   <213> Artificial Sequence
<220>
   <223> Synthetic
<400> 611
   cagagtgtta gcatcaggta c 21
<210> 612
   <211> 7
   <212> PRT
   <213> Artificial Sequence
<220>
   <223> Synthetic
<400> 612
   Gln Ser Val Ser Ile Arg Tyr
   1 5
<210> 613
   <211> 9
   <212> DNA
   <213> Artificial Sequence
<220>
   <223> Synthetic
<400> 613
   ggtgcatcc 9
<210> 614
   <211> 3
   <212> PRT
   <213> Artificial Sequence
<220>
   <223> Synthetic
<400> 614
   Gly Ala Ser
   1
<210> 615
   <211> 27
   <212> DNA
   <213> Artificial Sequence
<220>
   <223> Synthetic
<400> 615
   cagcaatatg gtagttcacc gctcact 27
<210> 616
   <211> 9
   <212> PRT
   <213> Artificial Sequence
<220>
   <223> Synthetic
<400> 616
   Gln Gln Tyr Gly Ser Ser Pro Leu Thr
   1               5
<210> 617
   <211> 366
   <212> DNA
   <213> Artificial Sequence
<220>
   <223> Synthetic
<400> 617
   gaggtgcagt tgttggagtc tgggggaggc ttggtacagc ctggggggtc cctgagactc 60
   tcctgtgcag cctctggatt cacgtttagt agctatgcca tgaactgggt ccgccaggct 120
   ccagggaagg ggctggattg ggtctcaggt atcagtggta atggtggtag cacctactac 180
   gcagactccg tgaagggccg gttcaccatc tccagagaca tttccaagaa cacgctgtat 240
   gtgcaaatgc acagcctgag agtcgaggac acggccgttt actactgtgc gaaagcccgt 300
   tattacgatt tttggggggg gaatttcgat ctctggggcc gtggcaccct ggtcactgtc 360
   tcctca                                                            366
<210> 618
   <211> 122
   <212> PRT
   <213> Artificial Sequence
<220>
   <223> Synthetic
<400> 618
   Glu Val Gln Leu Leu Glu Ser Gly Gly Gly Leu Val Gln Pro Gly Gly
    1               5                  10                  15
   Ser Leu Arg Leu Ser Cys Ala Ala Ser Gly Phe Thr Phe Ser Ser Tyr
               20                  25                  30
   Ala Met Asn Trp Val Arg Gln Ala Pro Gly Lys Gly Leu Asp Trp Val
           35                  40                  45
   Ser Gly Ile Ser Gly Asn Gly Gly Ser Thr Tyr Tyr Ala Asp Ser Val
       50                  55                  60
   Lys Gly Arg Phe Thr Ile Ser Arg Asp Ile Ser Lys Asn Thr Leu Tyr
   65                  70                  75                  80
   Val Gln Met His Ser Leu Arg Val Glu Asp Thr Ala Val Tyr Tyr Cys
                   85                  90                  95
   Ala Lys Ala Arg Tyr Tyr Asp Phe Trp Gly Gly Asn Phe Asp Leu Trp
               100                 105                 110
   Gly Arg Gly Thr Leu Val Thr Val Ser Ser
           115                 120
<210> 619
   <211> 324
   <212> DNA
   <213> Artificial Sequence
<220>
   <223> Synthetic
<400> 619
   gaaattgtgt tgacgcagtc tccaggcacc ctgtctttgt ctccagggga aagagccacc 60
   ctctcctgca gggccagtca gagtgttagc atcaggtact tagcctggta tcagcagaaa 120
   cctggccagg ctcccaggct cctcatctat ggtgcatcca gcagggccac tggcatccca 180
   gacaggttca gtgtcagtgt gtctgggaca gacttcactc tcaccatcac tagactggag 240
   cctgaagatt ttgcagtcta ttactgtcag caatatggta gttcaccgct cactttcggc 300
   ggagggacca aggtggagat caaa                                        324
<210> 620
   <211> 108
   <212> PRT
   <213> Artificial Sequence
<220>
   <223> Synthetic
<400> 620
   Glu Ile Val Leu Thr Gln Ser Pro Gly Thr Leu Ser Leu Ser Pro Gly
    1               5                  10                  15
   Glu Arg Ala Thr Leu Ser Cys Arg Ala Ser Gln Ser Val Ser Ile Arg
               20                  25                  30
   Tyr Leu Ala Trp Tyr Gln Gln Lys Pro Gly Gln Ala Pro Arg Leu Leu
           35                  40                  45
   Ile Tyr Gly Ala Ser Ser Arg Ala Thr Gly Ile Pro Asp Arg Phe Ser
       50                  55                  60
   Val Ser Val Ser Gly Thr Asp Phe Thr Leu Thr Ile Thr Arg Leu Glu
   65                  70                  75                  80
   Pro Glu Asp Phe Ala Val Tyr Tyr Cys Gln Gln Tyr Gly Ser Ser Pro
                   85                  90                  95
   Leu Thr Phe Gly Gly Gly Thr Lys Val Glu Ile Lys
               100                 105
<210> 621
   <211> 366
   <212> DNA
   <213> Artificial Sequence
<220>
   <223> Synthetic
<400> 621
   gaggtgcagc tgttggagtc tgggggaggc ttggtacagc ctggggggtc cctgagactc 60
   tcctgtgcag cctctggatt cacgtttagt agctatgcca tgagctgggt ccgccaggct 120
   ccagggaagg ggctggagtg ggtctcagct atcagtggta atggtggtag cacctactac 180
   gcagactccg tgaagggccg gttcaccatc tccagagaca attccaagaa cacgctgtat 240
   ctgcaaatga acagcctgag agccgaggac acggccgtat attactgtgc gaaagcccgt 300
   tattacgatt tttggggggg gaatttcgat ctctggggcc gtggcaccct ggtcactgtc 360
   tcctca                                                            366
<210> 622
   <211> 122
   <212> PRT
   <213> Artificial Sequence
<220>
   <223> Synthetic
<400> 622
   Glu Val Gln Leu Leu Glu Ser Gly Gly Gly Leu Val Gln Pro Gly Gly
    1               5                  10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Ala Ile Ser Gly Asn Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Ala Arg Tyr Tyr Asp Phe Trp Gly Gly Asn Phe Asp Leu Trp
               100                 105                 110
   Gly Arg Gly Thr Leu Val Thr Val Ser Ser
           115                 120
<210> 623
   <211> 324
   <212> DNA
   <213> Artificial Sequence
<220>
   <223> Synthetic
<400> 623
   gaaattgtgt tgacgcagtc tccaggcacc ctgtctttgt ctccagggga aagagccacc 60
   ctctcctgca gggccagtca gagtgttagc atcaggtact tagcctggta ccagcagaaa 120
   cctggccagg ctcccaggct cctcatctat ggtgcatcca gcagggccac tggcatccca 180
   gacaggttca gtggcagtgg gtctgggaca gacttcactc tcaccatcag cagactggag 240
   cctgaagatt ttgcagtgta ttactgtcag caatatggta gttcaccgct cactttcggc 300
   ggagggacca aggtggagat caaa                                        324
<210> 624
   <211> 108
   <212> PRT
   <213> Artificial Sequence
<220>
   <223> Synthetic
<400> 624
   Glu Ile Val Leu Thr Gln Ser Pro Gly Thr Leu Ser Leu Ser Pro Gly
    1               5                  10                  15
   Glu Arg Ala Thr Leu Ser Cys Arg Ala Ser Gln Ser Val Ser Ile Arg
               20                  25                  30
   Tyr Leu Ala Trp Tyr Gln Gln Lys Pro Gly Gln Ala Pro Arg Leu Leu
           35                  40                  45
   Ile Tyr Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Tyr Gly Ser Ser Pro
                   85                  90                  95
   Leu Thr Phe Gly Gly Gly Thr Lys Val Glu Ile Lys
               100                 105
<210> 625
   <211> 381
   <212> DNA
   <213> Artificial Sequence
<220>
   <223> Synthetic
<400> 625
   caggttcagc tggtgcagtc tggacctgag gtgaagaacc ctggggcctc agtgaaggtc 60
   tcctgcaagg cttctggtta cacctttacc acctatggta tcagttgggt acgacaggcc 120
   cctggacaag ggcttgagtg gatgggatgg atcagcggtt acaatggtaa aacaaacgat 180
   gcacagaagt tccaggacag agtcgccatg accacagaca catccacgag cacagcctac 240
   atggagctga ggagcctgag atctgacgac acggccattt attactgttc gagagatcgt 300
   ttagtagtac cacctgccct ttattattcc tactacgtta tggacgtctg gggccaaggg 360
   accacggtca ccgtctcctc a                                           381
<210> 626
   <211> 127
   <212> PRT
   <213> Artificial Sequence
<220>
   <223> Synthetic
<400> 626
   Gln Val Gln Leu Val Gln Ser Gly Pro Glu Val Lys Asn Pro Gly Ala
    1               5                  10                  15
   Ser Val Lys Val Ser Cys Lys Ala Ser Gly Tyr Thr Phe Thr Thr Tyr
               20                  25                  30
   Gly Ile Ser Trp Val Arg Gln Ala Pro Gly Gln Gly Leu Glu Trp Met
           35                  40                  45
   Gly Trp Ile Ser Gly Tyr Asn Gly Lys Thr Asn Asp Ala Gln Lys Phe
       50                  55                  60
   Gln Asp Arg Val Ala Met Thr Thr Asp Thr Ser Thr Ser Thr Ala Tyr
   65                  70                  75                  80
   Met Glu Leu Arg Ser Leu Arg Ser Asp Asp Thr Ala Ile Tyr Tyr Cys
                   85                  90                  95
   Ser Arg Asp Arg Leu Val Val Pro Pro Ala Leu Tyr Tyr Ser Tyr Tyr
               100                 105                 110
   Val Met Asp Val Trp Gly Gln Gly Thr Thr Val Thr Val Ser Ser
           115                 120                 125
<210> 627
   <211> 24
   <212> DNA
   <213> Artificial Sequence
<220>
   <223> Synthetic
<400> 627
   ggttacacct ttaccaccta tggt 24
<210> 628
   <211> 8
   <212> PRT
   <213> Artificial Sequence
<220>
   <223> Synthetic
<400> 628
   Gly Tyr Thr Phe Thr Thr Tyr Gly
   1 5
<210> 629
   <211> 24
   <212> DNA
   <213> Artificial Sequence
<220>
   <223> Synthetic
<400> 629
   atcagcggtt acaatggtaa aaca 24
<210> 630
   <211> 8
   <212> PRT
   <213> Artificial Sequence
<220>
   <223> Synthetic
<400> 630
   Ile Ser Gly Tyr Asn Gly Lys Thr
   1 5
<210> 631
   <211> 60
   <212> DNA
   <213> Artificial Sequence
<220>
   <223> Synthetic
<400> 631
   tcgagagatc gtttagtagt accacctgcc ctttattatt cctactacgt tatggacgtc 60
<210> 632
   <211> 20
   <212> PRT
   <213> Artificial Sequence
<220>
   <223> Synthetic
<400> 632
   Ser Arg Asp Arg Leu Val Val Pro Pro Ala Leu Tyr Tyr Ser Tyr Tyr
    1               5                  10                  15
   Val Met Asp Val
               20
<210> 633
   <211> 336
   <212> DNA
   <213> Artificial Sequence
<220>
   <223> Synthetic
<400> 633
   gatgttgtga tgactcagtc tccactctcc ctgcccgtca cccttggaca gccggcctcc 60
   atctcctgca ggtctagtca aagcctcgta tacagtgatg gaaacaccta cttgaattgg 120
   tttcagcaga ggccaggtca atctccaagg cgcctaattt ataaggtttc taaccgggac 180
   tctggggtcc cagacagatt cagcggcagt gggtcaggca ctgatttcac actgaaaatc 240
   agcagggtgg aggctgagga tgttggggtt tattactgca tgcaaggtac acactggccg 300
   tacacttttg gccaggggac caagctggag atcaaa                           336
<210> 634
   <211> 112
   <212> PRT
   <213> Artificial Sequence
<220>
   <223> Synthetic
<400> 634
   Asp Val Val Met Thr Gln Ser Pro Leu Ser Leu Pro Val Thr Leu Gly
    1               5                  10                  15
   Gln Pro Ala Ser Ile Ser Cys Arg Ser Ser Gln Ser Leu Val Tyr Ser
               20                  25                  30
   Asp Gly Asn Thr Tyr Leu Asn Trp Phe Gln Gln Arg Pro Gly Gln Ser
           35                  40                  45
   Pro Arg Arg Leu Ile Tyr Lys Val Ser Asn Arg Asp Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Gly
                   85                  90                  95
   Thr His Trp Pro Tyr Thr Phe Gly Gln Gly Thr Lys Leu Glu Ile Lys
               100                 105                 110
<210> 635
   <211> 33
   <212> DNA
   <213> Artificial Sequence
<220>
   <223> Synthetic
<400> 635
   caaagcctcg tatacagtga tggaaacacc tac 33
<210> 636
   <211> 11
   <212> PRT
   <213> Artificial Sequence
<220>
   <223> Synthetic
<400> 636
   Gln Ser Leu Val Tyr Ser Asp Gly Asn Thr Tyr
   1 5 10
<210> 637
   <211> 9
   <212> DNA
   <213> Artificial Sequence
<220>
   <223> Synthetic
<400> 637
   aaggtttct 9
<210> 638
   <211> 3
   <212> PRT
   <213> Artificial Sequence
<220>
   <223> Synthetic
<400> 638
   Lys Val Ser
   1
<210> 639
   <211> 27
   <212> DNA
   <213> Artificial Sequence
<220>
   <223> Synthetic
<400> 639
   atgcaaggta cacactggcc gtacact 27
<210> 640
   <211> 9
   <212> PRT
   <213> Artificial Sequence
<220>
   <223> Synthetic
<400> 640
   Met Gln Gly Thr His Trp Pro Tyr Thr
    1               5
<210> 641
   <211> 381
   <212> DNA
   <213> Artificial Sequence
<220>
   <223> Synthetic
<400> 641
   caggttcagc tggtgcagtc tggacctgag gtgaagaacc ctggggcctc agtgaaggtc 60
   tcctgcaagg cttctggtta cacctttacc acctatggta tcagttgggt acgacaggcc 120
   cctggacaag ggcttgagtg gatgggatgg atcagcggtt acaatggtaa aacaaacgat 180
   gcacagaagt tccaggacag agtcgccatg accacagaca catccacgag cacagcctac 240
   atggagctga ggagcctgag atctgacgac acggccattt attactgttc gagagatcgt 300
   ttagtagtac cacctgccct ttattattcc tactacgtta tggacgtctg gggccaaggg 360
   accacggtca ccgtctcctc a                                           381
<210> 642
   <211> 127
   <212> PRT
   <213> Artificial Sequence
<220>
   <223> Synthetic
<400> 642
   Gln Val Gln Leu Val Gln Ser Gly Pro Glu Val Lys Asn Pro Gly Ala
    1               5                  10                  15
   Ser Val Lys Val Ser Cys Lys Ala Ser Gly Tyr Thr Phe Thr Thr Tyr
               20                  25                  30
   Gly Ile Ser Trp Val Arg Gln Ala Pro Gly Gln Gly Leu Glu Trp Met
           35                  40                  45
   Gly Trp Ile Ser Gly Tyr Asn Gly Lys Thr Asn Asp Ala Gln Lys Phe
       50                  55                  60
   Gln Asp Arg Val Ala Met Thr Thr Asp Thr Ser Thr Ser Thr Ala Tyr
   65                  70                  75                  80
   Met Glu Leu Arg Ser Leu Arg Ser Asp Asp Thr Ala Ile Tyr Tyr Cys
                   85                  90                  95
   Ser Arg Asp Arg Leu Val Val Pro Pro Ala Leu Tyr Tyr Ser Tyr Tyr
               100                 105                 110
   Val Met Asp Val Trp Gly Gln Gly Thr Thr Val Thr Val Ser Ser
           115                 120                 125
<210> 643
   <211> 336
   <212> DNA
   <213> Artificial Sequence
<220>
   <223> Synthetic
<400> 643
   gatgttgtga tgactcagtc tccactctcc ctgcccgtca cccttggaca gccggcctcc 60
   atctcctgca ggtctagtca aagcctcgta tacagtgatg gaaacaccta cttgaattgg 120
   tttcagcaga ggccaggtca atctccaagg cgcctaattt ataaggtttc taaccgggac 180
   tctggggtcc cagacagatt cagcggcagt gggtcaggca ctgatttcac actgaaaatc 240
   agcagggtgg aggctgagga tgttggggtt tattactgca tgcaaggtac acactggccg 300
   tacacttttg gccaggggac caagctggag atcaaa                           336
<210> 644
   <211> 112
   <212> PRT
   <213> Artificial Sequence
<220>
   <223> Synthetic
<400> 644
   Asp Val Val Met Thr Gln Ser Pro Leu Ser Leu Pro Val Thr Leu Gly
    1               5                  10                  15
   Gln Pro Ala Ser Ile Ser Cys Arg Ser Ser Gln Ser Leu Val Tyr Ser
               20                  25                  30
   Asp Gly Asn Thr Tyr Leu Asn Trp Phe Gln Gln Arg Pro Gly Gln Ser
           35                  40                  45
   Pro Arg Arg Leu Ile Tyr Lys Val Ser Asn Arg Asp Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Gly
                   85                  90                  95
   Thr His Trp Pro Tyr Thr Phe Gly Gln Gly Thr Lys Leu Glu Ile Lys
               100                 105                 110
<210> 645
   <211> 381
   <212> DNA
   <213> Artificial Sequence
<220>
   <223> Synthetic
<400> 645
   caggttcagc tggtgcagtc tggagctgag gtgaagaagc ctggggcctc agtgaaggtc 60
   tcctgcaagg cttctggtta cacctttacc acctatggta tcagctgggt gcgacaggcc 120
   cctggacaag ggcttgagtg gatgggatgg atcagcggtt acaatggtaa aacaaactat 180
   gcacagaagc tccagggcag agtcaccatg accacagaca catccacgag cacagcctac 240
   atggagctga ggagcctgag atctgacgac acggccgtgt attactgttc gagagatcgt 300
   ttagtagtac cacctgccct ttattattcc tactacgtta tggacgtctg ggggcaaggg 360
   accacggtca ccgtctcctc a                                          381
<210> 646
   <211> 127
   <212> PRT
   <213> Artificial Sequence
<220>
   <223> Synthetic
<400> 646
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Thr Tyr
               20                  25                  30
   Gly Ile Ser Trp Val Arg Gln Ala Pro Gly Gln Gly Leu Glu Trp Met
           35                  40                  45
   Gly Trp Ile Ser Gly Tyr Asn Gly Lys Thr Asn Tyr Ala Gln Lys Leu
       50                  55                  60
   Gln Gly Arg Val Thr Met Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ser Arg Asp Arg Leu Val Val Pro Pro Ala Leu Tyr Tyr Ser Tyr Tyr
               100                 105                 110
   Val Met Asp Val Trp Gly Gln Gly Thr Thr Val Thr Val Ser Ser
           115                 120                 125
<210> 647
   <211> 336
   <212> DNA
   <213> Artificial Sequence
<220>
   <223> Synthetic
<400> 647
   gatgttgtga tgactcagtc tccactctcc ctgcccgtca cccttggaca gccggcctcc 60
   atctcctgca ggtctagtca aagcctcgta tacagtgatg gaaacaccta cttgaattgg 120
   tttcagcaga ggccaggcca atctccaagg cgcctaattt ataaggtttc taaccgggac 180
   tctggggtcc cagacagatt cagcggcagt gggtcaggca ctgatttcac actgaaaatc 240
   agcagggtgg aggctgagga tgttggggtt tattactgca tgcaaggtac acactggccg 300
   tacacttttg gccaggggac caagctggag atcaaa                          336
<210> 648
   <211> 112
   <212> PRT
   <213> Artificial Sequence
<220>
   <223> Synthetic
<400> 648
   Asp Val Val Met Thr Gln Ser Pro Leu Ser Leu Pro Val Thr Leu Gly
    1               5                  10                  15
   Gln Pro Ala Ser Ile Ser Cys Arg Ser Ser Gln Ser Leu Val Tyr Ser
               20                  25                  30
   Asp Gly Asn Thr Tyr Leu Asn Trp Phe Gln Gln Arg Pro Gly Gln Ser
           35                  40                  45
   Pro Arg Arg Leu Ile Tyr Lys Val Ser Asn Arg Asp Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Gly
                   85                  90                  95
   Thr His Trp Pro Tyr Thr Phe Gly Gln Gly Thr Lys Leu Glu Ile Lys
               100                 105                 110
<210> 649
   <211> 381
   <212> DNA
   <213> Artificial Sequence
<220>
   <223> Synthetic
<400> 649
   caggttcagc tggtgcagtc tggacctgag gtgaagaacc ctggggcctc agtgaaggtc 60
   tcctgcaagg cttctggtta cacctttacc acctatggta tcagttgggt acgacaggcc 120
   cctggacaag ggcttgagtg gatgggatgg atcagcggtt acaatggtaa aacaaacgat 180
   gcacagaagt tccaggacag agtcgccatg accacagaca catccacgag cacagcctac 240
   atggagctga ggagcctgag atctgacgac acggccattt attactgttc gagagatcgt 300
   ttagtagtac cacctgccct taattattac tactacgtta tggacgtctg gggccaaggg 360
   accacggtca ccgtctcctc a                                           381
<210> 650
   <211> 127
   <212> PRT
   <213> Artificial Sequence
<220>
   <223> Synthetic
<400> 650
   Gln Val Gln Leu Val Gln Ser Gly Pro Glu Val Lys Asn Pro Gly Ala
   1               5                   10                  15
   Ser Val Lys Val Ser Cys Lys Ala Ser Gly Tyr Thr Phe Thr Thr Tyr
               20                  25                  30
   Gly Ile Ser Trp Val Arg Gln Ala Pro Gly Gln Gly Leu Glu Trp Met
           35                  40                  45
   Gly Trp Ile Ser Gly Tyr Asn Gly Lys Thr Asn Asp Ala Gln Lys Phe
       50                  55                  60
   Gln Asp Arg Val Ala Met Thr Thr Asp Thr Ser Thr Ser Thr Ala Tyr
   65                  70                  75                  80
   Met Glu Leu Arg Ser Leu Arg Ser Asp Asp Thr Ala Ile Tyr Tyr Cys
                   85                  90                  95
   Ser Arg Asp Arg Leu Val Val Pro Pro Ala Leu Asn Tyr Tyr Tyr Tyr
               100                 105                 110
   Val Met Asp Val Trp Gly Gln Gly Thr Thr Val Thr Val Ser Ser
           115                 120                 125
<210> 651
   <211> 24
   <212> DNA
   <213> Artificial Sequence
<220>
   <223> Synthetic
<400> 651
   ggttacacct ttaccaccta tggt 24
<210> 652
   <211> 8
   <212> PRT
   <213> Artificial Sequence
<220>
   <223> Synthetic
<400> 652
   Gly Tyr Thr Phe Thr Thr Tyr Gly
   1               5
<210> 653
   <211> 24
   <212> DNA
   <213> Artificial Sequence
<220>
   <223> Synthetic
<400> 653
   atcagcggtt acaatggtaa aaca 24
<210> 654
   <211> 8
   <212> PRT
   <213> Artificial Sequence
<220>
   <223> Synthetic
<400> 654
   Ile Ser Gly Tyr Asn Gly Lys Thr
    1               5
<210> 655
   <211> 60
   <212> DNA
   <213> Artificial Sequence
<220>
   <223> Synthetic
<400> 655
   tcgagagatc gtttagtagt accacctgcc cttaattatt actactacgt tatggacgtc 60
<210> 656
   <211> 20
   <212> PRT
   <213> Artificial Sequence
<220>
   <223> Synthetic
<400> 656
   Ser Arg Asp Arg Leu Val Val Pro Pro Ala Leu Asn Tyr Tyr Tyr Tyr
    1               5                  10                  15
   Val Met Asp Val
               20
<210> 657
   <211> 336
   <212> DNA
   <213> Artificial Sequence
<220>
   <223> Synthetic
<400> 657
   gatgttgtga tgactcagtc tccactctcc ctgcccgtca cccttggaca gccggcctcc 60
   atctcctgca ggtctagtca aagcctcgta tacagtgatg gaaacaccta cttgaattgg 120
   tttcagcaga ggccaggtca atctccaagg cgcctaattt ataaggtttc taaccgggac 180
   tctggggtcc cagacagatt cagcggcagt gggtcaggca ctgatttcac actgaaaatc 240
   agcagggtgg aggctgagga tgttggggtt tattactgca tgcaaggtac acactggccg 300
   tacacttttg gccaggggac caagctggag atcaaa                           336
<210> 658
   <211> 112
   <212> PRT
   <213> Artificial Sequence
<220>
   <223> Synthetic
<400> 658
   Asp Val Val Met Thr Gln Ser Pro Leu Ser Leu Pro Val Thr Leu Gly
    1               5                  10                  15
   Gln Pro Ala Ser Ile Ser Cys Arg Ser Ser Gln Ser Leu Val Tyr Ser
               20                  25                  30
   Asp Gly Asn Thr Tyr Leu Asn Trp Phe Gln Gln Arg Pro Gly Gln Ser
           35                  40                  45
   Pro Arg Arg Leu Ile Tyr Lys Val Ser Asn Arg Asp Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Gly
                   85                  90                  95
   Thr His Trp Pro Tyr Thr Phe Gly Gln Gly Thr Lys Leu Glu Ile Lys
               100                 105                 110
<210> 659
   <211> 33
   <212> DNA
   <213> Artificial Sequence
<220>
   <223> Synthetic
<400> 659
   caaagcctcg tatacagtga tggaaacacc tac 33
<210> 660
   <211> 11
   <212> PRT
   <213> Artificial Sequence
<220>
   <223> Synthetic
<400> 660
   Gln Ser Leu Val Tyr Ser Asp Gly Asn Thr Tyr
    1               5                  10
<210> 661
   <211> 9
   <212> DNA
   <213> Artificial Sequence
<220>
   <223> Synthetic
<400> 661
   aaggtttct 9
<210> 662
   <211> 3
   <212> PRT
   <213> Artificial Sequence
<220>
   <223> Synthetic
<400> 662
   Lys Val Ser
   1
<210> 663
   <211> 27
   <212> DNA
   <213> Artificial Sequence
<220>
   <223> Synthetic
<400> 663
   atgcaaggta cacactggcc gtacact 27
<210> 664
   <211> 9
   <212> PRT
   <213> Artificial Sequence
<220>
   <223> Synthetic
<400> 664
   Met Gln Gly Thr His Trp Pro Tyr Thr
   1 5
<210> 665
   <211> 381
   <212> DNA
   <213> Artificial Sequence
<220>
   <223> Synthetic
<400> 665
   caggttcagc tggtgcagtc tggacctgag gtgaagaacc ctggggcctc agtgaaggtc 60
   tcctgcaagg cttctggtta cacctttacc acctatggta tcagttgggt acgacaggcc 120
   cctggacaag ggcttgagtg gatgggatgg atcagcggtt acaatggtaa aacaaacgat 180
   gcacagaagt tccaggacag agtcgccatg accacagaca catccacgag cacagcctac 240
   atggagctga ggagcctgag atctgacgac acggccattt attactgttc gagagatcgt 300
   ttagtagtac cacctgccct taattattac tactacgtta tggacgtctg ggccaagggg 360
   accacggtca ccgtctcctc a                                           381
<210> 666
   <211> 127
   <212> PRT
   <213> Artificial Sequence
<220>
   <223> Synthetic
<400> 666
   Gln Val Gln Leu Val Gln Ser Gly Pro Glu Val Lys Asn Pro Gly Ala
   1               5                   10                  15
   Ser Val Lys Val Ser Cys Lys Ala Ser Gly Tyr Thr Phe Thr Thr Tyr
               20                  25                  30
   Gly Ile Ser Trp Val Arg Gln Ala Pro Gly Gln Gly Leu Glu Trp Met
           35                  40                  45
   Gly Trp Ile Ser Gly Tyr Asn Gly Lys Thr Asn Asp Ala Gln Lys Phe
       50                  55                  60
   Gln Asp Arg Val Ala Met Thr Thr Asp Thr Ser Thr Ser Thr Ala Tyr
   65                  70                  75                  80
   Met Glu Leu Arg Ser Leu Arg Ser Asp Asp Thr Ala Ile Tyr Tyr Cys
                   85                  90                  95
   Ser Arg Asp Arg Leu Val Val Pro Pro Ala Leu Asn Tyr Tyr Tyr Tyr
               100                 105                 110
   Val Met Asp Val Trp Gly Gln Gly Thr Thr Val Thr Val Ser Ser
           115                 120                 125
<210> 667
   <211> 336
   <212> DNA
   <213> Artificial Sequence
<220>
   <223> Synthetic
<400> 667
   gatgttgtga tgactcagtc tccactctcc ctgcccgtca cccttggaca gccggcctcc 60
   atctcctgca ggtctagtca aagcctcgta tacagtgatg gaaacaccta cttgaattgg 120
   tttcagcaga ggccaggtca atctccaagg cgcctaattt ataaggtttc taaccgggac 180
   tctggggtcc cagacagatt cagcggcagt gggtcaggca ctgatttcac actgaaaatc 240
   agcagggtgg aggctgagga tgttggggtt tattactgca tgcaaggtac acactggccg 300
   tacacttttg gccaggggac caagctggag atcaaa                           336
<210> 668
   <211> 112
   <212> PRT
   <213> Artificial Sequence
<220>
   <223> Synthetic
<400> 668
   Asp Val Val Met Thr Gln Ser Pro Leu Ser Leu Pro Val Thr Leu Gly
   1               5                   10                  15
   Gln Pro Ala Ser Ile Ser Cys Arg Ser Ser Gln Ser Leu Val Tyr Ser
               20                  25                  30
   Asp Gly Asn Thr Tyr Leu Asn Trp Phe Gln Gln Arg Pro Gly Gln Ser
           35                  40                  45
   Pro Arg Arg Leu Ile Tyr Lys Val Ser Asn Arg Asp Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Gly
                   85                  90                  95
   Thr His Trp Pro Tyr Thr Phe Gly Gln Gly Thr Lys Leu Glu Ile Lys
               100                 105                 110
<210> 669
   <211> 381
   <212> DNA
   <213> Artificial Sequence
<220>
   <223> Synthetic
<400> 669
   caggttcagc tggtgcagtc tggagctgag gtgaagaagc ctggggcctc agtgaaggtc 60
   tcctgcaagg cttctggtta cacctttacc acctatggta tcagctgggt gcgacaggcc 120
   cctggacaag ggcttgagtg gatgggatgg atcagcggtt acaatggtaa aacaaactat 180
   gcacagaagc tccagggcag agtcaccatg accacagaca catccacgag cacagcctac 240
   atggagctga ggagcctgag atctgacgac acggccgtgt attactgttc gagagatcgt 300
   ttagtagtac cacctgccct taattattac tactacgtta tggacgtctg ggggcaaggg 360
   accacggtca ccgtctcctc a                                          381
<210> 670
   <211> 127
   <212> PRT
   <213> Artificial Sequence
<220>
   <223> Synthetic
<400> 670
   Gln Val Gln Leu Val Gln Ser Gly Ala Glu Val Lys Lys Pro Gly Ala
    1               5                  10                  15
   Ser Val Lys Val Ser Cys Lys Ala Ser Gly Tyr Thr Phe Thr Thr Tyr
               20                  25                  30
   Gly Ile Ser Trp Val Arg Gln Ala Pro Gly Gln Gly Leu Glu Trp Met
           35                  40                  45
   Gly Trp Ile Ser Gly Tyr Asn Gly Lys Thr Asn Tyr Ala Gln Lys Leu
       50                  55                  60
   Gln Gly Arg Val Thr Met Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ser Arg Asp Arg Leu Val Val Pro Pro Ala Leu Asn Tyr Tyr Tyr Tyr
               100                 105                 110
   Val Met Asp Val Trp Gly Gln Gly Thr Thr Val Thr Val Ser Ser
           115                 120                 125
<210> 671
   <211> 336
   <212> DNA
   <213> Artificial Sequence
<220>
   <223> Synthetic
<400> 671
   gatgttgtga tgactcagtc tccactctcc ctgcccgtca cccttggaca gccggcctcc 60
   atctcctgca ggtctagtca aagcctcgta tacagtgatg gaaacaccta cttgaattgg 120
   tttcagcaga ggccaggcca atctccaagg cgcctaattt ataaggtttc taaccgggac 180
   tctggggtcc cagacagatt cagcggcagt gggtcaggca ctgatttcac actgaaaatc 240
   agcagggtgg aggctgagga tgttggggtt tattactgca tgcaaggtac acactggccg 300
   tacacttttg gccaggggac caagctggag atcaaa                           336
<210> 672
   <211> 112
   <212> PRT
   <213> Artificial Sequence
<220>
   <223> Synthetic
<400> 672
   Asp Val Val Met Thr Gln Ser Pro Leu Ser Leu Pro Val Thr Leu Gly
    1               5                  10                  15
   Gln Pro Ala Ser Ile Ser Cys Arg Ser Ser Gln Ser Leu Val Tyr Ser
               20                  25                  30
   Asp Gly Asn Thr Tyr Leu Asn Trp Phe Gln Gln Arg Pro Gly Gln Ser
               35                  40                  45
   Pro Arg Arg Leu Ile Tyr Lys Val Ser Asn Arg Asp Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Gly
                   85                  90                  95
   Thr His Trp Pro Tyr Thr Phe Gly Gln Gly Thr Lys Leu Glu Ile Lys
               100                 105                 110
<210> 673
   <211> 381
   <212> DNA
   <213> Artificial Sequence
<220>
   <223> Synthetic
<400> 673
   caggttcagc tggtgcagtc tggacctgag gtgaagaacc ctggggcctc agtgaaggtc 60
   tcctgcaagg cttctggtta cacctttacc acctatggta tcagttgggt acgacaggcc 120
   cctggacaag ggcttgagtg gatgggatgg atcagcggtt acaatggtaa aacaaacgat 180
   gcacagaagt tccaggacag agtcgccatg accacagaca catccacgag cacagcctac 240
   atggagctga ggagcctgag atctgacgac acggccattt attactgttc gagagatcgt 300
   ttagtagtac cacctgccct ttattattac tactacgtta tggacgtctg gggccaaggg 360
   accacggtca ccgtctcctc a                                           381
<210> 674
   <211> 127
   <212> PRT
   <213> Artificial Sequence
<220>
   <223> Synthetic
<400> 674
   Gln Val Gln Leu Val Gln Ser Gly Pro Glu Val Lys Asn Pro Gly Ala
   1               5                   10                  15
   Ser Val Lys Val Ser Cys Lys Ala Ser Gly Tyr Thr Phe Thr Thr Tyr
               20                  25                  30
   Gly Ile Ser Trp Val Arg Gln Ala Pro Gly Gln Gly Leu Glu Trp Met
           35                  40                  45
   Gly Trp Ile Ser Gly Tyr Asn Gly Lys Thr Asn Asp Ala Gln Lys Phe
       50                  55                  60
   Gln Asp Arg Val Ala Met Thr Thr Asp Thr Ser Thr Ser Thr Ala Tyr
   65                  70                  75                  80
   Met Glu Leu Arg Ser Leu Arg Ser Asp Asp Thr Ala Ile Tyr Tyr Cys
                   85                  90                  95
   Ser Arg Asp Arg Leu Val Val Pro Pro Ala Leu Tyr Tyr Tyr Tyr Tyr
               100                 105                 110
   Val Met Asp Val Trp Gly Gln Gly Thr Thr Val Thr Val Ser Ser
           115                 120                 125
<210> 675
   <211> 24
   <212> DNA
   <213> Artificial Sequence
<220>
   <223> Synthetic
<400> 675
   ggttacacct ttaccaccta tggt 24
<210> 676
   <211> 8
   <212> PRT
   <213> Artificial Sequence
<220>
   <223> Synthetic
<400> 676
   Gly Tyr Thr Phe Thr Thr Tyr Gly
   1 5
<210> 677
   <211> 24
   <212> DNA
   <213> Artificial Sequence
<220>
   <223> Synthetic
<400> 677
   atcagcggtt acaatggtaa aaca 24
<210> 678
   <211> 8
   <212> PRT
   <213> Artificial Sequence
<220>
   <223> Synthetic
<400> 678
   Ile Ser Gly Tyr Asn Gly Lys Thr
   1 5
<210> 679
   <211> 60
   <212> DNA
   <213> Artificial Sequence
<220>
   <223> Synthetic
<400> 679
   tcgagagatc gtttagtagt accacctgcc ctttattatt actactacgt tatggacgtc 60
<210> 680
   <211> 20
   <212> PRT
   <213> Artificial Sequence
<220>
   <223> Synthetic
<400> 680
   Ser Arg Asp Arg Leu Val Val Pro Pro Ala Leu Tyr Tyr Tyr Tyr Tyr
    1               5                  10                  15
   Val Met Asp Val
               20
<210> 681
   <211> 336
   <212> DNA
   <213> Artificial Sequence
<220>
   <223> Synthetic
<400> 681
   gatgttgtga tgactcagtc tccactctcc ctgcccgtca cccttggaca gccggcctcc 60
   atctcctgca ggtctagtca aagcctcgta tacagtgatg gaaacaccta cttgaattgg 120
   tttcagcaga ggccaggtca atctccaagg cgcctaattt ataaggtttc taaccgggac 180
   tctggggtcc cagacagatt cagcggcagt gggtcaggca ctgatttcac actgaaaatc 240
   agcagggtgg aggctgagga tgttggggtt tattactgca tgcaaggtac acactggccg 300
   tacacttttg gccaggggac caagctggag atcaaa                          336
<210> 682
   <211> 112
   <212> PRT
   <213> Artificial Sequence
<220>
   <223> Synthetic
<400> 682
   Asp Val Val Met Thr Gln Ser Pro Leu Ser Leu Pro Val Thr Leu Gly
    1               5                  10                  15
   Gln Pro Ala Ser Ile Ser Cys Arg Ser Ser Gln Ser Leu Val Tyr Ser
               20                  25                  30
   Asp Gly Asn Thr Tyr Leu Asn Trp Phe Gln Gln Arg Pro Gly Gln Ser
           35                  40                  45
   Pro Arg Arg Leu Ile Tyr Lys Val Ser Asn Arg Asp Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Gly
                   85                  90                  95
   Thr His Trp Pro Tyr Thr Phe Gly Gln Gly Thr Lys Leu Glu Ile Lys
               100                 105                 110
<210> 683
   <211> 33
   <212> DNA
   <213> Artificial Sequence
<220>
   <223> Synthetic
<400> 683
   caaagcctcg tatacagtga tggaaacacc tac 33
<210> 684
   <211> 11
   <212> PRT
   <213> Artificial Sequence
<220>
   <223> Synthetic
<400> 684
   Gln Ser Leu Val Tyr Ser Asp Gly Asn Thr Tyr
    1               5                  10
<210> 685
   <211> 9
   <212> DNA
   <213> Artificial Sequence
<220>
   <223> Synthetic
<400> 685
   aaggtttct 9
<210> 686
   <211> 3
   <212> PRT
   <213> Artificial Sequence
<220>
   <223> Synthetic
<400> 686
   Lys Val Ser
   1
<210> 687
   <211> 27
   <212> DNA
   <213> Artificial Sequence
<220>
   <223> Synthetic
<400> 687
   atgcaaggta cacactggcc gtacact 27
<210> 688
   <211> 9
   <212> PRT
   <213> Artificial Sequence
<220>
   <223> Synthetic
<400> 688
   Met Gln Gly Thr His Trp Pro Tyr Thr
   1 5
<210> 689
   <211> 381
   <212> DNA
   <213> Artificial Sequence
<220>
   <223> Synthetic
<400> 689
   caggttcagc tggtgcagtc tggacctgag gtgaagaacc ctggggcctc agtgaaggtc 60
   tcctgcaagg cttctggtta cacctttacc acctatggta tcagttgggt acgacaggcc 120
   cctggacaag ggcttgagtg gatgggatgg atcagcggtt acaatggtaa aacaaacgat 180
   gcacagaagt tccaggacag agtcgccatg accacagaca catccacgag cacagcctac 240
   atggagctga ggagcctgag atctgacgac acggccattt attactgttc gagagatcgt 300
   ttagtagtac cacctgccct ttattattac tactacgtta tggacgtctg gggccaaggg 360
   accacggtca ccgtctcctc a                                           381
<210> 690
   <211> 127
   <212> PRT
   <213> Artificial Sequence
<220>
   <223> Synthetic
<400> 690
   Gln Val Gln Leu Val Gln Ser Gly Pro Glu Val Lys Asn Pro Gly Ala
    1               5                   10                  15
   Ser Val Lys Val Ser Cys Lys Ala Ser Gly Tyr Thr Phe Thr Thr Tyr
               20                  25                  30
   Gly Ile Ser Trp Val Arg Gln Ala Pro Gly Gln Gly Leu Glu Trp Met
           35                  40                  45
   Gly Trp Ile Ser Gly Tyr Asn Gly Lys Thr Asn Asp Ala Gln Lys Phe
       50                  55                  60
   Gln Asp Arg Val Ala Met Thr Thr Asp Thr Ser Thr Ser Thr Ala Tyr
   65                  70                  75                  80
   Met Glu Leu Arg Ser Leu Arg Ser Asp Asp Thr Ala Ile Tyr Tyr Cys
                   85                  90                  95
   Ser Arg Asp Arg Leu Val Val Pro Pro Ala Leu Tyr Tyr Tyr Tyr Tyr
               100                 105                 110
   Val Met Asp Val Trp Gly Gln Gly Thr Thr Val Thr Val Ser Ser
           115                 120                 125
<210> 691
   <211> 336
   <212> DNA
   <213> Artificial Sequence
<220>
   <223> Synthetic
<400> 691
   gatgttgtga tgactcagtc tccactctcc ctgcccgtca cccttggaca gccggcctcc 60
   atctcctgca ggtctagtca aagcctcgta tacagtgatg gaaacaccta cttgaattgg 120
   tttcagcaga ggccaggtca atctccaagg cgcctaattt ataaggtttc taaccgggac 180
   tctggggtcc cagacagatt cagcggcagt gggtcaggca ctgatttcac actgaaaatc 240
   agcagggtgg aggctgagga tgttggggtt tattactgca tgcaaggtac acactggccg 300
   tacacttttg gccaggggac caagctggag atcaaa                           336
<210> 692
   <211> 112
   <212> PRT
   <213> Artificial Sequence
<220>
   <223> Synthetic
<400> 692
   Asp Val Val Met Thr Gln Ser Pro Leu Ser Leu Pro Val Thr Leu Gly
    1               5                  10                  15
   Gln Pro Ala Ser Ile Ser Cys Arg Ser Ser Gln Ser Leu Val Tyr Ser
               20                  25                  30
   Asp Gly Asn Thr Tyr Leu Asn Trp Phe Gln Gln Arg Pro Gly Gln Ser
           35                  40                  45
   Pro Arg Arg Leu Ile Tyr Lys Val Ser Asn Arg Asp Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Gly
                   85                  90                  95
   Thr His Trp Pro Tyr Thr Phe Gly Gln Gly Thr Lys Leu Glu Ile Lys
               100                 105                 110
<210> 693
   <211> 381
   <212> DNA
   <213> Artificial Sequence
<220>
   <223> Synthetic
<400> 693
   caggttcagc tggtgcagtc tggagctgag gtgaagaagc ctggggcctc agtgaaggtc 60
   tcctgcaagg cttctggtta cacctttacc acctatggta tcagctgggt gcgacaggcc 120
   cctggacaag ggcttgagtg gatgggatgg atcagcggtt acaatggtaa aacaaactat 180
   gcacagaagc tccagggcag agtcaccatg accacagaca catccacgag cacagcctac 240
   atggagctga ggagcctgag atctgacgac acggccgtgt attactgttc gagagatcgt 300
   ttagtagtac cacctgccct ttattattac tactacgtta tggacgtctg gggccaaggg 360
   accacggtca ccgtctcctc a                                           381
<210> 694
   <211> 127
   <212> PRT
   <213> Artificial Sequence
<220>
   <223> Synthetic
<400> 694
   Gln Val Gln Leu Val Gln Ser Gly Ala Glu Val Lys Lys Pro Gly Ala
    1               5                  10                  15
   Ser Val Lys Val Ser Cys Lys Ala Ser Gly Tyr Thr Phe Thr Thr Tyr
               20                  25                  30
   Gly Ile Ser Trp Val Arg Gln Ala Pro Gly Gln Gly Leu Glu Trp Met
           35                  40                  45
   Gly Trp Ile Ser Gly Tyr Asn Gly Lys Thr Asn Tyr Ala Gln Lys Leu
       50                  55                  60
   Gln Gly Arg Val Thr Met Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ser Arg Asp Arg Leu Val Val Pro Pro Ala Leu Tyr Tyr Tyr Tyr Tyr
               100                 105                 110
   Val Met Asp Val Trp Gly Gln Gly Thr Thr Val Thr Val Ser Ser
           115                 120                 125
<210> 695
   <211> 336
   <212> DNA
   <213> Artificial Sequence
<220>
   <223> Synthetic
<400> 695
   gatgttgtga tgactcagtc tccactctcc ctgcccgtca cccttggaca gccggcctcc 60
   atctcctgca ggtctagtca aagcctcgta tacagtgatg gaaacaccta cttgaattgg 120
   tttcagcaga ggccaggcca atctccaagg cgcctaattt ataaggtttc taaccgggac 180
   tctggggtcc cagacagatt cagcggcagt gggtcaggca ctgatttcac actgaaaatc 240
   agcagggtgg aggctgagga tgttggggtt tattactgca tgcaaggtac acactggccg 300
   tacacttttg gccaggggac caagctggag atcaaa                           336
<210> 696
   <211> 112
   <212> PRT
   <213> Artificial Sequence
<220>
   <223> Synthetic
<400> 696
   Asp Val Val Met Thr Gln Ser Pro Leu Ser Leu Pro Val Thr Leu Gly
    1               5                  10                  15
   Gln Pro Ala Ser Ile Ser Cys Arg Ser Ser Gln Ser Leu Val Tyr Ser
               20                  25                  30
   Asp Gly Asn Thr Tyr Leu Asn Trp Phe Gln Gln Arg Pro Gly Gln Ser
           35                  40                  45
   Pro Arg Arg Leu Ile Tyr Lys Val Ser Asn Arg Asp Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Gly
                   85                  90                  95
   Thr His Trp Pro Tyr Thr Phe Gly Gln Gly Thr Lys Leu Glu Ile Lys
               100                 105                 110
<210> 697
   <211> 384
   <212> DNA
   <213> Artificial Sequence
<220>
   <223> Synthetic
<400> 697
   caggtgcacc tggtggagtc tgggggaggc ttggtcaagc ctggagggtc cctgagactc 60
   tcctgtgcag cctctggatt caccttcagt gaccactaca tgagctggat ccgccaggct 120
   ccagggaagg ggctggagtg gatttcatac attagtaatg atggtggtac caaatactat 180
   gtggactctg tggagggccg attcatcatt tccagggaca acgccaagaa ctcattgtat 240
   ctacatatga acagcctcag agccgacgac acggccgtgt attactgtgc gagagatcag 300
   ggatatattg gctacgactc gtattattac tattcctacg gtatggacgt ctggggccaa 360
   gggaccacgg tcaccgtcgc ctca                                        384
<210> 698
   <211> 128
   <212> PRT
   <213> Artificial Sequence
<220>
   <223> Synthetic
<400> 698
   Gln Val His Leu Val Glu Ser Gly Gly Gly Leu Val Lys Pro Gly Gly
    1               5                  10                  15
   Ser Leu Arg Leu Ser Cys Ala Ala Ser Gly Phe Thr Phe Ser Asp His
               20                  25                  30
   Tyr Met Ser Trp Ile Arg Gln Ala Pro Gly Lys Gly Leu Glu Trp Ile
           35                  40                  45
   Ser Tyr Ile Ser Asn Asp Gly Gly Thr Lys Tyr Tyr Val Asp Ser Val
       50                  55                  60
   Glu Gly Arg Phe Ile Ile Ser Arg Asp Asn Ala Lys Asn Ser Leu Tyr
   65                  70                  75                  80
   Leu His Met Asn Ser Leu Arg Ala Asp Asp Thr Ala Val Tyr Tyr Cys
                   85                  90                  95
   Ala Arg Asp Gln Gly Tyr Ile Gly Tyr Asp Ser Tyr Tyr Tyr Tyr Ser
               100                 105                 110
   Tyr Gly Met Asp Val Trp Gly Gln Gly Thr Thr Val Thr Val Ala Ser
           115                 120                 125
<210> 699
   <211> 24
   <212> DNA
   <213> Artificial Sequence
<220>
   <223> Synthetic
<400> 699
   ggattcacct tcagtgacca ctac 24
<210> 700
   <211> 8
   <212> PRT
   <213> Artificial Sequence
<220>
   <223> Synthetic
<400> 700
   Gly Phe Thr Phe Ser Asp His Tyr
   1 5
<210> 701
   <211> 24
   <212> DNA
   <213> Artificial Sequence
<220>
   <223> Synthetic
<400> 701
   attagtaatg atggtggtac caaa 24
<210> 702
   <211> 8
   <212> PRT
   <213> Artificial Sequence
<220>
   <223> Synthetic
<400> 702
   Ile Ser Asn Asp Gly Gly Thr Lys
   1 5
<210> 703
   <211> 63
   <212> DNA
   <213> Artificial Sequence
<220>
   <223> Synthetic
<400> 703
   gcgagagatc agggatatat tggctacgac tcgtattatt actattccta cggtatggac 60
   gtc 63
<210> 704
   <211> 21
   <212> PRT
   <213> Artificial Sequence
<220>
   <223> Synthetic
<400> 704
   Ala Arg Asp Gln Gly Tyr Ile Gly Tyr Asp Ser Tyr Tyr Tyr Tyr Ser
    1               5                  10                  15
   Tyr Gly Met Asp Val
               20
<210> 705
   <211> 321
   <212> DNA
   <213> Artificial Sequence
<220>
   <223> Synthetic
<400> 705
   aaaattgtgt tgacgcagtc tccaggcacc ctgcctttgt ttccagggga aagagccacc 60
   ctctcctgta gggccagtca gagtgttaac aacaaattct tagcctggta ccagcagaaa 120
   tctggccagg ctcccaggct cctcatctat ggtgcatcca gcagggccac tggcatccca 180
   gacaggttca gtggcagtgg gtctgggacc gacttcactc tcaccatcag cggactggag 240
   cctgaagatt ttgaagtgta ttattgtcaa gtatatggta actcactcac tttcggcgga 300
   gggaccaagg tggagatcaa g                                           321
<210> 706
   <211> 107
   <212> PRT
   <213> Artificial Sequence
<220>
   <223> Synthetic
<400> 706
   Lys Ile Val Leu Thr Gln Ser Pro Gly Thr Leu Pro Leu Phe Pro Gly
    1               5                  10                  15
   Glu Arg Ala Thr Leu Ser Cys Arg Ala Ser Gln Ser Val Asn Asn Lys
               20                  25                  30
   Phe Leu Ala Trp Tyr Gln Gln Lys Ser Gly Gln Ala Pro Arg Leu Leu
           35                  40                  45
   Ile Tyr Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Gly Leu Glu
   65                  70                  75                  80
   Pro Glu Asp Phe Glu Val Tyr Tyr Cys Gln Val Tyr Gly Asn Ser Leu
                   85                  90                  95
   Thr Phe Gly Gly Gly Thr Lys Val Glu Ile Lys
               100                 105
<210> 707
   <211> 21
   <212> DNA
   <213> Artificial Sequence
<220>
   <223> Synthetic
<400> 707
   cagagtgtta acaacaaatt c 21
<210> 708
   <211> 7
   <212> PRT
   <213> Artificial Sequence
<220>
   <223> Synthetic
<400> 708
   Gln Ser Val Asn Asn Lys Phe
   1 5
<210> 709
   <211> 9
   <212> DNA
   <213> Artificial Sequence
<220>
   <223> Synthetic
<400> 709
   ggtgcatcc 9
<210> 710
   <211> 3
   <212> PRT
   <213> Artificial Sequence
<220>
   <223> Synthetic
<400> 710
   Gly Ala Ser
   1
<210> 711
   <211> 24
   <212> DNA
   <213> Artificial Sequence
<220>
   <223> Synthetic
<400> 711
   caagtatatg gtaactcact cact 24
<210> 712
   <211> 8
   <212> PRT
   <213> Artificial Sequence
<220>
   <223> Synthetic
<400> 712
   Gln Val Tyr Gly Asn Ser Leu Thr
   1               5
<210> 713
   <211> 384
   <212> DNA
   <213> Artificial Sequence
<220>
   <223> Synthetic
<400> 713
   caggtgcagc tggtggagtc tgggggaggc ttggtcaagc ctggagggtc cctgagactc 60
   tcctgtgcag cctctggatt caccttcagt gaccactaca tgagctggat ccgccaggct 120
   ccagggaagg ggctggagtg gatttcatac attagtaatg atggtggtac caaatactat 180
   gtggactctg tggagggccg attcatcatt tccagggaca acgccaagaa ctcattgtat 240
   ctacatatga acagcctcag agccgacgac acggccgtgt attactgtgc gagagatcag 300
   ggatatattg gctacgactc gtattattac tattcctacg gtatggacgt ctggggccaa 360
   gggaccacgg tcaccgtctc ctca                                        384
<210> 714
   <211> 128
   <212> PRT
   <213> Artificial Sequence
<220>
   <223> Synthetic
<400> 714
   Gln Val Gln Leu Val Glu Ser Gly Gly Gly Leu Val Lys Pro Gly Gly
    1               5                  10                  15
   Ser Leu Arg Leu Ser Cys Ala Ala Ser Gly Phe Thr Phe Ser Asp His
               20                  25                  30
   Tyr Met Ser Trp Ile Arg Gln Ala Pro Gly Lys Gly Leu Glu Trp Ile
           35                  40                  45
   Ser Tyr Ile Ser Asn Asp Gly Gly Thr Lys Tyr Tyr Val Asp Ser Val
       50                  55                  60
   Glu Gly Arg Phe Ile Ile Ser Arg Asp Asn Ala Lys Asn Ser Leu Tyr
   65                  70                  75                  80
   Leu His Met Asn Ser Leu Arg Ala Asp Asp Thr Ala Val Tyr Tyr Cys
                   85                  90                  95
   Ala Arg Asp Gln Gly Tyr Ile Gly Tyr Asp Ser Tyr Tyr Tyr Tyr Ser
               100                 105                 110
   Tyr Gly Met Asp Val Trp Gly Gln Gly Thr Thr Val Thr Val Ser Ser
           115                 120                 125
<210> 715
   <211> 321
   <212> DNA
   <213> Artificial Sequence
<220>
   <223> Synthetic
<400> 715
   gaaattgtgt tgacgcagtc tccaggcacc ctgcctttgt ttccagggga aagagccacc 60
   ctctcctgta gggccagtca gagtgttaac aacaaattct tagcctggta ccagcagaaa 120
   tctggccagg ctcccaggct cctcatctat ggtgcatcca gcagggccac tggcatccca 180
   gacaggttca gtggcagtgg gtctgggacc gacttcactc tcaccatcag cggactggag 240
   cctgaagatt ttgaagtgta ttattgtcaa gtatatggta actcactcac tttcggcgga 300
   gggaccaagg tggagatcaa a                                           321
<210> 716
   <211> 107
   <212> PRT
   <213> Artificial Sequence
<220>
   <223> Synthetic
<400> 716
   Glu Ile Val Leu Thr Gln Ser Pro Gly Thr Leu Pro Leu Phe Pro Gly
    1               5                  10                  15
   Glu Arg Ala Thr Leu Ser Cys Arg Ala Ser Gln Ser Val Asn Asn Lys
               20                  25                  30
   Phe Leu Ala Trp Tyr Gln Gln Lys Ser Gly Gln Ala Pro Arg Leu Leu
           35                  40                  45
   Ile Tyr Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Gly Leu Glu
   65                  70                  75                  80
   Pro Glu Asp Phe Glu Val Tyr Tyr Cys Gln Val Tyr Gly Asn Ser Leu
                   85                  90                  95
   Thr Phe Gly Gly Gly Thr Lys Val Glu Ile Lys
               100                 105
<210> 717
   <211> 384
   <212> DNA
   <213> Artificial Sequence
<220>
   <223> Synthetic
<400> 717
   caggtgcagc tggtggagtc tgggggaggc ttggtcaagc ctggagggtc cctgagactc 60
   tcctgtgcag cctctggatt caccttcagt gaccactaca tgagctggat ccgccaggct 120
   ccagggaagg ggctggagtg ggtttcatac attagtaatg atggtggtac caaatactac 180
   gcagactctg tgaagggccg attcaccatc tccagggaca acgccaagaa ctcactgtat 240
   ctgcaaatga acagcctgag agccgaggac acggccgtgt attactgtgc gagagatcag 300
   ggatatattg gctacgactc gtattattac tattcctacg gtatggacgt ctgggggcaa 360
   gggaccacgg tcaccgtctc ctca                                        384
<210> 718
   <211> 128
   <212> PRT
   <213> Artificial Sequence
<220>
   <223> Synthetic
<400> 718
   Gln Val Gln Leu Val Glu Ser Gly Gly Gly Leu Val Lys Pro Gly Gly
    1               5                  10                  15
   Ser Leu Arg Leu Ser Cys Ala Ala Ser Gly Phe Thr Phe Ser Asp His
               20                  25                  30
   Tyr Met Ser Trp Ile Arg Gln Ala Pro Gly Lys Gly Leu Glu Trp Val
           35                  40                  45
   Ser Tyr Ile Ser Asn Asp Gly Gly Thr Lys Tyr Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Arg Asp Gln Gly Tyr Ile Gly Tyr Asp Ser Tyr Tyr Tyr Tyr Ser
               100                 105                 110
   Tyr Gly Met Asp Val Trp Gly Gln Gly Thr Thr Val Thr Val Ser Ser
           115                 120                 125
<210> 719
   <211> 321
   <212> DNA
   <213> Artificial Sequence
<220>
   <223> Synthetic
<400> 719
   gaaattgtgt tgacgcagtc tccaggcacc ctgtctttgt ctccagggga aagagccacc 60
   ctctcctgca gggccagtca gagtgttaac aacaaattct tagcctggta ccagcagaaa 120
   cctggccagg ctcccaggct cctcatctat ggtgcatcca gcagggccac tggcatccca 180
   gacaggttca gtggcagtgg gtctgggaca gacttcactc tcaccatcag cagactggag 240
   cctgaagatt ttgcagtgta ttactgtcaa gtatatggta actcactcac tttcggcgga 300
   gggaccaagg tggagatcaa a                                           321
<210> 720
   <211> 107
   <212> PRT
   <213> Artificial Sequence
<220>
   <223> Synthetic
<400> 720
   Glu Ile Val Leu Thr Gln Ser Pro Gly Thr Leu Ser Leu Ser Pro Gly
    1               5                  10                  15
   Glu Arg Ala Thr Leu Ser Cys Arg Ala Ser Gln Ser Val Asn Asn Lys
               20                  25                  30
   Phe Leu Ala Trp Tyr Gln Gln Lys Pro Gly Gln Ala Pro Arg Leu Leu
           35                  40                  45
   Ile Tyr Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Val Tyr Gly Asn Ser Leu
                   85                  90                  95
   Thr Phe Gly Gly Gly Thr Lys Val Glu Ile Lys
               100                 105
<210> 721
   <211> 378
   <212> DNA
   <213> Artificial Sequence
<220>
   <223> Synthetic
<400> 721
   caaattctgc tggtgcaatc tggacctgag gtgaaggagc ctggggcctc agtgaaggtc 60
   tcctgcaagg cttctggtta cacctttacc aactacgcta tcagctgggt gcgacaggtc 120
   cctggacaag ggcttgagtg gatgggatgg gtcagcgctt acaatggtca cacaaactat 180
   gcacatgaag tccagggcag agtcaccatg accacagaca catccacgac cacagcctac 240
   atggagctga ggagcctgag atctgacgac acggccatgt attactgtgc gagagggggt 300
   gtagtcgtgc cagttgctcc ccacttctac aacggtatgg acgtctgggg ccaagggacc 360
   acggtcaccg tctcctca                                                378
<210> 722
   <211> 126
   <212> PRT
   <213> Artificial Sequence
<220>
   <223> Synthetic
<400> 722
   Gln Ile Leu Leu Val Gln Ser Gly Pro Glu Val Lys Glu Pro Gly Ala
    1               5                  10                  15
   Ser Val Lys Val Ser Cys Lys Ala Ser Gly Tyr Thr Phe Thr Asn Tyr
               20                  25                  30
   Ala Ile Ser Trp Val Arg Gln Val Pro Gly Gln Gly Leu Glu Trp Met
           35                  40                  45
   Gly Trp Val Ser Ala Tyr Asn Gly His Thr Asn Tyr Ala His Glu Val
       50                  55                  60
   Gln Gly Arg Val Thr Met Thr Thr Asp Thr Ser Thr Thr Thr Ala Tyr
   65                  70                  75                  80
   Met Glu Leu Arg Ser Leu Arg Ser Asp Asp Thr Ala Met Tyr Tyr Cys
                   85                  90                  95
   Ala Arg Gly Gly Val Val Val Pro Val Ala Pro His Phe Tyr Asn Gly
               100                 105                 110
   Met Asp Val Trp Gly Gln Gly Thr Thr Val Thr Val Ser Ser
           115                 120                 125
<210> 723
   <211> 24
   <212> DNA
   <213> Artificial Sequence
<220>
   <223> Synthetic
<400> 723
   ggttacacct ttaccaacta cgct 24
<210> 724
   <211> 8
   <212> PRT
   <213> Artificial Sequence
<220>
   <223> Synthetic
<400> 724
   Gly Tyr Thr Phe Thr Asn Tyr Ala
   1               5
<210> 725
   <211> 24
   <212> DNA
   <213> Artificial Sequence
<220>
   <223> Synthetic
<400> 725
   gtcagcgctt acaatggtca caca 24
<210> 726
   <211> 8
   <212> PRT
   <213> Artificial Sequence
<220>
   <223> Synthetic
<400> 726
   Val Ser Ala Tyr Asn Gly His Thr
   1               5
<210> 727
   <211> 57
   <212> DNA
   <213> Artificial Sequence
<220>
   <223> Synthetic
<400> 727
   gcgagagggg gtgtagtcgt gccagttgct ccccacttct acaacggtat ggacgtc 57
<210> 728
   <211> 19
   <212> PRT
   <213> Artificial Sequence
<220>
   <223> Synthetic
<400> 728
   Ala Arg Gly Gly Val Val Val Pro Val Ala Pro His Phe Tyr Asn Gly
   1               5                   10                  15
   Met Asp Val
<210> 729
   <211> 336
   <212> DNA
   <213> Artificial Sequence
<220>
   <223> Synthetic
<400> 729
   gatattgtga tgactcagtt tccactctcc ctgcccgtca cccctggaga gccggcctcc 60
   atctcctgca ggtctagtca gagcctcctg catattaatg aatacaacta tttggattgg 120
   tacctaaaga agccagggca gtctccacag ctcctgatct atttgggttt taatcgggcc 180
   tccggggtcc ctgacaggtt cagtggcagt ggatcaggca cagattttac actgaaaatc 240
   agcagagtgg aggctgagga tgttggggtc tattactgca tgcaagctct tcaaactccg 300
   tggacgttcg gccaagggac caaggtggaa atcaaa                           336
<210> 730
   <211> 112
   <212> PRT
   <213> Artificial Sequence
<220>
   <223> Synthetic
<400> 730
   Asp Ile Val Met Thr Gln Phe Pro Leu Ser Leu Pro Val Thr Pro Gly
    1               5                  10                  15
   Glu Pro Ala Ser Ile Ser Cys Arg Ser Ser Gln Ser Leu Leu His Ile
               20                  25                  30
   Asn Glu Tyr Asn Tyr Leu Asp Trp Tyr Leu Lys Lys Pro Gly Gln Ser
           35                  40                  45
   Pro Gln Leu Leu Ile Tyr Leu Gly Phe Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ala
                   85                  90                  95
   Leu Gln Thr Pro Trp Thr Phe Gly Gln Gly Thr Lys Val Glu Ile Lys
               100                 105                 110
<210> 731
   <211> 33
   <212> DNA
   <213> Artificial Sequence
<220>
   <223> Synthetic
<400> 731
   cagagcctcc tgcatattaa tgaatacaac tat 33
<210> 732
   <211> 11
   <212> PRT
   <213> Artificial Sequence
<220>
   <223> Synthetic
<400> 732
   Gln Ser Leu Leu His Ile Asn Glu Tyr Asn Tyr
   1 5 10
<210> 733
   <211> 9
   <212> DNA
   <213> Artificial Sequence
<220>
   <223> Synthetic
<400> 733
   ttgggtttt 9
<210> 734
   <211> 3
   <212> PRT
   <213> Artificial Sequence
<220>
   <223> Synthetic
<400> 734
   Leu Gly Phe
   1
<210> 735
   <211> 27
   <212> DNA
   <213> Artificial Sequence
<220>
   <223> Synthetic
<400> 735
   atgcaagctc ttcaaactcc gtggacg 27
<210> 736
   <211> 9
   <212> PRT
   <213> Artificial Sequence
<220>
   <223> Synthetic
<400> 736
   Met Gln Ala Leu Gln Thr Pro Trp Thr
   1 5
<210> 737
   <211> 378
   <212> DNA
   <213> Artificial Sequence
<220>
   <223> Synthetic
<400> 737
   caggttcagc tggtgcagtc tggacctgag gtgaaggagc ctggggcctc agtgaaggtc 60
   tcctgcaagg cttctggtta cacctttacc aactacgcta tcagctgggt gcgacaggtc 120
   cctggacaag ggcttgagtg gatgggatgg gtcagcgctt acaatggtca cacaaactat 180
   gcacatgaag tccagggcag agtcaccatg accacagaca catccacgac cacagcctac 240
   atggagctga ggagcctgag atctgacgac acggccatgt attactgtgc gagagggggt 300
   gtagtcgtgc cagttgctcc ccacttctac aacggtatgg acgtctgggg ccaagggacc 360
   acggtcaccg tctcctca                                               378
<210> 738
   <211> 126
   <212> PRT
   <213> Artificial Sequence
<220>
   <223> Synthetic
<400> 738
   Gln Val Gln Leu Val Gln Ser Gly Pro Glu Val Lys Glu Pro Gly Ala
    1               5                  10                  15
   Ser Val Lys Val Ser Cys Lys Ala Ser Gly Tyr Thr Phe Thr Asn Tyr
               20                  25                  30
   Ala Ile Ser Trp Val Arg Gln Val Pro Gly Gln Gly Leu Glu Trp Met
           35                  40                  45
   Gly Trp Val Ser Ala Tyr Asn Gly His Thr Asn Tyr Ala His Glu Val
       50                  55                  60
   Gln Gly Arg Val Thr Met Thr Thr Asp Thr Ser Thr Thr Thr Ala Tyr
   65                  70                  75                  80
   Met Glu Leu Arg Ser Leu Arg Ser Asp Asp Thr Ala Met Tyr Tyr Cys
                   85                  90                  95
   Ala Arg Gly Gly Val Val Val Pro Val Ala Pro His Phe Tyr Asn Gly
               100                 105                 110
   Met Asp Val Trp Gly Gln Gly Thr Thr Val Thr Val Ser Ser
           115                 120                 125
<210> 739
   <211> 336
   <212> DNA
   <213> Artificial Sequence
<220>
   <223> Synthetic
<400> 739
   gatattgtga tgactcagtc tccactctcc ctgcccgtca cccctggaga gccggcctcc 60
   atctcctgca ggtctagtca gagcctcctg catattaatg aatacaacta tttggattgg 120
   tacctaaaga agccagggca gtctccacag ctcctgatct atttgggttt taatcgggcc 180
   tccggggtcc ctgacaggtt cagtggcagt ggatcaggca cagattttac actgaaaatc 240
   agcagagtgg aggctgagga tgttggggtc tattactgca tgcaagctct tcaaactccg 300
   tggacgttcg gccaagggac caaggtggaa atcaaa                           336
<210> 740
   <211> 112
   <212> PRT
   <213> Artificial Sequence
<220>
   <223> Synthetic
<400> 740
   Asp Ile Val Met Thr Gln Ser Pro Leu Ser Leu Pro Val Thr Pro Gly
    1               5                  10                  15
   Glu Pro Ala Ser Ile Ser Cys Arg Ser Ser Gln Ser Leu Leu His Ile
               20                  25                  30
   Asn Glu Tyr Asn Tyr Leu Asp Trp Tyr Leu Lys Lys Pro Gly Gln Ser
           35                  40                  45
   Pro Gln Leu Leu Ile Tyr Leu Gly Phe Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ala
                   85                  90                  95
   Leu Gln Thr Pro Trp Thr Phe Gly Gln Gly Thr Lys Val Glu Ile Lys
               100                 105                 110
<210> 741
   <211> 378
   <212> DNA
   <213> Artificial Sequence
<220>
   <223> Synthetic
<400> 741
   caggttcagc tggtgcagtc tggagctgag gtgaagaagc ctggggcctc agtgaaggtc 60
   tcctgcaagg cttctggtta cacctttacc aactacgcta tcagctgggt gcgacaggcc 120
   cctggacaag ggcttgagtg gatgggatgg gtcagcgctt acaatggtca cacaaactat 180
   gcacagaagc tccagggcag agtcaccatg accacagaca catccacgag cacagcctac 240
   atggagctga ggagcctgag atctgacgac acggccgtgt attactgtgc gagagggggt 300
   gtagtcgtgc cagttgctcc ccacttctac aacggtatgg acgtctgggg gcaagggacc 360
   acggtcaccg tctcctca                                               378
<210> 742
   <211> 126
   <212> PRT
   <213> Artificial Sequence
<220>
   <223> Synthetic
<400> 742
   Gln Val Gln Leu Val Gln Ser Gly Ala Glu Val Lys Lys Pro Gly Ala
    1               5                  10                  15
   Ser Val Lys Val Ser Cys Lys Ala Ser Gly Tyr Thr Phe Thr Asn Tyr
               20                  25                  30
   Ala Ile Ser Trp Val Arg Gln Ala Pro Gly Gln Gly Leu Glu Trp Met
           35                  40                  45
   Gly Trp Val Ser Ala Tyr Asn Gly His Thr Asn Tyr Ala Gln Lys Leu
       50                  55                  60
   Gln Gly Arg Val Thr Met Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Gly Gly Val Val Val Pro Val Ala Pro His Phe Tyr Asn Gly
               100                 105                 110
   Met Asp Val Trp Gly Gln Gly Thr Thr Val Thr Val Ser Ser
           115                 120                 125
<210> 743
   <211> 336
   <212> DNA
   <213> Artificial Sequence
<220>
   <223> Synthetic
<400> 743
   gatattgtga tgactcagtc tccactctcc ctgcccgtca cccctggaga gccggcctcc 60
   atctcctgca ggtctagtca gagcctcctg catattaatg aatacaacta tttggattgg 120
   tacctgcaga agccagggca gtctccacag ctcctgatct atttgggttc taatcgggcc 180
   tccggggtcc ctgacaggtt cagtggcagt ggatcaggca cagattttac actgaaaatc 240
   agcagagtgg aggctgagga tgttggggtt tattactgca tgcaagctct tcaaactccg 300
   tggacgttcg gccaagggac caaggtggaa atcaaa                           336
<210> 744
   <211> 112
   <212> PRT
   <213> Artificial Sequence
<220>
   <223> Synthetic
<400> 744
   Asp Ile Val Met Thr Gln Ser Pro Leu Ser Leu Pro Val Thr Pro Gly
    1               5                  10                  15
   Glu Pro Ala Ser Ile Ser Cys Arg Ser Ser Gln Ser Leu Leu His Ile
               20                  25                  30
   Asn Glu Tyr Asn Tyr Leu Asp Trp Tyr Leu Gln Lys Pro Gly Gln Ser
           35                  40                  45
   Pro Gln Leu Leu Ile Tyr Leu Gly Ser Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ala
                   85                  90                  95
   Leu Gln Thr Pro Trp Thr Phe Gly Gln Gly Thr Lys Val Glu Ile Lys
               100                 105                 110
<210> 745
   <211> 8
   <212> PRT
   <213> Artificial Sequence
<220>
   <223> Synthetic
<220>
   <221> VARIANT
   <222> (1)...(8)
   <223> Xaa = Any amino acid
<400> 745
   Xaa Xaa Xaa Xaa Xaa Xaa Xaa Xaa
    1               5
<210> 746
   <211> 8
   <212> PRT
   <213> Artificial Sequence
<220>
   <223> Synthetic
<220>
   <221> VARIANT
   <222> (1)...(8)
   <223> Xaa - Any amino acid
<400> 746
   Xaa Xaa Xaa Xaa Xaa Xaa Xaa Xaa
    1               5
<210> 747
   <211> 20
   <212> PRT
   <213> Artificial Sequence
<220>
   <223> Synthetic
<220>
   <221> VARIANT
   <222> (1)...(20)
   <223> Xaa = Any amino acid
<400> 747
   Xaa Xaa Xaa Xaa Xaa Xaa Xaa Xaa Xaa Xaa Xaa Xaa Xaa Xaa Xaa Xaa
    1               5                  10                  15
   Xaa Xaa Xaa Xaa
               20
<210> 748
   <211> 12
   <212> PRT
   <213> Artificial Sequence
<220>
   <223> Synthetic
<220>
   <221> VARIANT
   <222> (1)...(12)
   <223> Xaa = Any amino acid
<400> 748
   Xaa Xaa Xaa Xaa Xaa Xaa Xaa Xaa Xaa Xaa Xaa Xaa
    1               5                  10
<210> 749
   <211> 3
   <212> PRT
   <213> Artificial Sequence
<220>
   <223> Synthetic
<220>
   <221> VARIANT
   <222> (1) ... (3)
   <223> Xaa = Any amino acid
<400> 749
   Xaa Xaa Xaa
    1
<210> 750
   <211> 9
   <212> PRT
   <213> Artificial Sequence
<220>
   <223> Synthetic
<220>
   <221> VARIANT
   <222> (1)...(9)
   <223> Xaa = Any amino acid
<400> 750
   Xaa Xaa Xaa Xaa Xaa Xaa Xaa Xaa Xaa
    1               5
<210> 751
   <211> 330
   <212> PRT
   <213> Artificial Sequence
<220>
   <223> Synthetic
<400> 751
<210> 752
   <211> 327
   <212> PRT
   <213> Artificial Sequence
<220>
   <223> Synthetic
<400> 752
   Ala Ser Thr Lys Gly Pro Ser Val Phe Pro Leu Ala Pro Cys Ser Arg
    1               5                  10                  15
   Ser Thr Ser Glu Ser Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Lys Thr
   65                  70                  75                  80
   Tyr Thr Cys Asn Val Asp His Lys Pro Ser Asn Thr Lys Val Asp Lys
                   85                  90                  95
   Arg Val Glu Ser Lys Tyr Gly Pro Pro Cys Pro Ser Cys Pro Ala Pro
               100                 105                 110
   Glu Phe Leu Gly Gly Pro Ser Val Phe Leu Phe Pro Pro Lys Pro Lys
           115                 120                 125
   Asp Thr Leu Met Ile Ser Arg Thr Pro Glu Val Thr Cys Val Val Val
       130                 135                 140
   Asp Val Ser Gln Glu Asp Pro Glu Val Gln Phe Asn Trp Tyr Val Asp
   145                 150                 155                 160
   Gly Val Glu Val His Asn Ala Lys Thr Lys Pro Arg Glu Glu Gln Phe
                   165                 170                 175
   Asn Ser Thr Tyr Arg Val Val Ser Val Leu Thr Val Leu His Gln Asp
               180                 185                 190
   Trp Leu Asn Gly Lys Glu Tyr Lys Cys Lys Val Ser Asn Lys Gly Leu
           195                 200                 205
   Pro Ser Ser Ile Glu Lys Thr Ile Ser Lys Ala Lys Gly Gln Pro Arg
       210                 215                 220
   Glu Pro Gln Val Tyr Thr Leu Pro Pro Ser Gln Glu Glu Met Thr Lys
   225                 230                 235                 240
   Asn Gln Val Ser Leu Thr Cys Leu Val Lys Gly Phe Tyr Pro Ser Asp
                   245                 250                 255
   Ile Ala Val Glu Trp Glu Ser Asn Gly Gln Pro Glu Asn Asn Tyr Lys
               260                 265                 270
   Thr Thr Pro Pro Val Leu Asp Ser Asp Gly Ser Phe Phe Leu Tyr Ser
           275                 280                 285
   Arg Leu Thr Val Asp Lys Ser Arg Trp Gln Glu Gly Asn Val Phe Ser
       290                 295                 300
   Cys Ser Val Met His Glu Ala Leu His Asn His Tyr Thr Gln Lys Ser
   305                 310                 315                 320
   Leu Ser Leu Ser Leu Gly Lys
                   325
<210> 753
   <211> 327
   <212> PRT
   <213> Artificial Sequence
<220>
   <223> Synthetic
<400> 753
   Ala Ser Thr Lys Gly Pro Ser Val Phe Pro Leu Ala Pro Cys Ser Arg
    1               5                  10                  15
   Ser Thr Ser Glu Ser Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Lys Thr
   65                  70                  75                  80
   Tyr Thr Cys Asn Val Asp His Lys Pro Ser Asn Thr Lys Val Asp Lys
                   85                  90                  95
   Arg Val Glu Ser Lys Tyr Gly Pro Pro Cys Pro Pro Cys Pro Ala Pro
               100                 105                 110
   Glu Phe Leu Gly Gly Pro Ser Val Phe Leu Phe Pro Pro Lys Pro Lys
           115                 120                 125
   Asp Thr Leu Met Ile Ser Arg Thr Pro Glu Val Thr Cys Val Val Val
       130                 135                 140
   Asp Val Ser Gln Glu Asp Pro Glu Val Gln Phe Asn Trp Tyr Val Asp
   145                 150                 155                 160
   Gly Val Glu Val His Asn Ala Lys Thr Lys Pro Arg Glu Glu Gln Phe
                   165                 170                 175
   Asn Ser Thr Tyr Arg Val Val Ser Val Leu Thr Val Leu His Gln Asp
               180                 185                 190
   Trp Leu Asn Gly Lys Glu Tyr Lys Cys Lys Val Ser Asn Lys Gly Leu
           195                 200                 205
   Pro Ser Ser Ile Glu Lys Thr Ile Ser Lys Ala Lys Gly Gln Pro Arg
       210                 215                 220
   Glu Pro Gln Val Tyr Thr Leu Pro Pro Ser Gln Glu Glu Met Thr Lys
   225                 230                 235                 240
   Asn Gln Val Ser Leu Thr Cys Leu Val Lys Gly Phe Tyr Pro Ser Asp
                   245                 250                 255
   Ile Ala Val Glu Trp Glu Ser Asn Gly Gln Pro Glu Asn Asn Tyr Lys
               260                 265                 270
   Thr Thr Pro Pro Val Leu Asp Ser Asp Gly Ser Phe Phe Leu Tyr Ser
           275                 280                 285
   Arg Leu Thr Val Asp Lys Ser Arg Trp Gln Glu Gly Asn Val Phe Ser
       290                 295                 300
   Cys Ser Val Met His Glu Ala Leu His Asn His Tyr Thr Gln Lys Ser
   305                 310                 315                 320
   Leu Ser Leu Ser Leu Gly Lys
                   325
<210> 754
   <211> 2076
   <212> DNA
   <213> Homo sapiens
<400> 754
<210> 755
   <211> 692
   <212> PRT
   <213> Homo sapiens
<400> 755
   Met Gly Thr Val Ser Ser Arg Arg Ser Trp Trp Pro Leu Pro Leu Leu
    1               5                  10                  15
   Leu Leu Leu Leu Leu Leu Leu Gly Pro Ala Gly Ala Arg Ala Gln Glu
               20                  25                  30
   Asp Glu Asp Gly Asp Tyr Glu Glu Leu Val Leu Ala Leu Arg Ser Glu
           35                  40                  45
   Glu Asp Gly Leu Ala Glu Ala Pro Glu His Gly Thr Thr Ala Thr Phe
       50                  55                  60
   His Arg Cys Ala Lys Asp Pro Trp Arg Leu Pro Gly Thr Tyr Val Val
   65                  70                  75                  80
   Val Leu Lys Glu Glu Thr His Leu Ser Gln Ser Glu Arg Thr Ala Arg
                   85                  90                  95
   Arg Leu Gln Ala Gln Ala Ala Arg Arg Gly Tyr Leu Thr Lys Ile Leu
               100                 105                 110
   His Val Phe His Gly Leu Leu Pro Gly Phe Leu Val Lys Met Ser Gly
           115                 120                 125
   Asp Leu Leu Glu Leu Ala Leu Lys Leu Pro His Val Asp Tyr Ile Glu
       130                 135                 140
   Glu Asp Ser Ser Val Phe Ala Gln Ser Ile Pro Trp Asn Leu Glu Arg
   145                 150                 155                 160
   Ile Thr Pro Pro Arg Tyr Arg Ala Asp Glu Tyr Gln Pro Pro Asp Gly
                   165                 170                 175
   Gly Ser Leu Val Glu Val Tyr Leu Leu Asp Thr Ser Ile Gln Ser Asp
               180                 185                 190
   His Arg Glu Ile Glu Gly Arg Val Met Val Thr Asp Phe Glu Asn Val
           195                 200                 205
   Pro Glu Glu Asp Gly Thr Arg Phe His Arg Gln Ala Ser Lys Cys Asp
       210                 215                 220
   Ser His Gly Thr His Leu Ala Gly Val Val Ser Gly Arg Asp Ala Gly
   225                 230                 235                 240
   Val Ala Lys Gly Ala Ser Met Arg Ser Leu Arg Val Leu Asn Cys Gln
                   245                 250                 255
   Gly Lys Gly Thr Val Ser Gly Thr Leu Ile Gly Leu Glu Phe Ile Arg
               260                 265                 270
   Lys Ser Gln Leu Val Gln Pro Val Gly Pro Leu Val Val Leu Leu Pro
           275                 280                 285
   Leu Ala Gly Gly Tyr Ser Arg Val Leu Asn Ala Ala Cys Gln Arg Leu
       290                 295                 300
   Ala Arg Ala Gly Val Val Leu Val Thr Ala Ala Gly Asn Phe Arg Asp
   305                 310                 315                 320
   Asp Ala Cys Leu Tyr Ser Pro Ala Ser Ala Pro Glu Val Ile Thr Val
                   325                 330                 335
   Gly Ala Thr Asn Ala Gln Asp Gln Pro Val Thr Leu Gly Thr Leu Gly
               340                 345                 350
   Thr Asn Phe Gly Arg Cys Val Asp Leu Phe Ala Pro Gly Glu Asp Ile
           355                 360                 365
   Ile Gly Ala Ser Ser Asp Cys Ser Thr Cys Phe Val Ser Gln Ser Gly
       370                 375                 380
   Thr Ser Gln Ala Ala Ala His Val Ala Gly Ile Ala Ala Met Met Leu
   385                 390                 395                 400
   Ser Ala Glu Pro Glu Leu Thr Leu Ala Glu Leu Arg Gln Arg Leu Ile
                   405                 410                 415
   His Phe Ser Ala Lys Asp Val Ile Asn Glu Ala Trp Phe Pro Glu Asp
               420                 425                 430
   Gln Arg Val Leu Thr Pro Asn Leu Val Ala Ala Leu Pro Pro Ser Thr
           435                 440                 445
   His Gly Ala Gly Trp Gln Leu Phe Cys Arg Thr Val Trp Ser Ala His
       450                 455                 460
   Ser Gly Pro Thr Arg Met Ala Thr Ala Ile Ala Arg Cys Ala Pro Asp
   465                 470                 475                 480
   Glu Glu Leu Leu Ser Cys Ser Ser Phe Ser Arg Ser Gly Lys Arg Arg
                   485                 490                 495
   Gly Glu Arg Met Glu Ala Gln Gly Gly Lys Leu Val Cys Arg Ala His
               500                 505                 510
   Asn Ala Phe Gly Gly Glu Gly Val Tyr Ala Ile Ala Arg Cys Cys Leu
           515                 520                 525
   Leu Pro Gln Ala Asn Cys Ser Val His Thr Ala Pro Pro Ala Glu Ala
       530                 535                 540
   Ser Met Gly Thr Arg Val His Cys His Gln Gln Gly His Val Leu Thr
   545                 550                 555                 560
   Gly Cys Ser Ser His Trp Glu Val Glu Asp Leu Gly Thr His Lys Pro
                   565                 570                 575
   Pro Val Leu Arg Pro Arg Gly Gln Pro Asn Gln Cys Val Gly His Arg
               580                 585                 590
   Glu Ala Ser Ile His Ala Ser Cys Cys His Ala Pro Gly Leu Glu Cys
           595                 600                 605
   Lys Val Lys Glu His Gly Ile Pro Ala Pro Gln Glu Gln Val Thr Val
       610                 615                 620
   Ala Cys Glu Glu Gly Trp Thr Leu Thr Gly Cys Ser Ala Leu Pro Gly
   625                 630                 635                 640
   Thr Ser His Val Leu Gly Ala Tyr Ala Val Asp Asn Thr Cys Val Val
                   645                 650                 655
   Arg Ser Arg Asp Val Ser Thr Thr Gly Ser Thr Ser Glu Glu Ala Val
               660                 665                 670
   Thr Ala Val Ala Ile Cys Cys Arg Ser Arg His Leu Ala Gln Ala Ser
           675                 680                 685
   Gln Glu Leu Gln
       690
<210> 756
   <211> 692
   <212> PRT
   <213> Macaca mulata
<400> 756
   Met Gly Thr Val Ser Ser Arg Arg Ser Trp Trp Pro Leu Pro Leu Pro
    1               5                  10                  15
   Leu Leu Leu Leu Leu Leu Leu Gly Pro Ala Gly Ala Arg Ala Gln Glu
               20                  25                  30
   Asp Glu Asp Gly Asp Tyr Glu Glu Leu Val Leu Ala Leu Arg Ser Glu
           35                  40                  45
   Glu Asp Gly Leu Ala Asp Ala Pro Glu His Gly Ala Thr Ala Thr Phe
       50                  55                  60
   His Arg Cys Ala Lys Asp Pro Trp Arg Leu Pro Gly Thr Tyr Val Val
   65                  70                  75                  80
   Val Leu Lys Glu Glu Thr His Arg Ser Gln Ser Glu Arg Thr Ala Arg
                   85                  90                  95
   Arg Leu Gln Ala Gln Ala Ala Arg Arg Gly Tyr Leu Thr Lys Ile Leu
               100                 105                 110
   His Val Phe His His Leu Leu Pro Gly Phe Leu Val Lys Met Ser Gly
           115                 120                 125
   Asp Leu Leu Glu Leu Ala Leu Lys Leu Pro His Val Asp Tyr Ile Glu
       130                 135                 140
   Glu Asp Ser Ser Val Phe Ala Gln Ser Ile Pro Trp Asn Leu Glu Arg
   145                 150                 155                 160
   Ile Thr Pro Ala Arg Tyr Arg Ala Asp Glu Tyr Gln Pro Pro Lys Gly
                   165                 170                 175
   Gly Ser Leu Val Glu Val Tyr Leu Leu Asp Thr Ser Ile Gln Ser Asp
               180                 185                 190
   His Arg Glu Ile Glu Gly Arg Val Met Val Thr Asp Phe Glu Ser Val
           195                 200                 205
   Pro Glu Glu Asp Gly Thr Arg Phe His Arg Gln Ala Ser Lys Cys Asp
       210                 215                 220
   Ser His Gly Thr His Leu Ala Gly Val Val Ser Gly Arg Asp Ala Gly
   225                 230                 235                 240
   Val Ala Lys Gly Ala Gly Leu Arg Ser Leu Arg Val Leu Asn Cys Gln
                   245                 250                 255
   Gly Lys Gly Thr Val Ser Gly Thr Leu Ile Gly Leu Glu Phe Ile Arg
               260                 265                 270
   Lys Ser Gln Leu Val Gln Pro Val Gly Pro Leu Val Val Leu Leu Pro
           275                 280                 285
   Leu Ala Gly Gly Tyr Ser Arg Val Phe Asn Ala Ala Cys Gln Arg Leu
       290                 295                 300
   Ala Arg Ala Gly Val Val Leu Val Thr Ala Ala Gly Asn Phe Arg Asp
   305                 310                 315                 320
   Asp Ala Cys Leu Tyr Ser Pro Ala Ser Ala Pro Glu Val Ile Thr Val
                   325                 330                 335
   Gly Ala Thr Asn Ala Gln Asp Gln Pro Val Thr Leu Gly Thr Leu Gly
               340                 345                 350
   Thr Asn Phe Gly Arg Cys Val Asp Leu Phe Ala Pro Gly Glu Asp Ile
           355                 360                 365
   Ile Gly Ala Ser Ser Asp Cys Ser Thr Cys Phe Val Ser Arg Ser Gly
       370                 375                 380
   Thr Ser Gln Ala Ala Ala His Val Ala Gly Ile Ala Ala Met Met Leu
   385                 390                 395                 400
   Ser Ala Glu Pro Glu Leu Thr Leu Ala Glu Leu Arg Gln Arg Leu Ile
                   405                 410                 415
   His Phe Ser Ala Lys Asp Val Ile Asn Glu Ala Trp Phe Pro Glu Asp
               420                 425                 430
   Gln Arg Val Leu Thr Pro Asn Leu Val Ala Ala Leu Pro Pro Ser Thr
           435                 440                 445
   His Arg Ala Gly Trp Gln Leu Phe Cys Arg Thr Val Trp Ser Ala His
       450                 455                 460
   Ser Gly Pro Thr Arg Met Ala Thr Ala Val Ala Arg Cys Ala Gln Asp
   465                 470                 475                 480
   Glu Glu Leu Leu Ser Cys Ser Ser Phe Ser Arg Ser Gly Lys Arg Arg
                   485                 490                 495
   Gly Glu Arg Ile Glu Ala Gln Gly Gly Lys Arg Val Cys Arg Ala His
               500                 505                 510
   Asn Ala Phe Gly Gly Glu Gly Val Tyr Ala Ile Ala Arg Cys Cys Leu
           515                 520                 525
   Leu Pro Gln Val Asn Cys Ser Val His Thr Ala Pro Pro Ala Gly Ala
       530                 535                 540
   Ser Met Gly Thr Arg Val His Cys His Gln Gln Gly His Val Leu Thr
   545                 550                 555                 560
   Gly Cys Ser Ser His Trp Glu Val Glu Asp Leu Gly Thr His Lys Pro
                   565                 570                 575
   Pro Val Leu Arg Pro Arg Gly Gln Pro Asn Gln Cys Val Gly His Arg
               580                 585                 590
   Glu Ala Ser Ile His Ala Ser Cys Cys His Ala Pro Gly Leu Glu Cys
           595                 600                 605
   Lys Val Lys Glu His Gly Ile Pro Ala Pro Gln Glu Gln Val Ile Val
       610                 615                 620
   Ala Cys Glu Asp Gly Trp Thr Leu Thr Gly Cys Ser Pro Leu Pro Gly
   625                 630                 635                 640
   Thr Ser His Val Leu Gly Ala Tyr Ala Val Asp Asn Thr Cys Val Val
                   645                 650                 655
   Arg Ser Arg Asp Val Ser Thr Thr Gly Ser Thr Ser Lys Glu Ala Val
               660                 665                 670
   Ala Ala Val Ala Ile Cys Cys Arg Ser Arg His Leu Val Gln Ala Ser
           675                 680                 685
   Gln Glu Leu Gln
       690
<210> 757
   <211> 694
   <212> PRT
   <213> Mus muscular
<400> 757
   Met Gly Thr His Cys Ser Ala Trp Leu Arg Trp Pro Leu Leu Pro Leu
    1              5                   10                  15
   Leu Pro Pro Leu Leu Leu Leu Leu Leu Leu Leu Cys Pro Thr Gly Ala
               20                  25                  30
   Gly Ala Gln Asp Glu Asp Gly Asp Tyr Glu Glu Leu Met Leu Ala Leu
           35                  40                  45
   Pro Ser Gln Glu Asp Gly Leu Ala Asp Glu Ala Ala His Val Ala Thr
       50                  55                  60
   Ala Thr Phe Arg Arg Cys Ser Lys Glu Ala Trp Arg Leu Pro Gly Thr
   65                  70                  75                  80
   Tyr Ile Val Val Leu Met Glu Glu Thr Gln Arg Leu Gln Ile Glu Gln
                   85                  90                  95
   Thr Ala His Arg Leu Gln Thr Arg Ala Ala Arg Arg Gly Tyr Val Ile
               100                 105                 110
   Lys Val Leu His Ile Phe Tyr Asp Leu Phe Pro Gly Phe Leu Val Lys
               115                 120                 125
   Met Ser Ser Asp Leu Leu Gly Leu Ala Leu Lys Leu Pro His Val Glu
       130                 135                 140
   Tyr Ile Glu Glu Asp Ser Phe Val Phe Ala Gln Ser Ile Pro Trp Asn
   145                 150                 155                 160
   Leu Glu Arg Ile Ile Pro Ala Trp His Gln Thr Glu Glu Asp Arg Ser
                   165                 170                 175
   Pro Asp Gly Ser Ser Gln Val Glu Val Tyr Leu Leu Asp Thr Ser Ile
               180                 185                 190
   Gln Gly Ala His Arg Glu Ile Glu Gly Arg Val Thr Ile Thr Asp Phe
           195                 200                 205
   Asn Ser Val Pro Glu Glu Asp Gly Thr Arg Phe His Arg Gln Ala Ser
       210                 215                 220
   Lys Cys Asp Ser His Gly Thr His Leu Ala Gly Val Val Ser Gly Arg
   225                 230                 235                 240
   Asp Ala Gly Val Ala Lys Gly Thr Ser Leu His Ser Leu Arg Val Leu
                   245                 250                 255
   Asn Cys Gln Gly Lys Gly Thr Val Ser Gly Thr Leu Ile Gly Leu Glu
               260                 265                 270
   Phe Ile Arg Lys Ser Gln Leu Ile Gln Pro Ser Gly Pro Leu Val Val
           275                 280                 285
   Leu Leu Pro Leu Ala Gly Gly Tyr Ser Arg Ile Leu Asn Ala Ala Cys
       290                 295                 300
   Arg His Leu Ala Arg Thr Gly Val Val Leu Val Ala Ala Ala Gly Asn
   305                 310                 315                 320
   Phe Arg Asp Asp Ala Cys Leu Tyr Ser Pro Ala Ser Ala Pro Glu Val
                   325                 330                 335
   Ile Thr Val Gly Ala Thr Asn Ala Gln Asp Gln Pro Val Thr Leu Gly
               340                 345                 350
   Thr Leu Gly Thr Asn Phe Gly Arg Cys Val Asp Leu Phe Ala Pro Gly
           355                 360                 365
   Lys Asp Ile Ile Gly Ala Ser Ser Asp Cys Ser Thr Cys Phe Met Ser
       370                 375                 380
   Gln Ser Gly Thr Ser Gln Ala Ala Ala His Val Ala Gly Ile Val Ala
   385                 390                 395                 400
   Arg Met Leu Ser Arg Glu Pro Thr Leu Thr Leu Ala Glu Leu Arg Gln
                   405                 410                 415
   Arg Leu Ile His Phe Ser Thr Lys Asp Val Ile Asn Met Ala Trp Phe
               420                 425                 430
   Pro Glu Asp Gln Gln Val Leu Thr Pro Asn Leu Val Ala Thr Leu Pro
           435                 440                 445
   Pro Ser Thr His Glu Thr Gly Gly Gln Leu Leu Cys Arg Thr Val Trp
       450                 455                 460
   Ser Ala His Ser Gly Pro Thr Arg Thr Ala Thr Ala Thr Ala Arg Cys
   465                 470                 475                 480
   Ala Pro Glu Glu Glu Leu Leu Ser Cys Ser Ser Phe Ser Arg Ser Gly
                   485                 490                 495
   Arg Arg Arg Gly Asp Trp Ile Glu Ala Ile Gly Gly Gln Gln Val Cys
               500                 505                 510
   Lys Ala Leu Asn Ala Phe Gly Gly Glu Gly Val Tyr Ala Val Ala Arg
           515                 520                 525
   Cys Cys Leu Val Pro Arg Ala Asn Cys Ser Ile His Asn Thr Pro Ala
       530                 535                 540
   Ala Arg Ala Gly Leu Glu Thr His Val His Cys His Gln Lys Asp His
   545                 550                 555                 560
   Val Leu Thr Gly Cys Ser Phe His Trp Glu Val Glu Asp Leu Ser Val
                   565                 570                 575
   Arg Arg Gln Pro Ala Leu Arg Ser Arg Arg Gln Pro Gly Gln Cys Val
               580                 585                 590
   Gly His Gln Ala Ala Ser Val Tyr Ala Ser Cys Cys His Ala Pro Gly
           595                 600                 605
   Leu Glu Cys Lys Ile Lys Glu His Gly Ile Ser Gly Pro Ser Glu Gln
       610                 615                 620
   Val Thr Val Ala Cys Glu Ala Gly Trp Thr Leu Thr Gly Cys Asn Val
   625                 630                 635                 640
   Leu Pro Gly Ala Ser Leu Thr Leu Gly Ala Tyr Ser Val Asp Asn Leu
                   645                 650                 655
   Cys Val Ala Arg Val His Asp Thr Ala Arg Ala Asp Arg Thr Ser Gly
               660                 665                 670
   Glu Ala Thr Val Ala Ala Ala Ile Cys Cys Arg Ser Arg Pro Ser Ala
           675                 680                 685
   Lys Ala Ser Trp Val Gln
       690
<210> 758
   <211> 653
   <212> PRT
   <213> Homo sapiens
<400> 758
   Glu Phe Arg Cys His Asp Gly Lys Cys Ile Ser Arg Gln Phe Val Cys
    1               5                  10                  15
   Asp Ser Asp Arg Asp Cys Leu Asp Gly Ser Asp Glu Ala Ser Cys Pro
               20                  25                  30
   Val Leu Thr Cys Gly Pro Ala Ser Phe Gln Cys Asn Ser Ser Thr Cys
           35                  40                  45
   Ile Pro Gln Leu Trp Ala Cys Asp Asn Asp Pro Asp Cys Glu Asp Gly
       50                  55                  60
   Ser Asp Glu Trp Pro Gln Arg Cys Arg Gly Leu Tyr Val Phe Gln Gly
   65                  70                  75                  80
   Asp Ser Ser Pro Cys Ser Ala Phe Glu Phe His Cys Leu Ser Gly Glu
                   85                  90                  95
   Cys Ile His Ser Ser Trp Arg Cys Asp Gly Gly Pro Asp Cys Lys Asp
               100                 105                 110
   Lys Ser Asp Glu Glu Asn Cys Ala Val Ala Thr Cys Arg Pro Asp Glu
           115                 120                 125
   Phe Gln Cys Ser Asp Gly Asn Cys Ile His Gly Ser Arg Gln Cys Asp
       130                 135                 140
   Arg Glu Tyr Asp Cys Lys Asp Met Ser Asp Glu Val Gly Cys Val Asn
   145                 150                 155                 160
   Val Thr Leu Cys Glu Gly Pro Asn Lys Phe Lys Cys His Ser Gly Glu
                   165                 170                 175
   Cys Ile Thr Leu Asp Lys Val Cys Asn Met Ala Arg Asp Cys Arg Asp
               180                 185                 190
   Trp Ser Asp Glu Pro Ile Lys Glu Cys Gly Thr Asn Glu Cys Leu Asp
           195                 200                 205
   Asn Asn Gly Gly Cys Ser His Val Cys Asn Asp Leu Lys Ile Gly Tyr
       210                 215                 220
   Glu Cys Leu Cys Pro Asp Gly Phe Gln Leu Val Ala Gln Arg Arg Cys
   225                 230                 235                 240
   Glu Asp Ile Asp Glu Cys Gln Asp Pro Asp Thr Cys Ser Gln Leu Cys
                   245                 250                 255
   Val Asn Leu Glu Gly Gly Tyr Lys Cys Gln Cys Glu Glu Gly Phe Gln
               260                 265                 270
   Leu Asp Pro His Thr Lys Ala Cys Lys Ala Val Gly Ser Ile Ala Tyr
           275                 280                 285
   Leu Phe Phe Thr Asn Arg His Glu Val Arg Lys Met Thr Leu Asp Arg
       290                 295                 300
   Ser Glu Tyr Thr Ser Leu Ile Pro Asn Leu Arg Asn Val Val Ala Leu
   305                 310                 315                 320
   Asp Thr Glu Val Ala Ser Asn Arg Ile Tyr Trp Ser Asp Leu Ser Gln
                   325                 330                 335
   Arg Met Ile Cys Ser Thr Gln Leu Asp Arg Ala His Gly Val Ser Ser
               340                 345                 350
   Tyr Asp Thr Val Ile Ser Arg Asp Ile Gln Ala Pro Asp Gly Leu Ala
           355                 360                 365
   Val Asp Trp Ile His Ser Asn Ile Tyr Trp Thr Asp Ser Val Leu Gly
       370                 375                 380
   Thr Val Ser Val Ala Asp Thr Lys Gly Val Lys Arg Lys Thr Leu Phe
   385                 390                 395                 400
   Arg Glu Asn Gly Ser Lys Pro Arg Ala Ile Val Val Asp Pro Val His
                   405                 410                 415
   Gly Phe Met Tyr Trp Thr Asp Trp Gly Thr Pro Ala Lys Ile Lys Lys
               420                 425                 430
   Gly Gly Leu Asn Gly Val Asp Ile Tyr Ser Leu Val Thr Glu Asn Ile
           435                 440                 445
   Gln Trp Pro Asn Gly Ile Thr Leu Asp Leu Leu Ser Gly Arg Leu Tyr
       450                 455                 460
   Trp Val Asp Ser Lys Leu His Ser Ile Ser Ser Ile Asp Val Asn Gly
   465                 470                 475                 480
   Gly Asn Arg Lys Thr Ile Leu Glu Asp Glu Lys Arg Leu Ala His Pro
                   485                 490                 495
   Phe Ser Leu Ala Val Phe Glu Asp Lys Val Phe Trp Thr Asp Ile Ile
               500                 505                 510
   Asn Glu Ala Ile Phe Ser Ala Asn Arg Leu Thr Gly Ser Asp Val Asn
           515                 520                 525
   Leu Leu Ala Glu Asn Leu Leu Ser Pro Glu Asp Met Val Leu Phe His
       530                 535                 540
   Asn Leu Thr Gln Pro Arg Gly Val Asn Trp Cys Glu Arg Thr Thr Leu
   545                 550                 555                 560
   Ser Asn Gly Gly Cys Gln Tyr Leu Cys Leu Pro Ala Pro Gln Ile Asn
                   565                 570                 575
   Pro His Ser Pro Lys Phe Thr Cys Ala Cys Pro Asp Gly Met Leu Leu
               580                 585                 590
   Ala Arg Asp Met Arg Ser Cys Leu Thr Glu Ala Glu Ala Ala Val Ala
           595                 600                 605
   Thr Gln Glu Thr Ser Thr Val Arg Leu Lys Val Ser Ser Thr Ala Val
       610                 615                 620
   Arg Thr Gln His Thr Thr Thr Arg Pro Val Pro Asp Thr Ser Arg Leu
   625                 630                 635                 640
   Pro Gly Ala Thr Pro Gly Leu Thr Thr Val Glu Ile Val
                   645                 650
<210> 759
   <211> 753
   <212> PRT
   <213> Homo sapiens
<400> 759
<210> 760
   <211> 785
   <212> PRT
   <213> Homo sapiens
<400> 760
   Met Pro Lys Gly Arg Gln Lys Val Pro His Leu Asp Ala Pro Leu Gly
    1               5                  10                  15
   Leu Pro Thr Cys Leu Trp Leu Glu Leu Ala Gly Leu Phe Leu Leu Val
               20                  25                  30
   Pro Trp Val Met Gly Leu Ala Gly Thr Gly Gly Pro Asp Gly Gln Gly
           35                  40                  45
   Thr Gly Gly Pro Ser Trp Ala Val His Leu Glu Ser Leu Glu Gly Asp
       50                  55                  60
   Gly Glu Glu Glu Thr Leu Glu Gln Gln Ala Asp Ala Leu Ala Gln Ala
   65                  70                  75                  80
   Ala Gly Leu Val Asn Ala Gly Arg Ile Gly Glu Leu Gln Gly His Tyr
                   85                  90                  95
   Leu Phe Val Gln Pro Ala Gly His Arg Pro Ala Leu Glu Val Glu Ala
               100                 105                 110
   Ile Arg Gln Gln Val Glu Ala Val Leu Ala Gly His Glu Ala Val Arg
           115                 120                 125
   Trp His Ser Glu Gln Arg Leu Leu Arg Arg Ala Lys Arg Ser Val His
       130                 135                 140
   Phe Asn Asp Pro Lys Tyr Pro Gln Gln Trp His Leu Asn Asn Arg Arg
   145                 150                 155                 160
   Ser Pro Gly Arg Asp Ile Asn Val Thr Gly Val Trp Glu Arg Asn Val
                   165                 170                 175
   Thr Gly Arg Gly Val Thr Val Val Val Val Asp Asp Gly Val Glu His
               180                 185                 190
   Thr Ile Gln Asp Ile Ala Pro Asn Tyr Ser Pro Glu Gly Ser Tyr Asp
   195 200 205
   Leu Asn Ser Asn Asp Pro Asp Pro Met Pro His Pro Asp Val Glu Asn
   210 215 220
   Gly Asn His His Gly Thr Arg Cys Ala Gly Glu Ile Ala Ala Val Pro
   225 230 235 240
   Asn Asn Ser Phe Cys Ala Val Gly Val Ala Tyr Gly Ser Arg Ile Ala
   245 250 255
   Gly Ile Arg Val Leu Asp Gly Pro Leu Thr Asp Ser Met Glu Ala Val
   260 265 270
   Ala Phe Asn Lys His Tyr Gln Ile Asn Asp Ile Tyr Ser Cys Ser Trp
   275 280 285
   Gly Pro Asp Asp Asp Gly Lys Thr Val Asp Gly Pro His Gln Leu Gly
   290 295 300
   Lys Ala Ala Leu Gln His Gly Val Ile Ala Gly Arg Gln Gly Phe Gly
   305 310 315 320
   Ser Ile Phe Val Val Ala Ser Gly Asn Gly Gly Gln His Asn Asp Asn
   325 330 335
   Cys Asn Tyr Asp Gly Tyr Ala Asn Ser Ile Tyr Thr Val Thr Ile Gly
   340 345 350
   Ala Val Asp Glu Glu Gly Arg Met Pro Phe Tyr Ala Glu Glu Cys Ala
   355 360 365
   Ser Met Leu Ala Val Thr Phe Ser Gly Gly Asp Lys Met Leu Arg Ser
   370 375 380
   Ile Val Thr Thr Asp Trp Asp Leu Gln Lys Gly Thr Gly Cys Thr Glu
   385 390 395 400
   Gly His Thr Gly Thr Ser Ala Ala Ala Pro Leu Ala Ala Gly Met Ile
   405 410 415
   Ala Leu Met Leu Gln Val Arg Pro Cys Leu Thr Trp Arg Asp Val Gln
   420 425 430
   His Ile Ile Val Phe Thr Ala Thr Arg Tyr Glu Asp Arg Arg Ala Glu
   435 440 445
   Trp Val Thr Asn Glu Ala Gly Phe Ser His Ser His Gln His Gly Phe
   450 455 460
   Gly Leu Leu Asn Ala Trp Arg Leu Val Asn Ala Ala Lys Ile Trp Thr
   465 470 475 480
   Ser Val Pro Tyr Leu Ala Ser Tyr Val Ser Pro Val Leu Lys Glu Asn
   485 490 495
   Lys Ala Ile Pro Gln Ser Pro Arg Ser Leu Glu Val Leu Trp Asn Val
   500 505 510
   Ser Arg Met Asp Leu Glu Met Ser Gly Leu Lys Thr Leu Glu His Val
   515 520 525
   Ala Val Thr Val Ser Ile Thr His Pro Arg Arg Gly Ser Leu Glu Leu
   530 535 540
   Lys Leu Phe Cys Pro Ser Gly Met Met Ser Leu Ile Gly Ala Pro Arg
   545 550 555 560
   Ser Met Asp Ser Asp Pro Asn Gly Phe Asn Asp Trp Thr Phe Ser Thr
   565 570 575
   Val Arg Cys Trp Gly Glu Arg Ala Arg Gly Thr Tyr Arg Leu Val Ile
   580 585 590
   Arg Asp Val Gly Asp Glu Ser Phe Gln Val Gly Ile Leu Arg Gln Trp
   595 600 605
   Gln Leu Thr Leu Tyr Gly Ser Val Trp Ser Ala Val Asp Ile Arg Asp
   610 615 620
   Arg Gln Arg Leu Leu Glu Ser Ala Met Ser Gly Lys Tyr Leu His Asp
   625 630 635 640
   Asp Phe Ala Leu Pro Cys Pro Pro Gly Leu Lys Ile Pro Glu Glu Asp
   645 650 655
   Gly Tyr Thr Ile Thr Pro Asn Thr Leu Lys Thr Leu Val Leu Val Gly
   660 665 670
   Cys Phe Thr Val Phe Trp Thr Val Tyr Tyr Met Leu Glu Val Tyr Leu
           675                 680                 685
   Ser Gln Arg Asn Val Ala Ser Asn Gln Val Cys Arg Ser Gly Pro Cys
       690                 695                 700
   His Trp Pro His Arg Ser Arg Lys Ala Lys Glu Glu Gly Thr Glu Leu
   705                 710                 715                 720
   Glu Ser Val Pro Leu Cys Ser Ser Lys Asp Pro Asp Glu Val Glu Thr
                   725                 730                 735
   Glu Ser Arg Gly Pro Pro Thr Thr Ser Asp Leu Leu Ala Pro Asp Leu
               740                 745                 750
   Leu Glu Gln Gly Asp Trp Ser Leu Ser Gln Asn Lys Ser Ala Leu Asp
           755                 760                 765
   Cys Pro His Gln His Leu Asp Val Pro His Gly Lys Glu Glu Gln Ile
       770                 775                 780
   Cys
   785
<210> 761
   <211> 692
   <212> PRT
   <213> Macaca fascicularis
<400> 761
   Met Gly Thr Val Ser Ser Arg Arg Ser Trp Trp Pro Leu Pro Leu Pro
    1               5                  10                  15
   Leu Leu Leu Leu Leu Leu Leu Gly Pro Ala Gly Ala Arg Ala Gln Glu
               20                  25                  30
   Asp Glu Asp Gly Asp Tyr Glu Glu Leu Val Leu Ala Leu Arg Ser Glu
           35                  40                  45
   Glu Asp Gly Leu Ala Asp Ala Pro Glu His Gly Ala Thr Ala Thr Phe
       50                  55                  60
   His Arg Cys Ala Lys Asp Pro Trp Arg Leu Pro Gly Thr Tyr Val Val
   65                  70                  75                  80
   Val Leu Lys Glu Glu Thr His Arg Ser Gln Ser Glu Arg Thr Ala Arg
                   85                  90                  95
   Arg Leu Gln Ala Gln Ala Ala Arg Arg Gly Tyr Leu Thr Lys Ile Leu
               100                 105                 110
   His Val Phe His His Leu Leu Pro Gly Phe Leu Val Lys Met Ser Gly
           115                 120                 125
   Asp Leu Leu Glu Leu Ala Leu Lys Leu Pro His Val Asp Tyr Ile Glu
       130                 135                 140
   Glu Asp Ser Ser Val Phe Ala Gln Ser Ile Pro Trp Asn Leu Glu Arg
   145                 150                 155                 160
   Ile Thr Pro Ala Arg Tyr Arg Ala Asp Glu Tyr Gln Pro Pro Lys Gly
                   165                 170                 175
   Gly Ser Leu Val Glu Val Tyr Leu Leu Asp Thr Ser Ile Gln Ser Asp
               180                 185                 190
   His Arg Glu Ile Glu Gly Arg Val Met Val Thr Asp Phe Glu Ser Val
           195                 200                 205
   Pro Glu Glu Asp Gly Thr Arg Phe His Arg Gln Ala Ser Lys Cys Asp
       210                 215                 220
   Ser His Gly Thr His Leu Ala Gly Val Val Ser Gly Arg Asp Ala Gly
   225                 230                 235                 240
   Val Ala Lys Gly Ala Gly Leu Arg Ser Leu Arg Val Leu Asn Cys Gln
                   245                 250                 255
   Gly Lys Gly Thr Val Ser Gly Thr Leu Ile Gly Leu Glu Phe Ile Arg
               260                 265                 270
   Lys Ser Gln Leu Val Gln Pro Val Gly Pro Leu Val Val Leu Leu Pro
           275                 280                 285
   Leu Ala Gly Gly Tyr Ser Arg Val Phe Asn Ala Ala Cys Gln Arg Leu
       290                 295                 300
   Ala Arg Ala Gly Val Val Leu Val Thr Ala Ala Gly Asn Phe Arg Asp
   305                 310                 315                 320
   Asp Ala Cys Leu Tyr Ser Pro Ala Ser Ala Pro Glu Val Ile Thr Val
                   325                 330                 335
   Gly Ala Thr Asn Ala Gln Asp Gln Pro Val Thr Leu Gly Thr Leu Gly
               340                 345                 350
   Thr Asn Phe Gly Arg Cys Val Asp Leu Phe Ala Pro Gly Glu Asp Ile
           355                 360                 365
   Ile Gly Ala Ser Ser Asp Cys Ser Thr Cys Phe Val Ser Arg Ser Gly
       370                 375                 380
   Thr Ser Gln Ala Ala Ala His Val Ala Gly Ile Ala Ala Met Met Leu
   385                 390                 395                 400
   Ser Ala Glu Pro Glu Leu Thr Leu Ala Glu Leu Arg Gln Arg Leu Ile
                   405                 410                 415
   His Phe Ser Ala Lys Asp Val Ile Asn Glu Ala Trp Phe Pro Glu Asp
               420                 425                 430
   Gln Arg Val Leu Thr Pro Asn Leu Val Ala Ala Leu Pro Pro Ser Thr
           435                 440                 445
   His Arg Ala Gly Trp Gln Leu Phe Cys Arg Thr Val Trp Ser Ala His
       450                 455                 460
   Ser Gly Pro Thr Arg Met Ala Thr Ala Val Ala Arg Cys Ala Gln Asp
   465                 470                 475                 480
   Glu Glu Leu Leu Ser Cys Ser Ser Phe Ser Arg Ser Gly Lys Arg Arg
                   485                 490                 495
   Gly Glu Arg Ile Glu Ala Gln Gly Gly Lys Arg Val Cys Arg Ala His
               500                 505                 510
   Asn Ala Phe Gly Gly Glu Gly Val Tyr Ala Ile Ala Arg Cys Cys Leu
           515                 520                 525
   Leu Pro Gln Val Asn Cys Ser Val His Thr Ala Pro Pro Ala Gly Ala
       530                 535                 540
   Ser Met Gly Thr Arg Val His Cys His Gln Gln Gly His Val Leu Thr
   545                 550                 555                 560
   Gly Cys Ser Ser His Trp Glu Val Glu Asp Leu Gly Thr His Lys Pro
                   565                 570                 575
   Pro Val Leu Arg Pro Arg Gly Gln Pro Asn Gln Cys Val Gly His Arg
               580                 585                 590
   Glu Ala Ser Ile His Ala Ser Cys Cys His Ala Pro Gly Leu Glu Cys
           595                 600                 605
   Lys Val Lys Glu His Gly Ile Pro Ala Pro Gln Glu Gln Val Ile Val
       610                 615                 620
   Ala Cys Glu Asp Gly Trp Thr Leu Thr Gly Cys Ser Ala Leu Pro Gly
   625                 630                 635                 640
   Thr Ser His Val Leu Gly Ala Tyr Ala Val Asp Asn Thr Cys Val Val
                   645                 650                 655
   Arg Ser Arg Asp Val Ser Thr Thr Gly Ser Thr Ser Glu Glu Ala Val
               660                 665                 670
   Ala Ala Val Ala Ile Cys Cys Arg Ser Arg His Leu Val Gln Ala Ser
           675                 680                 685
   Gln Glu Leu Gln
       690
<210> 762
   <211> 698
   <212> PRT
   <213> Mesocricetus auratus
<400> 762
   Met Gly Thr Ser Cys Ser Ala Arg Pro Arg Trp Leu Leu Ser Pro Leu
    1               5                  10                  15
   Leu Leu Leu Leu Leu Leu Leu Arg Tyr Met Gly Ala Ser Ala Gln Asp
               20                  25                  30
   Glu Asp Ala Glu Tyr Glu Glu Leu Met Leu Thr Leu Gln Ser Gln Asp
           35                  40                  45
   Asp Gly Leu Ala Asp Glu Thr Asp Glu Ala Pro Gln Gly Ala Thr Ala
       50                  55                  60
   Ala Phe His Arg Cys Pro Glu Glu Ala Trp Arg Val Pro Gly Thr Tyr
   65                  70                  75                  80
   Ile Val Met Leu Ala Glu Glu Ala Gln Trp Val His Ile Glu Gln Thr
                   85                  90                  95
   Met His Arg Leu Gln Thr Gln Ala Ala Arg Arg Gly Tyr Val Ile Lys
               100                 105                 110
   Ile Gln His Ile Phe Tyr Asp Phe Leu Pro Ala Phe Val Val Lys Met
           115                 120                 125
   Ser Ser Asp Leu Leu Asp Leu Ala Leu Lys Leu Pro His Val Lys Tyr
       130                 135                 140
   Ile Glu Glu Asp Ser Leu Val Phe Ala Gln Ser Ile Pro Trp Asn Leu
   145                 150                 155                 160
   Asp Arg Ile Ile Pro Ala Gly Arg Gln Ala Gln Glu Tyr Ser Ser Ser
                   165                 170                 175
   Arg Lys Val Pro Ser Gly Ser Gly Gln Val Glu Val Tyr Leu Leu Asp
               180                 185                 190
   Thr Ser Ile Gln Ser Asp His Arg Glu Ile Glu Gly Arg Val Thr Val
           195                 200                 205
   Thr Asp Phe Asn Ser Val Pro Glu Glu Asp Gly Thr Arg Phe His Arg
       210                 215                 220
   Gln Ala Ser Lys Cys Asp Ser His Gly Thr His Leu Ala Gly Val Val
   225                 230                 235                 240
   Ser Gly Arg Asp Ala Gly Val Ala Lys Gly Thr Ile Leu His Gly Leu
                   245                 250                 255
   Arg Val Leu Asn Cys Gln Gly Lys Gly Ile Val Ser Gly Ile Leu Thr
               260                 265                 270
   Gly Leu Glu Phe Ile Trp Lys Ser Gln Leu Met Gln Pro Ser Gly Pro
           275                 280                 285
   Gln Val Val Leu Leu Pro Leu Ala Gly Arg Tyr Ser Arg Val Leu Asn
       290                 295                 300
   Thr Ala Cys Gln His Leu Ala Arg Thr Gly Val Val Leu Val Ala Ala
   305                 310                 315                 320
   Ala Gly Asn Phe Arg Asp Asp Ala Cys Leu Tyr Ser Pro Ala Ser Ala
                   325                 330                 335
   Pro Glu Val Ile Thr Val Gly Ala Thr Asp Val Gln Asp Gln Pro Val
               340                 345                 350
   Thr Leu Gly Thr Leu Gly Thr Asn Phe Gly Arg Cys Val Asp Leu Phe
           355                 360                 365
   Ala Pro Gly Lys Asp Ile Ile Gly Ala Ser Ser Asp Cys Ser Ala Cys
       370                 375                 380
   Phe Met Ser Gln Ser Gly Thr Ser Gln Ala Ala Ala His Val Ala Gly
   385                 390                 395                 400
   Ile Val Ala Met Met Leu Thr Leu Glu Pro Glu Leu Thr Leu Thr Glu
                   405                 410                 415
   Leu Arg Gln Arg Leu Ile His Phe Ser Thr Lys Asp Ala Ile Asn Met
               420                 425                 430
   Ala Trp Phe Pro Glu Asp Gln Arg Val Leu Thr Pro Asn Leu Val Ala
           435                 440                 445
   Thr Leu Pro Pro Ser Thr His Gly Thr Gly Gly Gln Leu Leu Cys Arg
       450                 455                 460
   Thr Val Trp Ser Ala His Ser Gly Pro Thr Arg Ala Ala Thr Ala Thr
   465                 470                 475                 480
   Ala Arg Cys Ala Pro Gly Glu Glu Leu Leu Ser Cys Ser Ser Phe Ser
                   485                 490                 495
   Arg Ser Gly Arg Arg Arg Gly Asp Arg Ile Glu Ala Ala Gly Thr Gln
               500                 505                 510
   Gln Val Cys Lys Ala Leu Asn Ala Phe Gly Gly Glu Gly Val Tyr Ala
           515                 520                 525
   Val Ala Arg Cys Cys Leu Leu Pro Arg Ala Asn Cys Ser Ile His Thr
       530                 535                 540
   Thr Pro Ala Ala Arg Thr Ser Leu Glu Thr His Ala His Cys His Gln
   545                 550                 555                 560
   Lys Asp His Val Leu Thr Gly Cys Ser Leu His Trp Glu Val Glu Gly
                   565                 570                 575
   Ile Gly Val Gln Pro Leu Ala Val Leu Arg Ser Arg His Gln Pro Gly
               580                 585                 590
   Gln Cys Thr Gly His Arg Glu Ala Ser Val His Ala Ser Cys Cys His
           595                 600                 605
   Ala Pro Gly Leu Glu Cys Lys Ile Lys Glu His Gly Ile Ser Gly Pro
       610                 615                 620
   Ala Glu Gln Val Thr Val Ala Cys Glu Ala Gly Trp Thr Leu Thr Gly
   625                 630                 635                 640
   Cys Asn Val Leu Pro Gly Ala Phe Ile Thr Leu Gly Ala Tyr Ala Val
                   645                 650                 655
   Asp Asn Thr Cys Val Ala Arg Ser Arg Val Thr Asp Thr Ala Gly Arg
               660                 665                 670
   Thr Gly Glu Glu Ala Thr Val Ala Ala Ala Ile Cys Cys Arg Asn Arg
           675                 680                 685
   Pro Ser Ala Lys Ala Ser Trp Val His Gln
       690                 695
<210> 763
   <211> 691
   <212> PRT
   <213> Rattus norvegicus
<400> 763
   Met Gly Ile Arg Cys Ser Thr Trp Leu Arg Trp Pro Leu Ser Pro Gln
    1               5                  10                  15
   Leu Leu Leu Leu Leu Leu Leu Cys Pro Thr Gly Ser Arg Ala Gln Asp
               20                  25                  30
   Glu Asp Gly Asp Tyr Glu Glu Leu Met Leu Ala Leu Pro Ser Gln Glu
           35                  40                  45
   Asp Ser Leu Val Asp Glu Ala Ser His Val Ala Thr Ala Thr Phe Arg
       50                  55                  60
   Arg Cys Ser Lys Glu Ala Trp Arg Leu Pro Gly Thr Tyr Val Val Val
   65                  70                  75                  80
   Leu Met Glu Glu Thr Gln Arg Leu Gln Val Glu Gln Thr Ala His Arg
                   85                  90                  95
   Leu Gln Thr Trp Ala Ala Arg Arg Gly Tyr Val Ile Lys Val Leu His
               100                 105                 110
   Val Phe Tyr Asp Leu Phe Pro Gly Phe Leu Val Lys Met Ser Ser Asp
           115                 120                 125
   Leu Leu Gly Leu Ala Leu Lys Leu Pro His Val Glu Tyr Ile Glu Glu
       130                 135                 140
   Asp Ser Leu Val Phe Ala Gln Ser Ile Pro Trp Asn Leu Glu Arg Ile
   145                 150                 155                 160
   Ile Pro Ala Trp Gln Gln Thr Glu Glu Asp Ser Ser Pro Asp Gly Ser
                   165                 170                 175
   Ser Gln Val Glu Val Tyr Leu Leu Asp Thr Ser Ile Gln Ser Gly His
               180                 185                 190
   Arg Glu Ile Glu Gly Arg Val Thr Ile Thr Asp Phe Asn Ser Val Pro
           195                 200                 205
   Glu Glu Asp Gly Thr Arg Phe His Arg Gln Ala Ser Lys Cys Asp Ser
       210                 215                 220
   His Gly Thr His Leu Ala Gly Val Val Ser Gly Arg Asp Ala Gly Val
   225                 230                 235                 240
   Ala Lys Gly Thr Ser Leu His Ser Leu Arg Val Leu Asn Cys Gln Gly
                   245                 250                 255
   Lys Gly Thr Val Ser Gly Thr Leu Ile Gly Leu Glu Phe Ile Arg Lys
               260                 265                 270
   Ser Gln Leu Ile Gln Pro Ser Gly Pro Leu Val Val Leu Leu Pro Leu
           275                 280                 285
   Ala Gly Gly Tyr Ser Arg Ile Leu Asn Thr Ala Cys Gln Arg Leu Ala
       290                 295                 300
   Arg Thr Gly Val Val Leu Val Ala Ala Ala Gly Asn Phe Arg Asp Asp
   305                 310                 315                 320
   Ala Cys Leu Tyr Ser Pro Ala Ser Ala Pro Glu Val Ile Thr Val Gly
                   325                 330                 335
   Ala Thr Asn Ala Gln Asp Gln Pro Val Thr Leu Gly Thr Leu Gly Thr
               340                 345                 350
   Asn Phe Gly Arg Cys Val Asp Leu Phe Ala Pro Gly Lys Asp Ile Ile
           355                 360                 365
   Gly Ala Ser Ser Asp Cys Ser Thr Cys Tyr Met Ser Gln Ser Gly Thr
       370                 375                 380
   Ser Gln Ala Ala Ala His Val Ala Gly Ile Val Ala Met Met Leu Asn
   385                 390                 395                 400
   Arg Asp Pro Ala Leu Thr Leu Ala Glu Leu Arg Gln Arg Leu Ile Leu
                   405                 410                 415
   Phe Ser Thr Lys Asp Val Ile Asn Met Ala Trp Phe Pro Glu Asp Gln
               420                 425                 430
   Arg Val Leu Thr Pro Asn Arg Val Ala Thr Leu Pro Pro Ser Thr Gln
           435                 440                 445
   Glu Thr Gly Gly Gln Leu Leu Cys Arg Thr Val Trp Ser Ala His Ser
       450                 455                 460
   Gly Pro Thr Arg Thr Ala Thr Ala Thr Ala Arg Cys Ala Pro Glu Glu
   465                 470                 475                 480
   Glu Leu Leu Ser Cys Ser Ser Phe Ser Arg Ser Gly Arg Arg Arg Gly
                   485                 490                 495
   Asp Arg Ile Glu Ala Ile Gly Gly Gln Gln Val Cys Lys Ala Leu Asn
               500                 505                 510
   Ala Phe Gly Gly Glu Gly Val Tyr Ala Val Ala Arg Cys Cys Leu Leu
           515                 520                 525
   Pro Arg Val Asn Cys Ser Ile His Asn Thr Pro Ala Ala Arg Ala Gly
       530                 535                 540
   Pro Gln Thr Pro Val His Cys His Gln Lys Asp His Val Leu Thr Gly
   545                 550                 555                 560
   Cys Ser Phe His Trp Glu Val Glu Asn Leu Arg Ala Gln Gln Gln Pro
                   565                 570                 575
   Leu Leu Arg Ser Arg His Gln Pro Gly Gln Cys Val Gly His Gln Glu
               580                 585                 590
   Ala Ser Val His Ala Ser Cys Cys His Ala Pro Gly Leu Glu Cys Lys
           595                 600                 605
   Ile Lys Glu His Gly Ile Ala Gly Pro Ala Glu Gln Val Thr Val Ala
       610                 615                 620
   Cys Glu Ala Gly Trp Thr Leu Thr Gly Cys Asn Val Leu Pro Gly Ala
   625                 630                 635                 640
   Ser Leu Pro Leu Gly Ala Tyr Ser Val Asp Asn Val Cys Val Ala Arg
                   645                 650                 655
   Ile Arg Asp Ala Gly Arg Ala Asp Arg Thr Ser Glu Glu Ala Thr Val
               660                 665                 670
   Ala Ala Ala Ile Cys Cys Arg Ser Arg Pro Ser Ala Lys Ala Ser Trp
                   675                 680                 685
   Val His Gln
       690
<210> 764
   <211> 447
   <212> PRT
   <213> Artificial Sequence
<220>
   <223> synthetic
<400> 764
   Glu Val Gln Leu Val Glu Ser Gly Gly Gly Leu Val Gln Pro Gly Gly
    1               5                  10                  15
   Ser Leu Arg Leu Ser Cys Ala Ala Ser Gly Phe Thr Phe Asn Asn Tyr
               20                  25                  30
   Ala Met Asn Trp Val Arg Gln Ala Pro Gly Lys Gly Leu Asp Trp Val
           35                  40                  45
   Ser Thr Ile Ser Gly Ser Gly Gly Thr Thr Asn Tyr Ala Asp Ser Val
       50                  55                  60
   Lys Gly Arg Phe Ile Ile Ser Arg Asp Ser Ser Lys His Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Asp Ser Asn Trp Gly Asn Phe Asp Leu Trp Gly Arg Gly Thr
               100                 105                 110
   Leu Val Thr Val Ser Ser Ala Ser Thr Lys Gly Pro Ser Val Phe Pro
           115                 120                 125
   Leu Ala Pro Ser Ser Lys Ser Thr Ser Gly Gly Thr Ala Ala Leu Gly
       130                 135                 140
   Cys Leu Val Lys Asp Tyr Phe Pro Glu Pro Val Thr Val Ser Trp Asn
   145                 150                 155                 160
   Ser Gly Ala Leu Thr Ser Gly Val His Thr Phe Pro Ala Val Leu Gln
                   165                 170                 175
   Ser Ser Gly Leu Tyr Ser Leu Ser Ser Val Val Thr Val Pro Ser Ser
               180                 185                 190
   Ser Leu Gly Thr Gln Thr Tyr Ile Cys Asn Val Asn His Lys Pro Ser
           195                 200                 205
   Asn Thr Lys Val Asp Lys Lys Val Glu Pro Lys Ser Cys Asp Lys Thr
       210                 215                 220
   His Thr Cys Pro Pro Cys Pro Ala Pro Glu Leu Leu Gly Gly Pro Ser
   225                 230                 235                 240
   Val Phe Leu Phe Pro Pro Lys Pro Lys Asp Thr Leu Met Ile Ser Arg
                   245                 250                 255
   Thr Pro Glu Val Thr Cys Val Val Val Asp Val Ser His Glu Asp Pro
               260                 265                 270
   Glu Val Lys Phe Asn Trp Tyr Val Asp Gly Val Glu Val His Asn Ala
           275                 280                 285
   Lys Thr Lys Pro Arg Glu Glu Gln Tyr Asn Ser Thr Tyr Arg Val Val
       290                 295                 300
   Ser Val Leu Thr Val Leu His Gln Asp Trp Leu Asn Gly Lys Glu Tyr
   305                 310                 315                 320
   Lys Cys Lys Val Ser Asn Lys Ala Leu Pro Ala Pro Ile Glu Lys Thr
                   325                 330                 335
   Ile Ser Lys Ala Lys Gly Gln Pro Arg Glu Pro Gln Val Tyr Thr Leu
               340                 345                 350
   Pro Pro Ser Arg Asp Glu Leu Thr Lys Asn Gln Val Ser Leu Thr Cys
           355                 360                 365
   Leu Val Lys Gly Phe Tyr Pro Ser Asp Ile Ala Val Glu Trp Glu Ser
       370                 375                 380
   Asn Gly Gln Pro Glu Asn Asn Tyr Lys Thr Thr Pro Pro Val Leu Asp
   385                 390                 395                 400
   Ser Asp Gly Ser Phe Phe Leu Tyr Ser Lys Leu Thr Val Asp Lys Ser
                   405                 410                 415
   Arg Trp Gln Gln Gly Asn Val Phe Ser Cys Ser Val Met His Glu Ala
               420                 425                 430
   Leu His Asn His Tyr Thr Gln Lys Ser Leu Ser Leu Ser Pro Gly
           435                 440                 445
<210> 765
   <211> 220
   <212> PRT
   <213> Artificial Sequence
<220>
   <223> synthetic
<400> 765
   Asp Ile Val Met Thr Gln Ser Pro Asp Ser Leu Ala Val Ser Leu Gly
    1               5                  10                  15
   Glu Arg Ala Thr Ile Asn Cys Lys Ser Ser Gln Ser Val Leu Tyr Arg
               20                  25                  30
   Ser Asn Asn Arg Asn Phe Leu Gly Trp Tyr Gln Gln Lys Pro Gly Gln
           35                  40                  45
   Pro Pro Asn Leu Leu Ile Tyr Trp Ala Ser Thr Arg Glu Ser Gly Val
       50                  55                  60
   Pro Asp Arg Phe Ser Gly Ser Gly Ser Gly Thr Asp Phe Thr Leu Thr
   65                  70                  75                  80
   Ile Ser Ser Leu Gln Ala Glu Asp Val Ala Val Tyr Tyr Cys Gln Gln
                   85                  90                  95
   Tyr Tyr Thr Thr Pro Tyr Thr Phe Gly Gln Gly Thr Lys Leu Glu Ile
               100                 105                 110
   Lys Arg Thr Val Ala Ala Pro Ser Val Phe Ile Phe Pro Pro Ser Asp
           115                 120                 125
   Glu Gln Leu Lys Ser Gly Thr Ala Ser Val Val Cys Leu Leu Asn Asn
       130                 135                 140
   Phe Tyr Pro Arg Glu Ala Lys Val Gln Trp Lys Val Asp Asn Ala Leu
   145                 150                 155                 160
   Gln Ser Gly Asn Ser Gln Glu Ser Val Thr Glu Gln Asp Ser Lys Asp
                   165                 170                 175
   Ser Thr Tyr Ser Leu Ser Ser Thr Leu Thr Leu Ser Lys Ala Asp Tyr
               180                 185                 190
   Glu Lys His Lys Val Tyr Ala Cys Glu Val Thr His Gln Gly Leu Ser
           195                 200                 205
   Ser Pro Val Thr Lys Ser Phe Asn Arg Gly Glu Cys
       210                 215                 220

## Description

### FIELD OF THE INVENTION

The present invention relates to the field of therapeutic treatments of diseases and disorders which are associated with elevated levels of lipoproteins. The administration of PCSK9 inhibitors to reduce the levels of serum remnant cholesterol and other lipoprotein subfractions in a patient is disclosed.

### BACKGROUND

Remnant cholesterol (also referred to as remnant lipoprotein) is a category of cholesterol that comprises non-HDL and non-LDL cholesterol. Remnant lipoproteins are products of VLDL lipolysis, and include VLDL₃ and intermediate-density lipoproteins (IDL, the direct precursor to LDL formation). Serum remnant cholesterol level has been identified as a predictive risk factor for coronary artery disease. In addition to remnant cholesterol, there are several subfractions of low density lipoprotein cholesterol (LDL-C) that may have relevance with regard to cardiovascular health. In particular, LDL-C is composed of a continuum of LDL particles of different densities and states of lipidation. Therapeutic reduction of serum remnant cholesterol and other lipoprotein subfraction levels may be a means for treating or reducing the risk of cardiovascular disorders.

PCSK9 is a proprotein convertase belonging to the proteinase K subfamily of the secretory subtilase family. The use of PCSK9 inhibitors (anti-PCSK9 antibodies) to reduce serum total cholesterol, LDL cholesterol and serum triglycerides is described in US Patent Nos. 8,062,640, 8,357,371, and US Patent Application Publication No. 2013/0064834.

WO 2012/154999 discloses methods of treating or preventing cholesterol related disorders, such as hypercholesterolemia, hyperlipidemia or dyslipidemia using antibodies against PCSK9. WO 2013/039969 discloses methods for reducing lipoprotein (a) Lp(a) in patients. The methods comprise selecting a patient who exhibits elevated serum Lp(a) and administering to the patient a pharmaceutical composition comprising a PCSK9 inhibitor. Varbo et al (2013) The Journal of the American College of Cardiology, 61, 427-436 discloses a study testing the hypothesis that elevated non-fasting remnant cholesterol is a causal risk factor for ischemic heart disease independent of reduced high-density lipoprotein cholesterol. Jorgensen et al (2012) European Heart Journal, 34, 1826-1833 discloses testing of whether genetically increased remnant cholesterol in hypertriglyceridemia due to genetic variation in the apolipoprotein A5 gene associates with an increased risk of myocardial infarction.

### BRIEF SUMMARY OF THE INVENTION

The present disclosure provides methods for reducing various lipoproteins and lipoprotein fractions in the serum of a patient. The patient can be a patient with hypercholesterolemia or at risk of developing hypercholesterolemia, or who has or is at risk of developing a cardiovascular disease.

The methods of the present disclosure comprise selecting a patient with an elevated serum level of a particular lipoprotein or lipoprotein fraction, and administering to the patient a pharmaceutical composition comprising an inhibitor of proprotein convertase subtilisin kexin-9 (PCSK9). According to certain aspects of the disclosure, the patient is selected on the basis of having an elevated serum remnant cholesterol level. According to certain other aspects of the disclosure, the patient is selected on the basis of having an elevated serum very low-density lipoprotein cholesterol level (*e*.*g*., elevated serum VLDL-C, VLDL₁-C, VLDL₂-C, VLDL₁₊₂-C, VLDL₃-C, etc.). According to certain other aspects of the disclosure, the patient is selected on the basis of having an elevated serum level of one or more low-density lipoprotein cholesterol (LDL-C) subfraction (*e*.*g*., elevated serum LDL₁-C, LDL₂-C, LDL₃-C, LDL₄-C, LDL₃₊₄-C, etc.). According to certain aspects of the present disclosure, the patient is additionally or alternatively selected on the basis of having an elevated serum level of Lp(a).

The present invention provides a pharmaceutical composition comprising a PCSK9 inhibitor for use in a method for reducing serum IDL-C in a patient and treating or preventing a cardiovascular disease or disorder, the method comprising selecting a patient with a serum IDL-C level of greater than 10 mg/dL as determined by vertical auto profile testing and who is diagnosed with or identified as being at risk of developing a cardiovascular disease or disorder, and administering the pharmaceutical composition to the patient, wherein the PCSK9 inhibitor is an antibody which comprises an HCVR having the amino acid sequence of SEQ ID NO:90 and an LCVR having the amino acid sequence of SEQ ID NO:92.

The patient may also be selected on the basis of exhibiting additional risk factors for such diseases and disorders in which a reduction in lipoprotein levels would be beneficial or risk-lowering. For example, patients with hypercholesterolemia (*e*.*g*., heFH, nonFH, etc.) may be good candidates for treatment in the present invention.

PCSK9 inhibitors which may be administered in accordance with the methods of the present disclosure include, *e*.*g*., anti-PCSK9 antibodies or antigen-binding fragments thereof. Specific exemplary anti-PCSK9 antibodies which may be used in the practice of the methods of the present disclosure include any antibodies or antigen-binding fragments as set forth in US Patent Nos. 8,062,640, and/or disclosed herein.

The PCSK9 inhibitor may be administered to a subject subcutaneously or intravenously. Furthermore, the PCSK9 inhibitor may be administered to a patient who is on a therapeutic statin regimen at the time of therapeutic intervention.

Other embodiments of the present invention will become apparent from a review of the ensuing detailed description.

### BRIEF DESCRIPTION OF THE FIGURES

**Figure 1** shows the dynamic relationship between mAb316P, free (unbound) PCSK9, and LDL-C levels following a single dose of mAb316P 150 mg SC in health subjects who were not receiving background statin therapy.
**Figure 2** shows the mean concentration of free mAb316P in HeFH and non-FH patients receiving mAb316P 50, 100, or 150 mg SC plus atorvastatin or mAb316P 150 mg SC plus diet only. Dotted line indicates quantitation limit for free PCSK9 (0.0312 mg/dL)
**Figure 3** shows the mean percentage change in LDL-C from baseline versus mean total mAb316P concentration (mg/dL) in patients (HeFH and non-FH combined; n-21 receiving mAb316P 150 mg SC on Study Days 1, 29, and 43. Points indicate sampling times; h, hour; d, day. The hysteresis curve moves in a clockwise direction over time, as indicated by the arrows, demonstrating the temporal relationship between mAb316P concentration and change in LDL-C.
**Figure 4** shows mean baseline free PCSK9 levels in non-FH patients receiving atorvastatin or diet alone (no statin).
**Figure 5** shows LDL-C efficacy curves for patients receiving mAb316P 150 mg SC or placebo ± atorvastatin.
**Figure 6** shows the mean percentage change in LDL-C from baseline over time in hypercholesterolemic patients on concomitant statin therapy following administration of mAb316P 50 mg, 100 mg, or 150 mg Q2W, 200 mg or 300 mg Q4W, or placebo. Delta symbol (Δ) corresponds to LOCF means.
**Figures 7-9** show the mean (SD) levels of cholesterol in VLDL and serum remnant lipoprotein subfractions and triglycerides before and after treatment for placebo and mAb316P 150 mg Q2W in three different clinical studies (A, B, and C, as summarized in Table 1 herein). **Figure 7** depicts the results for Study A, **Figure 8** depicts the results for Study B, and **Figure 9** depicts the results for Study C.
**Figures 10-12** show the mean (SD) levels of cholesterol in LDL subfractions before and after treatment for placebo and mAb316P 150 mg Q2W in three different clinical studies (A, B, and C, as summarized in Table 1 herein). **Figure 10** depicts the results for Study A, **Figure 11** depicts the results for Study B, and **Figure 12** depicts the results for Study C.
**Figure 13****, panels A and B,** show the dose response changes in apo CII and apo CIII, respectively, for all doses in Study A (as summarized in Table 1 herein).

### DETAILED DESCRIPTION

Before the present invention is described, it is to be understood that this invention is not limited to particular methods and experimental conditions described, as such methods and conditions may vary. It is also to be understood that the terminology used herein is for the purpose of describing particular embodiments only, and is not intended to be limiting, since the scope of the present invention will be limited only by the appended claims.

Unless defined otherwise, all technical and scientific terms used herein have the same meaning as commonly understood by one of ordinary skill in the art to which this invention belongs. As used herein, the term "about," when used in reference to a particular recited numerical value, means that the value may vary from the recited value by no more than 1%. For example, as used herein, the expression "about 100" includes 99 and 101 and all values in between (*e*.*g*., 99.1, 99.2, 99.3, 99.4, etc.).

Although any methods and materials similar or equivalent to those described herein can be used in the practice of the present invention, the preferred methods and materials are now described.

### Methods for Reducing Remnant Cholesterol and Other Lipoprotein Fractions

The present disclosure provides methods for reducing serum remnant cholesterol and other lipoprotein fractions in a patient. The methods of the disclosure comprise selecting a patient who exhibits an elevated serum level of one or more of remnant cholesterol (also referred to as "remnant lipoprotein cholesterol," "remnant lipoproteins," or RLP-C), very low density lipoprotein cholesterol (VLDL-C), intermediate density lipoprotein cholesterol (IDL-C), triglycerides (TG), and or Lp(a). In certain aspects, the methods of the disclosure comprise selecting a patient who exhibits elevated serum VLDL₁, VLDL₂, VLDL₁₊₂, VLDL₃, LDL₁, LDL₂, LDL₃, LDL₄, LDL₃₊₄, and/or other combinations thereof. The methods further comprise administering to the patient a pharmaceutical composition comprising a PCSK9 inhibitor.

As used herein, the expressions "remnant cholesterol," "remnant lipoproteins," "RLP," and the like, means the cholesterol content of triglyceride-rich lipoproteins, composed of VLDLs and IDLs in the fasting state and of these two lipoproteins together with chylomicron remnants in the nonfasting state. Remnant cholesterol can be calculated as: total cholesterol minus HDL-C minus LDL-C (*i.e*., non-[HDL-C + LDL-C]). Remnant cholesterol includes VLDL₃ and IDL.

Other lipoprotein fractions include, *e*.*g*., LDL₁-C ("large buoyant" LDL), LDL₂-C, LDL₃-C and LDL₄-C ("small dense" LDL). The serum levels of these lipoproteins may be ascertained by standard lipoprotein subfractionation techniques such as, *e*.*g*., vertical auto profile (VAP) testing, ion mobility, etc.

In the context of the present disclosure, "elevated serum lipoprotein" (*e*.*g*., elevated serum remnant cholesterol, elevated serum VLDL-C, elevated serum VLDL₁-C, elevated serum VLDL₂-C, elevated serum VLDL₁₊₂-C, elevated serum VLDL₃-C, elevated serum IDL-C, elevated serum LDL₁-C, elevated serum LDL₂-C, elevated serum LDL₃-C, elevated serum LDL₄-C, elevated serum LDL₃₊₄-C, etc.) means a serum level of the respective lipoprotein greater than about 8 mg/dL. In certain aspects, a patient is considered to exhibit an elevated serum lipoprotein if the level of the particular lipoprotein measured in the serum of a patient is greater than about 9 mg/dL, 10 mg/dL, 11 mg/dL, 12 mg/dL, 13 mg/dL, 14 mg/dL, 15 mg/dL, 16 mg/dL, 17 mg/dL, 18 mg/dL, 19 mg/dL, 20 mg/dL, 25 mg/dL, 30 mg/dL, 35 mg/dL, 40 mg/dL, 45 mg/dL, 50 mg/dL. The serum lipoprotein level can be measured in a patient post-prandial. In some embodiments, the lipoprotein level is measured after a period of time of fasting (*e*.*g*., after fasting for 6 hrs, 8 hrs, 10 hrs, 12 hrs or more). Any clinically acceptable diagnostic method can be used in the context of the present disclosure to measure serum lipoprotein in a patient.

According to the present disclosure, a reduction in serum remnant cholesterol or other lipoprotein subfraction (*e*.*g*., VLDL-C, VLDL₁-C, VLDL₂-C, VLDL₁₊₂-C, VLDL₃-C, IDL-C, IDL₁-C, IDL₂-C, IDL₁₊₂-C, LDL₁-C, LDL₂-C, LDL₂ₐ-C, LDL_{2b}-C, LDL₁₊₂ₐ-C, LDL₃-C, LDL₃ₐ-C, LDL_{3b}-C, LDL₄-C, LDL₄ₐ-C, LDL_{4b}-C, LDL_{4c}-C, LDL_{4a+4b+4c+3b}-C, LDL₃₊₄-C, etc.) means that the serum lipoprotein/lipoprotein subfraction level in the patient after receiving a pharmaceutical composition of the disclosure is reduced by about 5%, 10%, 15%, 20%, 25%, 30%, 35%, 40%, 45%, 50%, 55%, 60%, 65%, 70%, 75%, 80%, or more, from baseline. As used herein, "baseline level," when referring to a particular lipoprotein or lipoprotein subfraction, means the level of lipoprotein/lipoprotein subfraction measured in the serum of a subject prior to receiving a pharmaceutical composition of the present disclosure. The reduction in serum lipoprotein/lipoprotein subfraction resulting from administration of a pharmaceutical composition of the disclosure may be achieved and/or observed at 1 day, 2 days, 3 days, 4 days, 5 days, 6 days, 1 week, 2 weeks, 4 weeks, 6 weeks, 8 weeks, 10 weeks, 12 weeks , 14 weeks, 16 weeks, 18 weeks, 20 weeks, 22 weeks, or longer, following the initiation of a therapeutic regimen comprising administration of one or more doses of a pharmaceutical composition comprising a PCSK9 inhibitor, examples of which are disclosed elsewhere herein.

For example, the present disclosure includes methods for reducing serum remnant cholesterol in a patient by selecting a patient with elevated serum remnant cholesterol, and administering to the patient a pharmaceutical composition comprising a PCSK9 inhibitor, wherein serum remnant cholesterol is reduced in the patient by at least about 35% to about 45% from baseline following administration of the pharmaceutical composition.

The present disclosure also includes methods for reducing serum VLDL-C in a patient by selecting a patient with elevated serum VLDL-C, and administering to the patient a pharmaceutical composition comprising a PCSK9 inhibitor, wherein serum VLDL-C is reduced in the patient by at least about 20% to about 28% from baseline following administration of the pharmaceutical composition.

The present disclosure also includes methods for reducing serum VLDL₁₊₂-C in a patient by selecting a patient with elevated serum VLDL₁₊₂-C, and administering to the patient a pharmaceutical composition comprising a PCSK9 inhibitor, wherein serum VLDL₁₊₂-C is reduced in the patient by at least about 20% to about 32% from baseline following administration of the pharmaceutical composition.

The present disclosure also includes methods for reducing serum VLDL₃-C in a patient by selecting a patient with elevated serum VLDL₃-C, and administering to the patient a pharmaceutical composition comprising a PCSK9 inhibitor, wherein serum VLDL₃-C is reduced in the patient by at least about 20% to about 27% from baseline following administration of the pharmaceutical composition.

In the present invention, a serum IDL-C may be reduced in the patient by at least about 50% to about 56% from baseline following administration of the pharmaceutical composition.

The present disclosure also includes methods for reducing serum LDL₁-C in a patient by selecting a patient with elevated serum LDL₁-C, and administering to the patient a pharmaceutical composition comprising a PCSK9 inhibitor, wherein serum LDL₁-C is reduced in the patient by at least about 65% to about 78% from baseline following administration of the pharmaceutical composition.

The present disclosure also includes methods for reducing serum LDL₂-C in a patient by selecting a patient with elevated serum LDL₂-C, and administering to the patient a pharmaceutical composition comprising a PCSK9 inhibitor, wherein serum LDL₂-C is reduced in the patient by at least about 75% to about 85% from baseline following administration of the pharmaceutical composition.

The present disclosure also includes methods for reducing serum LDL₃₊₄-C in a patient by selecting a patient with elevated serum LDL₃₊₄-C, and administering to the patient a pharmaceutical composition comprising a PCSK9 inhibitor, wherein serum LDL₃₊₄-C is reduced in the patient by at least about 45% to about 70% from baseline following administration of the pharmaceutical composition.

The present disclosure also includes methods for reducing apolipoprotein (apo) CII and/or CIII in a patient by selecting a patient with hypercholesterolemia, and administering to the patient a pharmaceutical composition comprising a PCSK9 inhibitor. According to certain aspects, the patient has non-familial hypercholesterolemia (non-FH); in certain other aspects, the patient has heterozygous familial hypercholesterolemia (he-FH). According to certain instances of this aspect, apo CII is reduced in the patient by at least about 9% to about 30% from baseline, and apo CIII is reduced in the patient by at least about 20% to about 25%, following administration of the pharmaceutical composition.

The present disclosure also includes methods for reducing serum Lp(a) in a patient by selecting a patient with elevated serum Lp(a), and administering to the patient a pharmaceutical composition comprising a PCSK9 inhibitor, wherein serum Lp(a) is reduced in the patient by at least about 10% to about 40% (*e*.*g*., about 25%, about 30% or about 35%) from baseline following administration of the pharmaceutical composition.

The present disclosure also includes methods for reducing lipoprotein particle concentration in a patient by selecting a patient with hypercholesterolemia and/or elevated serum lipoprotein particle concentration, and administering to the patient a pharmaceutical composition comprising a PCSK9 inhibitor. The methods according to this aspect are useful for, *inter alia,* reducing the concentration of low-density lipoprotein particles (LDL-P), intermediate density lipoprotein particles (IDL-P), and very low-density lipoprotein particles (VLDL-P), including methods for reducing the serum concentrations of, *e*.*g*., small VLDL-P (diameter 29 to 42 mm); medium VLDL-P (diameter 42 to 60 nm); large VLDL-P (diameter > 60 nM); small LDL-P (diameter 18 to 20.5 nm); large LDL-P (diameter 20.5 to 23 nm); and IDL-P (diameter 23 to 29 nm).

### Patient Population

The methods of the present disclosure are useful for reducing serum remnant cholesterol and other lipoprotein fractions (*e*.*g*., VLDL-C, VLDL₁-C, VLDL₂-C, VLDL₁₊₂-C, VLDL₃-C, IDL-C, LDL₁-C, LDL₂-C, LDL₃-C, LDL₄-C, LDL₃₊₄-C, etc.) in a patient. In some instances the patient is otherwise healthy except for exhibiting elevated levels of one or more of the aforementioned serum lipoproteins. For example, the patient may not exhibit any other risk factor of cardiovascular, thrombotic or other diseases or disorders at the time of treatment. In other instances, however, the patient is selected on the basis of being diagnosed with, or at risk of developing, a disease or disorder that is caused by or correlated with elevated serum remnant cholesterol or with elevated serum levels of other lipoproteins or lipoprotein fractions. For example, at the time of, or prior to administration of the pharmaceutical composition, the patient may be diagnosed with or identified as being at risk of developing a cardiovascular disease or disorder, such as, *e*.*g*., coronary artery disease, acute myocardial infarction, asymptomatic carotid atherosclerosis, stroke, peripheral artery occlusive disease, etc. The cardiovascular disease or disorder, in some instances, is hypercholesterolemia. For example, a patient may be selected for treatment with the methods if the patient is diagnosed with or identified as being at risk of developing a hypercholesterolemia condition such as, *e*.*g*., heterozygous Familial Hypercholesterolemia (heFH), homozygous Familial Hypercholesterolemia (hoFH), Autosomal Dominant Hypercholesterolemia (ADH, *e*.*g*., ADH associated with one or more gain-of-function mutations in the PCSK9 gene), as well as incidences of hypercholesterolemia that are distinct from Familial Hypercholesterolemia (nonFH).

In other instances, at the time of, or prior to administration of the pharmaceutical composition, the patient may be diagnosed with or identified as being at risk of developing an arterial disorder such as cerebrovascular occlusive disease, peripheral vascular disease, peripheral arterial disorder, etc. The patient may be selected on the basis of being diagnosed with or at risk of developing a combination of two or more of the abovementioned diseases or disorders.

In yet other instances, the patient who is to be treated in the invention is selected on the basis of one or more factors selected from the group consisting of age (*e*.*g*., older than 40, 45, 50, 55, 60, 65, 70, 75, or 80 years), race, gender (male or female), exercise habits (*e*.*g*., regular exerciser, non-exerciser), other preexisting medical conditions (*e*.*g*., type-II diabetes, high blood pressure, etc.), and current medication status (*e*.*g*., currently taking statins [*e*.*g*.,cerivastatin, atorvastatin, simvastatin, pitavastatin, rosuvastatin, fluvastatin, lovastatin, pravastatin, etc.], beta blockers, niacin, etc.). The present disclosure also includes methods for reducing serum remnant cholesterol and/or other lipoprotein fraction levels in patients who are intolerant of, non-responsive to, or inadequately responsive to conventional statin therapy. Potential patients can be selected/screened on the basis of one or more of these factors (*e*.*g*., by questionnaire, diagnostic evaluation, etc.) before being treated with the methods.

### PCSK9 Inhibitors

The present invention comprises administering to a patient a therapeutic composition comprising a PCSK9 inhibitor. As used herein, a "PCSK9 inhibitor" is any agent which binds to or interacts with human PCSK9 and inhibits the normal biological function of PCSK9 *in vitro* or *in vivo.* Non-limiting examples of categories of PCSK9 inhibitors include small molecule PCSK9 antagonists, peptide-based PCSK9 antagonists (*e*.*g*., "peptibody" molecules), and antibodies or antigen-binding fragments of antibodies that specifically bind human PCSK9. In the invention, the inhibitor is an antibody as defined in the claims.

The term "human proprotein convertase subtilisin/kexin type 9" or "human PCSK9" or "hPCSK9", as used herein, refers to PCSK9 having the nucleic acid sequence shown in SEQ ID NO:754 and the amino acid sequence of SEQ ID NO:755, or a biologically active fragment thereof.

The term "antibody", as used herein, is intended to refer to immunoglobulin molecules comprising four polypeptide chains, two heavy (H) chains and two light (L) chains interconnected by disulfide bonds, as well as multimers thereof (*e*.*g*., IgM). Each heavy chain comprises a heavy chain variable region (abbreviated herein as HCVR or V_{H}) and a heavy chain constant region. The heavy chain constant region comprises three domains, C_{H}1, C_{H}2 and C_{H}3. Each light chain comprises a light chain variable region (abbreviated herein as LCVR or V_{L}) and a light chain constant region. The light chain constant region comprises one domain (C_{L}1). The V_{H} and V_{L} regions can be further subdivided into regions of hypervariability, termed complementarity determining regions (CDRs), interspersed with regions that are more conserved, termed framework regions (FR). Each V_{H} and V_{L} is composed of three CDRs and four FRs, arranged from amino-terminus to carboxy-terminus in the following order: FR1, CDR1, FR2, CDR2, FR3, CDR3, FR4. In different aspects of the disclosure, the FRs of the anti-PCSK9 antibody (or antigen-binding portion thereof) may be identical to the human germline sequences, or may be naturally or artificially modified. An amino acid consensus sequence may be defined based on a side-by-side analysis of two or more CDRs.

The term "antibody," as used herein, also includes antigen-binding fragments of full antibody molecules. The terms "antigen-binding portion" of an antibody, "antigen-binding fragment" of an antibody, and the like, as used herein, include any naturally occurring, enzymatically obtainable, synthetic, or genetically engineered polypeptide or glycoprotein that specifically binds an antigen to form a complex. Antigen-binding fragments of an antibody may be derived, *e*.*g*., from full antibody molecules using any suitable standard techniques such as proteolytic digestion or recombinant genetic engineering techniques involving the manipulation and expression of DNA encoding antibody variable and optionally constant domains. Such DNA is known and/or is readily available from, *e*.*g*., commercial sources, DNA libraries (including, *e*.*g*., phage-antibody libraries), or can be synthesized. The DNA may be sequenced and manipulated chemically or by using molecular biology techniques, for example, to arrange one or more variable and/or constant domains into a suitable configuration, or to introduce codons, create cysteine residues, modify, add or delete amino acids, etc.

Non-limiting examples of antigen-binding fragments include: (i) Fab fragments; (ii) F(ab')2 fragments; (iii) Fd fragments; (iv) Fv fragments; (v) single-chain Fv (scFv) molecules; (vi) dAb fragments; and (vii) minimal recognition units consisting of the amino acid residues that mimic the hypervariable region of an antibody (*e*.*g*., an isolated complementarity determining region (CDR) such as a CDR3 peptide), or a constrained FR3-CDR3-FR4 peptide. Other engineered molecules, such as domain-specific antibodies, single domain antibodies, domain-deleted antibodies, chimeric antibodies, CDR-grafted antibodies, diabodies, triabodies, tetrabodies, minibodies, nanobodies (*e*.*g*. monovalent nanobodies, bivalent nanobodies, etc.), small modular immunopharmaceuticals (SMIPs), and shark variable IgNAR domains, are also encompassed within the expression "antigen-binding fragment," as used herein.

An antigen-binding fragment of an antibody will typically comprise at least one variable domain. The variable domain may be of any size or amino acid composition and will generally comprise at least one CDR which is adjacent to or in frame with one or more framework sequences. In antigen-binding fragments having a V_{H} domain associated with a V_{L} domain, the V_{H} and V_{L} domains may be situated relative to one another in any suitable arrangement. For example, the variable region may be dimeric and contain V_{H}-V_{H}, V_{H}-V_{L} or V_{L}-V_{L} dimers. Alternatively, the antigen-binding fragment of an antibody may contain a monomeric V_{H} or V_{L} domain.

In certain aspects, an antigen-binding fragment of an antibody may contain at least one variable domain covalently linked to at least one constant domain. Non-limiting, exemplary configurations of variable and constant domains that may be found within an antigen-binding fragment of an antibody include: (i) V_{H}-C_{H}1; (ii) V_{H}-CH2; (iii) V_{H}-C_{H}3; (iv) V_{H}-C_{H}1-C_{H}2; (v) V_{H}-C_{H}1-C_{H}2-C_{H}3; (vi) V_{H}-C_{H}2-C_{H}3; (vii) V_{H}-C_{L}; (viii) V_{L}-C_{H}1; (ix) V_{L}-C_{H}2; (x) V_{L}-C_{H}3; (xi) V_{L}-C_{H}1-C_{H}2; (xii) V_{L}-C_{H}1-C_{H}2-C_{H}3; (xiii) V_{L}-C_{H}2-C_{H}3; and (xiv) V_{L}-C_{L}. In any configuration of variable and constant domains, including any of the exemplary configurations listed above, the variable and constant domains may be either directly linked to one another or may be linked by a full or partial hinge or linker region. A hinge region may consist of at least 2 (*e*.*g*., 5, 10, 15, 20, 40, 60 or more) amino acids which result in a flexible or semi-flexible linkage between adjacent variable and/or constant domains in a single polypeptide molecule. Moreover, an antigen-binding fragment of an antibody may comprise a homo-dimer or hetero-dimer (or other multimer) of any of the variable and constant domain configurations listed above in non-covalent association with one another and/or with one or more monomeric V_{H} or V_{L} domain (*e*.*g*., by disulfide bond(s)).

As with full antibody molecules, antigen-binding fragments may be monospecific or multispecific (*e*.*g*., bispecific). A multispecific antigen-binding fragment of an antibody will typically comprise at least two different variable domains, wherein each variable domain is capable of specifically binding to a separate antigen or to a different epitope on the same antigen. Any multispecific antibody format, including the exemplary bispecific antibody formats disclosed herein, may be adapted for use in the context of an antigen-binding fragment of an antibody using routine techniques available in the art.

The constant region of an antibody is important in the ability of an antibody to fix complement and mediate cell-dependent cytotoxicity. Thus, the isotype of an antibody may be selected on the basis of whether it is desirable for the antibody to mediate cytotoxicity.

The term "human antibody", as used herein, is intended to include antibodies having variable and constant regions derived from human germline immunoglobulin sequences. The human antibodies may nonetheless include amino acid residues not encoded by human germline immunoglobulin sequences (e.g., mutations introduced by random or site-specific mutagenesis *in vitro* or by somatic mutation *in vivo*), for example in the CDRs and in particular CDR3. However, the term "human antibody", as used herein, is not intended to include antibodies in which CDR sequences derived from the germline of another mammalian species, such as a mouse, have been grafted onto human framework sequences.

The term "recombinant human antibody", as used herein, is intended to include all human antibodies that are prepared, expressed, created or isolated by recombinant means, such as antibodies expressed using a recombinant expression vector transfected into a host cell (described further below), antibodies isolated from a recombinant, combinatorial human antibody library (described further below), antibodies isolated from an animal (e.g., a mouse) that is transgenic for human immunoglobulin genes (see e.g., Taylor et al. (1992) Nucl. Acids Res. 20:6287-6295) or antibodies prepared, expressed, created or isolated by any other means that involves splicing of human immunoglobulin gene sequences to other DNA sequences. Such recombinant human antibodies have variable and constant regions derived from human germline immunoglobulin sequences. In certain aspects, however, such recombinant human antibodies are subjected to *in vitro* mutagenesis (or, when an animal transgenic for human Ig sequences is used, *in vivo* somatic mutagenesis) and thus the amino acid sequences of the V_{H} and V_{L} regions of the recombinant antibodies are sequences that, while derived from and related to human germline V_{H} and V_{L} sequences, may not naturally exist within the human antibody germline repertoire *in vivo.*

Human antibodies can exist in two forms that are associated with hinge heterogeneity. In one form, an immunoglobulin molecule comprises a stable four chain construct of approximately 150-160 kDa in which the dimers are held together by an interchain heavy chain disulfide bond. In a second form, the dimers are not linked via inter-chain disulfide bonds and a molecule of about 75-80 kDa is formed composed of a covalently coupled light and heavy chain (half-antibody). These forms have been extremely difficult to separate, even after affinity purification.

The frequency of appearance of the second form in various intact IgG isotypes is due to, but not limited to, structural differences associated with the hinge region isotype of the antibody. A single amino acid substitution in the hinge region of the human IgG4 hinge can significantly reduce the appearance of the second form (Angal et al. (1993) Molecular Immunology 30:105) to levels typically observed using a human IgG1 hinge. The instant invention may utilize antibodies having one or more mutations in the hinge, C_{H}2 or C_{H}3 region which may be desirable, for example, in production, to improve the yield of the desired antibody form.

An "isolated antibody," as used herein, means an antibody that has been identified and separated and/or recovered from at least one component of its natural environment. For example, an antibody that has been separated or removed from at least one component of an organism, or from a tissue or cell in which the antibody naturally exists or is naturally produced, is an "isolated antibody" for purposes of the present invention. An isolated antibody also includes an antibody *in situ* within a recombinant cell. Isolated antibodies are antibodies that have been subjected to at least one purification or isolation step. According to certain embodiments, an isolated antibody may be substantially free of other cellular material and/or chemicals.

The term "specifically binds," or the like, means that an antibody or antigen-binding fragment thereof forms a complex with an antigen that is relatively stable under physiologic conditions. Methods for determining whether an antibody specifically binds to an antigen are well known in the art and include, for example, equilibrium dialysis, surface plasmon resonance, and the like. For example, an antibody that "specifically binds" PCSK9, as used in the context of the present invention, includes antibodies that bind PCSK9 or portion thereof with a K_{D} of less than about 1000 nM, less than about 500 nM, less than about 300 nM, less than about 200 nM, less than about 100 nM, less than about 90 nM, less than about 80 nM, less than about 70 nM, less than about 60 nM, less than about 50 nM, less than about 40 nM, less than about 30 nM, less than about 20 nM, less than about 10 nM, less than about 5 nM, less than about 4 nM, less than about 3 nM, less than about 2 nM, less than about 1 nM or less than about 0.5 nM, as measured in a surface plasmon resonance assay. An isolated antibody that specifically binds human PCSK9, however, have cross-reactivity to other antigens, such as PCSK9 molecules from other (non-human) species.

The anti-PCSK9 antibodies useful for the methods of the present disclosure may comprise one or more amino acid substitutions, insertions and/or deletions in the framework and/or CDR regions of the heavy and light chain variable domains as compared to the corresponding germline sequences from which the antibodies were derived. Such mutations can be readily ascertained by comparing the amino acid sequences disclosed herein to germline sequences available from, for example, public antibody sequence databases. The present disclosure includes methods involving the use of antibodies, and antigen-binding fragments thereof, which are derived from any of the amino acid sequences disclosed herein, wherein one or more amino acids within one or more framework and/or CDR regions are mutated to the corresponding residue(s) of the germline sequence from which the antibody was derived, or to the corresponding residue(s) of another human germline sequence, or to a conservative amino acid substitution of the corresponding germline residue(s) (such sequence changes are referred to herein collectively as "germline mutations"). A person of ordinary skill in the art, starting with the heavy and light chain variable region sequences disclosed herein, can easily produce numerous antibodies and antigen-binding fragments which comprise one or more individual germline mutations or combinations thereof. In certain instances, all of the framework and/or CDR residues within the V_{H} and/or V_{L} domains are mutated back to the residues found in the original germline sequence from which the antibody was derived. In other instances, only certain residues are mutated back to the original germline sequence, *e*.*g*., only the mutated residues found within the first 8 amino acids of FR1 or within the last 8 amino acids of FR4, or only the mutated residues found within CDR1, CDR2 or CDR3. In other instances, one or more of the framework and/or CDR residue(s) are mutated to the corresponding residue(s) of a different germline sequence (*i.e*., a germline sequence that is different from the germline sequence from which the antibody was originally derived). Furthermore, the antibodies may contain any combination of two or more germline mutations within the framework and/or CDR regions, e.g., wherein certain individual residues are mutated to the corresponding residue of a particular germline sequence while certain other residues that differ from the original germline sequence are maintained or are mutated to the corresponding residue of a different germline sequence. Once obtained, antibodies and antigen-binding fragments that contain one or more germline mutations can be easily tested for one or more desired property such as, improved binding specificity, increased binding affinity, improved or enhanced antagonistic or agonistic biological properties (as the case may be), reduced immunogenicity, etc. The use of antibodies and antigen-binding fragments obtained in this general manner are encompassed within the present disclosure.

The present disclosure also includes methods involving the use of anti-PCSK9 antibodies comprising variants of any of the HCVR, LCVR, and/or CDR amino acid sequences disclosed herein having one or more conservative substitutions. For example, the present disclosure includes the use of anti-PCSK9 antibodies having HCVR, LCVR, and/or CDR amino acid sequences with, *e*.*g*., 10 or fewer, 8 or fewer, 6 or fewer, 4 or fewer, etc. conservative amino acid substitutions relative to any of the HCVR, LCVR, and/or CDR amino acid sequences disclosed herein.

The term "surface plasmon resonance", as used herein, refers to an optical phenomenon that allows for the analysis of real-time interactions by detection of alterations in protein concentrations within a biosensor matrix, for example using the BIAcore™ system (Biacore Life Sciences division of GE Healthcare, Piscataway, NJ).

The term "K_{D}", as used herein, is intended to refer to the equilibrium dissociation constant of a particular antibody-antigen interaction.

The term "epitope" refers to an antigenic determinant that interacts with a specific antigen binding site in the variable region of an antibody molecule known as a paratope. A single antigen may have more than one epitope. Thus, different antibodies may bind to different areas on an antigen and may have different biological effects. Epitopes may be either conformational or linear. A conformational epitope is produced by spatially juxtaposed amino acids from different segments of the linear polypeptide chain. A linear epitope is one produced by adjacent amino acid residues in a polypeptide chain. In certain circumstance, an epitope may include moieties of saccharides, phosphoryl groups, or sulfonyl groups on the antigen.

According to certain instances, the anti-PCSK9 antibody used in the methods of the present disclosure is an antibody with pH-dependent binding characteristics. As used herein, the expression "pH-dependent binding" means that the antibody or antigen-binding fragment thereof exhibits "reduced binding to PCSK9 at acidic pH as compared to neutral pH" (for purposes of the present disclosure, both expressions may be used interchangeably). For the example, antibodies "with pH-dependent binding characteristics" includes antibodies and antigen-binding fragments thereof that bind PCSK9 with higher affinity at neutral pH than at acidic pH. In certain instances, the antibodies and antigen-binding fragments bind PCSK9 with at least 3, 5, 10, 15, 20, 25, 30, 35, 40, 45, 50, 55, 60, 65, 70, 75, 80, 85, 90, 95, 100, or more times higher affinity at neutral pH than at acidic pH.

According to this aspect, the anti-PCSK9 antibodies with pH-dependent binding characteristics may possess one or more amino acid variations relative to the parental anti-PCSK9 antibody. For example, an anti-PCSK9 antibody with pH-dependent binding characteristics may contain one or more histidine substitutions or insertions, *e.g*., in one or more CDRs of a parental anti-PCSK9 antibody. Thus, according to certain aspects of the disclosure, methods are provided comprising administering an anti-PCSK9 antibody which comprises CDR amino acid sequences (*e*.*g*., heavy and light chain CDRs) which are identical to the CDR amino acid sequences of a parental anti-PCSK9 antibody, except for the substitution of one or more amino acids of one or more CDRs of the parental antibody with a histidine residue. The anti-PCSK9 antibodies with pH-dependent binding may possess, *e*.*g*., 1, 2, 3, 4, 5, 6, 7, 8, 9, or more histidine substitutions, either within a single CDR of a parental antibody or distributed throughout multiple (*e*.*g*., 2, 3, 4, 5, or 6) CDRs of a parental anti-PCSK9 antibody. For example, the present disclosure includes the use of anti-PCSK9 antibodies with pH-dependent binding comprising one or more histidine substitutions in HCDR1, one or more histidine substitutions in HCDR2, one or more histidine substitutions in HCDR3, one or more histidine substitutions in LCDR1, one or more histidine substitutions in LCDR2, and/or one or more histidine substitutions in LCDR3, of a parental anti-PCSK9 antibody.

As used herein, the expression "acidic pH" means a pH of 6.0 or less (*e*.*g*., less than about 6.0, less than about 5.5, less than about 5.0, etc.). The expression "acidic pH" includes pH values of about 6.0, 5.95, 5.90, 5.85, 5.8, 5.75, 5.7, 5.65, 5.6, 5.55, 5.5, 5.45, 5.4, 5.35, 5.3, 5.25, 5.2, 5.15, 5.1, 5.05, 5.0, or less. As used herein, the expression "neutral pH" means a pH of about 7.0 to about 7.4. The expression "neutral pH" includes pH values of about 7.0, 7.05, 7.1, 7.15, 7.2, 7.25, 7.3, 7.35, and 7.4.

### Preparation of Human Antibodies

Methods for generating human antibodies in transgenic mice are known in the art. Any such known methods can be used to make human antibodies that specifically bind to human PCSK9.

Using VELOCIMMUNE™ technology (see, for example, US 6,596,541, Regeneron Pharmaceuticals) or any other known method for generating monoclonal antibodies, high affinity chimeric antibodies to PCSK9 are initially isolated having a human variable region and a mouse constant region. The VELOCIMMUNE® technology involves generation of a transgenic mouse having a genome comprising human heavy and light chain variable regions operably linked to endogenous mouse constant region loci such that the mouse produces an antibody comprising a human variable region and a mouse constant region in response to antigenic stimulation. The DNA encoding the variable regions of the heavy and light chains of the antibody are isolated and operably linked to DNA encoding the human heavy and light chain constant regions. The DNA is then expressed in a cell capable of expressing the fully human antibody.

Generally, a VELOCIMMUNE® mouse is challenged with the antigen of interest, and lymphatic cells (such as B-cells) are recovered from the mice that express antibodies. The lymphatic cells may be fused with a myeloma cell line to prepare immortal hybridoma cell lines, and such hybridoma cell lines are screened and selected to identify hybridoma cell lines that produce antibodies specific to the antigen of interest. DNA encoding the variable regions of the heavy chain and light chain may be isolated and linked to desirable isotypic constant regions of the heavy chain and light chain. Such an antibody protein may be produced in a cell, such as a CHO cell. Alternatively, DNA encoding the antigen-specific chimeric antibodies or the variable domains of the light and heavy chains may be isolated directly from antigen-specific lymphocytes.

Initially, high affinity chimeric antibodies are isolated having a human variable region and a mouse constant region. The antibodies are characterized and selected for desirable characteristics, including affinity, selectivity, epitope, etc, using standard procedures known to those skilled in the art. The mouse constant regions are replaced with a desired human constant region to generate the fully human antibody, for example wild-type or modified IgG1 or IgG4. While the constant region selected may vary according to specific use, high affinity antigen-binding and target specificity characteristics reside in the variable region.

In general, the antibodies that can be used in the methods possess high affinities, as described above, when measured by binding to antigen either immobilized on solid phase or in solution phase. The mouse constant regions are replaced with desired human constant regions to generate the fully human antibodies of the invention. While the constant region selected may vary according to specific use, high affinity antigen-binding and target specificity characteristics reside in the variable region.

Specific examples of human antibodies or antigen-binding fragments of antibodies that specifically bind PCSK9 which can be used in the context of the methods of the present disclosure include any antibody or antigen-binding fragment which comprises the three heavy chain CDRs (HCDR1, HCDR2 and HCDR3) contained within a heavy chain variable region (HCVR) having an amino acid sequence selected from the group consisting of SEQ ID NOs: 2, 18, 22, 26, 42, 46, 50, 66, 70, 74, 90, 94, 98, 114, 118, 122, 138, 142, 146, 162, 166, 170, 186, 190, 194, 210, 214, 218, 234, 238, 242, 258, 262, 266, 282, 286, 290, 306, 310, 314, 330, 334, 338, 354, 358, 362, 378, 382, 386, 402, 406, 410, 426, 430, 434, 450, 454, 458, 474, 478, 482, 498, 502, 506, 522, 526, 530, 546, 550, 554, 570, 574, 578, 594, 598, 602, 618, 622, 626, 642, 646, 650, 666, 670, 674, 690, 694, 698, 714, 718, 722, 738 and 742, or a substantially similar sequence thereof having at least 90%, at least 95%, at least 98% or at least 99% sequence identity. The antibody or antigen-binding fragment may comprise the three light chain CDRs (LCVR1, LCVR2, LCVR3) contained within a light chain variable region (LCVR) having an amino acid sequence selected from the group consisting of SEQ ID NOs: 10, 20, 24, 34, 44, 48, 58, 68, 72, 82, 92, 96, 106, 116, 120, 130, 140, 144, 154, 164, 168, 178, 188, 192, 202, 212, 216, 226, 236, 240, 250, 260, 264, 274, 284, 288, 298, 308, 312, 322, 332, 336, 346, 356, 360, 370, 380, 384, 394, 404, 408, 418, 428, 432, 442, 452, 456, 466, 476, 480, 490, 500, 504, 514, 524, 528, 538, 548, 552, 562, 572, 576, 586, 596, 600, 610, 620, 624, 634, 644, 648, 658, 668, 672, 682, 692, 696, 706, 716, 720, 730, 740 and 744, or a substantially similar sequence thereof having at least 90%, at least 95%, at least 98% or at least 99% sequence identity.

In certain aspects of the present disclosure, the antibody or antigen-binding fragment thereof comprises the six CDRs (HCDR1, HCDR2, HCDR3, LCDR1, LCDR2 and LCDR3) from the heavy and light chain variable region amino acid sequence pairs (HCVR/LCVR) selected from the group consisting of SEQ ID NOs: 2/10, 18/20, 22/24, 26/34, 42/44, 46/48, 50/58, 66/68, 70/72, 74/82, 90/92, 94/96, 98/106, 114/116, 118/120, 122/130, 138/140, 142/144, 146/154, 162/164, 166/168, 170/178, 186/188, 190/192, 194/202, 210/212, 214/216, 218/226, 234/236, 238/240, 242/250, 258/260, 262/264, 266/274, 282/284, 286/288, 290/298, 306/308, 310/312, 314/322, 330/332, 334/336, 338/346, 354/356, 358/360, 362/370, 378/380, 382/384, 386/394, 402/404, 406/408, 410/418, 426/428, 430/432, 434/442, 450/452, 454/456, 458/466, 474/476, 478/480, 482/490, 498/500, 502/504, 506/514, 522/524, 526/528, 530/538, 546/548, 550/552, 554/562, 570/572, 574/576, 578/586, 594/596, 598/600, 602/610, 618/620, 622/624, 626/634, 642/644, 646/648, 650/658, 666/668, 670/672, 674/682, 690/692, 694/696, 698/706, 714/716, 718/720, 722/730, 738/740 and 742/744.

In certain aspects of the present disclosure, the anti-PCSK9 antibody, or antigen-binding fragment thereof, that can be used in the methods of the present disclosure has HCDR1/HCDR2/HCDR3/LCDR1/LCDR2/LCDR3 amino acid sequences selected from SEQ ID NOs: 76/78/80/84/86/88 (mAb316P) and 220/222/224/228/230/232 (mAb300N) (See US Patent App. Publ No. 2010/0166768).

In certain aspects of the present disclosure, the antibody or antigen-binding fragment thereof comprises HCVR/LCVR amino acid sequence pairs selected from the group consisting of SEQ ID NOs: 2/10, 18/20, 22/24, 26/34, 42/44, 46/48, 50/58, 66/68, 70/72, 74/82, 90/92, 94/96, 98/106, 114/116, 118/120, 122/130, 138/140, 142/144, 146/154, 162/164, 166/168, 170/178, 186/188, 190/192, 194/202, 210/212, 214/216, 218/226, 234/236, 238/240, 242/250, 258/260, 262/264, 266/274, 282/284, 286/288, 290/298, 306/308, 310/312, 314/322, 330/332, 334/336, 338/346, 354/356, 358/360, 362/370, 378/380, 382/384, 386/394, 402/404, 406/408, 410/418, 426/428, 430/432, 434/442, 450/452, 454/456, 458/466, 474/476, 478/480, 482/490, 498/500, 502/504, 506/514, 522/524, 526/528, 530/538, 546/548, 550/552, 554/562, 570/572, 574/576, 578/586, 594/596, 598/600, 602/610, 618/620, 622/624, 626/634, 642/644, 646/648, 650/658, 666/668, 670/672, 674/682, 690/692, 694/696, 698/706, 714/716, 718/720, 722/730, 738/740 and 742/744. In the invention, the antibody comprises a HCVR having the amino acid sequence of SEQ ID NO: 90 and a LCVR having the amino sequence of SEQ ID NO: 92.

### Pharmaceutical Compositions and Methods of Administration

The present invention relates to methods which comprise administering a PCSK9 inhibitor to a patient, wherein the PCSK9 inhibitor is contained within a pharmaceutical composition. The pharmaceutical compositions are formulated with suitable carriers, excipients, and other agents that provide suitable transfer, delivery, tolerance, and the like. A multitude of appropriate formulations can be found in the formulary known to all pharmaceutical chemists: Remington's Pharmaceutical Sciences, Mack Publishing Company, Easton, PA. These formulations include, for example, powders, pastes, ointments, jellies, waxes, oils, lipids, lipid (cationic or anionic) containing vesicles (such as LIPOFECTIN™), DNA conjugates, anhydrous absorption pastes, oil-in-water and water-in-oil emulsions, emulsions carbowax (polyethylene glycols of various molecular weights), semi-solid gels, and semi-solid mixtures containing carbowax. See also Powell et al. "Compendium of excipients for parenteral formulations" PDA (1998) J Pharm Sci Technol 52:238-311.

Various delivery systems are known and can be used to administer the pharmaceutical composition, e.g., encapsulation in liposomes, microparticles, microcapsules, recombinant cells capable of expressing the mutant viruses, receptor mediated endocytosis (see, e.g., Wu et al., 1987, J. Biol. Chem. 262:4429-4432). Methods of administration include, but are not limited to, intradermal, intramuscular, intraperitoneal, intravenous, subcutaneous, intranasal, epidural, and oral routes. The composition may be administered by any convenient route, for example by infusion or bolus injection, by absorption through epithelial or mucocutaneous linings (e.g., oral mucosa, rectal and intestinal mucosa, etc.) and may be administered together with other biologically active agents.

A pharmaceutical composition can be delivered subcutaneously or intravenously with a standard needle and syringe. In addition, with respect to subcutaneous delivery, a pen delivery device readily has applications in delivering a pharmaceutical composition. Such a pen delivery device can be reusable or disposable. A reusable pen delivery device generally utilizes a replaceable cartridge that contains a pharmaceutical composition. Once all of the pharmaceutical composition within the cartridge has been administered and the cartridge is empty, the empty cartridge can readily be discarded and replaced with a new cartridge that contains the pharmaceutical composition. The pen delivery device can then be reused. In a disposable pen delivery device, there is no replaceable cartridge. Rather, the disposable pen delivery device comes prefilled with the pharmaceutical composition held in a reservoir within the device. Once the reservoir is emptied of the pharmaceutical composition, the entire device is discarded.

Numerous reusable pen and autoinjector delivery devices have applications in the subcutaneous delivery of a pharmaceutical composition of the present invention. Examples include, but are not limited to AUTOPEN™ (Owen Mumford, Inc., Woodstock, UK), DISETRONIC™ pen (Disetronic Medical Systems, Bergdorf, Switzerland), HUMALOG MIX 75/25™ pen, HUMALOG™ pen, HUMALIN 70/30™ pen (Eli Lilly and Co., Indianapolis, IN), NOVOPEN™ I, II and III (Novo Nordisk, Copenhagen, Denmark), NOVOPEN JUNIOR™ (Novo Nordisk, Copenhagen, Denmark), BD™ pen (Becton Dickinson, Franklin Lakes, NJ), OPTIPEN™, OPTIPEN PRO™, OPTIPEN STARLET™, and OPTICLIK™ (sanofi-aventis, Frankfurt, Germany), to name only a few. Examples of disposable pen delivery devices having applications in subcutaneous delivery of a pharmaceutical composition include, but are not limited to the SOLOSTAR™ pen (sanofi-aventis), the FLEXPEN™ (Novo Nordisk), and the KWIKPEN™ (Eli Lilly), the SURECLICK™ Autoinjector (Amgen, Thousand Oaks, CA), the PENLET™ (Haselmeier, Stuttgart, Germany), the EPIPEN (Dey, L.P.), and the HUMIRA™ Pen (Abbott Labs, Abbott Park IL), to name only a few.

In certain situations, the pharmaceutical composition can be delivered in a controlled release system. In one embodiment, a pump may be used (see Langer, supra; Sefton, 1987, CRC Crit. Ref. Biomed. Eng. 14:201). In another embodiment, polymeric materials can be used; see, Medical Applications of Controlled Release, Langer and Wise (eds.), 1974, CRC Pres., Boca Raton, Florida. In yet another embodiment, a controlled release system can be placed in proximity of the composition's target, thus requiring only a fraction of the systemic dose (see, *e*.*g*., Goodson, 1984, in Medical Applications of Controlled Release, supra, vol. 2, pp. 115-138). Other controlled release systems are discussed in the review by Langer, 1990, Science 249:1527-1533.

The injectable preparations may include dosage forms for intravenous, subcutaneous, intracutaneous and intramuscular injections, drip infusions, etc. These injectable preparations may be prepared by known methods. For example, the injectable preparations may be prepared, e.g., by dissolving, suspending or emulsifying the antibody or its salt described above in a sterile aqueous medium or an oily medium conventionally used for injections. As the aqueous medium for injections, there are, for example, physiological saline, an isotonic solution containing glucose and other auxiliary agents, etc., which may be used in combination with an appropriate solubilizing agent such as an alcohol (e.g., ethanol), a polyalcohol (e.g., propylene glycol, polyethylene glycol), a nonionic surfactant [e.g., polysorbate 80, HCO-50 (polyoxyethylene (50 mol) adduct of hydrogenated castor oil)], etc. As the oily medium, there are employed, e.g., sesame oil, soybean oil, etc., which may be used in combination with a solubilizing agent such as benzyl benzoate, benzyl alcohol, etc. The injection thus prepared is preferably filled in an appropriate ampoule.

Advantageously, the pharmaceutical compositions for oral or parenteral use described above are prepared into dosage forms in a unit dose suited to fit a dose of the active ingredients. Such dosage forms in a unit dose include, for example, tablets, pills, capsules, injections (ampoules), suppositories, etc.

### Dosage

The amount of PCSK9 inhibitor (*e*.*g*., anti-PCSK9 antibody) administered to a subject according to the methods disclosed herein is, generally, a therapeutically effective amount. As used herein, the phrase "therapeutically effective amount" means a dose of PCSK9 inhibitor that results in a detectable reduction (at least about 5%, 10%, 15%, 20%, 25%, 30%, 35%, 40%, 45%, 50%, 55%, 60%, 65%, 70%, 75%, or more from baseline) in one or more parameters selected from the group consisting of remnant cholesterol (RLP-C), VLDL-C, VLDL₁-C, VLDL₂-C, VLDL₁₊₂-C, VLDL₃-C, IDL-C, LDL₁-C, LDL₂-C, LDL₃-C, LDL₄-C, LDL₃₊₄-C. Alternatively, animal models can be used to establish whether a particular amount of a candidate PCSK9 inhibitor is a therapeutically effective amount.

In the case of an anti-PCSK9 antibody, a therapeutically effective amount can be from about 0.05 mg to about 600 mg, e.g., about 0.05 mg, about 0.1 mg, about 1.0 mg, about 1.5 mg, about 2.0 mg, about 10 mg, about 20 mg, about 30 mg, about 40 mg, about 50 mg, about 60 mg, about 70 mg, about 75 mg, about 80 mg, about 90 mg, about 100 mg, about 110 mg, about 120 mg, about 130 mg, about 140 mg, about 150 mg, about 160 mg, about 170 mg, about 180 mg, about 190 mg, about 200 mg, about 210 mg, about 220 mg, about 230 mg, about 240 mg, about 250 mg, about 260 mg, about 270 mg, about 280 mg, about 290 mg, about 300 mg, about 310 mg, about 320 mg, about 330 mg, about 340 mg, about 350 mg, about 360 mg, about 370 mg, about 380 mg, about 390 mg, about 400 mg, about 410 mg, about 420 mg, about 430 mg, about 440 mg, about 450 mg, about 460 mg, about 470 mg, about 480 mg, about 490 mg, about 500 mg, about 510 mg, about 520 mg, about 530 mg, about 540 mg, about 550 mg, about 560 mg, about 570 mg, about 580 mg, about 590 mg, or about 600 mg, of the anti-PCSK9 antibody.

The amount of anti-PCSK9 antibody contained within the individual doses may be expressed in terms of milligrams of antibody per kilogram of patient body weight (*i.e*., mg/kg). For example, the anti-PCSK9 antibody may be administered to a patient at a dose of about 0.0001 to about 10 mg/kg of patient body weight.

### Combination Therapies

The methods, according to certain embodiments, may comprise administering a pharmaceutical composition comprising an anti-PCSK9 antibody to a patient who is on a therapeutic regimen for the treatment of hypercholesterolemia at the time of, or just prior to, administration of the pharmaceutical composition. For example, a patient who has previously been diagnosed with hypercholesterolemia may have been prescribed and is taking a stable therapeutic regimen of another drug prior to and/or concurrent with administration of a pharmaceutical composition comprising an anti-PCSK9 antibody. The prior or concurrent therapeutic regimen may comprise, *e*.*g*., (1) an agent which induces a cellular depletion of cholesterol synthesis by inhibiting 3-hydroxy-3-methylglutaryl (HMG)-coenzyme A (CoA) reductase, such as a statin (e.g., cerivastatin, atorvastatin, simvastatin, pitavastatin, rosuvastatin, fluvastatin, lovastatin, pravastatin, etc.); (2) an agent which inhibits cholesterol uptake and or bile acid re-absorption; (3) an agent which increase lipoprotein catabolism (such as niacin); and/or (4) activators of the LXR transcription factor that plays a role in cholesterol elimination such as 22-hydroxycholesterol. In certain embodiments, the patient, prior to or concurrent with administration of an anti-PCSK9 antibody is on a fixed combination of therapeutic agents such as ezetimibe plus simvastatin; a statin with a bile resin (e.g., cholestyramine, colestipol, colesevelam); niacin plus a statin (e.g., niacin with lovastatin); or with other lipid lowering agents such as omega-3-fatty acid ethyl esters (for example, omacor).

### Administration Regimens

According to certain embodiments of the present invention, multiple doses of a PCSK9 inhibitor (*i.e*., a pharmaceutical composition comprising a PCSK9 inhibitor) may be administered to a subject over a defined time course. The methods according to this aspect of the invention comprise sequentially administering to a subject multiple doses of a PCSK9 inhibitor. As used herein, "sequentially administering" means that each dose of PCSK9 inhibitor is administered to the subject at a different point in time, *e*.*g*., on different days separated by a predetermined interval (*e*.*g*., hours, days, weeks or months). The present invention also includes sequentially administering to the patient a single initial dose of a PCSK9 inhibitor, followed by one or more secondary doses of the PCSK9 inhibitor, and optionally followed by one or more tertiary doses of the PCSK9 inhibitor.

The terms "initial dose," "secondary doses," and "tertiary doses," refer to the temporal sequence of administration of the individual doses of a pharmaceutical composition comprising a PCSK9 inhibitor. Thus, the "initial dose" is the dose which is administered at the beginning of the treatment regimen (also referred to as the "baseline dose"); the "secondary doses" are the doses which are administered after the initial dose; and the "tertiary doses" are the doses which are administered after the secondary doses. The initial, secondary, and tertiary doses may all contain the same amount of the PCSK9 inhibitor, but generally may differ from one another in terms of frequency of administration. In certain embodiments, however, the amount of PCSK9 inhibitor contained in the initial, secondary and/or tertiary doses varies from one another (*e*.*g*., adjusted up or down as appropriate) during the course of treatment. In certain embodiments, two or more (*e*.*g*., 2, 3, 4, or 5) doses are administered at the beginning of the treatment regimen as "loading doses" followed by subsequent doses that are administered on a less frequent basis (*e*.*g*., "maintenance doses").

According to exemplary embodiments of the present invention, each secondary and/or tertiary dose is administered 1 to 26 (*e*.*g*., 1, 1½, 2, 2½, 3, 3½, 4, 4½, 5, 5½, 6, 6½, 7, 7½, 8, 8½, 9, 9½, 10, 10½, 11, 11½, 12, 12½, 13, 13½, 14, 14½, 15, 15½, 16, 16½, 17, 17½, 18, 18½, 19, 19½, 20, 20½, 21, 21½, 22, 22½, 23, 23½, 24, 24½, 25, 25½, 26, 26½, or more) weeks after the immediately preceding dose. The phrase "the immediately preceding dose," as used herein, means, in a sequence of multiple administrations, the dose of antigen-binding molecule which is administered to a patient prior to the administration of the very next dose in the sequence with no intervening doses.

The methods according to this aspect of the invention may comprise administering to a patient any number of secondary and/or tertiary doses of a PCSK9 inhibitor. For example, in certain embodiments, only a single secondary dose is administered to the patient. In other embodiments, two or more (*e*.*g*., 2, 3, 4, 5, 6, 7, 8, or more) secondary doses are administered to the patient. Likewise, in certain embodiments, only a single tertiary dose is administered to the patient. In other embodiments, two or more (*e*.*g*., 2, 3, 4, 5, 6, 7, 8, or more) tertiary doses are administered to the patient.

In embodiments involving multiple secondary doses, each secondary dose may be administered at the same frequency as the other secondary doses. For example, each secondary dose may be administered to the patient 1 to 2, 4, 6, 8 or more weeks after the immediately preceding dose. Similarly, in embodiments involving multiple tertiary doses, each tertiary dose may be administered at the same frequency as the other tertiary doses. For example, each tertiary dose may be administered to the patient 1 to 2, 4, 6, 8 or more weeks after the immediately preceding dose. Alternatively, the frequency at which the secondary and/or tertiary doses are administered to a patient can vary over the course of the treatment regimen. The frequency of administration may also be adjusted during the course of treatment by a physician depending on the needs of the individual patient following clinical examination.

The present invention includes administration regimens comprising an up-titration option (also referred to herein as "dose modification"). As used herein, an "up-titration option" means that, after receiving a particular number of doses of a PCSK9 inhibitor, if a patient has not achieved a specified reduction in one or more defined therapeutic parameters, the dose of the PCSK9 inhibitor is thereafter increased. For example, in the case of a therapeutic regimen comprising administration of 75 mg doses of an anti-PCSK9 antibody to a patient at a frequency of once every two weeks, if after 8 weeks (i.e., 5 doses administered at Week 0, Week 2 and Week 4, Week 6 and Week 8), the patient has not achieved a serum LDL-C concentration of less than 70 mg/dL, then the dose of anti-PCSK9 antibody is increased to e.g., 150 mg administered once every two weeks thereafter (*e*.*g*., starting at Week 10 or Week 12, or later).

### EXAMPLES

The following examples are put forth so as to provide those of ordinary skill in the art with a complete disclosure and description of how to make and use the methods and compositions of the invention, and are not intended to limit the scope of what the inventors regard as their invention. Efforts have been made to ensure accuracy with respect to numbers used (e.g., amounts, temperature, etc.) but some experimental errors and deviations should be accounted for. Unless indicated otherwise, parts are parts by weight, molecular weight is average molecular weight, temperature is in degrees Centigrade, and pressure is at or near atmospheric.

### Example 1. Generation of Human Antibodies to Human PCSK9

Human anti-PCSK9 antibodies were generated as described in US Patent No. 8,062,640. The exemplary PCSK9 inhibitor used in the following Example is the human anti-PCSK9 antibody designated "mAb316P" (also referred to in the scientific literature as "alirocumab"). mAb316P has the following amino acid sequence characteristics: heavy chain variable region (HCVR) comprising SEQ ID NO:90; light chain variable domain (LCVR) comprising SEQ ID NO:92; heavy chain complementarity determining region 1 (HCDR1) comprising SEQ ID NO:76; HCDR2 comprising SEQ ID NO:78; HCDR3 comprising SEQ ID NO:80; light chain complementarity determining region 1 (LCDR1) comprising SEQ ID NO:84; LCDR2 comprising SEQ ID NO:86; and LCDR3 comprising SEQ ID NO:88.

### Example 2: Dynamics Between Anti-PCSK9 Antibody Levels and Low-Density Lipoprotein Cholesterol (LDL-C) Levels

### Introduction

Descriptive analyses of pharmacokinetic and pharmacodynamic data from Phase I and II studies of mAb316P were conducted to characterize relationships between mAb316P, PCSK9 and LDL-C levels. Three different clinical trials were included in this analysis:

(1) Single-dose Study: Phase I, single-dose study in healthy subjects not receiving background statin therapy in which subjects received single subcutaneous (SC) doses of mAb316P 50, 100 ,150, and 250 mg, or placebo.

(2) Multiple-dose Study: A Phase I study in cohorts of heterozygous familial hypercholesterolemia (HeFH) and non-familial hypercholesterolemia (FH) patients receiving atorvastatin therapy and non-FH patients receiving diet alone. Patients receiving atorvastatin were randomized to mAb316P (50, 100, or 150 mg) or placebo administered SC on Days 1, 29, and 43. Non-FH patients receiving diet alone were randomized to mAb316P 150 mg or placebo administered SC on Days 1 and 29.

(3) Phase II Study: A 12-week study in hypercholesterolemic patients receiving atorvastatin therapy: patients randomized to mAb316P 50, 100, or 150 mg SC every 2 weeks (Q2W), 200 or 300 mg SC every 4 weeks (Q4W), or placebo.

### Results

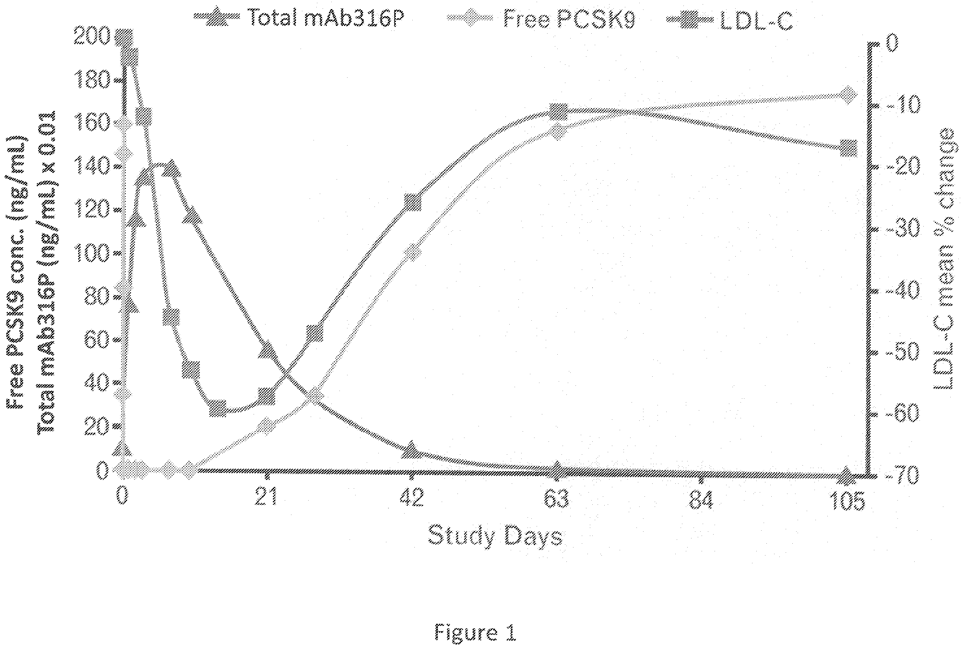

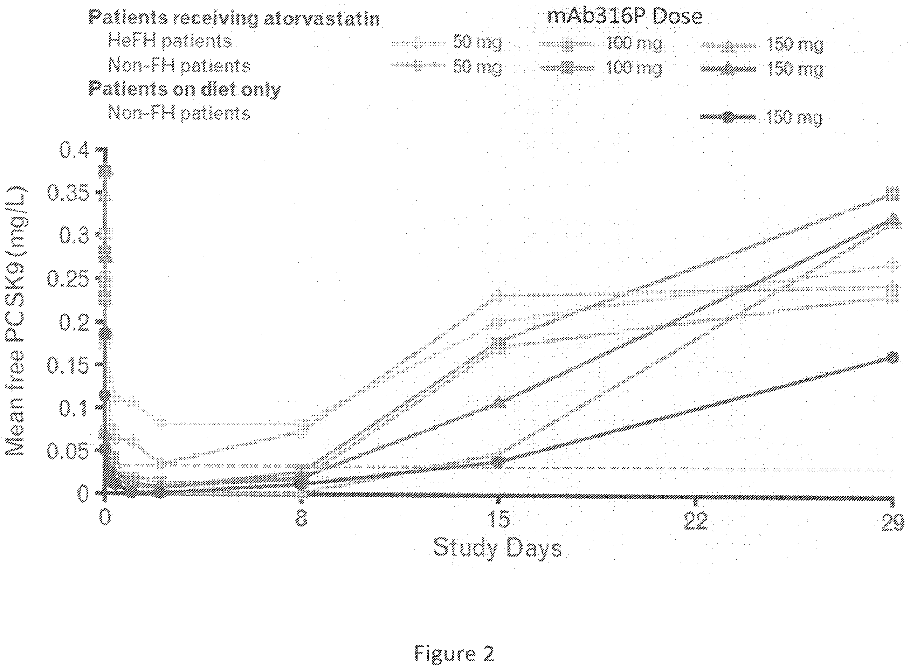

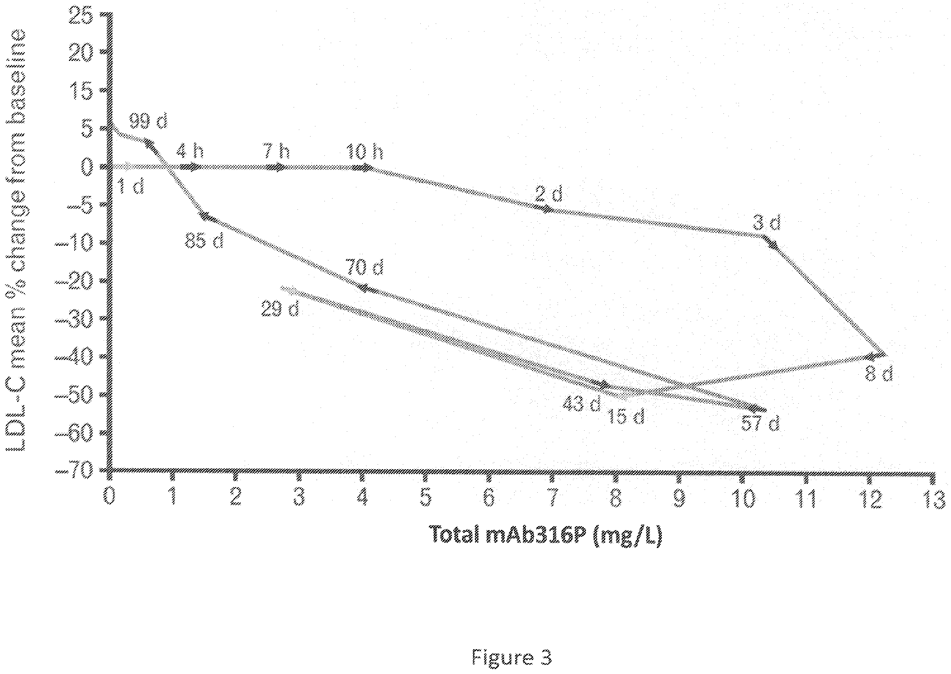

Single administration of mAb316P 150 mg SC rapidly bound to and rapidly reduced circulating free PCSK9 levels; this was followed by a drop in LDL-C. The return of free PCSK9 towards starting levels was noted to precede the return of LDL-C levels (Figure 1). Free PCSK9 levels reached nadir by Day 3 following administration of mAb316P 50, 100, or 150 mg SC in HeFH or non-FH patients (Figure 2). The return of free PCSK9 to starting levels was slower in the patients who received mAb316P plus diet alone versus patients who received mAb316P plus atorvastatin (Figure 2). The greatest mean LDL-C reductions were recorded on Day 8 for the 50 mg and 100 mg doses and Day 15 for the 150 mg dose; this delay relative to free PCSK9 concentrations may possibly be due to the time required for the production of new LDL-C receptors. A representative hysteresis curve showing the relationship between total mAb316P and LDL-C following administration of the 150 mg dose is shown in Figure 3.

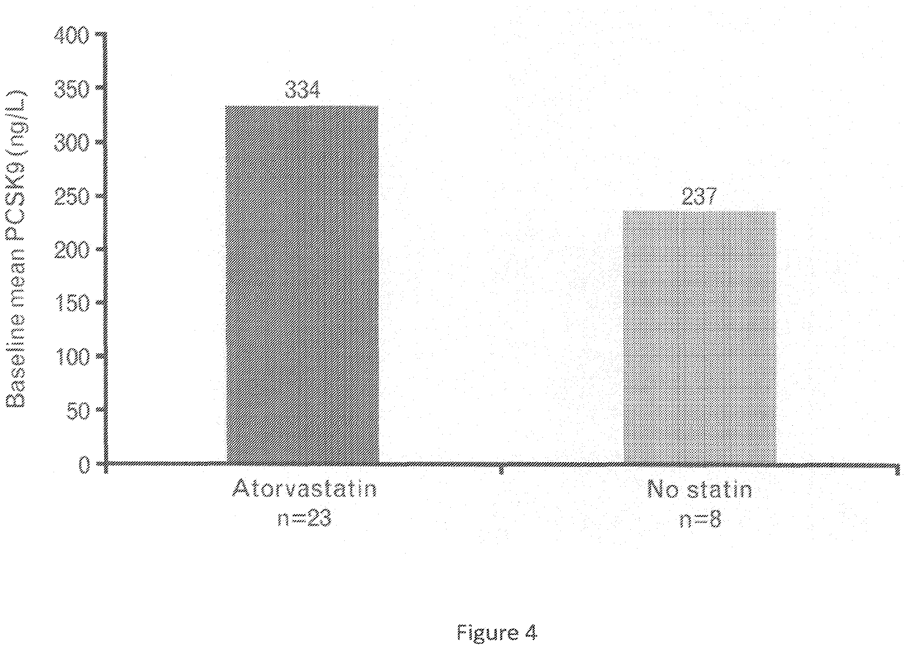

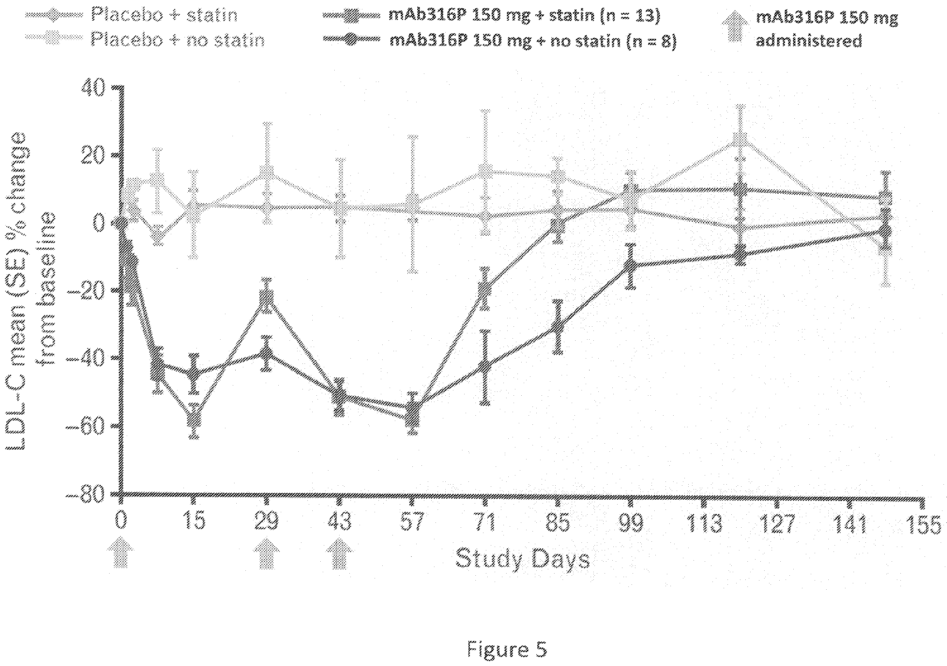

Higher baseline free PCSK9 levels were observed in patients taking statins as compared with diet alone (Figure 4). There was increased target-mediated clearance of mAb316P with concurrent statin therapy (due to higher free PCSK9 levels, likely reflective of an increase in production rate) compared with mAb316P and diet therapy alone. This appeared to affect the duration of LDL-C lowering over a 4-week dosing interval (Days 1 to 29), which was reduced with concurrent statin therapy versus diet only; however, this effect was not seen over the 2-week dosing interval (Days 29 to 43) (Figure 5).

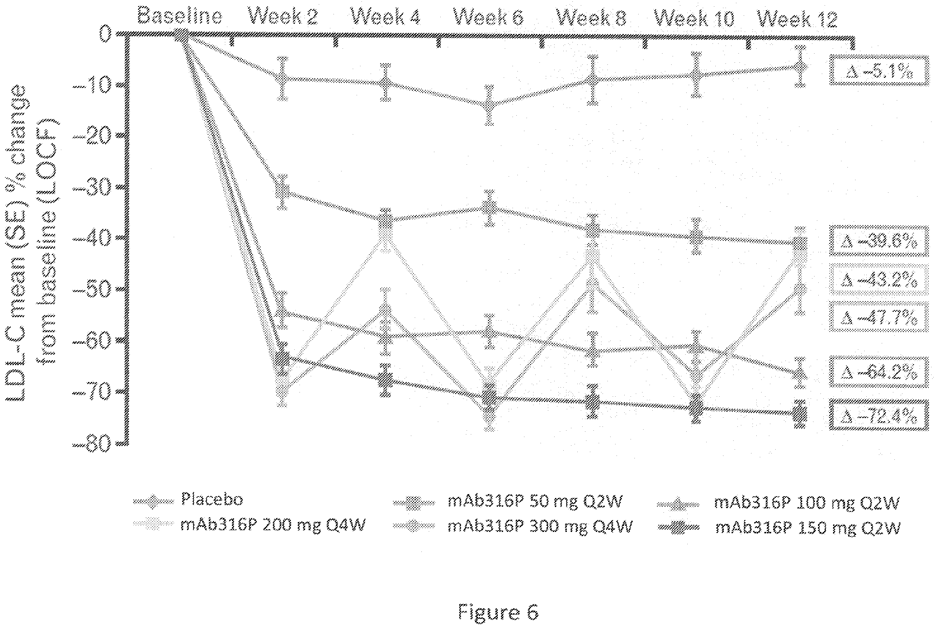

In hypercholesterolemic (non-FH) patients receiving concomitant statin therapy, mAb316P Q2W resulted in uniformly strong LDL-C lowering at 12 weeks. Patients on Q2W dosing regimens exhibited lower variability in the LDL-C response at Week 12 versus Q4W dosing (Figure 6); coefficient of variation 16% versus 49% for 150 mg Q2W and 300 mg Q4W, respectively.

### Conclusions

Dynamics were apparent between mAb316P, free PCSK9 and LDL-C levels. mAb316P treatment resulted in reductions in free PCSK9 levels within 3 days of dosing and peak reductions in LDL-C 8-15 days after dosing.

The clearance of mAb316P was accelerated by its binding to free PCSK9 (target-mediated clearance). Lower levels of free PCSK9 resulted in a longer duration of efficacy through lower target-mediated clearance. Previous studies have shown that administration of statins increases the production of free PCSK9. In the studies analyzed herein, patients who received mAb316P plus diet alone had lower baseline free PCSK9 levels and higher mAb316P blood concentrations compared with patients who received mAb316P plus atorvastatin therapy.

In patients receiving concomitant statin therapy, mAb316P administered Q2W achieved lower variation in LDL-C lowering at the end of the dosing interval as compared with Q4W dosing regimens. In Q4W dosing, patient-to-patient differences in target-mediated clearance (among other factors) appeared to contribute to differences in the duration of maximum LDL-C between Weeks 2 and 4 post dose and therefore wider differences in achieved efficacy 4 weeks after dosing. These differences were not observed when doses were administered Q2W.

### Example 3A: An Anti-PCSK9 Antibody Reduces Cholesterol Concentrations of Serum Remnant Lipoprotein Fractions, Very Low Density Lipoproteins, Triglycerides, and Lipoprotein(a) [Lp(a)]

### Introduction

Increased very low-density lipoproteins (VLDL) levels form part of a pattern of atherogenic dyslipidemia, which predisposes to premature atherosclerosis. Remnant lipoproteins are products of VLDL lipolysis, and include VLDL₃ and intermediate-density lipoproteins (IDL, the direct precursor to LDL formation).

Lipoprotein(a) [Lp(a)] consists of apolipoprotein(a) [apo(a)] covalently bound to the apo B component of a cholesterol-rich lipoprotein which is approximately the same size as a low-density lipoprotein (LDL) particle. Lp(a) metabolism is under mostly genetic regulation, in that more than 90% of its plasma concentration is influenced by quantitative and qualitative polymorphisms at the apo(a) gene (*LPA*)*.* Elevated Lp(a) levels are considered to be an independent risk factor for cardiovascular disease, with risk continuing to increase as levels rise. Although elevated Lp(a) may promote atherosclerosis through intimal deposition of cholesterol, it may also promote thrombosis due to a high homology with plasminogen, although it lacks protease activity. In patients undergoing coronary angiography, Lp(a) concentrations >30 mg/dL have been shown to be associated with an increased risk of angiographic stenosis and major coronary events. Furthermore, Lp(a) levels and genotype have been shown to be associated with aortic-valve calcification, suggesting it may play a role in causation.

The objective of this Example was to test the hypothesis that mAb316P, a fully human monoclonal antibody to proprotein convertase subtilisin/kexin type 9 (PCSK9), reduces serum levels of VLDL and lipoprotein remnants, contributing to its ability to reduce serum LDL-C and non-HDL-C, and to confirm the Lp(a) lowering activity of mAb316P in patients.

Three multicenter, double-blind, parallel-group, placebo-controlled trials were conducted in patients with primary hypercholesterolemia (Study A, n=183; Study B, n=92) or heterozygous familial hypercholesterolemia (Study C, n=77). A summary of the designs and dosing for the three trials is shown in Table 1.

**Table 1. Summary of Designs and Dosing for mAb316P Clinical Trials**

| **Study** | A | B | C |
|---|---|---|---|
| **Duration** | 12 weeks | 8 weeks | 12 weeks |
| **Patients** | hypercholesterolemia (n = 183) | hypercholesterolemia (n = 92) | heterozygous familial hypercholesterolemeia (n = 77) |
| **mAb316P Doses** | 200-300 mg Q4W | 150 mg Q2W + ATV 10 to 80 mg | 150, 200, 300 mg Q4W |
| | 50-100 mg Q2W | | 150 mg Q2W (n=16) |
| | 150 mg Q2W (n=31) | 150 mg Q2W + ATV 10 to 80 mg (total n=61) | |
| **Placebo** | n = 31 | Placebo + ATV 10 to 80 mg (n = 31) | n = 15 |
| | | | |
| **PooledAnalysis Population** | mAb316P 150 mg Q2W (n = 108) | | Placebo (n = 77) |

In Study B, all patients received atorvastatin (ATV) 10 mg prior to randomization, which was uptitrated to ATV 80 mg at the start of randomized treatment in the placebo and one mAb316P arm.

Patients on background atorvastatin or statins+/-ezetimibe received mAb316P 50-300 mg administered subcutaneously (SC) either every 2 or 4 weeks (Q2W, Q4W), depending on the study. The mAb316P 150 mg Q2W dose was common to all three trials.

In *post hoc* analyses, lipoproteins were subfractionated by vertical auto profile (VAP) testing. The VAP method is a single, direct ultracentrifugation test that separates the lipoprotein fractions according to their densities in a vertical rotor, which allows high resolution of each lipoprotein class and subclass. The bottom of the tube is punctured and the cholesterol in each layer is measured with a spectrophotometer after the addition of an enzymatic cholesterol reagent. Percent changes in VLDL-C, VLDL₁₊₂-C (a measure of cholesterol in "large, buoyant" VLDL particles), triglycerides (TG), VLDL₃-C, IDL-C and total remnant lipoprotein cholesterol levels (RLP-C; VLDL₃-C + IDL-C) in patients treated with mAb316P 150 mg Q2W vs. placebo were analyzed at week 12 (Study A), week 8 (Study B) and week 6 (Study C) using ANCOVA.

In addition, baseline and on-treatment Lp(a) levels were assessed in patients from the three different Phase 2 studies receiving mAb316P 150 mg Q2W or placebo. In all Phase 2 studies Lp(a) levels were measured at the same laboratory and using the same method. Data on Lp(a) levels at baseline and end of treatment (Week 8/12 on-treatment value or the last available on-treatment value carried forward) from the modified intention-to-treat populations of the three studies were pooled, and percentage changes from baseline for mAb316P 150 mg Q2W and placebo were compared using analysis of covariance with treatment group and study as fixed effects and baseline Lp(a) as covariate. P-values associated with these exploratory analyses are provided for descriptive purposes only and were not adjusted for multiplicity. The relationship between the percentage changes from baseline in Lp(a) and LDL-C was assessed using linear regression, and the Spearman's correlation coefficient was calculated.

### Results/Conclusions

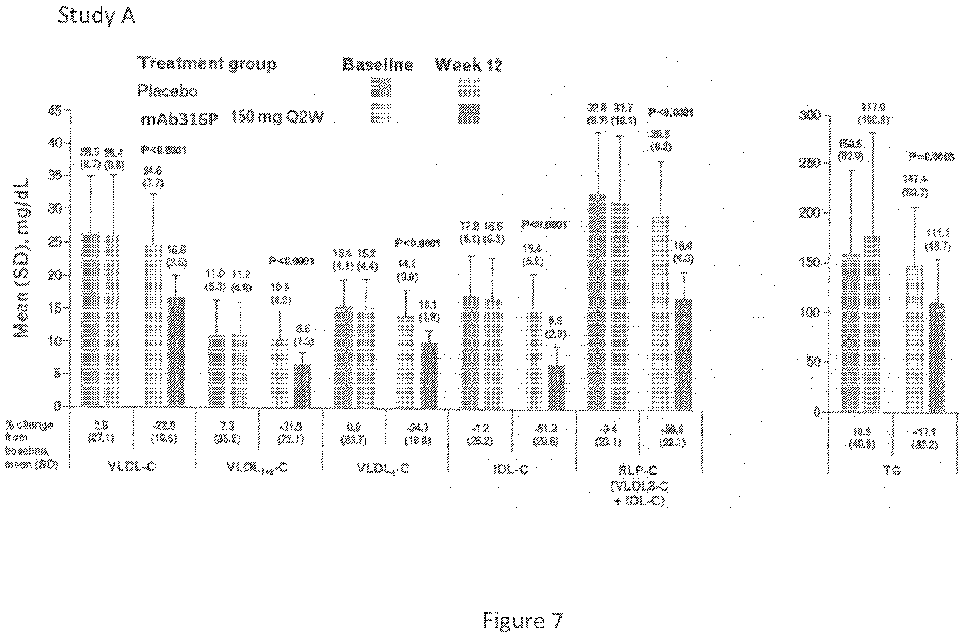

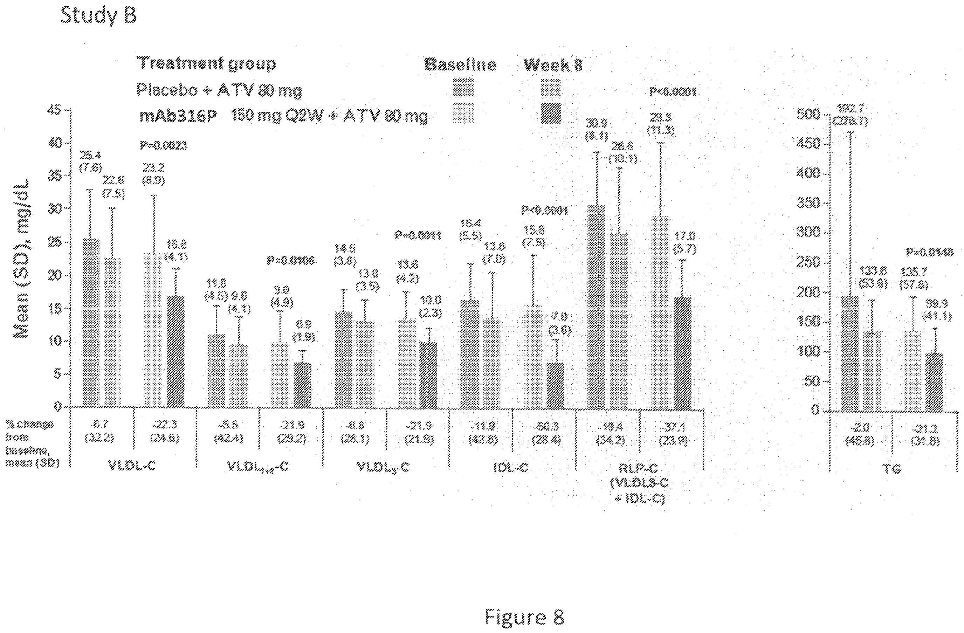

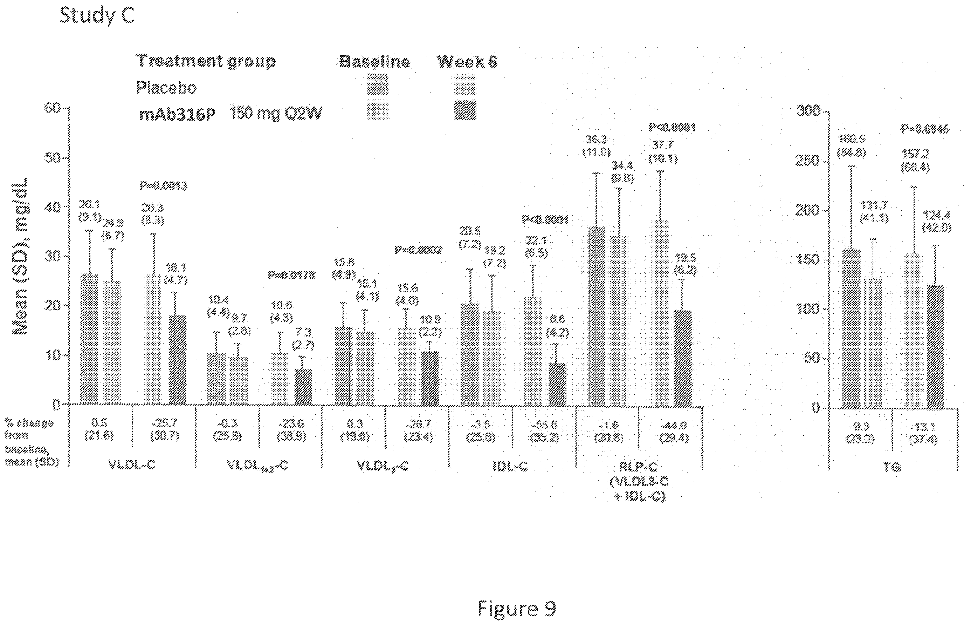

In the 3 studies, reductions in TG, VLDL-C, and in the cholesterol content of remnant lipoprotein were observed with mAb316P vs. placebo. Results are summarized in Table 2 and Figures 7-9.

**Table 2**

| | Baseline (mg/dL) | Endpoint (mg/dL) | Percent Change | p-value(s) |
|---|---|---|---|---|
| VLDL | 23.24 to 26.31 | 16.62 to 18.12 | -22.33 to -27.95 | 0.0023 to <0.0001 |
| VLDL₁₊₂-C | 9.79 to 10.59 | 6.59 to 7.31 | -21.87 to -31.45 | 0.0178 to <0.0001 |
| VLDL₃ | 13.55 to 15.62 | 10.03 to 10.88 | -21.9 to -26.66 | 0.0011 to <0.0001 |
| TG | 135.72 to 157.19 | 99.9 to 124.44 | -13.07 to -21.19 | 0.6945 to 0.0003 |
| IDL | 15.38 to 22.06 | 6.79 to 8.62 | -50.28 to -55.75 | <0.0001 |
| RLPC | 29.34 to 37.69 | 16.86 to 19.5 | -37.05 to -44.04 | <0.0001 |

The entire spectrum of atherogenic lipoproteins (LDL-C, IDL-C, VLDL-C and RLP subfractions) was reduced by mAb316P, against a background of lipid lowering therapy (statins ± ezetimibe), in heFH/non-FH patients. This Example therefore illustrates that mAb316P significantly reduced serum TG and VLDL-C, in addition to significant reductions in cholesterol concentrations of remnant lipoprotein fractions separable by VAP, including VLDL₃-C and IDL-C.

Baseline and on-treatment Lp(a) data were available for 102 of the 108 patients who received mAb316P 150 mg Q2W and 74 of the 77 patients who received placebo in the Pooled Analysis Population. Baseline values are shown in Table 3.

**Table 3. Baseline Lp(a) Levels in Patients Included in the Pooled Analysis**

| **Lp(a) values in mg/dL** | | |
|---|---|---|
| | **Placebo (n=74)** | **mAb316P 150 mg Q2W (n=102)** |
| Overall Population, median (IQR) | 19.0 (6.0-77.0) | 29.5 (8.0-70.0) |
| Range | 1.5-299.0 | 1.5-181.0 |

| **Number of Patients Subdivided by Baseline Lp(a)** | | |
|---|---|---|
| | **Placebo (n=74)** | **mAb316P 150 mg Q2W (n=102)** |
| ≤ 50 mg/dL, n (%) | 49 (66.2) | 68 (66.7) |
| > 50 mg/dL, n (%) | 25 (33.8) | 36 (35.3) |

| | | |
|---|---|---|
| *Patients from the mITT population with Lp(a) data available at baseline and end of treatment (Week 8/12 on-treatment value or the last available on-treatment value carried forward). | | |

As shown in Table 3, 36 (35%) patients treated with mAb316P and 25 (33%) patients treated with placebo had baseline Lp(a) >50 mg/dL, considered as the high-risk cut-point by the EAS guidelines.

Absolute and percentage median reductions from baseline in Lp(a) are summarized in Table 4.

**Table 4. Lp(a) Change From Baseline**

| | | **Patients Subdivided by Baseline Lp(a)** | | | |
|---|---|---|---|---|---|
| **All Patients** | | **≤ 50 mg/dL** | | **> 50 mg/dL** | |
| **pooled placebo treated (n=74)** | **pooled mAb316P treated (n=102** | **pooled placebo treated (n=49)** | **pooled mAb316P treated (n=68)** | **pooled placebo treated (n=25)** | **pooled mAb316P treated (n=34** |
| **Median Lp(a) Change From Baseline, mg/dL (IQR; LOCF)** | | | | | |
| -0.5 (-5.0- 2.0) | -9.0 (-19.0- -2.0)* | 0.0 (-3.0- 1.5) | -3.5 (-11.5- -1.5)* | -5.0 (-11.0- 6.0) | -26.5 (-39.0-16.0)* |

| **Median Lp(a) Percent Change From Baseline, (IQR; LOCF)** | | | | | |
|---|---|---|---|---|---|
| -0.3% (-16.7- 11.5) | -30.3% (-50.0-19.4)* | 0.0% (-16.7- 16.7) | -36.1% (-51.1- - 17.4)* | -4.4% (-9.4-7.1) | -27.0% (-32.6-19.6)* |

| | | | | | |
|---|---|---|---|---|---|
| * *P*<0.0001 vs placebo. | | | | | |

As shown in Table 4, the median percentage reduction from baseline in Lp(a) was - 30.3% with mAb316P 150 mg Q2W versus -0.3% with placebo (P<0.0001). The absolute median reductions in Lp(a) from baseline were substantially greater in patients with the higher baseline Lp(a). In summary, an analysis of data pooled from three Phase 2 trials conducted with mAb316P 150 mg Q2W demonstrated significant reductions in Lp(a) versus placebo, including in patients with baseline Lp(a) >50 mg/dL. In those patients considered at higher cardiovascular disease risk due to elevated Lp(a), the percentage reductions in Lp(a) appeared to be of similar magnitude resulting in greater absolute reductions in Lp(a).

### Example 3B: An Anti-PCSK9 Antibody Reduces Apolipoprotein CII and CIII Levels in Serum

### Introduction

Apoprotein (apo) CIII inhibits lipoprotein lipase (LPL)-mediated catabolism of VLDL triglycerides. Apo CII appears to have a more complex relationship with VLDL and LPL activity that may depend on baseline triglyceride levels. Apo CII is, in general, an important activator of LPL

LPL hydrolyzes the triglyceride mass of VLDL and its remnants. In this Example, the ability of an anti-PCSK9 antibody, mAb316P, to reduce VLDL-C and remnants by impacting serum levels of apoproteins CII and CIII was investigated.

Three multicenter, double-blind, parallel-group, placebo-controlled trials were conducted in patients with primary (non-familial) hypercholesterolemia (non-FH) (Study A, n=183; Study B, n=92) or heterozygous familial hypercholesterolemia (heFH) (Study C, n=77). Patients (who were receiving atorvastatin or statins+/-ezetimibe) were treated with mAb316P 50-150 mg every 2 wks (Q2W) or 150-300mg every 4 wks (Q4W) depending on the study. The mAb316P 150 mg Q2W dose was common to all three trials. (See Example 3A, Table 1).

In *post hoc* analyses, apo CII and CIII concentrations were measured via immunoassay. (using reagent kits from Randox Laboratories Limited, UK [apo CII, Cat. No. LP3866; apo CIII, Cat. No. LP3865] and an Architect Ci8200 analyzer [Abbott Laboratories, IL]). The immunoassay methods were based on the reaction of a sample containing human apo CII (or apo CIII) and specific antiserum to apo CII (or CIII) to form an insoluble complex whose concentration can be measured turbidimetrically at 340 nm. Both assays were validated for analytical performance.

Percent change from baseline in apo CII and apo CIII in the placebo group was compared with patients treated with mAb316P, analyzed at week 12 (Study A), week 8 (Study B) and week 6 (Study C) using analysis of covariance. Study C had a Week 12 endpoint; however, 6-week data were used due to reduced number of patients with available stored samples at 12 weeks (n=17) compared with 6 weeks (n=75).

### Results

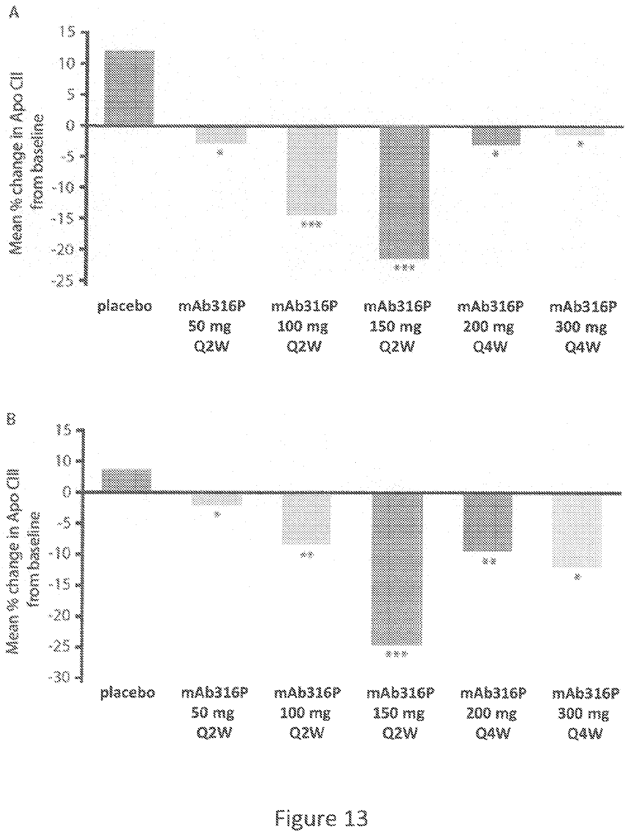

Percent change in apo CII and CIII from baseline by treatment group for mAb316P 150 mg Q2W in Studies A, B and C are shown in Table 5. Values shown are mean (SD), except for apo CIII, where values are median (IQR). Data are from Week 12 (study A), Week 8 (study B) and Week 6 (study C). Figure 13 shows the dose response changes in apo CII (panel A) and CIII (panel B) for study A.

**Table 5**

| **Study A** | **Apo CII % change** | **P-value versus placebo** | **Apo CII % change** | **P-value versus placebo** |
|---|---|---|---|---|
| Placebo | 12.0 (33.0) | | 3.76 (-5.5 to 27.6) | |
| mAb316P 150mg Q2W | -21.4 (26.5) | <0.00001 | -24.7 (-28.4 to -2.9) | <0.00001 |

| **Study B** | | | | |
|---|---|---|---|---|
| Placebo | -10.6 (30.2) | | -7.0 (29.8 to 7.3) | |
| mAb316P 150mg Q2W + atorvastatin 10 mg | -10.0 (22.7) | 0.98 | -4.9 (-24.4 to 1.0) | 0.4426 |
| mAb316P 150mg Q2W + atorvastatin 80 mg | -29.3 (19.29) | 0.0067 | -22.9 (-28 to -14.0) | 0.0165 |

| **Study C** | | | | |
|---|---|---|---|---|
| Placebo | 1.6 (15.5) | | -3.3 (-15.3 to 5.6) | |
| mAb316P 150mg Q2W | -9.4 (28.1) | 0.0794 | -20.2 (-29.0 to 2.5) | 0.019 |

mAb316P reduced apo CII and CIII at all doses, but a clear dose response was observed (Table 5, Figure 13). In all three studies, mAb316P reduced apo CII in patients from baseline (Table 5). The mean reductions from baseline at Week 12 (study A), Week 8 (study B) and Week 6 (study C) were 21.4% (*P*<0.00001), 29.3% (*P*=0.0067) and 9.4% (*P*=0.08), respectively (*P*-values compared with placebo). Apo CIII was reduced by 24.7% (*P*<0.00001), 22.9% (*P*=0.017) and 20.2% (*P*=0.019), for studies A, B and C, respectively (*P*-values compared with placebo).

### Conclusions

Therapy with anti-PCSK9 antibody mAb316P was associated with significant reductions in serum apo CII and CIII. There was a nearly 1:1 reduction in both apo CII and CIII in patients with non-FH. In patients with heFH, apo CIII decreased approximately two-fold more than apo CII. The reductions in apo CII and CIII may be a manifestation of either increased clearance or reduced production/secretion of VLDL particles, known carriers of these apoproteins.

### Example 4: An Anti-PCSK9 Antibody Reduces Cholesterol Concentrations of All Serum Low-Density Lipoprotein Fractions

### Introduction

Low-density lipoprotein cholesterol (LDL-C) is composed of a continuum of LDL particles of different densities and states of lipidation. Analysis of effects of lipid-modifying therapies on LDL particle composition may aid understanding of treatment mode-of-action. The purpose of this Example was to test the hypothesis that mAb316P, a fully human monoclonal antibody to proprotein convertase subtilisin/kexin 9 (PCSK9), reduces LDL-C by decreasing multiple LDL fractions.

Three multicenter, double-blind, parallel-group, placebo-controlled trials were conducted in patients with primary hypercholesterolemia (Study A, n=183; Study B, n=92) or heterozygous familial hypercholesterolemia (Study C, n=77). Patients (who were receiving atorvastatin or statins+/-ezetimibe) were treated with mAb316P 50-150 mg every 2 wks (Q2W) or 150-300mg every 4 wks (Q4W) depending on the study. The mAb316P 150 mg Q2W dose was common to all three trials. (See Example 3A, Table 1).

In *post hoc* analyses, lipoproteins were subfractionated by vertical auto profile (VAP) testing. Percent change in LDL₁-C ("large buoyant" LDL), LDL₂-C, LDL₃-C, and LDL₄-C ("small, dense" LDL) levels in patients receiving mAb316P 150 mg Q2W vs. placebo from baseline to week 12 for Study A, week 8 for Study B, and week 6 for Study C were assessed using ANCOVA. The LDL₃-C and LDL₄-C fractions were analyzed individually and in a pooled analysis. This was done because LDL₄-C typically exists at substantially lower concentrations than LDL₃₋₄-C. There was much more substantial variation in percent reductions in LDL₄-C. The sum of LDL₃₊₄-C represents a sum of the two smallest and densest LDL-C fractions.

### Results/Conclusions

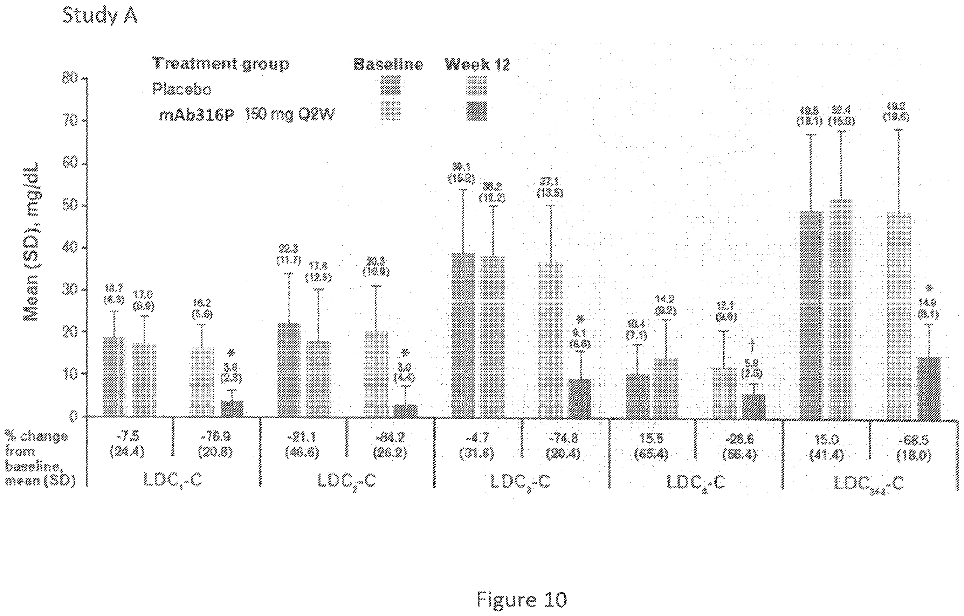

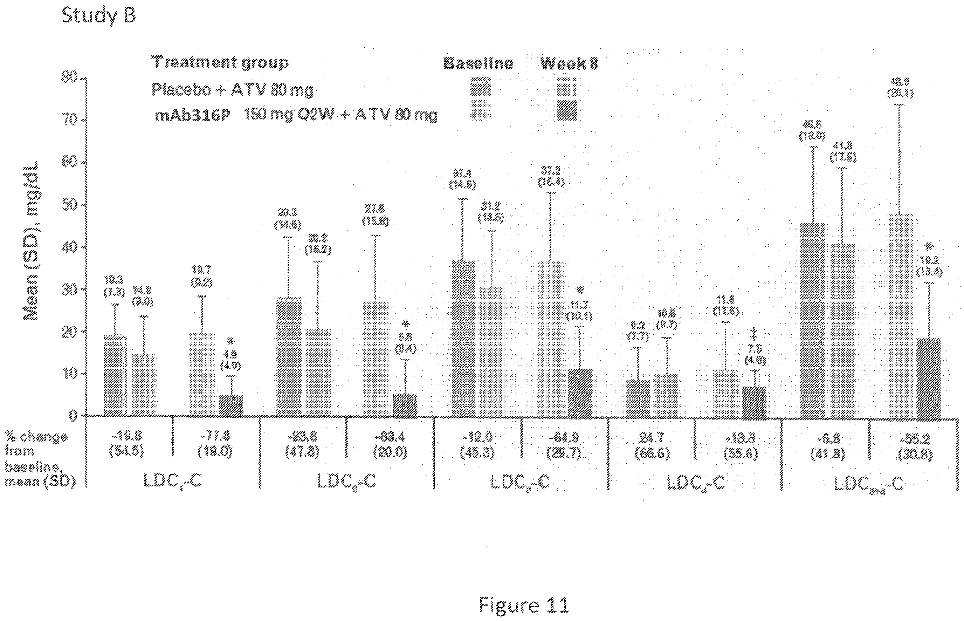

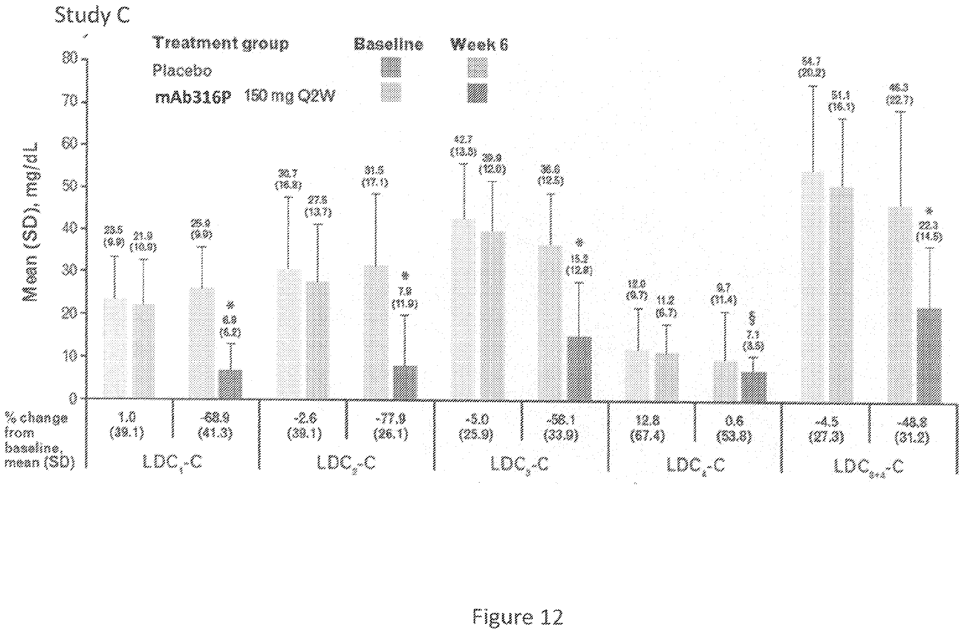

Significant reductions from baseline in cholesterol content of all LDL subfractions separable by VAP were observed in patients receiving mAb316P 150mg Q2W in the 3 studies, vs. placebo. Results are summarized in Table 6 and Figures 10-12.

**Table 6**

| | Baseline(mg/dL) | Endpoint (mg/dL) | Percent Change | p-value |
|---|---|---|---|---|
| LDL₁ | 16.18 to 25.92 | 3.56 to 6.81 | -68.87 to -77.84 | <0.0001 |
| LDL₂ | 20.29 to 31.46 | 3.04 to 7.84 | -77.85 to -84.17 | <0.0001 |
| LDL₃₄ | 46.33 to 49.16 | 14.9 to 22.29 | -48.77 to -68.49 | <0.0001 |

This Example therefore illustrates that therapy with a monoclonal antibody directed against PCSK9 (mAb316P) significantly reduced cholesterol concentrations in LDL fractions separable by VAP. The previously demonstrated LDL-C reductions with mAb316P were confirmed across the spectrum of LDL-C sub-fractions.

### Example 5: Effects of an Anti-PCSK9 Antibody on Lipoprotein Particle Concentrations

### Background

The cholesterol content of low-density lipoprotein particles (LDL-P) and high-density lipoprotein particles (HDL-P) can vary greatly among individuals. For example, one individual may have larger, cholesterol-rich LDL-P, and another will have smaller, cholesterol-poor LDL-P. Therefore measurements of serum levels of low-density lipoprotein cholesterol (LDL-C) and high-density lipoprotein cholesterol (HDL-C) often do not correlate with circulating LDL-P and HDL-P number.

Epidemiological studies have demonstrated that atherosclerotic cardiovascular disease risk tracks with both LDL-C and LDL-P. However, in patient populations where these measures are discordant (e.g., patients with diabetes or metabolic syndrome), risk has been shown to track more consistently with LDL-P than LDL-C. Consequently, multiple expert panels and consensus statements have advocated the adjunctive use of LDL-P as a target of therapy in the management of high-risk patients.

Nuclear magnetic resonance (NMR) technology can accurately measure the number of circulating lipoprotein particles to identify patients with discordantly high LDL-P levels that require intensification of therapy.

The anti-PCSK9 antibody mAb316P, has been shown to reduce LDL-C by up to 72.4% in patients on stable doses of atorvastatin over 12 weeks with similar rates of adverse events between placebo and mAb316P-treatment groups. mAb316P reduced apolipoprotein B by up to 56% proportionally with the changes in LDL-C. The effect of mAb316P on lipoprotein particle numbers in hypercholesterolemic patients has heretofore not been reported.

The present Example sets forth an evaluation of the effects of mAb316P on the concentration and size of lipoprotein particles.

### Methods

A sub-study of a randomized double-blind Phase II trial was carried out with respect to patients with LDL-C ≥1 00mg/dL who received placebo (n=31) or mAb316P 150 mg administered subcutaneously (SC) every 2 weeks (Q2W) (n=28) on top of stable daily atorvastatin (10, 20 or 40 mg daily). Lipid and lipoprotein tests were performed using samples collected after a 12-hour overnight fast. Lipoprotein particle profiles were measured by NMR spectroscopy using the LipoProfile-3 algorithm at LipoScience, Inc. (Raleigh, NC). LDL-P and HDL-P subclasses were quantified from the amplitudes of their spectroscopically distinct lipid methyl group NMR signals, and weighted-average LDL and HDL sizes were derived from the sum of the diameter of each subclass multiplied by its relative mass percentage based on the amplitude of its methyl NMR signal.

Diameter range estimates for the subclasses were as follows:

VLDL-P: small VLDL-P = 29 to 42 nm; medium VLDL-P = 42 to 60 nm; large VLDL-P >60 nm;

LDL-P: small LDL-P = 18 to 20.5 nm; large LDL-P = 20.5 to 23 nm; intermediate-density lipoprotein particles (IDL-P) = 23 to 29 nm;

HDL-P: small HDL-P = 7.3 to 8.2 nm; medium HDL-P = 8.2 to 9.4 nm; and large HDL-P = 9.4 to 14 nm.

Total VLDL-P, LDL-P, and HDL-P equal the sum of the particle number concentrations of the VLDL, LDL, and HDL subclasses, respectively. Endpoints for the analysis were percentage change in the concentration of LDL-P, VLDL-P and HDL-P from baseline to Week 12.

Any variables that were not normally distributed were transformed for all statistical tests, but the untransformed variables are presented in the tables herein. Mean (standard deviation [SD]) are reported for continuous normally distributed variables, while median (interquartile range [IQR]) are reported for any non-normally distributed variables. Count and percentage are reported for categorical variables. To determine if the treatment group was significantly different than placebo, Fisher's exact tests were performed and the associated *P-*values are reported. To determine if each of the treatment groups were significantly different than placebo, *t*-tests were performed and the associated *P*-values are reported. To determine if there was a significant difference in percent change between baseline and Week 12 for the placebo compared to the treatment groups analysis of covariances were performed with the baseline value as a covariate.

### Results

Table 7 shows lipoprotein particle concentrations before and after mAb316P 150mg SC Q2W compared to placebo.

**Table 7. Lipoprotein particle concentrations (nmol/L) for placebo (n=31) and mAb316P 150mg Q2W (n=28). *P<0.05; †P<0.0001 vs. placebo**

| Mean (sd) or median (Q1:Q3) [proportion of total] | | **PLACEBO** | | |
|---|---|---|---|---|
| | | **Baseline** | **Week 12** | **% change** |
| LDL | Total LDL | 1422.5 (321.3) | 1383.8 (327.9) | -1.0% |
| | Intermediate density lipoprotein | 110 (51:166.5) | 57 (24.5:144.5) | -15.0% |
| | Large LDL | 546.6 (205.3) | 431.8 (217.4) | -21.8% |
| | Small LDL | 755.3 (304.9) | 847.6 (375.1) | 17.8% |
| VLDL +chylomicron | Total VLDL | 61.9 (47.8:95.6) | 83.9 (45:102.2) | 33.4% |
| | +chylomicron | | | |
| | Large VLDL +chylomicron | 3.5 (2.1:8.5) | 4.3 (1.8:9.4) | 14.3% |
| | Medium VLDL | 19.3 (13.1:33.9) | 33.1 (13.1:51.9) | 24.0% |
| | Small VLDL | 35.3 (28.2:47.8) | 37.3 (23.4:56.3) | 21.4% |
| HDL | Total HDL | 32.9 (6.4) | 33.2 (7.4) | 1.4% |
| | Large HDL | 3.8 (2.0) | 3.9 (2.2) | 6.99% |
| | Medium HDL | 9.2 (5.9:14.4) | 8 (5.4:10.2) | -13.9% |
| | Small HDL | 18.8(5.3) | 21.3 (5.8) | 18.4% |
| | | | | |

| | | **mAb316P 150 mg Q2W** | | |
|---|---|---|---|---|
| | | **Baseline** | **Week 12** | **% change** |
| LDL | Total LDL | 1320 (304.0) | 475.4 (167.3)† | -63.3%† |
| | Intermediate density lipoprotein | 84.5 (33:115) | 37 (12:66)* | -52.8% |
| | Large LDL | 532.2 (212.9) | 152.4 (107.6)† | -71.3% |
| | Small LDL | 666.5 (333.8) | 279.9 (191.0)† | -54.0% |
| VLDL +chylomicron | Total VLDL +chylomicron | 71.5 (36.9:94.1) | 42.0 (30.5:54.1)† | -36.4%† |
| | Large VLDL +chylomicron | 3.6 (1.8:8.5) | 3.1 (1.7:6.9) | 5.37% |
| | Medium VLDL | 18.3 (10.7:50) | 14.4 (8.2:26.9)† | -38.92% |
| | Small VLDL | 35.7 (25:49.5) | 21.4 (19.9:26.7)* | -33.4% |
| HDL | Total HDL | 32.6 (6.3) | 36.1 (6.5)* | 11.2%* |
| | Large HDL | 4.8 (3.1) | 6.1 (3.5)* | 44.6% |
| | Medium HDL | 7.8 (5.6:10.7) | 9.8 (6.6:11.3) | 17.65% |
| | Small HDL | 19.4 (4.1) | 20.0 (5.7)* | 2.8% |

mAb316P reduced mean LDL-P by 63% vs 1% for placebo (P<0.0001) and median VLDL-P by 36% vs an increase of 33% for placebo (P<0.0001). HDL-P levels increased 11% for mAb316P vs 1% for placebo (P<0.05). Changes in all particle subclasses were directionally similar.

### Conclusion

mAb316P significantly reduced LDL-P and other lipoprotein particles in a manner similar to its previously reported effect on LDL-C and apolipoprotein B.

### Example 6: Effects of an Anti-PCSK9 Antibody on Lipoprotein Subfractions As Determined by Ion Mobility

### Background

mAb316P (also known as Alirocumab) has demonstrated significant reductions in low-density lipoprotein cholesterol (LDL-C) in Phase 2 clinical trials. LDL-C tracks closely with cardiovascular disease (CVD) risk and subfractions of LDL, defined by differences in particle size and density, have also been associated with varying degrees of CVD risk. Analysis of lipoprotein subfractions may provide further insight into CVD risk evaluation and the effects of lipid-lowering treatments seen in individual patients.

### Methods

A Phase 2 randomized, double-blind, clinical trial was conducted in patients with hypercholesterolemia and LDL-C levels ≥100mg/dL, in which 31 patients received placebo and 27 patients received alirocumab 150 mg every 2 weeks (Q2W) via a 1-mL subcutaneous auto-injection in addition to stable atorvastatin (10-40 mg daily). In the present sub-study, lipoprotein subfractions were determined at baseline and at Week 12 using ion mobility.

### Results

Changes in lipoprotein subfractions and lipid parameters from baseline to Week 12 are set forth in Tables 8A (placebo-treated) and 8B (mAb316P-treated).

**Table 8A: Placebo**

| **Lipoprotein subfractions** | | | |
|---|---|---|---|
| | **Baseline** | **Week 12** | **Percent Change** |
| VLDL + IDL + Total LDL | 1648.44 (453.5) | 1728.88 (434.58) | 8.25 |
| VLDL (large + intermediate + small) | 134.97 (109.01 to 152.85) | 140.48 (106.18 to 193.49) | 6.64 |
| IDL (IDL 1+2) | 314.28 (282.74 to 407.95) | 303.35 (277.99 to 376.17) | -3.3 |
| Total LDL | 1172.99 (362.85) | 1233.76 (331.13) | 8.8 |
| Large LDL (LDL 1+2a) | 448.23 (173.52) | 419.36 (160.98) | -0.26 |
| Medium LDL (LDL 2b) | 233.2 (105.34) | 228.92 (88.12) | 5.07 |
| Small LDL (LDL 3a) | 195.17 (85.53) | 225.98 (105.1) | 24.39 |
| Very Small LDL (LDL 4a+4b+4c+ 3b) | 296.4 (91.75) | 359.49 (136.92) | 24.61 |
| HDL (HDL 2b; HDL3+2a) | 23532.48 (5392.57) | 24400.97 (5798.21) | 0.06 |

| **Lipid parameters** | | | |
|---|---|---|---|
| | **Baseline** | **Week 12** | **Percent Change** |
| LDL-C, mg/dL | 130.2 (27.3) | 120.5 (27.0) | -5.1 |
| HDL-C, mg/dL | 49.0 (10.3) | 48.9 (13.2) | -1.0 |
| TG, mg/dL | 124.0 (92.0 to 187.5) | 127.0 (98.0 to 197.0) | 9.7 |
| Apo-B, mg/dL | 108.3 (19.3) | 109.2 (27.0) | 2.2 |
| Apo-A1, g/L | 1.4 (1.3 to 1.6) | 1.4 (1.3 to 1.7) | 0.0 |
| Lp(a), g/L | 0.2 (0.1 to 0.9) | 0.2 (0.07 to 0.85) | 0.0 |

**Table 8B: mAb316P 150 mg Q2W**

| **Lipoprotein subfractions** | | | |
|---|---|---|---|
| | **Baseline** | **Week 12** | **Percent Change (p-value)** |
| VLDL + IDL + Total LDL | 1542.82 (435.5) | 767.78 (212.39) | -48.68 (<0.0001) |
| VLDL (large + intermediate + small) | 138.17 (96.41 to 167.8) | 77.2 (51.81 to 95.8) | -51.37 (<0.0001) |
| IDL (IDL 1+2) | 309.64 (268.32 to 363.95) | 155.19 (134.05 to 194.5) | -52.29 (<0.0001) |
| Total LDL | 1078.28 (347.61) | 529.35 (171) | -49.84 (<0.0001) |
| Large LDL (LDL 1+2a) | 421.08 (130.03) | 152.66 (53.4) | -62.66 (<0.0001) |
| Medium LDL (LDL 2b) | 200.86 (91.4) | 78.67 (40.76) | -58.69 (<0.0001) |
| Small LDL (LDL 3a) | 172.02 (104.89) | 79.24 (38.36) | -48.56 (<0.0001) |
| Very Small LDL (LDL 4a+4b+4c+ 3b) | 284.31 (101.87) | 218.77 (55.66) | -17.91 (<0.0001) |
| HDL (HDL 2b; HDL3+2a) | 23480.25 (5281.63) | 21888.87 (4515.15) | -0.05 (0.0339) |

| **Lipid parameters** | | | |
|---|---|---|---|
| | **Baseline** | **Week 12** | **Percent Change (p-value)** |
| LDL-C, mg/dL | 123.9 (26.7) | 34.2 (15.6) | -72.4 (<0.0001)* |
| HDL-C, mg/dL | 53.3 (16.1) | 55.1 (14.8) | 5.5 (0.570)‡ |
| TG, mg/dL | 140.5 (92.5 to 177.5) | 99.0 (79.0 to 139.0) | -18.9 (0.0006)‡ |
| Apo-B, mg/dL | 101.6 (26.6) | 44.1 (14.1) | -56.1 (<0.0001)‡ |
| Apo-A1, g/L | 1.5 (1.3 to 1.7) | 1.6 (1.4 to 1.7) | 1.4 (0.1524)‡ |
| Lp(a), g/L | 0.3 (0.1 to 0.6) | 0.1 (0.05 to 0.41) | -28.6 (<0.0001)‡ |
| *Statistically significant p-value according to the hierarchical procedure. ‡P-values are not adjusted for multiplicity and are for descriptive purposes only. | | | |

Tables 8A and 8B illustrate changes in lipoprotein subfractions and lipid parameters in placebo and mAb316P 150 mg Q2W groups, respectively, from baseline to Week 12. Compared with placebo, mAb316P reduced very low-density lipoprotein (VLDL) + intermediate-density lipoprotein (IDL) + total LDL by 48.7% (p<0.0001), VLDL by 51.4% (p<0.0001), IDL by 52.3% (p<0.0001) and total LDL by 49.8% (p<0.0001); changes in other subfractions were directionally similar. No significant change was noted for HDL. There was a highly significant difference in percent change reduction in very small LDL versus the other LDL fractions (p<0.0001).

### Conclusions

As determined by ion mobility, mAb316P significantly reduced LDL subfractions when added to stable atorvastatin therapy, similar to its previously reported effect on LDL-C.

## Claims

1. A pharmaceutical composition comprising a PCSK9 inhibitor for use in a method for reducing serum IDL-C in a patient and treating or preventing a cardiovascular disease or disorder, the method comprising selecting a patient with a serum IDL-C level of greater than 10 mg/dL as determined by vertical auto profile testing and who is diagnosed with or identified as being at risk of developing a cardiovascular disease or disorder, and administering the pharmaceutical composition to the patient, wherein the PCSK9 inhibitor is an antibody which comprises an HCVR having the amino acid sequence of SEQ ID NO:90 and an LCVR having the amino acid sequence of SEQ ID NO:92.

2. The pharmaceutical composition for use according to claim 1,wherein serum IDL-C is reduced in the patient by at least 50% to 56% from baseline following administration of the pharmaceutical composition.

3. The pharmaceutical composition for use according to claim 1 or 2, wherein the patient has a serum IDL-C level of greater than 11 mg/dL, 12 mg/dL, 13 mg/dL, 14 mg/dL, 15 mg/dL, 16 mg/dL, 17 mg/dL, 18 mg/dL, 19 mg/dL, 20 mg/dL, 25 mg/dL, 30 mg/dL, 35 mg/dL, 40 mg/dL, 45 mg/dL or 50 mg/dL.

4. The pharmaceutical composition for use according to any one of the preceding claims, wherein the serum IDL-C level is measured in the patient post-prandial or after a period of time of fasting.

5. The pharmaceutical composition for use according to any one of claims 1-4, wherein the cardiovascular disease or disorder is selected from the group consisting of coronary artery disease, acute myocardial infarction, asymptomatic carotid atherosclerosis, stroke and peripheral artery occlusive disease.

6. The pharmaceutical composition for use according to any one of claims 1-4, wherein the patient has or is at risk of developing hypercholesterolemia.

7. The pharmaceutical composition for use according to claim 6, wherein the hypercholesterolemia is heterozygous familial hypercholesterolemia (heFH) or the hypercholesterolemia is not familial hypercholesterolemia (nonFH).

8. The pharmaceutical composition for use in the method of any one of claims 1 to 7, wherein:
(a) the pharmaceutical composition comprises 20 mg to 200 mg of the PCSK9 inhibitor;
(b) the pharmaceutical composition comprises 50 mg to 150 mg of the PCSK9 inhibitor;
(c) the pharmaceutical composition comprises 50 mg of the PCSK9 inhibitor;
(d) the pharmaceutical composition comprises 75 mg of the PCSK9 inhibitor;
(e) the pharmaceutical composition comprises 100 mg of the PCSK9 inhibitor; or
(f) the pharmaceutical composition comprises 150 mg of the PCSK9 inhibitor.

9. The pharmaceutical composition for use in the method of any one of claims 1 to 8, wherein:
(a) the patient is on a therapeutic statin regimen at the time of or just prior to administration of the pharmaceutical composition, optionally wherein the therapeutic statin regimen comprises a statin selected from the group consisting of cerivastatin, atorvastatin, simvastatin, pitavastatin, rosuvastatin, fluvastatin, lovastatin and pravastatin;
(b) the patient is on atorvastatin at the time of or just prior to administration of the pharmaceutical composition; or
(c) the patient is not on a therapeutic statin regimen at the time of administration of the pharmaceutical composition.

## Patentansprüche

1. Pharmazeutische Zusammensetzung, umfassend einen PCSK9-Hemmer zur Verwendung in einem Verfahren zum Reduzieren von Serum-IDL-C bei einem Patienten und Behandeln oder Vorbeugen einer kardiovaskulären Erkrankung oder Störung, wobei das Verfahren ein Auswählen eines Patienten mit einem Serum-IDL-C-Spiegel von größer als 10 mg/dl, wie durch den Vertical-Auto-Profile-Test bestimmt, und der mit einer kardiovaskulären Erkrankung oder Störung diagnostiziert wurde oder der als gefährdet, eine solche zu entwickeln, identifiziert wurde, und ein Verabreichen der pharmazeutischen Zusammensetzung an den Patienten umfasst, wobei der PCSK9-Hemmer ein Antikörper ist, der eine HCVR mit der Aminosäuresequenz von SEQ ID NO: 90 und eine LCVR mit der Aminosäuresequenz von SEQ ID NO: 92 umfasst.

2. Pharmazeutische Zusammensetzung zur Verwendung nach Anspruch 1, wobei Serum-IDL-C bei dem Patienten um mindestens 50 % bis 56 % vom Ausgangswert nach Verabreichung der pharmazeutischen Zusammensetzung reduziert ist.

3. Pharmazeutische Zusammensetzung zur Verwendung nach Anspruch 1 oder 2, wobei der Patient einen Serum-IDL-C-Spiegel von größer als 11 mg/dl, 12 mg/dl, 13 mg/dl, 14 mg/dl, 15 mg/dL, 16 mg/dl, 17 mg/dl, 18 mg/dl, 19 mg/dl, 20 mg/dl, 25 mg/dl, 30 mg/dl, 35 mg/dl, 40 mg/dl, 45 mg/dl oder 50 mg/dl aufweist.

4. Pharmazeutische Zusammensetzung zur Verwendung nach einem der vorhergehenden Ansprüche, wobei der Serum-IDL-C-Spiegel bei dem Patienten postprandial oder nach einem Zeitraum des Fastens gemessen wird.

5. Pharmazeutische Zusammensetzung zur Verwendung nach einem der Ansprüche 1-4, wobei die kardiovaskuläre Erkrankung oder Störung aus der Gruppe bestehend aus koronarer Herzkrankheit, akutem Myokardinfarkt, asymptomatischer Karotis-Atherosklerose, Schlaganfall und peripherer arterieller Verschlusskrankheit ausgewählt ist.

6. Pharmazeutische Zusammensetzung zur Verwendung nach einem der Ansprüche 1-4, wobei der Patient Hypercholesterinämie aufweist oder gefährdet ist, diese zu entwickeln.

7. Pharmazeutische Zusammensetzung zur Verwendung nach Anspruch 6, wobei die Hypercholesterinämie heterzygote familiäre Hypercholesterinämie (heFH) ist oder die Hypercholesterinämie nicht-familiäre Hypercholesterinämie (nonFH) ist.

8. Pharmazeutische Zusammensetzung zur Verwendung in dem Verfahren nach einem der Ansprüche 1 bis 7, wobei:
(a) die pharmazeutische Zusammensetzung 20 mg bis 200 mg des PCSK9-Hemmers umfasst;
(b) die pharmazeutische Zusammensetzung 50 mg bis 150 mg des PCSK9-Hemmers umfasst;
(c) die pharmazeutische Zusammensetzung 50 mg des PCSK9-Hemmers umfasst;
(d) die pharmazeutische Zusammensetzung 75 mg des PCSK9-Hemmers umfasst;
(e) die pharmazeutische Zusammensetzung 100 mg des PCSK9-Hemmers umfasst; oder
(d) die pharmazeutische Zusammensetzung 150 mg des PCSK9-Hemmers umfasst.

9. Pharmazeutische Zusammensetzung zur Verwendung in dem Verfahren nach einem der Ansprüche 1 bis 8, wobei:
(a) der Patient einem therapeutischen Statin-Therapieschema zum Zeitpunkt oder kurz vor der Verabreichung der pharmazeutischen Zusammensetzung folgt, optional wobei das therapeutische Statin-Therapieschema ein Statin umfasst, das aus der Gruppe bestehend aus Cerivastatin, Atorvastatin, Simvastatin, Pitavastatin, Rosuvastatin, Fluvastatin, Lovastatin und Pravastatin ausgewählt ist;
(b) der Patient Atorvastatin zum Zeitpunkt oder kurz vor der Verabreichung der pharmazeutischen Zusammensetzung erhält; oder
(c) der Patient keinem therapeutischen Statin-Therapieschema zum Zeitpunkt der Verabreichung der pharmazeutischen Zusammensetzung folgt.

## Revendications

1. Composition pharmaceutique comprenant un inhibiteur de PCSK9 destinée à une utilisation dans un procédé permettant de réduire l'IDL-C sérique chez un patient et de traiter ou de prévenir une maladie ou d'un trouble cardiovasculaire, le procédé comprenant la sélection d'un patient présentant un taux d'IDL-C sérique supérieur à 10 mg/dL, tel que déterminé par un test de profil automatique vertical et chez qui on a diagnostiqué ou identifié un risque de développement d'une maladie ou d'un trouble cardiovasculaire, et l'administration de la composition pharmaceutique au patient, l'inhibiteur de PCSK9 étant un anticorps qui comprend une HCVR ayant la séquence d'acides aminés de SEQ ID N° : 90 et une LCVR ayant la séquence d'acides aminés de SEQ ID N° : 92.

2. Composition pharmaceutique destinée à une utilisation selon la revendication 1, dans laquelle l'IDL-C sérique est réduite chez le patient d'au moins 50 % à 56 % par rapport à la ligne de base après l'administration de la composition pharmaceutique.

3. Composition pharmaceutique destinée à une utilisation selon la revendication 1 ou 2, dans laquelle le patient présente un taux d'IDL-C sérique supérieur à 11 mg/dL, 12 mg/dL, 13 mg/dL, 14 mg/dL, 15 mg/dL, 16 mg/dL, 17 mg/dL, 18 mg/dL, 19 mg/dL, 20 mg/dL, 25 mg/dL, 30 mg/dL, 35 mg/dL, 40 mg/dL, 45 mg/dL ou 50 mg/dL.

4. Composition pharmaceutique destinée à une utilisation selon l'une quelconque des revendications précédentes, dans laquelle le taux d'IDL-C est mesuré chez le patient après un repas ou après une période de jeûne.

5. Composition pharmaceutique destiné à une utilisation selon l'une quelconque des revendications 1 à 4, dans laquelle la maladie ou le trouble cardiovasculaire est choisi dans le groupe constitué par une maladie coronarienne, l'infarctus du myocarde aigu, une athérosclérose carotidienne asymptomatique, un accident vasculaire cérébral et une maladie occlusive des artères périphériques.

6. Composition pharmaceutique destiné à une utilisation selon l'une quelconque des revendications 1 à 4, dans laquelle la patient est atteint ou présente un risque de développement d'hypercholestérolémie

7. Composition pharmaceutique destiné à une utilisation selon la revendication 6, dans laquelle l'hypercholestérolémie est une hypercholestérolémie familiale hétérozygote (heFH) ou l'hypercholestérolémie est une hypercholestérolémie non familiale (nonFH).

8. Composition pharmaceutique destinée à une utilisation dans le procédé de l'une quelconque des revendications 1 à 7, dans laquelle :
(a) la composition pharmaceutique comprend 20 mg à 200 mg de l'inhibiteur de PCSK9 ;
(b) la composition pharmaceutique comprend 50 mg à 150 mg de l'inhibiteur de PCSK9 ;
(c) la composition pharmaceutique comprend 50 mg de l'inhibiteur de PCSK9;
(d) la composition pharmaceutique comprend 75 mg de l'inhibiteur de PCSK9;
(d) la composition pharmaceutique comprend 100 mg de l'inhibiteur de PCSK9 ; ou
(e) la composition pharmaceutique comprend 150 mg de l'inhibiteur de PCSK9.

9. Composition pharmaceutique destinée à une utilisation dans le procédé de l'une quelconque des revendications 1 à 8, dans laquelle :
(a) le patient suit un régime thérapeutique à base de statine au moment de l'administration de la composition pharmaceutique ou juste avant celle-ci, le régime thérapeutique à base de statine comprenant éventuellement une statine choisie dans le groupe constitué par la cérivastatine, l'atorvastatine, la simvastatine, la pitavastatine, la rosuvastatine, la fluvastatine, la lovastatine et la pravastatine ;
(b) le patient est sous atorvastatine au moment de l'administration de la composition pharmaceutique ou juste avant celle-ci ; ou
(c) le patient ne suit pas de régime thérapeutique à base de statine au moment de l'administration de la composition pharmaceutique.
